(19) Europäisches Patentamt
European Patent Office
Office européen des brevets

(11) EP 3 978 029 A1

# (12) EUROPEAN PATENT APPLICATION
published in accordance with Art. 153(4) EPC

(43) Date of publication:
**06.04.2022 Bulletin 2022/14**

(21) Application number: **20814338.8**

(22) Date of filing: **21.05.2020**

(51) International Patent Classification (IPC):
***A61K 48/00*** (2006.01) ***C12N 15/113*** (2010.01)
***A61P 21/04*** (2006.01) ***A61P 37/00*** (2006.01)

(52) Cooperative Patent Classification (CPC):
**A61K 48/00; A61P 21/04; A61P 37/00; C12N 15/113**

(86) International application number:
**PCT/CN2020/091624**

(87) International publication number:
**WO 2020/238763 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.05.2019 CN 201910440575**

(71) Applicant: **Suzhou Ribo Life Science Co., Ltd.**
**Suzhou, Jiangsu 215300 (CN)**

(72) Inventors:
- **ZHANG, Hongyan**
**Suzhou, Jiangsu 215300 (CN)**
- **GAO, Shan**
**Suzhou, Jiangsu 215300 (CN)**
- **KANG, Daiwu**
**Suzhou, Jiangsu 215300 (CN)**

(74) Representative: **SSM Sandmair Patentanwälte Rechtsanwalt Partnerschaft mbB**
**Joseph-Wild-Straße 20**
**81829 München (DE)**

## (54) NUCLEIC ACID, PHARMACEUTICAL COMPOSITION AND CONJUGATE, PREPARATION METHOD AND USE

(57) The present disclosure provides an siRNA capable of inhibiting expression of a complement protein 5(C5) gene, and a pharmaceutical composition and conjugate containing the siRNA. Each nucleotide in the siRNA is an independent modified or unmodified nucleotide. The siRNA comprises a sense strand and an antisense strand. The sense strand comprises a nucleotide sequence I, and the nucleotide sequence I has the same length and no more than three nucleotide differences from a nucleotide sequence shown in SEQ ID NO: 1. The antisense strand comprises a nucleotide sequence II, and the nucleotide sequence II has the same length and no more than three nucleotide differences from a nucleotide sequence shown in SEQ ID NO: 2. The siRNA, the pharmaceutical composition and the conjugate thereof provided by the present disclosure can effectively treat and/or prevent complement-mediated related diseases, such as myasthenia gravis (MG).

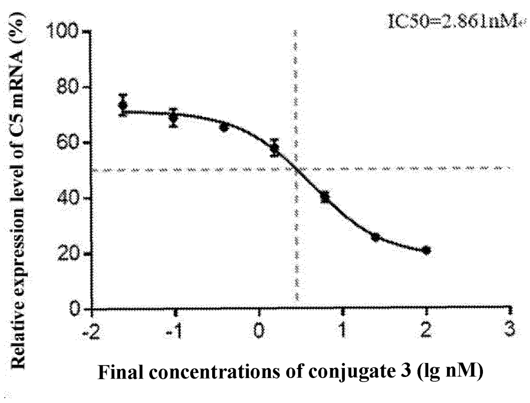


Figure 1C

EP 3 978 029 A1

Sequence Listing

<110> SUZHOU RIBO LIFE SCIENCE CO., LTD.

<120> NUCLEIC ACID, PHARMACEUTICAL COMPOSITION AND CONJUGATE, PREPARATION METHOD AND USE

<130> DSP1V211765ZX

<150> CN201910440575.8
<151> 2019-05-24

<160> 388

<170> PatentIn version 3.5

<210> 1
<211> 19
<212> RNA
<213> Artificial Sequence

<220>
<223> siRNA

<220>
<222> (19)..(19)
<221> misc_feature
<223> n is Z1, and Z1 is A

<400> 1
cuucauucau acagacaan 19

<210> 2
<211> 19
<212> RNA
<213> Artificial Sequence

<220>
<223> siRNA

<220>
<221> misc_feature
<222> (1)..(1)
<223> n is Z2, and Z2 is U

<400> 2
nuugucugua ugaaugaag 19

<210> 3
<211> 19
<212> RNA
<213> Artificial Sequence

<220>
<223> siRNA

<220>
<221> misc_feature
<222> (19)..(19)
<223> n is Z3, and Z3 is selected from A, U, G or C

<400> 3
cuucauucau acagacaan 19

<210> 4
<211> 19
<212> RNA
<213> Artificial Sequence

<220>
<223> siRNA

<220>
<221> misc_feature
<222> (1)..(1)
<223> n is Z4, Z4 is a nucleotide complementary to Z3, and Z3 is selected from A, U, G or C

<400> 4
nuugucugua ugaaugaag 19

<210> 5
<211> 19
<212> RNA
<213> Artificial Sequence

<220>
<223> siRNA

<220>
<221> misc_feature
<222> (19)..(19)
<223> n is Z3, and Z3 is selected from A, U, G or C

<400> 5
cuucauucau acagacaan 19

<210> 6
<211> 21
<212> RNA
<213> Artificial Sequence

<220>
<223> siRNA

<220>
<221> misc_feature
<222> (1)..(1)
<223> n is Z4, Z4 is a nucleotide complementary to Z3, and Z3 is selected from A, U, G or C

<400> 6
nuugucugua ugaaugaaga g 21

<210> 7
<211> 21
<212> RNA
<213> Artificial Sequence

<220>
<223> siRNA

<220>
<221> misc_feature
<222> (21)..(21)
<223> n is Z3, and Z3 is selected from A, U, G or C

<400> 7
uucuucauuc auacagacaa n 21

<210> 8
<211> 23
<212> RNA
<213> Artificial Sequence

<220>
<223> siRNA

<220>
<222> (1)..(1)
<221> misc_feature
<223> n is Z4, Z4 is a nucleotide complementary to Z3, and Z3 is selected from A, U, G or C

<400> 8
nuugucugua ugaaugaaga gaa 23

<210> 9
<211> 19
<212> RNA
<213> Artificial Sequence

<220>
<223> siRNA

<400> 9
cuucauucau acagacaaa 19

<210> 10
<211> 21
<212> RNA
<213> Artificial Sequence

<220>
<223> siRNA

<400> 10
uuugucugua ugaaugaaga g 21

<210> 11
<211> 21
<212> RNA
<213> Artificial Sequence

<220>
<223> siRNA

<400> 11

cucuucauuc auacagacaa a 21

<210> 12
<211> 23
<212> RNA
<213> Artificial Sequence

<220>
<223> siRNA

<400> 12
uuugucugua ugaaugaaga gaa 23

<210> 13
<211> 19
<212> RNA
<213> Artificial Sequence

<220>
<223> siRNA

<400> 13
cuucauucau acagacaaa 19

<210> 14
<211> 21
<212> RNA
<213> Artificial Sequence

<220>
<223> siRNA

<400> 14
uuugucugua ugaaugaaga g 21

<210> 15
<211> 19
<212> RNA
<213> Artificial Sequence

<220>
<223> siRNA

<400> 15
cuucauucau acagacaaa 19

<210> 16
<211> 21
<212> RNA
<213> Artificial Sequence

<220>
<223> siRNA

<400> 16
uuugucugua ugaaugaaga g 21

<210> 17
<211> 19
<212> RNA
<213> Artificial Sequence

<220>
<223> siRNA

<400> 17
cuucauucau acagacaaa 19

<210> 18
<211> 21
<212> RNA
<213> Artificial Sequence

<220>
<223> siRNA

<400> 18
uuugucugua ugaaugaaga g 21

<210> 19
<211> 21
<212> RNA
<213> Artificial Sequence

<220>
<223> siRNA

<400> 19
cucuucauuc auacagacaa a 21

<210> 20
<211> 23
<212> RNA
<213> Artificial Sequence

<220>
<223> siRNA

<400> 20
uuugucugua ugaaugaaga gaa 23

<210> 21
<211> 21
<212> RNA
<213> Artificial Sequence

<220>
<223> siRNA

<400> 21
cucuucauuc auacagacaa a 21

<210> 22
<211> 23
<212> RNA

<213> Artificial Sequence

<220>
<223> siRNA

<400> 22
uuugucugua ugaaugaaga gaa 23

<210> 23
<211> 21
<212> RNA
<213> Artificial Sequence

<220>
<223> siRNA

<400> 23
cucuucauuc auacagacaa a 21

<210> 24
<211> 23
<212> RNA
<213> Artificial Sequence

<220>
<223> siRNA

<400> 24
uuugucugua ugaaugaaga gaa 23

<210> 25
<211> 19
<212> RNA
<213> Artificial Sequence

<220>
<223> siRNA

<400> 25
cuucauucau acagacaaa 19

<210> 26
<211> 21
<212> RNA
<213> Artificial Sequence

<220>
<223> siRNA

<400> 26
uuugucugua ugaaugaaga g 21

<210> 27
<211> 19
<212> RNA
<213> Artificial Sequence

<220>

<223> siRNA

<400> 27
cuucauucau acagacaaa 19

<210> 28
<211> 21
<212> RNA
<213> Artificial Sequence

<220>
<223> siRNA

<400> 28
uuugucugua ugaaugaaga g 21

<210> 29
<211> 19
<212> RNA
<213> Artificial Sequence

<220>
<223> siRNA

<400> 29
cuucauucau acagacaaa 19

<210> 30
<211> 21
<212> RNA
<213> Artificial Sequence

<220>
<223> siRNA

<400> 30
uuugucugua ugaaugaaga g 21

<210> 31
<211> 21
<212> RNA
<213> Artificial Sequence

<220>
<223> siRNA

<400> 31
cucuucauuc auacagacaa a 21

<210> 32
<211> 23
<212> RNA
<213> Artificial Sequence

<220>
<223> siRNA

<400> 32

uuugucugua ugaaugaaga gaa 23

<210> 33
<211> 21
<212> RNA
<213> Artificial Sequence

<220>
<223> siRNA

<400> 33
cucuucauuc auacagacaa a 21

<210> 34
<211> 23
<212> RNA
<213> Artificial Sequence

<220>
<223> siRNA

<400> 34
uuugucugua ugaaugaaga gaa 23

<210> 35
<211> 21
<212> RNA
<213> Artificial Sequence

<220>
<223> siRNA

<400> 35
cucuucauuc auacagacaa a 21

<210> 36
<211> 23
<212> RNA
<213> Artificial Sequence

<220>
<223> siRNA

<400> 36
uuugucugua ugaaugaaga gaa 23

<210> 37
<211> 19
<212> RNA
<213> Artificial Sequence

<220>
<223> siRNA

<400> 37
cuucauucau acagacaaa 19

<210> 38
<211> 21
<212> RNA
<213> Artificial Sequence

<220>
<223> siRNA

<400> 38
uuugucugua ugaaugaaga g 21

<210> 39
<211> 19
<212> RNA
<213> Artificial Sequence

<220>
<223> siRNA

<400> 39
cuucauucau acagacaaa 19

<210> 40
<211> 21
<212> RNA
<213> Artificial Sequence

<220>
<223> siRNA

<400> 40
uuugucugua ugaaugaaga g 21

<210> 41
<211> 19
<212> RNA
<213> Artificial Sequence

<220>
<223> siRNA

<400> 41
cuucauucau acagacaaa 19

<210> 42
<211> 21
<212> RNA
<213> Artificial Sequence

<220>
<223> siRNA

<400> 42
uuugucugua ugaaugaaga g 21

<210> 43
<211> 21
<212> RNA

<213> Artificial Sequence

<220>
<223> siRNA

<400> 43
cucuucauuc auacagacaa a 21

<210> 44
<211> 23
<212> RNA
<213> Artificial Sequence

<220>
<223> siRNA

<400> 44
uuugucugua ugaaugaaga gaa 23

<210> 45
<211> 21
<212> RNA
<213> Artificial Sequence

<220>
<223> siRNA

<400> 45
cucuucauuc auacagacaa a 21

<210> 46
<211> 23
<212> RNA
<213> Artificial Sequence

<220>
<223> siRNA

<400> 46
uuugucugua ugaaugaaga gaa 23

<210> 47
<211> 21
<212> RNA
<213> Artificial Sequence

<220>
<223> siRNA

<400> 47
cucuucauuc auacagacaa a 21

<210> 48
<211> 23
<212> RNA
<213> Artificial Sequence

<220>

<223> siRNA

<400> 48
uuugucugua ugaaugaaga gaa 23

<210> 49
<211> 19
<212> RNA
<213> Artificial Sequence

<220>
<223> siRNA

<400> 49
cuucauucau acagacaaa 19

<210> 50
<211> 21
<212> RNA
<213> Artificial Sequence

<220>
<223> siRNA

<400> 50
uuugucugua ugaaugaaga g 21

<210> 51
<211> 19
<212> RNA
<213> Artificial Sequence

<220>
<223> siRNA

<400> 51
cuucauucau acagacaaa 19

<210> 52
<211> 21
<212> RNA
<213> Artificial Sequence

<220>
<223> siRNA

<400> 52
uuugucugua ugaaugaaga g 21

<210> 53
<211> 19
<212> RNA
<213> Artificial Sequence

<220>
<223> siRNA

<400> 53

cuucauucau acagacaaa 19

<210> 54
<211> 21
<212> RNA
<213> Artificial Sequence

<220>
<223> siRNA

<400> 54
uuugucugua ugaaugaaga g 21

<210> 55
<211> 21
<212> RNA
<213> Artificial Sequence

<220>
<223> siRNA

<400> 55
cucuucauuc auacagacaa a 21

<210> 56
<211> 23
<212> RNA
<213> Artificial Sequence

<220>
<223> siRNA

<400> 56
uuugucugua ugaaugaaga gaa 23

<210> 57
<211> 21
<212> RNA
<213> Artificial Sequence

<220>
<223> siRNA

<400> 57
cucuucauuc auacagacaa a 21

<210> 58
<211> 23
<212> RNA
<213> Artificial Sequence

<220>
<223> siRNA

<400> 58
uuugucugua ugaaugaaga gaa 23

<210> 59
<211> 21
<212> RNA
<213> Artificial Sequence

<220>
<223> siRNA

<400> 59
cucuucauuc auacagacaa a 21

<210> 60
<211> 23
<212> RNA
<213> Artificial Sequence

<220>
<223> siRNA

<400> 60
uuugucugua ugaaugaaga gaa 23

<210> 61
<211> 19
<212> RNA
<213> Artificial Sequence

<220>
<223> siRNA

<220>
<221> misc_feature
<222> (19)..(19)
<223> N is Z5, and Z5 is A

<400> 61
cuacaguuua gaagauuun 19

<210> 62
<211> 19
<212> RNA
<213> Artificial Sequence

<220>
<223> siRNA

<220>
<221> misc_feature
<222> (1)..(1)
<223> n is Z6, and Z6 is U

<400> 62
naaaucuucu aaacuguag 19

<210> 63
<211> 19
<212> RNA
<213> Artificial Sequence

<220>
<223> siRNA

<220>
<221> misc_feature
<222> (19)..(19)
<223> n is Z7, and Z7 is selected from A, U, G or C

<400> 63
cuacaguuua gaagauuun 19

<210> 64
<211> 19
<212> RNA
<213> Artificial Sequence

<220>
<223> siRNA

<220>
<221> misc_feature
<222> (1)..(1)
<223> n is Z8, Z8 is a nucleotide complementary to Z8, and Z7 is selected from A, U, G or C

<400> 64
naaaucuucu aaacuguag 19

<210> 65
<211> 19
<212> RNA
<213> Artificial Sequence

<220>
<223> siRNA

<220>
<221> misc_feature
<222> (19)..(19)
<223> n is Z7, and Z7 is selected from A, U, G or C

<400> 65
cuacaguuua gaagauuun 19

<210> 66
<211> 21
<212> RNA
<213> Artificial Sequence

<220>
<223> siRNA

<220>
<221> misc_feature
<222> (1)..(1)
<223> n is Z8, Z8 is a nucleotide complementary to Z8, and Z7 is selected from A, U, G or C

<400> 66
naaaucuucu aaacuguagu a 21

<210> 67
<211> 21
<212> RNA
<213> Artificial Sequence

<220>
<223> siRNA

<220>
<221> misc_feature
<222> (21)..(21)
<223> n is Z7, and Z7 is selected from A, U, G or C

<400> 67
uacuacaguu uagaagauuu n 21

<210> 68
<211> 23
<212> RNA
<213> Artificial Sequence

<220>
<223> siRNA

<220>
<221> misc_feature
<222> (1)..(1)
<223> n is Z8, Z8 is a nucleotide complementary to Z8, and Z7 is selected from A, U, G or C

<400> 68
naaaucuucu aaacuguagu aug 23

<210> 69
<211> 19
<212> RNA
<213> Artificial Sequence

<220>
<223> siRNA

<400> 69
cuacaguuua gaagauuua 19

<210> 70
<211> 21
<212> RNA
<213> Artificial Sequence

<220>
<223> siRNA

<400> 70
uaaaucuucu aaacuguagu a 21

<210> 71
<211> 21

<212> RNA
<213> Artificial Sequence

<220>
<223> siRNA

<400> 71
uacuacaguu uagaagauuu a 21

<210> 72
<211> 23
<212> RNA
<213> Artificial Sequence

<220>
<223> siRNA

<400> 72
uaaaucuucu aaacuguagu aug 23

<210> 73
<211> 19
<212> RNA
<213> Artificial Sequence

<220>
<223> siRNA

<400> 73
cuacaguuua gaagauuua 19

<210> 74
<211> 21
<212> RNA
<213> Artificial Sequence

<220>
<223> siRNA

<400> 74
uaaaucuucu aaacuguagu a 21

<210> 75
<211> 19
<212> RNA
<213> Artificial Sequence

<220>
<223> siRNA

<400> 75
cuacaguuua gaagauuua 19

<210> 76
<211> 21
<212> RNA
<213> Artificial Sequence

<220>
<223> siRNA

<400> 76
uaaaucuucu aaacuguagu a 21

<210> 77
<211> 19
<212> RNA
<213> Artificial Sequence

<220>
<223> siRNA

<400> 77
cuacaguuua gaagauuua 19

<210> 78
<211> 21
<212> RNA
<213> Artificial Sequence

<220>
<223> siRNA

<400> 78
uaaaucuucu aaacuguagu a 21

<210> 79
<211> 21
<212> RNA
<213> Artificial Sequence

<220>
<223> siRNA

<400> 79
uacuacaguu uagaagauuu a 21

<210> 80
<211> 23
<212> RNA
<213> Artificial Sequence

<220>
<223> siRNA

<400> 80
uaaaucuucu aaacuguagu aug 23

<210> 81
<211> 21
<212> RNA
<213> Artificial Sequence

<220>
<223> siRNA

<400> 81
uacuacaguu uagaagauuu a 21

<210> 82
<211> 23
<212> RNA
<213> Artificial Sequence

<220>
<223> siRNA

<400> 82
uaaaucuucu aaacuguagu aug 23

<210> 83
<211> 21
<212> RNA
<213> Artificial Sequence

<220>
<223> siRNA

<400> 83
uacuacaguu uagaagauuu a 21

<210> 84
<211> 23
<212> RNA
<213> Artificial Sequence

<220>
<223> siRNA

<400> 84
uaaaucuucu aaacuguagu aug 23

<210> 85
<211> 19
<212> RNA
<213> Artificial Sequence

<220>
<223> siRNA

<400> 85
cuacaguuua gaagauuua 19

<210> 86
<211> 21
<212> RNA
<213> Artificial Sequence

<220>
<223> siRNA

<400> 86
uaaaucuucu aaacuguagu a 21

<210> 87
<211> 19
<212> RNA
<213> Artificial Sequence

<220>
<223> siRNA

<400> 87
cuacaguuua gaagauuua 19

<210> 88
<211> 21
<212> RNA
<213> Artificial Sequence

<220>
<223> siRNA

<400> 88
uaaaucuucu aaacuguagu a 21

<210> 89
<211> 19
<212> RNA
<213> Artificial Sequence

<220>
<223> siRNA

<400> 89
cuacaguuua gaagauuua 19

<210> 90
<211> 21
<212> RNA
<213> Artificial Sequence

<220>
<223> siRNA

<400> 90
uaaaucuucu aaacuguagu a 21

<210> 91
<211> 21
<212> RNA
<213> Artificial Sequence

<220>
<223> siRNA

<400> 91
uacuacaguu uagaagauuu a 21

<210> 92
<211> 23

<212> RNA
<213> Artificial Sequence

<220>
<223> siRNA

<400> 92
uaaaucuucu aaacuguagu aug 23

<210> 93
<211> 21
<212> RNA
<213> Artificial Sequence

<220>
<223> siRNA

<400> 93
uacuacaguu uagaagauuu a 21

<210> 94
<211> 23
<212> RNA
<213> Artificial Sequence

<220>
<223> siRNA

<400> 94
uaaaucuucu aaacuguagu aug 23

<210> 95
<211> 21
<212> RNA
<213> Artificial Sequence

<220>
<223> siRNA

<400> 95
uacuacaguu uagaagauuu a 21

<210> 96
<211> 23
<212> RNA
<213> Artificial Sequence

<220>
<223> siRNA

<400> 96
uaaaucuucu aaacuguagu aug 23

<210> 97
<211> 19
<212> RNA
<213> Artificial Sequence

<220>
<223> siRNA

<400> 97
cuacaguuua gaagauuua 19

<210> 98
<211> 21
<212> RNA
<213> Artificial Sequence

<220>
<223> siRNA

<400> 98
uaaaucuucu aaacuguagu a 21

<210> 99
<211> 19
<212> RNA
<213> Artificial Sequence

<220>
<223> siRNA

<400> 99
cuacaguuua gaagauuua 19

<210> 100
<211> 21
<212> RNA
<213> Artificial Sequence

<220>
<223> siRNA

<400> 100
uaaaucuucu aaacuguagu a 21

<210> 101
<211> 19
<212> RNA
<213> Artificial Sequence

<220>
<223> siRNA

<400> 101
cuacaguuua gaagauuua 19

<210> 102
<211> 21
<212> RNA
<213> Artificial Sequence

<220>
<223> siRNA

<400> 102
uaaaucuucu aaacuguagu a 21

<210> 103
<211> 21
<212> RNA
<213> Artificial Sequence

<220>
<223> siRNA

<400> 103
uacuacaguu uagaagauuu a 21

<210> 104
<211> 23
<212> RNA
<213> Artificial Sequence

<220>
<223> siRNA

<400> 104
uaaaucuucu aaacuguagu aug 23

<210> 105
<211> 21
<212> RNA
<213> Artificial Sequence

<220>
<223> siRNA

<400> 105
uacuacaguu uagaagauuu a 21

<210> 106
<211> 23
<212> RNA
<213> Artificial Sequence

<220>
<223> siRNA

<400> 106
uaaaucuucu aaacuguagu aug 23

<210> 107
<211> 21
<212> RNA
<213> Artificial Sequence

<220>
<223> siRNA

<400> 107
uacuacaguu uagaagauuu a 21

<210> 108
<211> 23
<212> RNA
<213> Artificial Sequence

<220>
<223> siRNA

<400> 108
uaaaucuucu aaacuguagu aug 23

<210> 109
<211> 19
<212> RNA
<213> Artificial Sequence

<220>
<223> siRNA

<400> 109
cuacaguuua gaagauuua 19

<210> 110
<211> 21
<212> RNA
<213> Artificial Sequence

<220>
<223> siRNA

<400> 110
uaaaucuucu aaacuguagu a 21

<210> 111
<211> 19
<212> RNA
<213> Artificial Sequence

<220>
<223> siRNA

<400> 111
cuacaguuua gaagauuua 19

<210> 112
<211> 21
<212> RNA
<213> Artificial Sequence

<220>
<223> siRNA

<400> 112
uaaaucuucu aaacuguagu a 21

<210> 113
<211> 19

<212> RNA
<213> Artificial Sequence

<220>
<223> siRNA

<400> 113
cuacaguuua gaagauuua 19

<210> 114
<211> 21
<212> RNA
<213> Artificial Sequence

<220>
<223> siRNA

<400> 114
uaaaucuucu aaacuguagu a 21

<210> 115
<211> 21
<212> RNA
<213> Artificial Sequence

<220>
<223> siRNA

<400> 115
uacuacaguu uagaagauuu a 21

<210> 116
<211> 23
<212> RNA
<213> Artificial Sequence

<220>
<223> siRNA

<400> 116
uaaaucuucu aaacuguagu aug 23

<210> 117
<211> 21
<212> RNA
<213> Artificial Sequence

<220>
<223> siRNA

<400> 117
uacuacaguu uagaagauuu a 21

<210> 118
<211> 23
<212> RNA
<213> Artificial Sequence

<220>
<223> siRNA

<400> 118
uaaaucuucu aaacuguagu aug 23

<210> 119
<211> 21
<212> RNA
<213> Artificial Sequence

<220>
<223> siRNA

<400> 119
uacuacaguu uagaagauuu a 21

<210> 120
<211> 23
<212> RNA
<213> Artificial Sequence

<220>
<223> siRNA

<400> 120
uaaaucuucu aaacuguagu aug 23

<210> 121
<211> 19
<212> RNA
<213> Artificial Sequence

<220>
<223> siRNA

<220>
<221> misc_feature
<222> (19)..(19)
<223> n is Z9, and Z9 is A

<400> 121
ggaagguuac cgagcaaun 19

<210> 122
<211> 19
<212> RNA
<213> Artificial Sequence

<220>
<223> siRNA

<220>
<221> misc_feature
<222> (1)..(1)
<223> n is Z10, and Z10 is U

<400> 122
nauugcucgg uaaccuucc 19

<210> 123
<211> 19
<212> RNA
<213> Artificial Sequence

<220>
<223> siRNA

<220>
<221> misc_feature
<222> (19)..(19)
<223> n is Z11, and Z11 is selected from A, U, G or C

<400> 123
ggaagguuac cgagcaaun 19

<210> 124
<211> 19
<212> RNA
<213> Artificial Sequence

<220>
<223> siRNA

<220>
<221> misc_feature
<222> (1)..(1)
<223> N is Z12, Z12 is a nucleotide complementary to Z11, and Z11 is selected from A, U, G or C

<400> 124
nauugcucgg uaaccuucc 19

<210> 125
<211> 19
<212> RNA
<213> Artificial Sequence

<220>
<223> siRNA

<220>
<221> misc_feature
<222> (19)..(19)
<223> n is Z11, and Z11 is selected from A, U, G or C

<400> 125
ggaagguuac cgagcaaun 19

<210> 126
<211> 21
<212> RNA
<213> Artificial Sequence

<220>
<223> siRNA

<220>

<221> misc_feature
<222> (1)..(1)
<223> N is Z12, Z12 is a nucleotide complementary to Z11, and Z11 is selected from A, U, G or C

<400> 126
nauugcucgg uaaccuuccc u 21

<210> 127
<211> 21
<212> RNA
<213> Artificial Sequence

<220>
<223> siRNA

<220>
<221> misc_feature
<222> (21)..(21)
<223> n is Z11, and Z11 is selected from A, U, G or C

<400> 127
agggaagguu accgagcaau n 21

<210> 128
<211> 23
<212> RNA
<213> Artificial Sequence

<220>
<223> siRNA

<220>
<221> misc_feature
<222> (1)..(1)
<223> N is Z12, Z12 is a nucleotide complementary to Z11, and Z11 is selected from A, U, G or C

<400> 128
nauugcucgg uaaccuuccc ugg 23

<210> 129
<211> 19
<212> RNA
<213> Artificial Sequence

<220>
<223> siRNA

<400> 129
ggaagguuac cgagcaaua 19

<210> 130
<211> 21
<212> RNA
<213> Artificial Sequence

<220>
<223> siRNA

<400> 130
uauugcucgg uaaccuuccc u 21

<210> 131
<211> 21
<212> RNA
<213> Artificial Sequence

<220>
<223> siRNA

<400> 131
agggaagguu accgagcaau a 21

<210> 132
<211> 23
<212> RNA
<213> Artificial Sequence

<220>
<223> siRNA

<400> 132
uauugcucgg uaaccuuccc ugg 23

<210> 133
<211> 19
<212> RNA
<213> Artificial Sequence

<220>
<223> siRNA

<400> 133
ggaagguuac cgagcaaua 19

<210> 134
<211> 21
<212> RNA
<213> Artificial Sequence

<220>
<223> siRNA

<400> 134
uauugcucgg uaaccuuccc u 21

<210> 135
<211> 19
<212> RNA
<213> Artificial Sequence

<220>
<223> siRNA

<400> 135
ggaagguuac cgagcaaua 19

<210> 136
<211> 21
<212> RNA
<213> Artificial Sequence

<220>
<223> siRNA

<400> 136
uauugcucgg uaaccuuccc u 21

<210> 137
<211> 19
<212> RNA
<213> Artificial Sequence

<220>
<223> siRNA

<400> 137
ggaagguuac cgagcaaua 19

<210> 138
<211> 21
<212> RNA
<213> Artificial Sequence

<220>
<223> siRNA

<400> 138
uauugcucgg uaaccuuccc u 21

<210> 139
<211> 21
<212> RNA
<213> Artificial Sequence

<220>
<223> siRNA

<400> 139
agggaagguu accgagcaau a 21

<210> 140
<211> 23
<212> RNA
<213> Artificial Sequence

<220>
<223> siRNA

<400> 140
uauugcucgg uaaccuuccc ugg 23

<210> 141

<211> 21
<212> RNA
<213> Artificial Sequence

<220>
<223> siRNA

<400> 141
agggaagguu accgagcaau a 21

<210> 142
<211> 23
<212> RNA
<213> Artificial Sequence

<220>
<223> siRNA

<400> 142
uauugcucgg uaaccuuccc ugg 23

<210> 143
<211> 21
<212> RNA
<213> Artificial Sequence

<220>
<223> siRNA

<400> 143
agggaagguu accgagcaau a 21

<210> 144
<211> 23
<212> RNA
<213> Artificial Sequence

<220>
<223> siRNA

<400> 144
uauugcucgg uaaccuuccc ugg 23

<210> 145
<211> 19
<212> RNA
<213> Artificial Sequence

<220>
<223> siRNA

<400> 145
ggaagguuac cgagcaaua 19

<210> 146
<211> 21
<212> RNA
<213> Artificial Sequence

<220>
<223> siRNA

<400> 146
uauugcucgg uaaccuuccc u 21

<210> 147
<211> 19
<212> RNA
<213> Artificial Sequence

<220>
<223> siRNA

<400> 147
ggaagguuac cgagcaaua 19

<210> 148
<211> 21
<212> RNA
<213> Artificial Sequence

<220>
<223> siRNA

<400> 148
uauugcucgg uaaccuuccc u 21

<210> 149
<211> 19
<212> RNA
<213> Artificial Sequence

<220>
<223> siRNA

<400> 149
ggaagguuac cgagcaaua 19

<210> 150
<211> 21
<212> RNA
<213> Artificial Sequence

<220>
<223> siRNA

<400> 150
uauugcucgg uaaccuuccc u 21

<210> 151
<211> 21
<212> RNA
<213> Artificial Sequence

<220>
<223> siRNA

<400> 151
agggaagguu accgagcaau a 21

<210> 152
<211> 23
<212> RNA
<213> Artificial Sequence

<220>
<223> siRNA

<400> 152
uauugcucgg uaaccuuccc ugg 23

<210> 153
<211> 21
<212> RNA
<213> Artificial Sequence

<220>
<223> siRNA

<400> 153
agggaagguu accgagcaau a 21

<210> 154
<211> 23
<212> RNA
<213> Artificial Sequence

<220>
<223> siRNA

<400> 154
uauugcucgg uaaccuuccc ugg 23

<210> 155
<211> 21
<212> RNA
<213> Artificial Sequence

<220>
<223> siRNA

<400> 155
agggaagguu accgagcaau a 21

<210> 156
<211> 23
<212> RNA
<213> Artificial Sequence

<220>
<223> siRNA

<400> 156
uauugcucgg uaaccuuccc ugg 23

<210> 157
<211> 19
<212> RNA
<213> Artificial Sequence

<220>
<223> siRNA

<400> 157
ggaagguuac cgagcaaua 19

<210> 158
<211> 21
<212> RNA
<213> Artificial Sequence

<220>
<223> siRNA

<400> 158
uauugcucgg uaaccuuccc u 21

<210> 159
<211> 19
<212> RNA
<213> Artificial Sequence

<220>
<223> siRNA

<400> 159
ggaagguuac cgagcaaua 19

<210> 160
<211> 21
<212> RNA
<213> Artificial Sequence

<220>
<223> siRNA

<400> 160
uauugcucgg uaaccuuccc u 21

<210> 161
<211> 19
<212> RNA
<213> Artificial Sequence

<220>
<223> siRNA

<400> 161
ggaagguuac cgagcaaua 19

<210> 162

<211> 21
<212> RNA
<213> Artificial Sequence

<220>
<223> siRNA

<400> 162
uauugcucgg uaaccuuccc u 21

<210> 163
<211> 21
<212> RNA
<213> Artificial Sequence

<220>
<223> siRNA

<400> 163
agggaagguu accgagcaau a 21

<210> 164
<211> 23
<212> RNA
<213> Artificial Sequence

<220>
<223> siRNA

<400> 164
uauugcucgg uaaccuuccc ugg 23

<210> 165
<211> 21
<212> RNA
<213> Artificial Sequence

<220>
<223> siRNA

<400> 165
agggaagguu accgagcaau a 21

<210> 166
<211> 23
<212> RNA
<213> Artificial Sequence

<220>
<223> siRNA

<400> 166
uauugcucgg uaaccuuccc ugg 23

<210> 167
<211> 21
<212> RNA
<213> Artificial Sequence

<220>
<223> siRNA

<400> 167
agggaagguu accgagcaau a 21

<210> 168
<211> 23
<212> RNA
<213> Artificial Sequence

<220>
<223> siRNA

<400> 168
uauugcucgg uaaccuuccc ugg 23

<210> 169
<211> 19
<212> RNA
<213> Artificial Sequence

<220>
<223> siRNA

<400> 169
ggaagguuac cgagcaaua 19

<210> 170
<211> 21
<212> RNA
<213> Artificial Sequence

<220>
<223> siRNA

<400> 170
uauugcucgg uaaccuuccc u 21

<210> 171
<211> 19
<212> RNA
<213> Artificial Sequence

<220>
<223> siRNA

<400> 171
ggaagguuac cgagcaaua 19

<210> 172
<211> 21
<212> RNA
<213> Artificial Sequence

<220>
<223> siRNA

<400> 172
uauugcucgg uaaccuuccc u 21

<210> 173
<211> 19
<212> RNA
<213> Artificial Sequence

<220>
<223> siRNA

<400> 173
ggaagguuac cgagcaaua 19

<210> 174
<211> 21
<212> RNA
<213> Artificial Sequence

<220>
<223> siRNA

<400> 174
uauugcucgg uaaccuuccc u 21

<210> 175
<211> 21
<212> RNA
<213> Artificial Sequence

<220>
<223> siRNA

<400> 175
agggaagguu accgagcaau a 21

<210> 176
<211> 23
<212> RNA
<213> Artificial Sequence

<220>
<223> siRNA

<400> 176
uauugcucgg uaaccuuccc ugg 23

<210> 177
<211> 21
<212> RNA
<213> Artificial Sequence

<220>
<223> siRNA

<400> 177
agggaagguu accgagcaau a 21

<210> 178
<211> 23
<212> RNA
<213> Artificial Sequence

<220>
<223> siRNA

<400> 178
uauugcucgg uaaccuuccc ugg 23

<210> 179
<211> 21
<212> RNA
<213> Artificial Sequence

<220>
<223> siRNA

<400> 179
agggaagguu accgagcaau a 21

<210> 180
<211> 23
<212> RNA
<213> Artificial Sequence

<220>
<223> siRNA

<400> 180
uauugcucgg uaaccuuccc ugg 23

<210> 181
<211> 19
<212> RNA
<213> Artificial Sequence

<220>
<223> siRNA

<220>
<221> misc_feature
<222> (19)..(19)
<223> N is Z13, and Z13 is A

<400> 181
agaacagaca gcagaauun 19

<210> 182
<211> 19
<212> RNA
<213> Artificial Sequence

<220>
<223> siRNA

<220>
<221> misc_feature
<222> (1)..(1)
<223> N is Z14, and Z14 is U

<400> 182
naauucugcu gucuguucu 19

<210> 183
<211> 19
<212> RNA
<213> Artificial Sequence

<220>
<223> siRNA

<220>
<221> misc_feature
<222> (19)..(19)
<223> n is Z15, and Z15 is selected from A, U, G or C

<400> 183
agaacagaca gcagaauun 19

<210> 184
<211> 19
<212> RNA
<213> Artificial Sequence

<220>
<223> siRNA

<220>
<221> misc_feature
<222> (1)..(1)
<223> N is Z16, Z16 is a nucleotide complementary to Z15, and Z15 is selected from A, U, G or C

<400> 184
naauucugcu gucuguucu 19

<210> 185
<211> 19
<212> RNA
<213> Artificial Sequence

<220>
<223> siRNA

<220>
<221> misc_feature
<222> (19)..(19)
<223> n is Z15, and Z15 is selected from A, U, G or C

<400> 185
agaacagaca gcagaauun 19

<210> 186
<211> 21

<212> RNA
<213> Artificial Sequence

<220>
<223> siRNA

<220>
<221> misc_feature
<222> (1)..(1)
<223> N is Z16, Z16 is a nucleotide complementary to Z15, and Z15 is selected from A, U, G or C

<400> 186
naauucugcu gucuguucuc c 21

<210> 187
<211> 21
<212> RNA
<213> Artificial Sequence

<220>
<223> siRNA

<220>
<221> misc_feature
<222> (21)..(21)
<223> n is Z15, and Z15 is selected from A, U, G or C

<400> 187
ggagaacaga cagcagaauu n 21

<210> 188
<211> 23
<212> RNA
<213> Artificial Sequence

<220>
<223> siRNA

<220>
<221> misc_feature
<222> (1)..(1)
<223> N is Z16, Z16 is a nucleotide complementary to Z15, and Z15 is selected from A, U, G or C

<400> 188
naauucugcu gucuguucuc cug 23

<210> 189
<211> 19
<212> RNA
<213> Artificial Sequence

<220>
<223> siRNA

<400> 189
agaacagaca gcagaauua 19

<210> 190
<211> 21
<212> RNA
<213> Artificial Sequence

<220>
<223> siRNA

<400> 190
uaauucugcu gucuguucuc c 21

<210> 191
<211> 21
<212> RNA
<213> Artificial Sequence

<220>
<223> siRNA

<400> 191
ggagaacaga cagcagaauu a 21

<210> 192
<211> 23
<212> RNA
<213> Artificial Sequence

<220>
<223> siRNA

<400> 192
uaauucugcu gucuguucuc cug 23

<210> 193
<211> 19
<212> RNA
<213> Artificial Sequence

<220>
<223> siRNA

<400> 193
agaacagaca gcagaauua 19

<210> 194
<211> 21
<212> RNA
<213> Artificial Sequence

<220>
<223> siRNA

<400> 194
uaauucugcu gucuguucuc c 21

<210> 195
<211> 19
<212> RNA

<213> Artificial Sequence

<220>
<223> siRNA

<400> 195
agaacagaca gcagaauua 19

<210> 196
<211> 21
<212> RNA
<213> Artificial Sequence

<220>
<223> siRNA

<400> 196
uaauucugcu gucuguucuc c 21

<210> 197
<211> 19
<212> RNA
<213> Artificial Sequence

<220>
<223> siRNA

<400> 197
agaacagaca gcagaauua 19

<210> 198
<211> 21
<212> RNA
<213> Artificial Sequence

<220>
<223> siRNA

<400> 198
uaauucugcu gucuguucuc c 21

<210> 199
<211> 21
<212> RNA
<213> Artificial Sequence

<220>
<223> siRNA

<400> 199
ggagaacaga cagcagaauu a 21

<210> 200
<211> 23
<212> RNA
<213> Artificial Sequence

<220>

<223>  siRNA

<400>  200
uaauucugcu gucuguucuc cug                                              23

<210>  201
<211>  21
<212>  RNA
<213>  Artificial Sequence

<220>
<223>  siRNA

<400>  201
ggagaacaga cagcagaauu a                                                21

<210>  202
<211>  23
<212>  RNA
<213>  Artificial Sequence

<220>
<223>  siRNA

<400>  202
uaauucugcu gucuguucuc cug                                              23

<210>  203
<211>  21
<212>  RNA
<213>  Artificial Sequence

<220>
<223>  siRNA

<400>  203
ggagaacaga cagcagaauu a                                                21

<210>  204
<211>  23
<212>  RNA
<213>  Artificial Sequence

<220>
<223>  siRNA

<400>  204
uaauucugcu gucuguucuc cug                                              23

<210>  205
<211>  19
<212>  RNA
<213>  Artificial Sequence

<220>
<223>  siRNA

<400>  205

agaacagaca gcagaauua 19

<210> 206
<211> 21
<212> RNA
<213> Artificial Sequence

<220>
<223> siRNA

<400> 206
uaauucugcu gucuguucuc c 21

<210> 207
<211> 19
<212> RNA
<213> Artificial Sequence

<220>
<223> siRNA

<400> 207
agaacagaca gcagaauua 19

<210> 208
<211> 21
<212> RNA
<213> Artificial Sequence

<220>
<223> siRNA

<400> 208
uaauucugcu gucuguucuc c 21

<210> 209
<211> 19
<212> RNA
<213> Artificial Sequence

<220>
<223> siRNA

<400> 209
agaacagaca gcagaauua 19

<210> 210
<211> 21
<212> RNA
<213> Artificial Sequence

<220>
<223> siRNA

<400> 210
uaauucugcu gucuguucuc c 21

<210> 211
<211> 21
<212> RNA
<213> Artificial Sequence

<220>
<223> siRNA

<400> 211
ggagaacaga cagcagaauu a 21

<210> 212
<211> 23
<212> RNA
<213> Artificial Sequence

<220>
<223> siRNA

<400> 212
uaauucugcu gucuguucuc cug 23

<210> 213
<211> 21
<212> RNA
<213> Artificial Sequence

<220>
<223> siRNA

<400> 213
ggagaacaga cagcagaauu a 21

<210> 214
<211> 23
<212> RNA
<213> Artificial Sequence

<220>
<223> siRNA

<400> 214
uaauucugcu gucuguucuc cug 23

<210> 215
<211> 21
<212> RNA
<213> Artificial Sequence

<220>
<223> siRNA

<400> 215
ggagaacaga cagcagaauu a 21

<210> 216
<211> 23
<212> RNA

<213> Artificial Sequence

<220>
<223> siRNA

<400> 216
uaauucugcu gucuguucuc cug 23

<210> 217
<211> 19
<212> RNA
<213> Artificial Sequence

<220>
<223> siRNA

<400> 217
agaacagaca gcagaauua 19

<210> 218
<211> 21
<212> RNA
<213> Artificial Sequence

<220>
<223> siRNA

<400> 218
uaauucugcu gucuguucuc c 21

<210> 219
<211> 19
<212> RNA
<213> Artificial Sequence

<220>
<223> siRNA

<400> 219
agaacagaca gcagaauua 19

<210> 220
<211> 21
<212> RNA
<213> Artificial Sequence

<220>
<223> siRNA

<400> 220
uaauucugcu gucuguucuc c 21

<210> 221
<211> 19
<212> RNA
<213> Artificial Sequence

<220>

<223> siRNA

<400> 221
agaacagaca gcagaauua 19

<210> 222
<211> 21
<212> RNA
<213> Artificial Sequence

<220>
<223> siRNA

<400> 222
uaauucugcu gucuguucuc c 21

<210> 223
<211> 21
<212> RNA
<213> Artificial Sequence

<220>
<223> siRNA

<400> 223
ggagaacaga cagcagaauu a 21

<210> 224
<211> 23
<212> RNA
<213> Artificial Sequence

<220>
<223> siRNA

<400> 224
uaauucugcu gucuguucuc cug 23

<210> 225
<211> 21
<212> RNA
<213> Artificial Sequence

<220>
<223> siRNA

<400> 225
ggagaacaga cagcagaauu a 21

<210> 226
<211> 23
<212> RNA
<213> Artificial Sequence

<220>
<223> siRNA

<400> 226

uaauucugcu gucuguucuc cug 23

<210> 227
<211> 21
<212> RNA
<213> Artificial Sequence

<220>
<223> siRNA

<400> 227
ggagaacaga cagcagaauu a 21

<210> 228
<211> 23
<212> RNA
<213> Artificial Sequence

<220>
<223> siRNA

<400> 228
uaauucugcu gucuguucuc cug 23

<210> 229
<211> 19
<212> RNA
<213> Artificial Sequence

<220>
<223> siRNA

<400> 229
agaacagaca gcagaauua 19

<210> 230
<211> 21
<212> RNA
<213> Artificial Sequence

<220>
<223> siRNA

<400> 230
uaauucugcu gucuguucuc c 21

<210> 231
<211> 19
<212> RNA
<213> Artificial Sequence

<220>
<223> siRNA

<400> 231
agaacagaca gcagaauua 19

<210> 232
<211> 21
<212> RNA
<213> Artificial Sequence

<220>
<223> siRNA

<400> 232
uaauucugcu gucuguucuc c 21

<210> 233
<211> 19
<212> RNA
<213> Artificial Sequence

<220>
<223> siRNA

<400> 233
agaacagaca gcagaauua 19

<210> 234
<211> 21
<212> RNA
<213> Artificial Sequence

<220>
<223> siRNA

<400> 234
uaauucugcu gucuguucuc c 21

<210> 235
<211> 21
<212> RNA
<213> Artificial Sequence

<220>
<223> siRNA

<400> 235
ggagaacaga cagcagaauu a 21

<210> 236
<211> 23
<212> RNA
<213> Artificial Sequence

<220>
<223> siRNA

<400> 236
uaauucugcu gucuguucuc cug 23

<210> 237
<211> 21
<212> RNA

<213> Artificial Sequence

<220>
<223> siRNA

<400> 237
ggagaacaga cagcagaauu a 21

<210> 238
<211> 23
<212> RNA
<213> Artificial Sequence

<220>
<223> siRNA

<400> 238
uaauucugcu gucuguucuc cug 23

<210> 239
<211> 21
<212> RNA
<213> Artificial Sequence

<220>
<223> siRNA

<400> 239
ggagaacaga cagcagaauu a 21

<210> 240
<211> 23
<212> RNA
<213> Artificial Sequence

<220>
<223> siRNA

<400> 240
uaauucugcu gucuguucuc cug 23

<210> 241
<211> 19
<212> RNA
<213> Artificial Sequence

<220>
<223> siRNA

<220>
<221> misc_feature
<222> (19)..(19)
<223> n is Z17, and Z17 is A

<400> 241
ccaagaagaa cgcugcaan 19

<210> 242

<211> 19
<212> RNA
<213> Artificial Sequence

<220>
<223> siRNA

<220>
<221> misc_feature
<222> (1)..(1)
<223> n is Z18, and Z18 is U

<400> 242
nuugcagcgu ucuucuugg 19

<210> 243
<211> 19
<212> RNA
<213> Artificial Sequence

<220>
<223> siRNA

<220>
<221> misc_feature
<222> (19)..(19)
<223> n is Z19,and Z19 is selected from A, U, G or C

<400> 243
ccaagaagaa cgcugcaan 19

<210> 244
<211> 19
<212> RNA
<213> Artificial Sequence

<220>
<223> siRNA

<220>
<221> misc_feature
<222> (1)..(1)
<223> n is Z20, Z20 is a nucleotide complementary to Z19,and Z19 is selected from A, U, G or C

<400> 244
nuugcagcgu ucuucuugg 19

<210> 245
<211> 19
<212> RNA
<213> Artificial Sequence

<220>
<223> siRNA

<220>
<221> misc_feature
<222> (19)..(19)
<223> n is Z19, and Z19 is selected from A, U, G or C

<400> 245
ccaagaagaa cgcugcaan 19

<210> 246
<211> 21
<212> RNA
<213> Artificial Sequence

<220>
<223> siRNA

<220>
<221> misc_feature
<222> (1)..(1)
<223> n is Z20, Z20 is a nucleotide complementary to Z19,and Z19 is selected from A, U, G or C

<400> 246
nuugcagcgu ucuucuuggc c 21

<210> 247
<211> 21
<212> RNA
<213> Artificial Sequence

<220>
<223> siRNA

<220>
<221> misc_feature
<222> (21)..(21)
<223> n is Z19,and Z19 is selected from A, U, G or C

<400> 247
ggccaagaag aacgcugcaa n 21

<210> 248
<211> 23
<212> RNA
<213> Artificial Sequence

<220>
<223> siRNA

<220>
<221> misc_feature
<222> (1)..(1)
<223> n is Z20, Z20 is a nucleotide complementary to Z19,and Z19 is selected from A, U, G or C

<400> 248
nuugcagcgu ucuucuuggc cug 23

<210> 249
<211> 19
<212> RNA
<213> Artificial Sequence

<220>
<223> siRNA

<400> 249
ccaagaagaa cgcugcaaa 19

<210> 250
<211> 21
<212> RNA
<213> Artificial Sequence

<220>
<223> siRNA

<400> 250
uuugcagcgu ucuucuuggc c 21

<210> 251
<211> 21
<212> RNA
<213> Artificial Sequence

<220>
<223> siRNA

<400> 251
ggccaagaag aacgcugcaa a 21

<210> 252
<211> 23
<212> RNA
<213> Artificial Sequence

<220>
<223> siRNA

<400> 252
uuugcagcgu ucuucuuggc cug 23

<210> 253
<211> 19
<212> RNA
<213> Artificial Sequence

<220>
<223> siRNA

<400> 253
ccaagaagaa cgcugcaaa 19

<210> 254
<211> 21
<212> RNA
<213> Artificial Sequence

<220>
<223> siRNA

<400> 254
uuugcagcgu ucuucuuggc c 21

<210> 255
<211> 19
<212> RNA
<213> Artificial Sequence

<220>
<223> siRNA

<400> 255
ccaagaagaa cgcugcaaa 19

<210> 256
<211> 21
<212> RNA
<213> Artificial Sequence

<220>
<223> siRNA

<400> 256
uuugcagcgu ucuucuuggc c 21

<210> 257
<211> 19
<212> RNA
<213> Artificial Sequence

<220>
<223> siRNA

<400> 257
ccaagaagaa cgcugcaaa 19

<210> 258
<211> 21
<212> RNA
<213> Artificial Sequence

<220>
<223> siRNA

<400> 258
uuugcagcgu ucuucuuggc c 21

<210> 259
<211> 21
<212> RNA
<213> Artificial Sequence

<220>
<223> siRNA

<400> 259
ggccaagaag aacgcugcaa a 21

<210> 260
<211> 23
<212> RNA
<213> Artificial Sequence

<220>
<223> siRNA

<400> 260
uuugcagcgu ucuucuuggc cug 23

<210> 261
<211> 21
<212> RNA
<213> Artificial Sequence

<220>
<223> siRNA

<400> 261
ggccaagaag aacgcugcaa a 21

<210> 262
<211> 23
<212> RNA
<213> Artificial Sequence

<220>
<223> siRNA

<400> 262
uuugcagcgu ucuucuuggc cug 23

<210> 263
<211> 21
<212> RNA
<213> Artificial Sequence

<220>
<223> siRNA

<400> 263
ggccaagaag aacgcugcaa a 21

<210> 264
<211> 23
<212> RNA
<213> Artificial Sequence

<220>
<223> siRNA

<400> 264
uuugcagcgu ucuucuuggc cug 23

<210> 265
<211> 19

<212>  RNA
<213>  Artificial Sequence

<220>
<223>  siRNA

<400>  265
ccaagaagaa cgcugcaaa 19

<210>  266
<211>  21
<212>  RNA
<213>  Artificial Sequence

<220>
<223>  siRNA

<400>  266
uuugcagcgu ucuucuuggc c 21

<210>  267
<211>  19
<212>  RNA
<213>  Artificial Sequence

<220>
<223>  siRNA

<400>  267
ccaagaagaa cgcugcaaa 19

<210>  268
<211>  21
<212>  RNA
<213>  Artificial Sequence

<220>
<223>  siRNA

<400>  268
uuugcagcgu ucuucuuggc c 21

<210>  269
<211>  19
<212>  RNA
<213>  Artificial Sequence

<220>
<223>  siRNA

<400>  269
ccaagaagaa cgcugcaaa 19

<210>  270
<211>  21
<212>  RNA
<213>  Artificial Sequence

<220>
<223> siRNA

<400> 270
uuugcagcgu ucuucuuggc c 21

<210> 271
<211> 21
<212> RNA
<213> Artificial Sequence

<220>
<223> siRNA

<400> 271
ggccaagaag aacgcugcaa a 21

<210> 272
<211> 23
<212> RNA
<213> Artificial Sequence

<220>
<223> siRNA

<400> 272
uuugcagcgu ucuucuuggc cug 23

<210> 273
<211> 21
<212> RNA
<213> Artificial Sequence

<220>
<223> siRNA

<400> 273
ggccaagaag aacgcugcaa a 21

<210> 274
<211> 23
<212> RNA
<213> Artificial Sequence

<220>
<223> siRNA

<400> 274
uuugcagcgu ucuucuuggc cug 23

<210> 275
<211> 21
<212> RNA
<213> Artificial Sequence

<220>
<223> siRNA

<400> 275
ggccaagaag aacgcugcaa a 21

<210> 276
<211> 23
<212> RNA
<213> Artificial Sequence

<220>
<223> siRNA

<400> 276
uuugcagcgu ucuucuuggc cug 23

<210> 277
<211> 19
<212> RNA
<213> Artificial Sequence

<220>
<223> siRNA

<400> 277
ccaagaagaa cgcugcaaa 19

<210> 278
<211> 21
<212> RNA
<213> Artificial Sequence

<220>
<223> siRNA

<400> 278
uuugcagcgu ucuucuuggc c 21

<210> 279
<211> 19
<212> RNA
<213> Artificial Sequence

<220>
<223> siRNA

<400> 279
ccaagaagaa cgcugcaaa 19

<210> 280
<211> 21
<212> RNA
<213> Artificial Sequence

<220>
<223> siRNA

<400> 280
uuugcagcgu ucuucuuggc c 21

<210> 281
<211> 19
<212> RNA
<213> Artificial Sequence

<220>
<223> siRNA

<400> 281
ccaagaagaa cgcugcaaa 19

<210> 282
<211> 21
<212> RNA
<213> Artificial Sequence

<220>
<223> siRNA

<400> 282
uuugcagcgu ucuucuuggc c 21

<210> 283
<211> 21
<212> RNA
<213> Artificial Sequence

<220>
<223> siRNA

<400> 283
ggccaagaag aacgcugcaa a 21

<210> 284
<211> 23
<212> RNA
<213> Artificial Sequence

<220>
<223> siRNA

<400> 284
uuugcagcgu ucuucuuggc cug 23

<210> 285
<211> 21
<212> RNA
<213> Artificial Sequence

<220>
<223> siRNA

<400> 285
ggccaagaag aacgcugcaa a 21

<210> 286
<211> 23

<212> RNA
<213> Artificial Sequence

<220>
<223> siRNA

<400> 286
uuugcagcgu ucuucuuggc cug 23

<210> 287
<211> 21
<212> RNA
<213> Artificial Sequence

<220>
<223> siRNA

<400> 287
ggccaagaag aacgcugcaa a 21

<210> 288
<211> 23
<212> RNA
<213> Artificial Sequence

<220>
<223> siRNA

<400> 288
uuugcagcgu ucuucuuggc cug 23

<210> 289
<211> 19
<212> RNA
<213> Artificial Sequence

<220>
<223> siRNA

<400> 289
ccaagaagaa cgcugcaaa 19

<210> 290
<211> 21
<212> RNA
<213> Artificial Sequence

<220>
<223> siRNA

<400> 290
uuugcagcgu ucuucuuggc c 21

<210> 291
<211> 19
<212> RNA
<213> Artificial Sequence

<220>
<223> siRNA

<400> 291
ccaagaagaa cgcugcaaa 19

<210> 292
<211> 21
<212> RNA
<213> Artificial Sequence

<220>
<223> siRNA

<400> 292
uuugcagcgu ucuucuuggc c 21

<210> 293
<211> 19
<212> RNA
<213> Artificial Sequence

<220>
<223> siRNA

<400> 293
ccaagaagaa cgcugcaaa 19

<210> 294
<211> 21
<212> RNA
<213> Artificial Sequence

<220>
<223> siRNA

<400> 294
uuugcagcgu ucuucuuggc c 21

<210> 295
<211> 21
<212> RNA
<213> Artificial Sequence

<220>
<223> siRNA

<400> 295
ggccaagaag aacgcugcaa a 21

<210> 296
<211> 23
<212> RNA
<213> Artificial Sequence

<220>
<223> siRNA

<400> 296
uuugcagcgu ucuucuuggc cug 23

<210> 297
<211> 21
<212> RNA
<213> Artificial Sequence

<220>
<223> siRNA

<400> 297
ggccaagaag aacgcugcaa a 21

<210> 298
<211> 23
<212> RNA
<213> Artificial Sequence

<220>
<223> siRNA

<400> 298
uuugcagcgu ucuucuuggc cug 23

<210> 299
<211> 21
<212> RNA
<213> Artificial Sequence

<220>
<223> siRNA

<400> 299
ggccaagaag aacgcugcaa a 21

<210> 300
<211> 23
<212> RNA
<213> Artificial Sequence

<220>
<223> siRNA

<400> 300
uuugcagcgu ucuucuuggc cug 23

<210> 301
<211> 19
<212> RNA
<213> Artificial Sequence

<220>
<223> siRNA

<220>
<221> misc_feature
<222> (19)..(19)

<223> n is Z21,and Z21 is A

<400> 301
ccaguaagca agccagaan 19

<210> 302
<211> 19
<212> RNA
<213> Artificial Sequence

<220>
<223> siRNA

<220>
<221> misc_feature
<222> (1)..(1)
<223> n is Z22,and Z22 is U

<400> 302
nuucuggcuu gcuuacugg 19

<210> 303
<211> 19
<212> RNA
<213> Artificial Sequence

<220>
<223> siRNA

<220>
<221> misc_feature
<222> (19)..(19)
<223> n is Z23, and Z23 is selected from A, U, G or C

<400> 303
ccaguaagca agccagaan 19

<210> 304
<211> 19
<212> RNA
<213> Artificial Sequence

<220>
<223> siRNA

<220>
<221> misc_feature
<222> (1)..(1)
<223> n is Z24, Z24 is a nucleotide complementary to Z23,and Z23 is selected from A, U, G or C

<400> 304
nuucuggcuu gcuuacugg 19

<210> 305
<211> 19
<212> RNA
<213> Artificial Sequence

<220>
<223> siRNA

<220>
<221> misc_feature
<222> (19)..(19)
<223> n is Z23, and Z23 is selected from A, U, G or C

<400> 305
ccaguaagca agccagaan 19

<210> 306
<211> 21
<212> RNA
<213> Artificial Sequence

<220>
<223> siRNA

<220>
<221> misc_feature
<222> (1)..(1)
<223> n is Z24, Z24 is a nucleotide complementary to Z23,and Z23 is selected from A, U, G or C

<400> 306
nuucuggcuu gcuuacuggu a 21

<210> 307
<211> 21
<212> RNA
<213> Artificial Sequence

<220>
<223> siRNA

<220>
<221> misc_feature
<222> (21)..(21)
<223> n is Z23, and Z23 is selected from A, U, G or C

<400> 307
uaccaguaag caagccagaa n 21

<210> 308
<211> 23
<212> RNA
<213> Artificial Sequence

<220>
<223> siRNA

<220>
<221> misc_feature
<222> (1)..(1)
<223> n is Z24, Z24 is a nucleotide complementary to Z23,and Z23 is selected from A, U, G or C

<400> 308
nuucuggcuu gcuuacuggu aac 23

<210> 309
<211> 19
<212> RNA
<213> Artificial Sequence

<220>
<223> siRNA

<400> 309
ccaguaagca agccagaaa 19

<210> 310
<211> 21
<212> RNA
<213> Artificial Sequence

<220>
<223> siRNA

<400> 310
uuucuggcuu gcuuacuggu a 21

<210> 311
<211> 21
<212> RNA
<213> Artificial Sequence

<220>
<223> siRNA

<400> 311
uaccaguaag caagccagaa a 21

<210> 312
<211> 23
<212> RNA
<213> Artificial Sequence

<220>
<223> siRNA

<400> 312
uuucuggcuu gcuuacuggu aac 23

<210> 313
<211> 19
<212> RNA
<213> Artificial Sequence

<220>
<223> siRNA

<400> 313
ccaguaagca agccagaaa 19

<210> 314

<211> 21
<212> RNA
<213> Artificial Sequence

<220>
<223> siRNA

<400> 314
uuucuggcuu gcuuacuggu a 21

<210> 315
<211> 19
<212> RNA
<213> Artificial Sequence

<220>
<223> siRNA

<400> 315
ccaguaagca agccagaaa 19

<210> 316
<211> 21
<212> RNA
<213> Artificial Sequence

<220>
<223> siRNA

<400> 316
uuucuggcuu gcuuacuggu a 21

<210> 317
<211> 19
<212> RNA
<213> Artificial Sequence

<220>
<223> siRNA

<400> 317
ccaguaagca agccagaaa 19

<210> 318
<211> 21
<212> RNA
<213> Artificial Sequence

<220>
<223> siRNA

<400> 318
uuucuggcuu gcuuacuggu a 21

<210> 319
<211> 21
<212> RNA
<213> Artificial Sequence

<220>
<223> siRNA

<400> 319
uaccaguaag caagccagaa a 21

<210> 320
<211> 23
<212> RNA
<213> Artificial Sequence

<220>
<223> siRNA

<400> 320
uuucuggcuu gcuuacuggu aac 23

<210> 321
<211> 21
<212> RNA
<213> Artificial Sequence

<220>
<223> siRNA

<400> 321
uaccaguaag caagccagaa a 21

<210> 322
<211> 23
<212> RNA
<213> Artificial Sequence

<220>
<223> siRNA

<400> 322
uuucuggcuu gcuuacuggu aac 23

<210> 323
<211> 21
<212> RNA
<213> Artificial Sequence

<220>
<223> siRNA

<400> 323
uaccaguaag caagccagaa a 21

<210> 324
<211> 23
<212> RNA
<213> Artificial Sequence

<220>
<223> siRNA

<400> 324
uuucuggcuu gcuuacuggu aac 23

<210> 325
<211> 19
<212> RNA
<213> Artificial Sequence

<220>
<223> siRNA

<400> 325
ccaguaagca agccagaaa 19

<210> 326
<211> 21
<212> RNA
<213> Artificial Sequence

<220>
<223> siRNA

<400> 326
uuucuggcuu gcuuacuggu a 21

<210> 327
<211> 19
<212> RNA
<213> Artificial Sequence

<220>
<223> siRNA

<400> 327
ccaguaagca agccagaaa 19

<210> 328
<211> 21
<212> RNA
<213> Artificial Sequence

<220>
<223> siRNA

<400> 328
uuucuggcuu gcuuacuggu a 21

<210> 329
<211> 19
<212> RNA
<213> Artificial Sequence

<220>
<223> siRNA

<400> 329
ccaguaagca agccagaaa 19

<210> 330
<211> 21
<212> RNA
<213> Artificial Sequence

<220>
<223> siRNA

<400> 330
uuucuggcuu gcuuacuggu a 21

<210> 331
<211> 21
<212> RNA
<213> Artificial Sequence

<220>
<223> siRNA

<400> 331
uaccaguaag caagccagaa a 21

<210> 332
<211> 23
<212> RNA
<213> Artificial Sequence

<220>
<223> siRNA

<400> 332
uuucuggcuu gcuuacuggu aac 23

<210> 333
<211> 21
<212> RNA
<213> Artificial Sequence

<220>
<223> siRNA

<400> 333
uaccaguaag caagccagaa a 21

<210> 334
<211> 23
<212> RNA
<213> Artificial Sequence

<220>
<223> siRNA

<400> 334
uuucuggcuu gcuuacuggu aac 23

<210> 335

<211> 21
<212> RNA
<213> Artificial Sequence

<220>
<223> siRNA

<400> 335
uaccaguaag caagccagaa a 21

<210> 336
<211> 23
<212> RNA
<213> Artificial Sequence

<220>
<223> siRNA

<400> 336
uuucuggcuu gcuuacuggu aac 23

<210> 337
<211> 19
<212> RNA
<213> Artificial Sequence

<220>
<223> siRNA

<400> 337
ccaguaagca agccagaaa 19

<210> 338
<211> 21
<212> RNA
<213> Artificial Sequence

<220>
<223> siRNA

<400> 338
uuucuggcuu gcuuacuggu a 21

<210> 339
<211> 19
<212> RNA
<213> Artificial Sequence

<220>
<223> siRNA

<400> 339
ccaguaagca agccagaaa 19

<210> 340
<211> 21
<212> RNA
<213> Artificial Sequence

<220>
<223> siRNA

<400> 340
uuucuggcuu gcuuacuggu a 21

<210> 341
<211> 19
<212> RNA
<213> Artificial Sequence

<220>
<223> siRNA

<400> 341
ccaguaagca agccagaaa 19

<210> 342
<211> 21
<212> RNA
<213> Artificial Sequence

<220>
<223> siRNA

<400> 342
uuucuggcuu gcuuacuggu a 21

<210> 343
<211> 21
<212> RNA
<213> Artificial Sequence

<220>
<223> siRNA

<400> 343
uaccaguaag caagccagaa a 21

<210> 344
<211> 23
<212> RNA
<213> Artificial Sequence

<220>
<223> siRNA

<400> 344
uuucuggcuu gcuuacuggu aac 23

<210> 345
<211> 21
<212> RNA
<213> Artificial Sequence

<220>
<223> siRNA

<400> 345
uaccaguaag caagccagaa a 21

<210> 346
<211> 23
<212> RNA
<213> Artificial Sequence

<220>
<223> siRNA

<400> 346
uuucuggcuu gcuuacuggu aac 23

<210> 347
<211> 21
<212> RNA
<213> Artificial Sequence

<220>
<223> siRNA

<400> 347
uaccaguaag caagccagaa a 21

<210> 348
<211> 23
<212> RNA
<213> Artificial Sequence

<220>
<223> siRNA

<400> 348
uuucuggcuu gcuuacuggu aac 23

<210> 349
<211> 19
<212> RNA
<213> Artificial Sequence

<220>
<223> siRNA

<400> 349
ccaguaagca agccagaaa 19

<210> 350
<211> 21
<212> RNA
<213> Artificial Sequence

<220>
<223> siRNA

<400> 350
uuucuggcuu gcuuacuggu a 21

<210> 351
<211> 19
<212> RNA
<213> Artificial Sequence

<220>
<223> siRNA

<400> 351
ccaguaagca agccagaaa 19

<210> 352
<211> 21
<212> RNA
<213> Artificial Sequence

<220>
<223> siRNA

<400> 352
uuucuggcuu gcuuacuggu a 21

<210> 353
<211> 19
<212> RNA
<213> Artificial Sequence

<220>
<223> siRNA

<400> 353
ccaguaagca agccagaaa 19

<210> 354
<211> 21
<212> RNA
<213> Artificial Sequence

<220>
<223> siRNA

<400> 354
uuucuggcuu gcuuacuggu a 21

<210> 355
<211> 21
<212> RNA
<213> Artificial Sequence

<220>
<223> siRNA

<400> 355
uaccaguaag caagccagaa a 21

<210> 356

<211> 23
<212> RNA
<213> Artificial Sequence

<220>
<223> siRNA

<400> 356
uuucuggcuu gcuuacuggu aac 23

<210> 357
<211> 21
<212> RNA
<213> Artificial Sequence

<220>
<223> siRNA

<400> 357
uaccaguaag caagccagaa a 21

<210> 358
<211> 23
<212> RNA
<213> Artificial Sequence

<220>
<223> siRNA

<400> 358
uuucuggcuu gcuuacuggu aac 23

<210> 359
<211> 21
<212> RNA
<213> Artificial Sequence

<220>
<223> siRNA

<400> 359
uaccaguaag caagccagaa a 21

<210> 360
<211> 23
<212> RNA
<213> Artificial Sequence

<220>
<223> siRNA

<400> 360
uuucuggcuu gcuuacuggu aac 23

<210> 361
<211> 19
<212> RNA
<213> Artificial Sequence

<220>
<223> siRNA

<400> 361
cuucauucau acagacaaa 19

<210> 362
<211> 21
<212> RNA
<213> Artificial Sequence

<220>
<223> siRNA

<400> 362
uuugucugua ugaaugaaga g 21

<210> 363
<211> 19
<212> RNA
<213> Artificial Sequence

<220>
<223> siRNA

<400> 363
cuacaguuua gaagauuua 19

<210> 364
<211> 21
<212> RNA
<213> Artificial Sequence

<220>
<223> siRNA

<400> 364
uaaaucuucu aaacuguagu a 21

<210> 365
<211> 19
<212> RNA
<213> Artificial Sequence

<220>
<223> siRNA

<400> 365
ggaagguuac cgagcaaua 19

<210> 366
<211> 21
<212> RNA
<213> Artificial Sequence

<220>
<223> siRNA

<400> 366
uauugcucgg uaaccuuccc u 21

<210> 367
<211> 19
<212> RNA
<213> Artificial Sequence

<220>
<223> siRNA

<400> 367
agaacagaca gcagaauua 19

<210> 368
<211> 21
<212> RNA
<213> Artificial Sequence

<220>
<223> siRNA

<400> 368
uaauucugcu gucuguucuc c 21

<210> 369
<211> 19
<212> RNA
<213> Artificial Sequence

<220>
<223> siRNA

<400> 369
ccaagaagaa cgcugcaaa 19

<210> 370
<211> 21
<212> RNA
<213> Artificial Sequence

<220>
<223> siRNA

<400> 370
uuugcagcgu ucuucuuggc c 21

<210> 371
<211> 19
<212> RNA
<213> Artificial Sequence

<220>
<223> siRNA

<400> 371
ccaguaagca agccagaaa 19

<210> 372
<211> 21
<212> RNA
<213> Artificial Sequence

<220>
<223> siRNA

<400> 372
uuucuggcuu gcuuacuggu a 21

<210> 373
<211> 19
<212> RNA
<213> Artificial Sequence

<220>
<223> siRNA

<400> 373
cuucauucau acagacaaa 19

<210> 374
<211> 21
<212> RNA
<213> Artificial Sequence

<220>
<223> siRNA

<400> 374
uuugucugua ugaaugaaga g 21

<210> 375
<211> 19
<212> RNA
<213> Artificial Sequence

<220>
<223> siRNA

<400> 375
cuacaguuua gaagauuua 19

<210> 376
<211> 21
<212> RNA
<213> Artificial Sequence

<220>
<223> siRNA

<400> 376
uaaaucuucu aaacuguagu a 21

<210> 377

<211> 19
<212> RNA
<213> Artificial Sequence

<220>
<223> siRNA

<400> 377
ggaagguuac cgagcaaua 19

<210> 378
<211> 21
<212> RNA
<213> Artificial Sequence

<220>
<223> siRNA

<400> 378
uauugcucgg uaaccuuccc u 21

<210> 379
<211> 19
<212> RNA
<213> Artificial Sequence

<220>
<223> siRNA

<400> 379
agaacagaca gcagaauua 19

<210> 380
<211> 21
<212> RNA
<213> Artificial Sequence

<220>
<223> siRNA

<400> 380
uaauucugcu gucuguucuc c 21

<210> 381
<211> 19
<212> RNA
<213> Artificial Sequence

<220>
<223> siRNA

<400> 381
ccaagaagaa cgcugcaaa 19

<210> 382
<211> 21
<212> RNA
<213> Artificial Sequence

<220>
<223> siRNA

<400> 382
uuugcagcgu ucuucuuggc c 21

<210> 383
<211> 19
<212> RNA
<213> Artificial Sequence

<220>
<223> siRNA

<400> 383
ccaguaagca agccagaaa 19

<210> 384
<211> 21
<212> RNA
<213> Artificial Sequence

<220>
<223> siRNA

<400> 384
uuucuggcuu gcuuacuggu a 21

<210> 385
<211> 20
<212> DNA
<213> Artificial Sequence

<220>
<223> Primer

<400> 385
atcaggccag ggaaggttac 20

<210> 386
<211> 20
<212> DNA
<213> Artificial Sequence

<220>
<223> Primer

<400> 386
tcgggatgaa ggaaccatgt 20

<210> 387
<211> 19
<212> DNA
<213> Artificial Sequence

<220>
<223> Primer

<400> 387
ggtcggagtc aacggattt 19

<210> 388
<211> 19
<212> DNA
<213> Artificial Sequence

<220>
<223> Primer

<400> 388
ccagcatcgc cccacttga 19

## Description

### TECHNICAL FIELD

The present disclosure relates to a nucleic acid, a pharmaceutical composition and a siRNA conjugate capable of inhibiting expression of a complement protein 5(C5) gene. The present disclosure also relates to a preparation method and use of the conjugate of the nucleic acid, the pharmaceutical composition and the siRNA conjugate.

### BACKGROUND

Myasthenia gravis (MG) is an acquired autoimmune disease, which is mainly mediated by Acetylcholine Receptor Antibody (AchR-Ab), depended on cellular immunity, participated by complements, and involves the Acetylcholine Receptor (AChR) on the postsynaptic membrane of neuromuscular junction.

Complement protein (C5) is one of the key targets for treating myasthenia gravis.With the participation of complements, AchR-Ab is combined with AchR, which destroys a large number of AchR through complement-mediated cell membrane lysis, resulting in muscle weakness due to the obstacle of acetylcholine transmission in the postsynaptic membrane. Studies have shown that by combining drugs with complement protein C5 specifically, C5 can be prevented from cracking into C5a and C5b, thus preventing the formation of a membrane attack complex, blocking the destruction of the membrane attack complex on the neuromuscular junction and the subsequent production of proinflammatory factors, thus exerting immunosuppression and treating myasthenia gravis.

Small interfering RNA (siRNA), based on the mechanism of RNA interference (RNAi), can inhibit or block the expression of interested target genes in a sequence-specific way, thus achieving the purpose of treating diseases. It will undoubtedly be the most ideal treatment if the expression of C5 gene can be inhibited to block the production of complement proteins, maintain the normal immune functions and inhibit the abnormal immune response from an mRNA level.

The key to develop siRNA drugs for inhibiting the expression of the C5 gene and treating myasthenia gravis lies in finding a suitable siRNA and modification and an effective delivery system thereof.

### SUMMARY OF THE INVENTION

The inventors of the present disclosure have surprisingly found that the siRNA conjugate provided by the present disclosure herein can specifically inhibit the expression of the C5 gene, specifically target the liver, inhibit the expression of the C5 gene in the liver, and realize the treatment or prevention of myasthenia gravis. Moreover, the inventors have also invented a siRNA with high activity and a pharmaceutical composition.

In some embodiments, the present disclosure provides a siRNA conjugate, wherein the siRNA conjugate has a structure as shown by Formula (308):

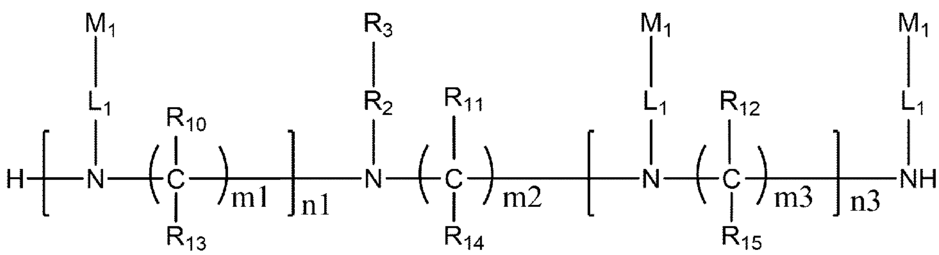

Formula (308),

wherein: n1 is an integer of 1-3, and n3 is an integer of 0-4; each of m1, m2, and m3 is independently an integer of 2-10; each of $R_{10}$, $R_{11}$, $R_{12}$, $R_{13}$, $R_{14}$ or $R_{15}$ is independently H or selected from the group consisting of $C_1$-$C_{10}$ alkyl, $C_1$-$C_{10}$ haloalkyl and $C_1$-$C_{10}$ alkoxy;

$R_3$ is a group having a structure as shown by Formula A59:

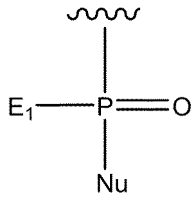

(A59)

wherein, $E_1$ is OH, SH or $BH_2$; and

Nu is siRNA; the siRNA comprises a sense strand and an antisense strand, each nucleotide in the siRNA is independently a modified or unmodified nucleotide, wherein the sense strand comprises a nucleotide sequence I, and the antisense strand comprises a nucleotide sequence II; the nucleotide sequence I and the nucleotide sequence II are at least partly reverse complementary to form a double-stranded region; and the nucleotide sequence I and the nucleotide sequence II are selected from a group of sequences shown in the following i) - vi):

i) the nucleotide sequence I has the same length and no more than three nucleotides difference from the nucleotide sequence shown in SEQ ID NO: 1; and the nucleotide sequence II has the same length and no more than three nucleotides difference from the nucleotide sequence shown in SEQ ID NO: 2:

5'-CUUCAUUCAUACAGACAAZi-3' (SEQ ID NO: 1);

5'-$Z_2$UUGUCUGUAUGAAUGAAG-3' (SEQ ID NO: 2),

wherein, $Z_1$ is A, $Z_2$ is U, the nucleotide sequence I comprises a nucleotide $Z_3$ at a corresponding site to $Z_1$, the nucleotide sequence II comprises a nucleotide $Z_4$ at a corresponding site to $Z_2$, and $Z_4$ is the first nucleotide from the 5' terminal of the antisense strand ;

ii) the nucleotide sequence I has the same length and no more than three nucleotides differences from the nucleotide sequence shown in SEQ ID NO: 61; and the nucleotide sequence II has the same length and no more than three nucleotides differences from the nucleotide sequence shown in SEQ ID NO: 62:

5'-CUACAGUUUAGAAGAUUU$Z_5$-3' (SEQ ID NO: 61);

5'-$Z_6$AAAUCUUCUAAACUGUAG-3' (SEQ ID NO: 62),

wherein, $Z_5$ is A, $Z_6$ is U, the nucleotide sequence I comprises a nucleotide $Z_7$ at a corresponding site to $Z_5$, the nucleotide sequence II comprises a nucleotide $Z_8$ at a corresponding site to $Z_6$, and $Z_8$ is the first nucleotide from the 5' terminal of the antisense strand;

iii) the nucleotide sequence I has the same length and no more than three nucleotides differences from the nucleotide sequence shown in SEQ ID NO: 121; and the nucleotide sequence II has the same length and no more than three nucleotides differences from the nucleotide sequence shown in SEQ ID NO: 122:

5'-GGAAGGUUACCGAGCAAU$Z_9$-3' (SEQ ID NO: 121);

5'-$Z_{10}$AUUGCUCGGUAACCUUCC-3' (SEQ ID NO: 122),

wherein, $Z_9$ is A, $Z_{10}$ is U, the nucleotide sequence I comprises a nucleotide $Z_{11}$ at a corresponding site to $Z_9$, the nucleotide sequence II comprises a nucleotide $Z_{12}$ at a corresponding site to $Z_{10}$, and $Z_{12}$ is the first nucleotide from the 5' terminal of the antisense strand;

iv) the nucleotide sequence I has the same length and no more than three nucleotides differences from the nucleotide sequence shown in SEQ ID NO: 181; and the nucleotide sequence II has the same length and no more than three nucleotides differences from the nucleotide sequence shown in SEQ ID NO: 182:

5'-AGAACAGACAGCAGAAUU$Z_{13}$-3' (SEQ ID NO: 181);

5'-$Z_{14}$AAUUCUGCUGUCUGUUCU-3' (SEQ ID NO: 182),

wherein, $Z_{13}$ is A, $Z_{14}$ is U, the nucleotide sequence I comprises a nucleotide $Z_{15}$ at a corresponding site to $Z_{13}$, the nucleotide sequence II comprises a nucleotide $Z_{16}$ at a corresponding site to $Z_{14}$, and $Z_{16}$ is the first nucleotide from the 5' terminal of the antisense strand;

v) the nucleotide sequence I has the same length and no more than three nucleotides differences from the nucleotide sequence shown in SEQ ID NO: 241; and the nucleotide sequence II has the same length and no more than three nucleotides differences from the nucleotide sequence shown in SEQ ID NO: 242:

5'-CCAAGAAGAACGCUGCAA$Z_{17}$-3' (SEQ ID NO: 241);

5'-$Z_{18}$UUGCAGCGUUCUUCUUGG-3' (SEQ ID NO: 242),

wherein, $Z_{17}$ is A, $Z_{18}$ is U, the nucleotide sequence I comprises a nucleotide $Z_{19}$ at a corresponding site to $Z_{17}$, the nucleotide sequence II comprises a nucleotide $Z_{20}$ at a corresponding site to $Z_{18}$, and $Z_{20}$ is the first nucleotide from the 5' terminal of the antisense strand; and,

vi) the nucleotide sequence I has the same length and no more than three nucleotides differences from the nucleotide sequence shown in SEQ ID NO: 301; and the nucleotide sequence II has the same length and no more than three nucleotides differences from the nucleotide sequence shown in SEQ ID NO: 302:

5'-CCAGUAAGCAAGCCAGAA$Z_{21}$-3' (SEQ ID NO: 301);

5'-$Z_{22}$UUCUGGCUUGCUUACUGG-3' (SEQ ID NO: 302),

wherein, $Z_{21}$ is A, $Z_{22}$ is U, the nucleotide sequence I comprises a nucleotide $Z_{23}$ at a corresponding site to $Z_{21}$, the nucleotide sequence II comprises a nucleotide $Z_{24}$ at a corresponding site to $Z_{22}$, and $Z_{24}$ is the first nucleotide from the 5' terminal of the antisense strand;

$R_2$ is a linear alkylene of 1-20 carbon atoms in length, wherein one or more carbon atoms are optionally replaced with any one or more of the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $R_2$ is optionally substituted by any one or more of the group consisting of: $C_1$-$C_{10}$ alkyl, $C_6$-$C_{10}$ aryl, $C_5$-$C_{10}$ heteroaryl, $C_1$-$C_{10}$ haloalkyl, -O$C_1$-$C_{10}$ alkyl, -O$C_1$-$C_{10}$ alkylphenyl, -$C_1$-$C_{10}$ alkyl-OH, -O$C_1$-$C_{10}$ haloalkyl, -S$C_1$-$C_{10}$ alkyl, -S$C_1$-$C_{10}$ alkylphenyl, -$C_1$-$C_{10}$ alkyl-SH, -S$C_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -CO2H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -NH$SO_2$($C_1$-$C_{10}$ alkyl), -NH$SO_2$(phenyl), and -NH$SO_2$($C_1$-$C_{10}$ haloalkyl);

each $L_1$ is independently a linear alkylene of 1-70 carbon atoms in length, wherein one or more carbon atoms are optionally replaced with any one or more of the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ is optionally substituted by any one or more of the group consisting of: $C_1$-$C_{10}$ alkyl, $C_6$-$C_{10}$ aryl, $C_5$-$C_{10}$ heteroaryl, $C_1$-$C_{10}$ haloalkyl, -O$C_1$-$C_{10}$ alkyl, -O$C_1$-$C_{10}$ alkylphenyl, -$C_1$-$C_{10}$ alkyl-OH, -O$C_1$-$C_{10}$ haloalkyl, -S$C_1$-$C_{10}$ alkyl, -S$C_1$-$C_{10}$ alkylphenyl, -$C_1$-$C_{10}$ alkyl-SH, -S$C_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -NH$SO_2$($C_1$-$C_{10}$ alkyl), -NH$SO_2$(phenyl), and -NH$SO_2$($C_1$-$C_{10}$ haloalkyl); and

 represents a site where the group is covalently linked; and $M_1$ represents a targeting group.

In some embodiments, the present disclosure provides a siRNA capable of inhibiting expression of a C5 gene, wherein the siRNA comprises a sense strand and an antisense strand, and each nucleotide in the sense strand and the antisense strand is independently a fluoro modified nucleotide or a non-fluoro modified nucleotide; the sense strand comprises a nucleotide sequence I, the antisense strand comprises a nucleotide sequence II, the nucleotide sequence I and the nucleotide sequence II are at least partly reverse complementary to form a double-stranded region, the fluoro modified nucleotides are located in the nucleotide sequence I and the nucleotide sequence II, and, in the direction from 5' terminal to 3' terminal, the nucleotides at positions 7, 8 and 9 of the nucleotide sequence I in the sense strand are fluoro modified nucleotides, and the nucleotides at the rest of positions in the sense strand are non-fluoro modified nucleotides; in the direction from 5' terminal to 3' terminal, the nucleotides at positions 2, 6, 14 and 16 of the nucleotide sequence II in the antisense strand are fluoro modified nucleotides, and the nucleotides at the rest of positions in the antisense strand are non-fluoro modified nucleotides, and, the nucleotide sequence I and the nucleotide sequence II are selected from one of the above i) - vi).

In some embodiments, the present disclosure provides a pharmaceutical composition, wherein the pharmaceutical composition comprises the above-mentioned siRNA of the present disclosure and a pharmaceutically acceptable carrier.

In some embodiments, the present disclosure provides a siRNA conjugate, wherein the siRNA conjugate comprises the above-mentioned siRNA provided by the present disclosure and a conjugating group conjugatively linked to the siRNA.

In some embodiments, the present disclosure provides use of the siRNA and/or the pharmaceutical composition and/or the siRNA conjugate according to the present disclosure in the manufacture of a medicament for treating and/or preventing myasthenia gravis.

In some embodiments, the present disclosure provides a method for treating and/or preventing myasthenia gravis, wherein the method comprises administering an effective amount of the siRNA and/or the pharmaceutical composition and/or the siRNA conjugate of the present disclosure to a subject suffering from myasthenia gravis.

In some embodiments, the present disclosure provides a method for inhibiting expression of a C5 gene in a hepatocyte, wherein the method comprises contacting an effective amount of the siRNA and/or the pharmaceutical composition and/or the siRNA conjugate of the present disclosure to the hepatocyte.

In some embodiments, the present disclosure provides a kit, wherein the kit comprises the siRNA and/or the pharmaceutical composition and/or the siRNA conjugate of the present disclosure.

**Advantageous Effects**

In some embodiments, the siRNA, the pharmaceutical composition and the siRNA conjugate provided by the present disclosure have better stability, higher C5 mRNA inhibitory activity and lower off-target effect, and/or can significantly treat or relieve myasthenia gravis symptoms.

In some embodiments, the siRNA, the pharmaceutical composition or the siRNA conjugate provided by the present disclosure exhibits excellent target mRNA inhibitory activity in cell experiments *in vitro.* In some embodiments, the siRNA, the pharmaceutical composition or the siRNA conjugate provided by the present disclosure exhibits an inhibition percentage to target mRNA in a hepatocyte of at least 20%, 30%, 40%, 50%, 60%, 70%, 80%, 90%, or 95%.

In some embodiments, the siRNA provided by the present disclosure exhibits higher inhibitory activity in HepG2 cells, and the $IC_{50}$ for C5 mRNA is between 1.494 nM and 9.688 nM. In some embodiments, the siRNA conjugate according to the present disclosure with fluorescent label is injected subcutaneously into C57 mice to perform real-time fluorescence imaging on the mice and observe distribution of fluorescence in organs. After 48 hours, the mice are killed for organ dissection, and it is found that almost all siRNA conjugates are gathered in the liver, indicating that the siRNA conjugate provided by the present disclosure can effectively deliver siRNA to the liver specifically, indicating that the conjugate can specifically inhibit the expression of target mRNA in the liver.

In some embodiments, the siRNA, the pharmaceutical composition or the siRNA conjugate provided by the present disclosure may exhibit higher stability and/or higher activity *in vivo.* In some embodiments, the siRNA, the pharmaceutical composition or the siRNA conjugate provided by the present disclosure exhibits an inhibition percentage to target mRNA target mRNA of at least 20%, 30%, 40%, 50%, 60%, 70%, 80%, 90%, or 95% *in vivo.* In some embodiments, the siRNA, the pharmaceutical composition or the siRNA conjugate provided by the present disclosure exhibits an inhibition percentage to C5 mRNA expression of at least 20%, 30%, 40%, 50%, 60%, 70%, 80%, 90%, or 95% *in vivo.* In some embodiments, the siRNA, the pharmaceutical composition or the siRNA conjugate provided by the present disclosure exhibits an inhibition percentage to C5 mRNA expression in liver of at least 20%, 30%, 40%, 50%, 60%, 70%, 80%, 90%, or 95% *in vivo.* In some embodiments, the siRNA, the pharmaceutical composition or the siRNA conjugate provided by the present disclosure exhibits an inhibition percentage to C5 mRNA expression in liver in animal models of at least 20%, 30%, 40%, 50%, 60%, 70%, 80%, 90%, or 95% *in vivo.* In some embodiments, the siRNA, the pharmaceutical composition or the siRNA conjugate provided by the present disclosure exhibits an inhibition percentage to

C5 mRNA expression in liver in human subjects of at least 20%, 30%, 40%, 50%, 60%, 70%, 80%, 90%, or 95% *in vivo.*

In some embodiments, the siRNA, the pharmaceutical composition or the siRNA conjugate provided by the present disclosure exhibits no significant off-target effect. An off-target effect may be, for example, inhibition on normal expression of a gene which is not the target gene. It is considered insignificant if the binding/inhibition of off-target gene expression is at a level of lower than 50%, 40%, 30%, 20%, or 10% of the on-target effect.

In this way, it is indicated that the siRNA, the pharmaceutical composition and the siRNA conjugate provided by the present disclosure can inhibit the expression of C5 mRNA, effectively treat and/or prevent myasthenia gravis symptoms, and have good application prospects.

Other features and advantages of the present disclosure will be described in detail in the detailed description section that follows.

## DESCRIPTION OF THE DRAWINGS

FIGs. 1A-1H are dose-response curves fitted according to relative expression levels of C5 mRNA in HepG2 cells after transfection of different conjugates 1-8.

FIG. 2A is a fluorescence imaging photograph of various organs in C57 mice after administration of 5 ml/kg 1 × PBS, 3 mg/kg Cy5-siRNA 1 or 3 mg/kg Cy5-conjugate 1 for 48 hours.

FIG. 2B is a fluorescence imaging photograph of various organs in C57 mice after administration of 5 ml/kg 1 × PBS, 3 mg/kg Cy5-siRNA 2 or 3 mg/kg Cy5-conjugate 2 for 48 hours.

## DETAILED DESCRIPTION OF THE INVENTION

The specific embodiments of the present disclosure are described in detail as below. It should be understood that the specific embodiments described herein are only for the purpose of illustration and explanation of the present disclosure and are not intended to limit the present disclosure in any respect.

In the present disclosure, C5 mRNA refers to the mRNA with the sequence shown in Genbank registration number M57729.1. Furthermore, unless otherwise stated, the term "target gene" used in the present disclosure refers to a gene capable of transcribing the above C5 mRNA, and the term "target mRNA" refers to the above C5 mRNA.

### Definitions

In the context of the present disclosure, unless otherwise specified, capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the lowercase letter s indicates that the two nucleotides adjacent to the left and right of the letter s are linked by phosphorothioate; Pl represents that the nucleotide adjacent to the right side of Pl is a 5'-phosphate nucleotide or a 5'-phosphate analogue modified nucleotide, the letter combination VP represents that the nucleotide adjacent to the right side of the letter combination VP is a vinyl phosphate modified nucleotide, the letter combination Ps represents that the nucleotide adjacent to the right side of the letter combination Ps is a phosphorothioate modified nucleotide, and the capital letter P represents that the nucleotide adjacent to the right side of the letter P is a 5'-phosphate nucleotide.

In the context of the present disclosure, the "fluoro modified nucleotide" refers to a nucleotide formed by substituting the 2'-hydroxy of the ribose group of the nucleotide with a fluoro, and the "non-fluoro modified nucleotide" refers to a nucleotide formed by substituting the 2'-hydroxy of the ribose group of the nucleotide with a non-fluoro group, or a nucleotide analogue. The "nucleotide analogue" refers to a group that can replace a nucleotide in a nucleic acid, while structurally differs from an adenine ribonucleotide, a guanine ribonucleotide, a cytosine ribonucleotide, a uracil ribonucleotide or a thymidine deoxyribonucleotide, such as an isonucleotide, a bridged nucleic acid (BNA) nucleotide or an acyclic nucleotide. The "methoxy modified nucleotide" refers to a nucleotide formed by substituting the 2'-hydroxy of the ribose group with a methoxy group.

In the context of the present disclosure, expressions "complementary" and "reverse complementary" can be interchangeably used, and have a well-known meaning in the art, namely, the bases in one strand are complementarily paired with those in the other strand of a double-stranded nucleic acid molecule. In DNA, a purine base adenine (A) is always paired with a pyrimidine base thymine (T) (or uracil (U) in RNAs); and a purine base guanine (G) is always paired with a pyrimidine base cytosine (C). Each base pair comprises a purine and a pyrimidine. While adenines in one strand

are always paired with thymines (or uracils) in another strand, and guanines are always paired with cytosines, these two strands are considered as being complementary each other; and the sequence of a strand may be deduced from the sequence of its complementary strand. Correspondingly, a "mispairing" means that in a double-stranded nucleic acid, the bases at corresponding sites are not presented in a manner of being complementarily paired.

In the context of the present disclosure, unless otherwise specified, "basically reverse complementary" means that there are no more than 3 base mispairings between two nucleotide sequences. "Substantially reverse complementary" means that there is no more than 1 base mispairing between two nucleotide sequences. "Completely complementary" means that there is no based mispairing between two nucleotide sequences.

In the context of the present disclosure, when a nucleotide sequence has "nucleotide difference" from another nucleotide sequence, the bases of the nucleotides at the same position therebetween are changed. For example, if a nucleotide base in the second sequence is A and the nucleotide base at the same position in the first sequence is U, C, G or T, these two nucleotide sequences are considered as having a nucleotide difference at this position. In some embodiments, if a nucleotide at a position is replaced with an abasic nucleotide or a nucleotide analogue, it is also considered that there is a nucleotide difference at the position.

In the context of the present disclosure, particularly in the description of the method for preparing the siRNA, the composition comprising the siRNA or the siRNA conjugate of the present disclosure, unless otherwise specified, the nucleoside monomer refers to, according to the kind and sequence of the nucleotides in the siRNA or siRNA conjugate to be prepared, unmodified or modified RNA phosphoramidites used in a solid phase phosphoramidite synthesis (the RNA phosphoramidites are also called as Nucleoside phosphoramidites elsewhere). Solid phase phosphoramidite synthesis is a well-known method used in RNA synthesis to those skilled in the art. Nucleoside monomers used in the present disclosure can all be commercially available.

In the context of the present disclosure, unless otherwise stated, "conjugating" refers to two or more chemical moieties each with specific function being linked to each other via a covalent linkage. Correspondingly, a "conjugate" refers to a compound formed by covalent linkage of individual chemical moieties. Further, a "siRNA conjugate" represents a compound formed by covalently linking one or more chemical moieties with specific functions to siRNA. Hereinafter, the siRNA conjugate of the present disclosure is sometimes abbreviated as "conjugate". The siRNA conjugate should be understood according to the context as the generic term of a plurality of siRNA conjugates or siRNA conjugates shown in certain chemical formulae. In the context of the present disclosure, a "conjugating molecule" should be understood as a specific compound capable of being conjugated to a siRNA via reactions, thus finally forming the siRNA conjugate of the present disclosure.

As used herein, "optional" or "optionally" means that the subsequently described event or condition may or may not occur, and that the description includes instances wherein the event or condition may or may not occur. For example, "optionally substituted" "alkyl" encompasses both "alkyl" and "substituted alkyl" as defined below. Those skilled in the art would understand, with respect to any group containing one or more substituents, that such groups are not intended to introduce any substitution or substitution patterns that are sterically impractical, synthetically infeasible and/or inherently unstable.

As used herein, "alkyl" refers to straight chain and branched chain having the indicated number of carbon atoms, usually 1 to 20 carbon atoms, for example 1 to 10 carbon atoms, such as 1 to 8 or 1 to 6 carbon atoms. For example, $C_1$-$C_6$ alkyl encompasses both straight and branched chain alkyl of 1 to 6 carbon atoms. When naming an alkyl residue having a specific number of carbon atoms, all branched and straight chain forms having that number of carbon atoms are intended to be encompassed; thus, for example, "butyl" is meant to include n-butyl, sec-butyl, isobutyl and t-butyl; and "propyl" includes n-propyl and isopropyl. Alkylene is a subset of alkyl, referring to the same residues as alkyl, but having two attachment positions.

As used herein, "alkenyl" refers to an unsaturated branched or linear alkyl having at least one carbon-carbon double bond which is obtained by respectively removing one hydrogen molecule from two adjacent carbon atoms of the parent alkyl. The group may be in either cis or trans configuration of the double bond. Typical alkenyl groups include, but not limited to, ethenyl; propenyls such as prop-1-en-1-yl, prop-1-en-2-yl, prop-2-en-1-yl (allyl), and prop-2-en-2-yl; and butenyls such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, and the like. In certain embodiments, an alkenyl group has 2 to 20 carbon atoms, and in other embodiments, 2 to 10, 2 to 8, or 2 to 6 carbon atoms. Alkenylene is a subset of alkenyl, referring to the same residues as alkenyl, but having two attachment positions.

As used herein, "alkynyl" refers to an unsaturated branched or linear alkyl having at least one carbon-carbon triple bond which is obtained by respectively removing two hydrogen molecules from two adjacent carbon atoms of the parent alkyl. Typical alkynyl groups include, but not limited to, ethynyl; propynyls such as prop-1-yn-1-yl, and prop-2-yn-1-yl; and butynyls such as but-1-yn-1-yl, but-1-yn-3-yl, but-3-yn-1-yl, and the like. In certain embodiments, an alkynyl group has 2 to 20 carbon atoms, and in other embodiments, 2 to 10, 2 to 8, or 2 to 6 carbon atoms. Alkynylene is a subset of alkynyl, referring to the same residues as alkynyl, but having two attachment positions.

As used herein, "alkoxy" refers to an alkyl group of the indicated number of carbon atoms attached through an

oxygen bridge, such as, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. An alkoxy usually has 1 to 10, 1 to 8, 1 to 6, or 1 to 4 carbon atoms attached through oxygen bridge.

As used herein, "aryl" refers to a group derived from an aromatic monocyclic or multicyclic hydrocarbon ring system by removing a hydrogen atom from a ring carbon atom. The aromatic monocyclic or multicyclic hydrocarbon ring system contains only hydrogen and 6 to 18 carbon atoms, wherein at least one ring in the ring system is fully unsaturated, i.e., containing a cyclic, delocalized $(4n+2)\pi$-electron system in accordance with the Hückel theory. Aryl groups include, but not limited to, phenyl, fluorenyl, naphthyl and the like. Arylene is a subset of aryl, referring to the same residues as aryl, but having two attachment positions.

As used herein, "halo substituent" or " halogen" refers to fluoro, chloro, bromo, and iodo, and the term "halogen" includes fluorine, chlorine, bromine, or iodine.

As used herein, "haloalkyl" refers to the alkyl as defined above with the specified number of carbon atoms being substituted with one or more halogen atoms, up to the maximum allowable number of halogen atoms. Examples of haloalkyl include, but not limited to, trifluoromethyl, difluoromethyl, 2-fluoroethyl, and pentafluoroethyl.

"Heterocyclyl" refers to a stable 3- to 18-membered non-aromatic ring radical that comprises 2-12 carbon atoms and 1-6 heteroatoms selected from nitrogen, oxygen or sulfur. Unless stated otherwise in the description, heterocyclyl is a monocyclic, bicyclic, tricyclic, or tetracyclic ring system, which may include fused or bridged ring systems. The heteroatoms in the heterocyclyl may be optionally oxidized. One or more nitrogen atoms, if present, are optionally quaternized. The heterocyclyl is partially or fully saturated. The heterocyclyl may be linked to the rest of the molecule through any atom of the ring. Examples of such heterocyclyl include, but not limited to, dioxanyl, thienyl[1,3]disulfonyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl.

"Heteroaryl" refers to a group derived from a 3- to 18-membered aromatic ring radical that comprises 2 to 17 carbon atoms and 1 to 6 heteroatoms selected from nitrogen, oxygen and sulfur. As used herein, the heteroaryl may be a monocyclic, bicyclic, tricyclic or tetracyclic ring system, wherein at least one ring in the ring system is fully unsaturated, i.e., containing a cyclic, delocalized $(4n+2)\pi$-electron system in accordance with the Hückel theory. The heteroaryl includes fused or bridged ring systems. The heteroatoms in the heteroaryl are optionally oxidized. One or more nitrogen atoms, if present, are optionally quaternized. The heteroaryl may be linked to the rest of the molecule through any atom of the ring. Examples of such heteroaryl include, but not limited to, azepinyl, acridinyl, benzimidazolyl, benzindolyl, 1,3-benzodioxazolyl, benzofuranyl, benzoxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10-hexahydrocycloocta[d] pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1H-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl, and thiophenyl/thienyl.

Various hydroxy protecting groups may be used in the present disclosure. In general, protecting groups render chemical functional groups inert to specific reaction conditions, and may be attached to and removed from such functional groups in a molecule without substantially damaging the remainder of the molecule. Representative hydroxy protecting groups are disclosed in Tetrahedron 1992, 48, 2223-2311 written by Beaucage, et al., and also in Greene and Wuts, Protective Groups in Organic Synthesis, Chapter 2, 2d ed, John Wiley & Sons, New York, 1991, each of which is hereby incorporated by reference in their entirety. In some embodiments, the protecting group is stable under basic conditions but can be removed under acidic conditions. In some embodiments, non-exclusive examples of the hydroxy protecting groups used herein include dimethoxytrityl (DMT), monomethoxytrityl, 9-phenylxanthen-9-yl (Pixyl), or 9-(p-methoxyphenyl)xanthen-9-yl (Mox). In some embodiments, non-exclusive examples of the hydroxy protecting groups used herein include Tr(trityl), MMTr(4-methoxytrityl), DMTr(4,4'-dimethoxytrityl), and TMTr(4,4',4"-trimethoxytrityl).

The term "subject", as used herein, refers to any animal, e.g., mammal or marsupial. The subject of the present disclosure includes, but not limited to, human, non-human primate (e.g., rhesus or other kinds of macaque), mouse, pig,

horse, donkey, cow, sheep, rat and any kind of poultry.

As used herein, "treatment" refers to a method for obtaining advantageous or desired result, including but not limited to, therapeutic benefit. "Therapeutic benefit" means eradication or improvement of potential disorder to be treated. Moreover, the therapeutic benefit is achieved by eradicating or ameliorating one or more of physiological symptoms associated with the potential disorder such that an improvement is observed in the subject, notwithstanding that the subject may still be afflicted with the potential disorder.

As used herein, "prevention" refers to a method for obtaining advantageous or desired result, including but not limited to, prophylactic benefit. For obtaining the "prophylactic benefit", the siRNA conjugate or composition may be administered to the subject at risk of developing a particular disease, or to the subject reporting one or more physiological symptoms of a disease, even though the diagnosis of this disease may not have been made.

siRNA

In one aspect, the present disclosure provides six types of siRNAs capable of inhibiting expression of a C5 gene.

The siRNA of the present disclosure comprises nucleotides as basic structural units. It is well-known to those skilled in the art that the nucleotide comprises a phosphate group, a ribose group and a base. Detailed illustrations relating to such groups are omitted herein.

The siRNA of the present disclosure comprises a sense strand and an antisense strand, wherein lengths of the sense strand and the antisense strand are the same or different, the length of the sense strand is 19-23 nucleotides, and the length of the antisense strand is 19-26 nucleotides. In this way, a length ratio of the sense strand to the antisense strand of the siRNA provided by the present disclosure may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, the length ratio of the sense strand to the antisense strand of the siRNA is 19/21, 21/23 or 23/25.

The first siRNA

According to the present disclosure, the siRNA may be the first siRNA.

The first siRNA comprises a sense strand and an antisense strand. Each nucleotide in the first siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length and no more than three nucleotide differences from the nucleotide sequence shown in SEQ ID NO: 1; and the nucleotide sequence II has the same length and no more than three nucleotide differences from the nucleotide sequence shown in SEQ ID NO: 2:

5'-CUUCAUUCAUACAGACAAZi-3' (SEQ ID NO: 1);

5'-$Z_2$UUGUCUGUAUGAAUGAAG-3' (SEQ ID NO: 2),

wherein, $Z_1$ is A, $Z_2$ is U, the nucleotide sequence I comprises a nucleotide $Z_3$ at a corresponding site to $Z_1$, the nucleotide sequence II comprises a nucleotide $Z_4$ at a corresponding site to $Z_2$, and $Z_4$ is the first nucleotide from the 5' terminal of the antisense strand .

In this context, the term "corresponding site" means being at the same site in the nucleotide sequence by counting from the same terminal of the nucleotide sequence. For example, the first nucleotide at the 3' terminal of the nucleotide sequence I is a nucleotide at the corresponding site to the first nucleotide at the 3' terminal of SEQ ID NO: 1.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 1, and/or the nucleotide sequence II has no more than one nucleotide difference from the nucleotide sequence shown in SEQ ID NO: 2.

In some embodiments, the nucleotide difference between the nucleotide sequence II and the nucleotide sequence shown in SEQ ID NO: 2 comprises a difference at the site of $Z_4$, and $Z_4$ is selected from A, C or G. In some embodiments, the nucleotide difference is a difference at the site of $Z_4$, and $Z_4$ is selected from A, C or G. In some embodiments, $Z_3$ is a nucleotide complementary to $Z_4$. The siRNAs having the above nucleotide difference has higher ability of the siRNAs to inhibit the target mRNA, and these siRNAs are also within the scope of the present disclosure.

In some embodiments, the nucleotide sequence I is basically reverse complementary, substantially reverse complementary, or completely reverse complementary to the nucleotide sequence II. The basically reverse complementary refers to no more than three base mispairings between two nucleotide sequences; the substantially reverse complementary refers to no more than one base mispairing between two nucleotide sequences; and the completely reverse complementary refers to no base mispairing between two nucleotide sequences.

In some embodiments, the nucleotide sequence I is the nucleotide sequence shown in SEQ ID NO: 3, and the nucleotide sequence II is the nucleotide sequence shown in SEQ ID NO: 4:

5'-CUUCAUUCAUACAGACAA$Z_3$-3' (SEQ ID NO: 3);

5'-$Z_4$UUGUCUGUAUGAAUGAAG-3' (SEQ ID NO: 4),

wherein, $Z_4$ is the first nucleotide from 5' terminal of the antisense strand; $Z_3$ is selected from A, U, G or C; and $Z_4$ is a nucleotide complementary to $Z_3$; and in some embodiments, $Z_3$ is A, and $Z_4$ is U.

In some embodiments, the sense strand further comprises a nucleotide sequence III, the antisense strand further comprises a nucleotide sequence IV, and the nucleotide sequence III and the nucleotide sequence IV each independently has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. In some embodiments, the nucleotide sequence IV is substantially reverse complementary or completely reverse complementary to the nucleotide sequence II, and the nucleotide sequence II refers to the nucleotide sequence adjacent to the 5' terminal of the nucleotide sequence represented by SEQ ID NO: 1 in the target mRNA and having the same length as the nucleotide sequence IV.

In some embodiments, the nucleotide sequence III and the nucleotide sequence IV both have a length of one nucleotide. The base of the nucleotide sequence III is U, and the base of the nucleotide sequence IV is A; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is CU, and the base composition of the nucleotide sequence IV is AG; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is UCU, and the base composition of the nucleotide sequence IV is AGA; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is UUCU, and the base composition of the nucleotide sequence IV is AGAA; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is CU, and the base composition of the nucleotide sequence IV is AG; in this case, the length ratio of the sense strand to the antisense strand is 21/21.

In some embodiments, the nucleotide sequence III is completely reverse complementary to the nucleotide sequence IV. Thus, if the base(s) of the nucleotide sequence III is provided, the base(s) of the nucleotide sequence IV is also determined.

The second siRNA

According to the present disclosure, the siRNA may be the second siRNA.

The second siRNA comprises a sense strand and an antisense strand. Each nucleotide in the second siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length and no more than three nucleotide differences from the nucleotide sequence shown in SEQ ID NO: 61; and the nucleotide sequence II has the same length and no more than three nucleotide differences from the nucleotide sequence shown in SEQ ID NO: 62:

5'-CUACAGUUUAGAAGAUUU$Z_5$-3' (SEQ ID NO: 61);

5'-$Z_6$AAAUCUUCUAAACUGUAG-3' (SEQ ID NO: 62),

wherein, Zs is A, $Z_6$ is U, the nucleotide sequence I comprises a nucleotide $Z_7$ at a corresponding site to $Z_5$, the nucleotide sequence II comprises a nucleotide $Z_8$ at a corresponding site to $Z_6$, and $Z_8$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 61, and/or the nucleotide sequence II has no more than one nucleotide difference from the nucleotide sequence shown in SEQ ID NO: 62.

In some embodiments, the nucleotide difference between the nucleotide sequence II and the nucleotide sequence shown in SEQ ID NO: 62 comprises a difference at the site of $Z_8$, and $Z_8$ is selected from A, C or G. In some embodiments, the nucleotide difference is a difference at the site of $Z_8$, and $Z_8$ is selected from A, C or G. In some embodiments, $Z_7$ is a nucleotide complementary to $Z_8$. The siRNAs having the above nucleotide difference has higher ability of the siRNAs to inhibit the target mRNA, and these siRNAs are also within the scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 63, and the nucleotide sequence II is the nucleotide sequence shown in SEQ ID NO: 64:

5'-CUACAGUUUAGAAGAUUU$Z_7$-3' (SEQ ID NO: 63);

5'-$Z_8$AAAUCUUCUAAACUGUAG-3' (SEQ ID NO: 64),

wherein, $Z_8$ is the first nucleotide from 5' terminal of the antisense strand; $Z_7$ is selected from A, U, G or C; and $Z_8$ is a nucleotide complementary to $Z_7$; and in some embodiments, $Z_7$ is A, and $Z_8$ is U.

In some embodiments, the sense strand further comprises a nucleotide sequence III, the antisense strand further comprises a nucleotide sequence IV, and the nucleotide sequence III and the nucleotide sequence IV each independently has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the nucleotide sequence II, and the nucleotide sequence II refers to the nucleotide sequence adjacent to the 5' terminal of the nucleotide sequence represented by SEQ ID NO: 61 in the target mRNA and having the same length as the nucleotide sequence IV.

In some embodiments, the nucleotide sequence III and the nucleotide sequence IV both have a length of one nucleotide. The base of the nucleotide sequence III is A, and the base of the nucleotide sequence IV is U; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is UA, and the base composition of the nucleotide sequence IV is UA; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is AUA, and the base composition of the nucleotide sequence IV is UAU; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is CAUA, and the base composition of the nucleotide sequence IV is UAUG; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is UA, and the base composition of the nucleotide sequence IV is UA; in this case, the length ratio of the sense strand to the antisense strand is 21/21.

In some embodiments, the nucleotide sequence III is completely reverse complementary to the nucleotide sequence IV. Thus, if the base(s) of the nucleotide sequence III is provided, the base(s) of the nucleotide sequence IV is also determined.

<u>The third siRNA</u>

According to the present disclosure, the siRNA may be the third siRNA.

The third siRNA comprises a sense strand and an antisense strand. Each nucleotide in the third siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length and no more than three nucleotide differences from the nucleotide sequence shown in SEQ ID NO: 121; and the nucleotide sequence II has the same length and no more than three nucleotide differences from the nucleotide

sequence shown in SEQ ID NO: 122:

5'-GGAAGGUUACCGAGCAAU$Z_9$-3' (SEQ ID NO: 121);

5'-$Z_{10}$AUUGCUCGGUAACCUUCC-3' (SEQ ID NO: 122),

wherein, $Z_9$ is A, $Z_{10}$ is U, the nucleotide sequence I comprises a nucleotide $Z_{11}$ at a corresponding site to $Z_9$, the nucleotide sequence II comprises a nucleotide $Z_{12}$ at a corresponding site to $Z_{10}$, and $Z_{12}$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 121, and/or the nucleotide sequence II has no more than one nucleotide difference from the nucleotide sequence shown in SEQ ID NO: 122.

In some embodiments, the nucleotide difference between the nucleotide sequence II and the nucleotide sequence shown in SEQ ID NO: 122 comprises a difference at the site of $Z_{12}$, and $Z_{12}$ is selected from A, C or G. In some embodiments, the nucleotide difference is a difference at the site of $Z_{12}$, and $Z_{12}$ is selected from A, C or G. In some embodiments, $Z_{11}$ is a nucleotide complementary to $Z_{12}$. The siRNAs having the above nucleotide difference has higher ability of the siRNAs to inhibit the target mRNA, and these siRNAs are also within the scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 123, and the nucleotide sequence II is the nucleotide sequence shown in SEQ ID NO: 124:

5'-GGAAGGUUACCGAGCAAUZn-3' (SEQ ID NO: 123);

5'-$Z_{12}$AUUGCUCGGUAACCUUCC-3' (SEQ ID NO: 124),

wherein, $Z_{12}$ is the first nucleotide from 5' terminal of the antisense strand; $Z_{11}$ is selected from A, U, G or C; and $Z_{12}$ is a nucleotide complementary to $Z_{11}$; and in some embodiments, $Z_{11}$ is A, and $Z_{12}$ is U.

In some embodiments, the sense strand further comprises a nucleotide sequence III, the antisense strand further comprises a nucleotide sequence IV, and the nucleotide sequence III and the nucleotide sequence IV each independently has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the nucleotide sequence II, and the nucleotide sequence II refers to the nucleotide sequence adjacent to the 5' terminal of the nucleotide sequence represented by SEQ ID NO: 121 in the target mRNA and having the same length as the nucleotide sequence IV.

In some embodiments, the nucleotide sequence III and the nucleotide sequence IV both have a length of one nucleotide. The base of the nucleotide sequence III is G, and the base of the nucleotide sequence IV is C; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is AG, and the base composition of the nucleotide sequence IV is CU; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is CAG, and the base composition of the nucleotide sequence IV is CUG; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is CCAG, and the base composition of the nucleotide sequence IV is CUGG; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is AG, and the base composition of the nucleotide sequence IV is CU; in this case, the length ratio of the sense strand to the antisense strand is 21/21.

In some embodiments, the nucleotide sequence III is completely reverse complementary to the nucleotide sequence IV. Thus, if the base(s) of the nucleotide sequence III is provided, the base(s) of the nucleotide sequence IV is also determined.

The fourth siRNA

According to the present disclosure, the siRNA may be the fourth siRNA.

The fourth siRNA comprises a sense strand and an antisense strand. Each nucleotide in the fourth siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length and no more than three nucleotide differences from the nucleotide sequence shown in SEQ ID NO: 181; and the nucleotide sequence II has the same length and no more than three nucleotide differences from the nucleotide sequence shown in SEQ ID NO: 182:

5'-AGAACAGACAGCAGAAUU$Z_{13}$-3' (SEQ ID NO: 181);

5'-$Z_{14}$AAUUCUGCUGUCUGUUCU-3' (SEQ ID NO: 182),

wherein, $Z_{13}$ is A, $Z_{14}$ is U, the nucleotide sequence I comprises a nucleotide $Z_{15}$ at a corresponding site to $Z_{13}$, the nucleotide sequence II comprises a nucleotide $Z_{16}$ at a corresponding site to $Z_{14}$, and $Z_{16}$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 181, and/or the nucleotide sequence II has no more than one nucleotide difference from the nucleotide sequence shown in SEQ ID NO: 182.

In some embodiments, the nucleotide difference between the nucleotide sequence II and the nucleotide sequence shown in SEQ ID NO: 182 comprises a difference at the site of $Z_{16}$, and $Z_{16}$ is selected from A, C or G. In some embodiments, the nucleotide difference is a difference at the site of $Z_{16}$, and $Z_{16}$ is selected from A, C or G. In some embodiments, $Z_{15}$ is a nucleotide complementary to $Z_{16}$. The siRNAs having the above nucleotide difference has higher ability of the siRNAs to inhibit the target mRNA, and these siRNAs are also within the scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 183, and the nucleotide sequence II is the nucleotide sequence shown in SEQ ID NO: 184:

5'-AGAACAGACAGCAGAAUU$Z_{15}$-3' (SEQ ID NO: 183);

5'-$Z_{16}$AAUUCUGCUGUCUGUUCU-3' (SEQ ID NO: 184),

wherein, $Z_{16}$ is the first nucleotide from 5' terminal of the antisense strand; $Z_{15}$ is selected from A, U, G or C; and $Z_{16}$ is a nucleotide complementary to $Z_{15}$; and in some embodiments, $Z_{15}$ is A, and $Z_{16}$ is U.

In some embodiments, the sense strand further comprises a nucleotide sequence III, the antisense strand further comprises a nucleotide sequence IV, and the nucleotide sequence III and the nucleotide sequence IV each independently has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the nucleotide sequence II, and the nucleotide sequence II refers to the nucleotide sequence adjacent to the 5' terminal of the nucleotide sequence represented by SEQ ID NO: 181 in the target mRNA and having the same length as the nucleotide sequence IV.

In some embodiments, the nucleotide sequence III and the nucleotide sequence IV both have a length of one nucleotide. The base of the nucleotide sequence III is G, and the base of the nucleotide sequence IV is C; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is GG, and the base composition of the nucleotide sequence IV is CC; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is AGG, and the base composition of the nucleotide sequence IV is CCU; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence

III is CAGG, and the base composition of the nucleotide sequence IV is CCUG; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is GG, and the base composition of the nucleotide sequence IV is CC; in this case, the length ratio of the sense strand to the antisense strand is 21/21.

In some embodiments, the nucleotide sequence III is completely reverse complementary to the nucleotide sequence IV. Thus, if the base(s) of the nucleotide sequence III is provided, the base(s) of the nucleotide sequence IV is also determined.

## The fifth siRNA

According to the present disclosure, the siRNA may be the fifth siRNA.

The fifth siRNA comprises a sense strand and an antisense strand. Each nucleotide in the fifth siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length and no more than three nucleotide differences from the nucleotide sequence shown in SEQ ID NO: 241; and the nucleotide sequence II has the same length and no more than three nucleotide differences from the nucleotide sequence shown in SEQ ID NO: 242:

5'-CCAAGAAGAACGCUGCAA$Z_{17}$-3' (SEQ ID NO: 241);
5'-$Z_{18}$UUGCAGCGUUCUUCUUGG-3' (SEQ ID NO: 242),

wherein, $Z_{17}$ is A, $Z_{18}$ is U, the nucleotide sequence I comprises a nucleotide $Z_{19}$ at a corresponding site to $Z_{17}$, the nucleotide sequence II comprises a nucleotide $Z_{20}$ at a corresponding site to $Z_{18}$, and $Z_{20}$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 241, and/or the nucleotide sequence II has no more than one nucleotide difference from the nucleotide sequence shown in SEQ ID NO: 242.

In some embodiments, the nucleotide difference between the nucleotide sequence II and the nucleotide sequence shown in SEQ ID NO: 242 comprises a difference at the site of $Z_{20}$, and $Z_{20}$ is selected from A, C or G. In some embodiments, the nucleotide difference is a difference at the site of $Z_{20}$, and $Z_{20}$ is selected from A, C or G. In some embodiments, $Z_{19}$ is a nucleotide complementary to $Z_{20}$. The siRNAs having the above nucleotide difference has higher ability of the siRNAs to inhibit the target mRNA, and these siRNAs are also within the scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 243, and the nucleotide sequence II is the nucleotide sequence shown in SEQ ID NO: 244:

5'-CCAAGAAGAACGCUGCAA$Z_{19}$-3' (SEQ ID NO: 243);

5'-$Z_{20}$UUGCAGCGUUCUUCUUGG-3' (SEQ ID NO: 244),

wherein, $Z_{20}$ is the first nucleotide from 5' terminal of the antisense strand; $Z_{19}$ is selected from A, U, G or C; and $Z_{20}$ is a nucleotide complementary to $Z_{19}$; and in some embodiments, $Z_{19}$ is A, and $Z_{20}$ is U.

In some embodiments, the sense strand further comprises a nucleotide sequence III, the antisense strand further comprises a nucleotide sequence IV, and the nucleotide sequence III and the nucleotide sequence IV each independently has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the nucleotide sequence II, and the nucleotide sequence II refers to the nucleotide sequence adjacent to the 5' terminal of the nucleotide sequence represented by SEQ ID NO: 241 in the target mRNA and having the same length as the nucleotide sequence IV.

In some embodiments, the nucleotide sequence III and the nucleotide sequence IV both have a length of one nucleotide. The base of the nucleotide sequence III is G, and the base of the nucleotide sequence IV is C; in this case,

the length ratio of the sense strand to the antisense strand is 20/20; or, the nucleotide sequences III and IV both have a length of two nucleotides, and in the direction from the 5' terminal to the 3' terminal, the base composition of the nucleotide sequence III is GG, and the base composition of the nucleotide sequence IV is CC; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is AGG, and the base composition of the nucleotide sequence IV is CCU; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is CAGG, and the base composition of the nucleotide sequence IV is CCUG; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is GG, and the base composition of the nucleotide sequence IV is CC; in this case, the length ratio of the sense strand to the antisense strand is 21/21.

In some embodiments, the nucleotide sequence III is completely reverse complementary to the nucleotide sequence IV. Thus, if the base(s) of the nucleotide sequence III is provided, the base(s) of the nucleotide sequence IV is also determined.

The sixth siRNA

According to the present disclosure, the siRNA may be the sixth siRNA.

The sixth siRNA comprises a sense strand and an antisense strand. Each nucleotide in the sixth siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length and no more than three nucleotide differences from the nucleotide sequence shown in SEQ ID NO: 301; and the nucleotide sequence II has the same length and no more than three nucleotide differences from the nucleotide sequence shown in SEQ ID NO: 302:

5'-CCAGUAAGCAAGCCAGAA$Z_{21}$-3' (SEQ ID NO: 301);

5'-$Z_{22}$UUCUGGCUUGCUUACUGG-3' (SEQ ID NO: 302),

wherein, $Z_{21}$ is A, $Z_{22}$ is U, the nucleotide sequence I comprises a nucleotide $Z_{23}$ at a corresponding site to $Z_{21}$, the nucleotide sequence II comprises a nucleotide $Z_{24}$ at a corresponding site to $Z_{22}$, and $Z_{24}$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 301, and/or the nucleotide sequence II has no more than one nucleotide difference from the nucleotide sequence shown in SEQ ID NO: 302.

In some embodiments, the nucleotide difference between the nucleotide sequence II and the nucleotide sequence shown in SEQ ID NO: 302 comprises a difference at the site of $Z_{24}$, and $Z_{24}$ is selected from A, C or G. In some embodiments, the nucleotide difference is a difference at the site of $Z_{24}$, and $Z_{24}$ is selected from A, C or G. In some embodiments, $Z_{23}$ is a nucleotide complementary to $Z_{24}$. The siRNAs having the above nucleotide difference has higher ability of the siRNAs to inhibit the target mRNA, and these siRNAs are also within the scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 303, and the nucleotide sequence II is the nucleotide sequence shown in SEQ ID NO: 304:

5'-CCAGUAAGCAAGCCAGAA$Z_{23}$-3' (SEQ ID NO: 303);

5'-$Z_{24}$UUCUGGCUUGCUUACUGG-3' (SEQ ID NO: 304),

wherein, $Z_{24}$ is the first nucleotide from 5' terminal of the antisense strand; $Z_{23}$ is selected from A, U, G or C; and $Z_{24}$ is a nucleotide complementary to $Z_{23}$; and in some embodiments, $Z_{23}$ is A, and $Z_{24}$ is U.

In some embodiments, the sense strand further comprises a nucleotide sequence III, the antisense strand further comprises a nucleotide sequence IV, and the nucleotide sequence III and the nucleotide sequence IV each

independently has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the nucleotide sequence II, and the nucleotide sequence II refers to the nucleotide sequence adjacent to the 5' terminal of the nucleotide sequence represented by SEQ ID NO: 301 in the target mRNA and having the same length as the nucleotide sequence IV.

In some embodiments, the nucleotide sequence III and the nucleotide sequence IV both have a length of one nucleotide. The base of the nucleotide sequence III is A, and the base of the nucleotide sequence IV is U; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is UA, and the base composition of the nucleotide sequence IV is UA; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is UUA, and the base composition of the nucleotide sequence IV is UAA; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is GUUA, and the base composition of the nucleotide sequence IV is UAAC; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is UA, and the base composition of the nucleotide sequence IV is UA; in this case, the length ratio of the sense strand to the antisense strand is 21/21.

In some embodiments, the nucleotide sequence III is completely reverse complementary to the nucleotide sequence IV. Thus, if the base(s) of the nucleotide sequence III is provided, the base(s) of the nucleotide sequence IV is also determined.

Overhang and modification of the siRNA

The following description of the nucleotide sequence V, the nucleic acid sequence, the nucleotide modification in the siRNA and the modified sequence is applicable to any one of the first siRNA to the sixth siRNA. That is, unless otherwise specified, the following description of the siRNA should be regarded as describing the first siRNA, the second siRNA, the third siRNA, the fourth siRNA, the fifth siRNA, and the sixth siRNA one by one. For example, if no specific siRNA is specified, "the siRNA further comprises a nucleotide sequence V" means "the first siRNA, the second siRNA, the third siRNA, the fourth siRNA, the fifth siRNA, or the sixth siRNA further comprises a nucleotide sequence V".

In some embodiments, the sense strand and the antisense strand have different lengths. The nucleotide sequence II further comprises a nucleotide sequence V, which has a length of 1-3 nucleotides and is linked to the 3' terminal of the antisense strand, thereby constituting a 3' overhang of the antisense strand. As such, the length ratio of the sense strand to the antisense strand in the siRNA of the present disclosure may be 19/20, 19/21, 19/22, 20/21, 20/22, 20/23, 21/22, 21/23, 21/24, 22/23, 22/24, 22/25, 23/24, 23/25, or 23/26. In some embodiments, the nucleotide sequence V has a length of 2 nucleotides. As such, the length ratio of the sense strand to the antisense strand in the siRNA of the present disclosure may be 19/21, 21/23 or 23/25.

Each nucleotide in the nucleotide sequence V may be any nucleotide. In order to facilitate synthesis and save synthesis cost, the nucleotide sequence V is 2 continuous thymidine deoxyribonucleotides (dTdT) or 2 continuous uracil ribonucleotides (UU); or, in order to improve the affinity of the antisense strand of the siRNA to the target mRNA, the nucleotide sequence V is complementary to the nucleotides at the corresponding site of the target mRNA. Therefore, in some embodiments, the length ratio of the sense strand to the antisense strand of the siRNA of the present disclosure is 19/21 or 21/23. In this case, the siRNA of the present disclosure has better silencing activity against mRNA.

The nucleotide at the corresponding site of the target mRNA refers to a nucleotide or nucleotide sequence adjacent to the nucleotide sequence III of the target mRNA at the 5' terminal. The nucleotide sequence III is substantially reverse complementary or completely reverse complementary to the nucleotide sequence II, or, is a nucleotide sequence which is substantially reverse complementary or completely reverse complementary to the nucleotide sequence formed by the nucleotide sequence II and the nucleotide sequence IV.

In some embodiments, for the first siRNA, the sense strand of the siRNA comprises the nucleotide sequence shown in SEQ ID NO: 5, and the antisense strand comprises the nucleotide sequence shown in SEQ ID NO: 6;

5'-CUUCAUUCAUACAGACAA$Z_3$-3' (SEQ ID NO: 5);

5'-$Z_4$UUGUCUGUAUGAAUGAAGAG-3' (SEQ ID NO: 6);

or, the sense strand of the siRNA comprises the nucleotide sequence shown in SEQ ID NO: 7, and the antisense strand of the siRNA comprises the nucleotide sequence shown in SEQ ID NO: 8;

5'-CUCUUCAUUCAUACAGACAA$Z_3$-3' (SEQ ID NO: 7);

5'-$Z_4$UUGUCUGUAUGAAUGAAGAGAA-3' (SEQ ID NO: 8);

wherein, $Z_4$ is the first nucleotide from 5' terminal of the antisense strand; $Z_3$ is selected from A, U, G or C; and $Z_4$ is a nucleotide complementary to $Z_3$.

In some embodiments, for the siRNA, the sense strand of the siRNA comprises the nucleotide sequence shown in SEQ ID NO: 65, and the antisense strand comprises the nucleotide sequence shown in SEQ ID NO: 66:

5'-CUACAGUUUAGAAGAUUU$Z_7$-3' (SEQ ID NO: 65);

5'-$Z_8$AAAUCUUCUAAACUGUAGUA-3' (SEQ ID NO: 66),

or, the sense strand of the siRNA comprises the nucleotide sequence shown in SEQ ID NO: 67, and the antisense strand of the siRNA comprises the nucleotide sequence shown in SEQ ID NO: 68:

5'-UACUACAGUUUAGAAGAUUU$Z_7$-3' (SEQ ID NO: 67);

5'-$Z_8$AAAUCUUCUAAACUGUAGUAUG-3' (SEQ ID NO: 68),

wherein, $Z_8$ is the first nucleotide from 5' terminal of the antisense strand; $Z_7$ is selected from A, U, G or C; and Zs is a nucleotide complementary to $Z_7$.

In some embodiments, for the third siRNA, the sense strand of the siRNA comprises the nucleotide sequence shown in SEQ ID NO: 125, and the antisense strand of the siRNA comprises the nucleotide sequence shown in SEQ ID NO: 126:

5'-GGAAGGUUACCGAGCAAU$Z_{11}$-3' (SEQ ID NO: 125);

5'-$Z_{12}$AUUGCUCGGUAACCUUCCCU-3' (SEQ ID NO: 126),

or, the sense strand of the siRNA comprises the nucleotide sequence shown in SEQ ID NO: 127, and the antisense strand of the siRNA comprises the nucleotide sequence shown in SEQ ID NO: 128:

5'-AGGGAAGGUUACCGAGCAAU$Z_{11}$-3' (SEQ ID NO: 127);

5'-$Z_{12}$AUUGCUCGGUAACCUUCCCUGG-3' (SEQ ID NO: 128),

wherein, $Z_{12}$ is the first nucleotide from 5' terminal of the antisense strand; $Z_{11}$ is selected from A, U, G or C; and $Z_{12}$ is a nucleotide complementary to $Z_{11}$.

In some embodiments, for the fourth siRNA, the sense strand of the siRNA comprises the nucleotide sequence shown in SEQ ID NO: 185, and the antisense strand of the siRNA comprises the nucleotide sequence shown in SEQ ID NO: 186:

5'-AGAACAGACAGCAGAAUU$Z_{15}$-3' (SEQ ID NO: 185);

5'-$Z_{16}$AAUUCUGCUGUCUGUUCUCC-3' (SEQ ID NO: 186),

or, the sense strand of the siRNA comprises the nucleotide sequence shown in SEQ ID NO: 187, and the antisense strand of the siRNA comprises the nucleotide sequence shown in SEQ ID NO: 188:

5'-GGAGAACAGACAGCAGAAUU$Z_{15}$-3' (SEQ ID NO: 187);

5'-$Z_{16}$AAUUCUGCUGUCUGUUCUCCUG-3' (SEQ ID NO: 188),

wherein, $Z_{16}$ is the first nucleotide from 5' terminal of the antisense strand; $Z_{15}$ is selected from A, U, G or C; and $Z_{16}$ is

a nucleotide complementary to $Z_{15}$.

In some embodiments, for the fifth siRNA, the sense strand of the siRNA comprises the nucleotide sequence shown in SEQ ID NO: 245, and the antisense strand of the siRNA comprises the nucleotide sequence shown in SEQ ID NO: 246:

5'-CCAAGAAGAACGCUGCAA$Z_{19}$-3' (SEQ ID NO: 245);

5'-$Z_{20}$UUGCAGCGUUCUUCUUGGCC-3' (SEQ ID NO: 246),

or, the sense strand of the siRNA comprises the nucleotide sequence shown in SEQ ID NO: 247, and the antisense strand of the siRNA comprises the nucleotide sequence shown in SEQ ID NO: 248:

5'-GGCCAAGAAGAACGCUGCAA$Z_{19}$-3' (SEQ ID NO: 247);

5'-$Z_{20}$UUGCAGCGUUCUUCUUGGCCUG-3' (SEQ ID NO: 248),

wherein, $Z_{20}$ is the first nucleotide from 5' terminal of the antisense strand; $Z_{19}$ is selected from A, U, G or C; and $Z_{20}$ is a nucleotide complementary to $Z_{19}$.

In some embodiments, for the sixth siRNA, the sense strand of the siRNA comprises the nucleotide sequence shown in SEQ ID NO: 305, and the antisense strand of the siRNA comprises the nucleotide sequence shown in SEQ ID NO: 306:

5'-CCAGUAAGCAAGCCAGAA$Z_{23}$-3' (SEQ ID NO: 305);

5'-$Z_{24}$UUCUGGCUUGCUUACUGGUA-3' (SEQ ID NO: 306),

or, the sense strand of the siRNA comprises the nucleotide sequence shown in SEQ ID NO: 307, and the antisense strand of the siRNA comprises the nucleotide sequence shown in SEQ ID NO: 308:

5'-UACCAGUAAGCAAGCCAGAA$Z_{23}$-3' (SEQ ID NO: 307);

5'-$Z_{24}$UUCUGGCUUGCUUACUGGUAAC-3' (SEQ ID NO: 308),

wherein, $Z_{24}$ is the first nucleotide from 5' terminal of the antisense strand; $Z_{23}$ is selected from A, U, G or C; and $Z_{24}$ is a nucleotide complementary to $Z_{23}$.

In some embodiments, the siRNA of the present disclosure is siC5a1, siC5a2, siC5b1, siC5b2, siC5c1, siC5c2, siC5d1, siC5d2, siC5e1, siC5e2, siC5f1 or siC5f2 listed in Tables 1a-1f.

As described above, the nucleotides in the siRNA of the present disclosure are each independently modified or unmodified nucleotides. In some embodiments, the nucleotides in the siRNA of the present disclosure are unmodified nucleotides. In some embodiments, some or all nucleotides in the siRNA of the present disclosure are modified nucleotides. Such modifications on the nucleotides would not cause significant decrease or loss of the function of the siRNA of the present disclosure to inhibit the expression of C5 genes.

In some embodiments, the siRNA of the present disclosure comprises at least one modified nucleotide. In the context of the present disclosure, the term "modified nucleotide" employed herein refers to a nucleotide formed by substituting the 2'-hydroxy of the ribose group of a nucleotide with other groups, a nucleotide analogue, or a nucleotide with modified base. Such modified nucleotides would not cause significant decrease or loss of the function of the siRNA to inhibit the expression of genes. For example, the modified nucleotides disclosed in Chemically Modified siRNA: tools and applications. Drug Discov Today, 2008.13(19-20): 842-55 written by J.K. Watts, G. F. Deleavey and M. J.Damha may be selected.

In some embodiments, at least one nucleotide in the sense strand or the antisense strand of the siRNA provided by the present disclosure is a modified nucleotide, and/or at least one phosphate is a phosphate group with modified group. In other words, at least a portion of the phosphate group and/or ribose group in phosphate-ribose backbone of at least one single strand in the sense strand and the antisense strand are phosphate group with modified group and/or ribose group with modified group.

In some embodiments, all nucleotides in the sense strand and/or the antisense strand are modified nucleotides. In some embodiments, each nucleotide in the sense strand and the antisense strand of the siRNA provided by the present disclosure is independently a fluoro modified nucleotide or a non-fluoro modified nucleotide.

The inventors of the present disclosure have surprisingly found that the siRNA of the present disclosure has

achieved a high degree of balance between the stability in serum and the gene silencing efficiency in animal experiments.

In some embodiments, the fluoro modified nucleotides are located in the nucleotide sequence I and the nucleotide sequence II; and in the direction from 5' terminal to 3' terminal, the nucleotides at positions 7, 8 and 9 of the nucleotide sequence I are fluoro modified nucleotides; and in the direction from 5' terminal to 3' terminal, the nucleotides at positions 2, 6, 14 and 16 of the nucleotide sequence ii are fluoro modified nucleotides.

In some embodiments, the fluoro modified nucleotides are located in the nucleotide sequence I and the nucleotide sequence II; no more than 5 fluoro modified nucleotides are present in the nucleotide sequence I, and in the direction from 5' terminal to 3' terminal, the nucleotides at positions 7, 8 and 9 in the nucleotide sequence I are fluoro modified nucleotides; no more than 7 fluoro modified nucleotides are present in the nucleotide sequence II, and the nucleotides at positions 2, 6, 14 and 16 in the nucleotide sequence II are fluoro modified nucleotides.

In some embodiments, in the direction from 5' terminal to 3' terminal, the nucleotides at positions 7, 8 and 9 or 5, 7, 8 and 9 of the nucleotide sequence I in the sense strand are fluoro modified nucleotides, and the nucleotides at the rest of positions in the sense strand are non-fluoro modified nucleotides; and in the direction from 5' terminal to 3' terminal, the nucleotides at positions 2, 6, 14 and 16 or 2, 6, 8, 9, 14 and 16 of the nucleotide sequence II in the antisense strand are fluoro modified nucleotides, and the nucleotides at the rest of positions in the antisense strand are non-fluoro modified nucleotides.

In the context of the present disclosure, a "fluoro modified nucleotide" refers to a nucleotide which is formed by substituting the 2'-hydroxy of the ribose group of a nucleotide with fluoro, which has a structure as shown by Formula (7). A "non-fluoro modified nucleotide", refers to a nucleotide which is formed by substituting the 2'-hydroxy of the ribose group of a nucleotide with a non-fluoro group, or a nucleotide analogue. In some embodiments, each non-fluoro modified nucleotide is independently selected from a nucleotide formed by substituting the 2'-hydroxy of the ribose group of the nucleotide with the non-fluoro group, or the nucleotide analogue.

These nucleotides formed by substituting the 2'-hydroxy of the ribose group with the non fluoro group are well-known to those skilled in the art, and these nucleotides may be selected from one of a 2' alkoxy modified nucleotide, a 2'-substituted alkoxy modified nucleotide, a 2'-alkyl modified nucleotide, a 2'-substituted alkyl modified nucleotide, a 2'-amino modified nucleotide, a 2' substituted amino modified nucleotide and a 2'-deoxy nucleotide.

In some embodiments, the 2'-alkoxy modified nucleotide is a 2'-methoxy modified nucleotide (2'-OMe), as shown by Formula (8). In some embodiments, the 2'-substituted alkoxy modified nucleotide is, for example, a 2'-O-methoxyethoxy modified nucleotide (2' MOE) as shown by Formula (9). In some embodiments, the 2'-amino modified nucleotide (2'-$NH_2$) is as shown by Formula (10). In some embodiments, the 2'-deoxy nucleotide (DNA) is as shown by Formula (11):

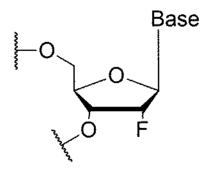 , 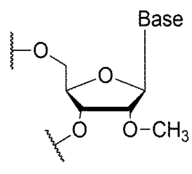 , 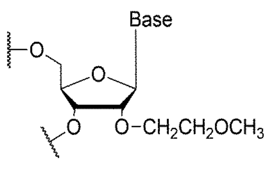 , 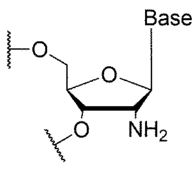 , 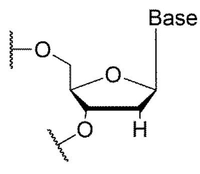

Formula (7) Formula (8) Formula (9) Formula (10) Formula (11).

The nucleotide analogue refers to a group that can replace a nucleotide in a nucleic acid, while structurally differs from an adenine ribonucleotide, a guanine ribonucleotide, a cytosine ribonucleotide, a uracil ribonucleotide or a thymidine deoxyribonucleotide. In some embodiments, the nucleotide analogue may be an isonucleotide, a bridged nucleic acid or an acyclic nucleotide.

The bridged nucleic acid (BNA) is a nucleotide that is constrained or is not accessible. The BNA may contain a 5-membered ring, 6-membered ring or 7-membered ring bridged structure with a "fixed" C3'-endo sugar puckering. The bridge is typically incorporated at the 2'- and 4'-position of the ribose to afford a 2',4'-BNA nucleotide. In some embodiments, the BNA may be an LNA, an ENA and a cET BNA, wherein the LNA is as shown by Formula (12), the ENA is as shown by Formula (13) and the cET BNA is as shown by Formula (14):

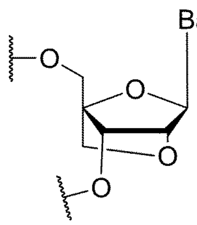 , 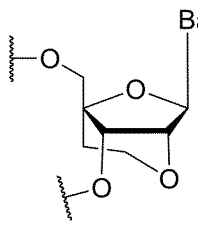 , 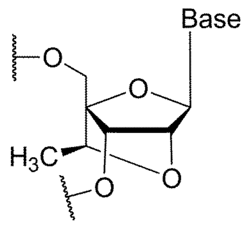

Formula (12) Formula (13) Formula (14).

An acyclic nucleotide is a nucleotide in which a ribose ring is opened. In some embodiments, the acyclic nucleotide may be an unlocked nucleic acid (UNA) or a glycerol nucleic acid (GNA), wherein the UNA is as shown by Formula (15), and the GNA is as shown by Formula (16):

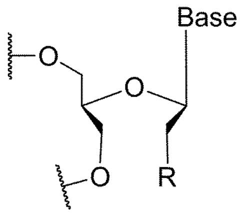 , 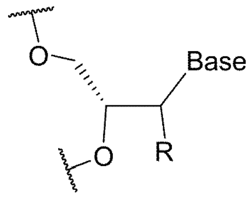

Formula (15) Formula (16).

In the Formula (15) and the Formula (16), R is selected from H, OH or alkoxy (O-alkyl).

An isonucleotide is a compound which is formed by that a nucleotide in which a position of a base on a ribose ring alters. In some embodiments, the isonucleotide may be a compound in which the base is transposed from position-1' to position-2' or position-3' on the ribose ring, as shown by Formula (17) or (18):

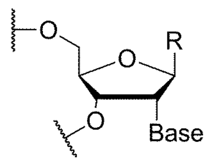 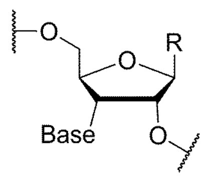

Formula (17) Formula (18).

In the compounds as shown by the Formula (17) and Formula (18) above, Base represents a base, such as A, U, G, C or T; and R is selected from H, OH, F or a non-fluoro group described above.

In some embodiments, the nucleotide analogue is selected from one of an isonucleotide, an LNA, an ENA, a cET, a UNA and a GNA. In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group.

In the context of the present disclosure, a "fluoro modified nucleotide", a "2'-fluoro modified nucleotide", a "nucleotide in which the 2'-hydroxy of the ribose group is substituted with fluoro" and a "2'-fluororibosyl" have the same meaning, referring to the compound formed by substituting the 2'-hydroxy of the ribose group of the nucleotide with fluoro, having a structure as shown by Formula (7). A "methoxy modified nucleotide", a "2'-methoxy modified nucleotide", a "nucleotide in which the 2'-hydroxy of the ribose group is substituted with methoxy" and a "2'-methoxyribosyl" have the same meaning, referring to the compound formed by substituting the 2'-hydroxy of the ribose group of the nucleotide with methoxy, having a structure as shown by Formula (8).

In some embodiments, the siRNA of the present disclosure is a siRNA with the following modifications: in the direction from 5' terminal to 3' terminal, the nucleotides at positions 7, 8 and 9 or 5, 7, 8 and 9 of the nucleotide sequence

I in the sense strand are fluoro modified nucleotides, and the nucleotides at the rest of positions in the sense strand are methoxy modified nucleotides; and the nucleotides at positions 2, 6, 14 and 16 or 2, 6, 8, 9, 14 and 16 of the nucleotide sequence II in the antisense strand are fluoro modified nucleotides, and the nucleotides at the rest of positions in the antisense strand are methoxy modified nucleotides.

In some embodiments, the siRNA of the present disclosure is a siRNA with the following modifications: in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 8, 9, 14 and 16 of the nucleotide sequence II in the antisense strand of the siRNA are fluoro modified nucleotides, and the nucleotides at the rest of positions in the antisense strand of the siRNA are methoxy modified nucleotides;

or, in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides;

or, in the direction from 5' terminal to 3' terminal, the nucleotides at positions 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides.

In some embodiments, the siRNA provided by the present disclosure is any one of siC5a1-M1, siC5a1-M2, siC5a1-M3, siC5a2-M1, siC5a2-M2, siC5a2-M3, siC5b1-M1, siC5b1-M2, siC5b1-M3, siC5b2-M1, siC5b2-M2, siC5b2-M3, siC5c1-M1, siC5c1-M2, siC5c1-M3, siC5c2-M1, siC5c2-M2, siC5c2-M3, siC5d1-M1, siC5d1-M2, siC5d1-M3, siC5d2-M1, siC5d2-M2, siC5d2-M3, siC5e1-M1, siC5e1-M2, siC5e1-M3, siC5e2-M1, siC5e2-M2, siC5e2-M3, siC5f1-M1, siC5f1-M2, siC5f1-M3, siC5f2-M1, siC5f2-M2 or siC5f2-M3 listed in Tables 1a-1f.

The siRNAs with the above modifications can not only be afforded at lower costs, but also allow the ribonucleases in the blood to be less liable to cleaving the nucleic acid so as to increase the stability of the nucleic acid and enable the nucleic acid to have stronger resistance against nuclease hydrolysis.

In some embodiments, at least a portion of the phosphate group in phosphate-ribose backbone of at least one single strand in the sense strand and the antisense strand of the siRNA provided by the present disclosure is a phosphate group with modified group. In some embodiments, the phosphate group with modified group is a phosphorothioate group formed by substituting at least one oxygen atom in a phosphodiester bond in the phosphate group with a sulfur atom; and in some embodiments, the phosphate group with modified group is a phosphorothioate group having a structure as shown by Formula (1):

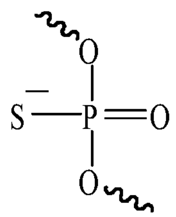

Formula (1).

This modification can stabilize the double-stranded structure of the siRNA, thereby maintaining high specificity and high affinity for base pairing.

In some embodiments, in the siRNA provided by the present disclosure, a phosphorothioate linkage exists in at least one of the following positions: the position between the first nucleotide and second nucleotides at either terminal of the sense strand or antisense strand; the position between the second and third nucleotides at either terminal of the sense strand or antisense strand; or any combination thereof. In some embodiments, a phosphorothioate linkage exists at all the above positions except for 5' terminal of the sense strand. In some embodiments, a phosphorothioate linkage exists at all the above positions except for 3' terminal of the sense strand. In some embodiments, a phosphorothioate

linkage exists in at least one of the following positions:

the position between the first nucleotide and the second nucleotide at 5' terminal of the sense strand;

the position between the second nucleotide and the third nucleotide at 5' terminal of the sense strand;

the position between the first nucleotide and the second nucleotide at 3' terminal of the sense strand;

the position between the second nucleotide and the third nucleotide at 3' terminal of the sense strand;

the position between the first nucleotide and the second nucleotide at 5' terminal of the antisense strand;

the position between the second nucleotide and the third nucleotide at 5' terminal of the antisense strand;

the position between the first nucleotide and the second nucleotide at 3' terminal of the antisense strand; and

the position between the second nucleotide and the third nucleotide at 3' terminal of the antisense strand.

In some embodiments, the siRNA provided by the present disclosure is any one of siC5a1-M1S, siC5a1-M2S, siC5a1-M3S, siC5a2-M1S, siC5a2-M2S, siC5a2-M3S, siC5b1-M1S, siC5b1-M2S, siC5b1-M3S, siC5b2-M1S, siC5b2-M2S, siC5b2-M3S, siC5c1-M1S, siC5c1-M2S, siC5c1-M3S, siC5c2-M1S, siC5c2-M2S, siC5c2-M3S, siC5d1-M1S, siC5d1-M2S, siC5d1-M3S, siC5d2-M1S, siC5d2-M2S, siC5d2-M3S, siC5e1-M1S, siC5e1-M2S, siC5e1-M3S, siC5e2-M1S, siC5e2-M2S, siC5e2-M3S, siC5f1-M1S, siC5f1-M2S, siC5f1-M3S, siC5f2-M1S, siC5f2-M2S or siC5f2-M3S listed in Tables 1a-1f.

In some embodiments, the 5'-terminal nucleotide in the antisense strand of the siRNA is a 5'-phosphate nucleotide or a 5'-phosphate analogue modified nucleotide.

Common types of the 5'-phosphate nucleotides or 5'-phosphate analogue modified nucleotides are well known to those skilled in the art, for example, the 5'-phosphate nucleotides may have the following structure:

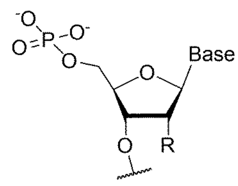
Formula (2);

For another example, The chemical evolution of oligonucleotide therapies of clinical utility. Nature Biotechnology, 2017, 35(3): 238-48 written by Anastasia Khvorova and Jonathan K. Watts, discloses the following four 5'-phosphate analogue modified nucleotides:

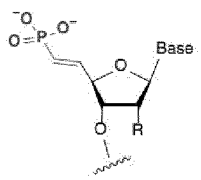 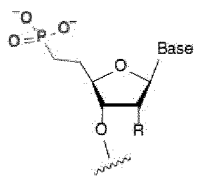 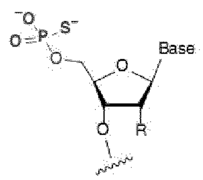 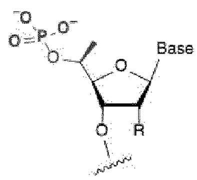

Formula (3) Formula (4) Formula (5) Formula (6)

wherein, R is selected from H, OH, methoxy or F; and Base represents a base selected from A, U, C, G, or T.

In some embodiments, the 5'-phosphate nucleotide is a nucleotide with 5'-phosphate modification as shown by Formula (2); the 5'-phosphate analogue modified nucleotide is a nucleotide with 5'-(*E*)-vinylphosphonate (*E*-VP) modification as shown by Formula (3) or a phosphorothioate modified nucleotide as shown by Formula (5).

In some embodiments, the siRNA provided by the present disclosure is any one of siC5a1-M1P1, siC5a1-M2P1, siC5a1-M3P1, siC5a2-M1P1, siC5a2-M2P1, siC5a2-M3P1, siC5b1-M1P1, siC5b1-M2P1, siC5b1-M3P1, siC5b2-M1P1, siC5b2-M2P1, siC5b2-M3P1, siC5c1-M1P1, siC5c1-M2P1, siC5c1-M3P1, siC5c2-M1P1, siC5c2-M2P1, siC5c2-M3P1, siC5d1-M1P1, siC5d1-M2P1, siC5d1-M3P1, siC5d2-M1P1, siC5d2-M2P1, siC5d2-M3P1, siC5e1-M1P1, siC5e1-M2P1, siC5e1-M3P1, siC5e2-M1P1, siC5e2-M2P1, siC5e2-M3P1, siC5f1-M1P1, siC5f1-M2P1, siC5f1-M3P1, siC5f2-M1P1, siC5f2-M2P1, siC5f2-M3P1, siC5a1-M1SP1, siC5a1-M2SP1, siC5a1-M3SP1, siC5a2-M1SP1, siC5a2-M2SP1, siC5a2-M3SP1, siC5b1-M1SP1, siC5b1-M2SP1, siC5b1-M3SP1, siC5b2-M1SP1, siC5b2-M2SP1, siC5b2-M3SP1, siC5c1-M1SP1, siC5c1-M2SP1, siC5c1-M3SP1, siC5c2-M1SP1, siC5c2-M2SP1, siC5c2-M3SP1, siC5d1-M1SP1, siC5d1-M2SP1, siC5d1-M3SP1, siC5d2-M1SP1, siC5d2-M2SP1, siC5d2-M3SP1, siC5e1-M1SP1, siC5e1-M2SP1, siC5e1-M3SP1, siC5e2-M1SP1, siC5e2-M2SP1, siC5e2-M3SP1, siC5f1-M1SP1, siC5f1-M2SP1, siC5f1-M3SP1, siC5f2-M1SP1, siC5f2-M2SP1 or siC5f2-M3SP1 listed in Tables 1a-1f.

The inventors of the present disclosure have surprisingly found that the siRNA provided by the present disclosure has significantly enhanced plasma and lysosomal stability, and has higher inhibitory activity of target mRNA.

The siRNA provided by the present disclosure can be obtained by conventional methods for preparing siRNAs in the art (e.g., solid phase synthesis and liquid phase synthesis methods). Commercial customization services have already been available for solid phase synthesis. Modified nucleotides can be introduced into the siRNAs of the present disclosure by using a nucleotide monomer having a corresponding modification, wherein the methods for preparing a nucleotide monomer having a corresponding modification and the methods for introducing a modified nucleotide into an siRNA are also well-known to those skilled in the art. Modified nucleotide groups may be introduced into the siRNA of the present disclosure by using a nucleotide monomer having a corresponding modification, wherein the methods for preparing the nucleotide monomer having the corresponding modification and the methods for introducing the modified nucleotide group into the siRNA are also well-known to those skilled in the art.

Pharmaceutical composition

The present disclosure provides a pharmaceutical composition, wherein the pharmaceutical composition comprises the siRNA described above as an active ingredient, and a pharmaceutically acceptable carrier.

The pharmaceutically acceptable carrier may be a carrier conventionally used in the field of siRNA administration, for example, but not limited to, one or more of magnetic nanoparticles (such as $Fe_3O_4$ or $Fe_2O_3$-based nanoparticle), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In the pharmaceutical composition, there are no special requirements for the contents of the siRNA and the pharmaceutically acceptable carrier, which may be the conventional content of each component. In some embodiments, the weight ratio of the siRNA to the pharmaceutically acceptable carrier is 1: (1-500), and in some embodiments, the weight ratio above is 1: (1-50).

In some embodiments, the pharmaceutical composition may also comprise other pharmaceutically acceptable excipients, which may be one or more of various conventional formulations or compounds in the art. For example, the other pharmaceutically acceptable excipients may comprise at least one of a pH buffer solution, a protective agent and an osmotic pressure regulator.

The pH buffer solution may be a tris(hydroxymethyl) aminomethane hydrochloride buffer solution with a pH of 7.5-8.5, and/or a phosphate buffer solution with a pH of 5.5-8.5, preferably a phosphate buffer solution with a pH of 5.5-8.5.

The protective agent may be at least one of inositol, sorbitol, sucrose, trehalose, mannose, maltose, lactose, and glucose. The content of the protective agent may be from 0.01 wt% to 30 wt% on the basis of the total weight of the pharmaceutical composition.

The osmotic pressure regulator may be sodium chloride and/or potassium chloride. The content of the osmotic pressure regulator allows an osmotic pressure of the pharmaceutical composition to be 200-700 milliosmol/kg (mOsm/kg). Depending on the desired osmotic pressure, those skilled in the art can readily determine the content of the osmotic pressure regulator.

In some embodiments, the pharmaceutical composition may be a liquid formulation, for example, an injection solution; or a lyophilized powder for injection, which is mixed with a liquid excipient to form a liquid formulation upon administration. The liquid formulation may be administered by, but not limited to, subcutaneous, intramuscular or intravenous injection routes, and also may be administered to, but not limited to, lung by spray, or other organs (such as liver) via lung by spray. In some embodiments, the pharmaceutical composition is administered by intravenous injection.

In some embodiments, the pharmaceutical composition may be in the form of a liposome formulation. In some embodiments, the pharmaceutically acceptable carrier used in the liposome formulation comprises an amine-containing transfection compound (hereinafter also referred to as an organic amine), a helper lipid and/or a pegylated lipid. The organic amine, the helper lipid and the pegylated lipid may be respectively selected from one or more of the amine-containing transfection compounds or the pharmaceutically acceptable salts or derivatives thereof, the helper lipids and the pegylated lipids as described in CN103380113A (which is incorporated herein by reference in its entirety).

In some embodiments, the organic amine may be a compound as shown by Formula (201) as described in CN103380113A or a pharmaceutically acceptable salt thereof:

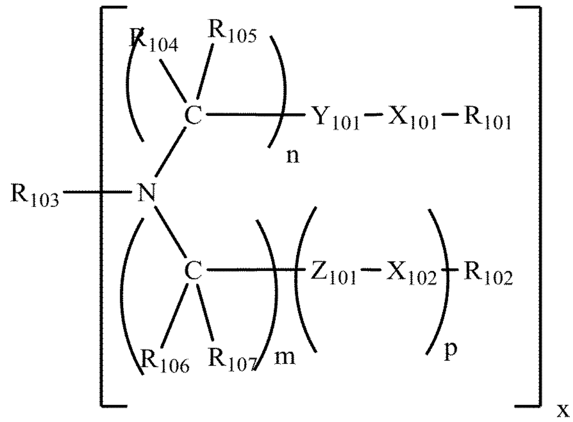

Formula (201),

wherein:

$X_{101}$ or $X_{102}$ is independently selected from O, S, N-A or C-A, wherein A is hydrogen or a C1-C20 hydrocarbon chain;

$Y_{101}$ or $Z_{101}$ is independently selected from C=O, C=S, S=O, CH-OH or $SO_2$;

$R_{101}$, $R_{102}$, $R_{103}$, $R_{104}$, $R_{105}$, $R_{106}$ or $R_{107}$ is independently selected from hydrogen; a cyclic or aliphatic, substituted or unsubstituted, branched or linear aliphatic group; a cyclic or aliphatic, substituted or unsubstituted, branched or linear heteroaliphatic group; a substituted or unsubstituted, branched or linear acyl group; a substituted or unsubstituted, branched or linear aryl, or a substituted or unsubstituted, branched or linear heteroaryl;

x is an integer of 1-10;

n is an integer of 1-3, m is an integer of 0-20, and p is 0 or 1, wherein if m and p are both 0, then $R_{102}$ is hydrogen, and

if at least one of n or m has is 2, then $R_{103}$ and the nitrogen in Formula (201) form a structure as shown by Formula (202) or (203):

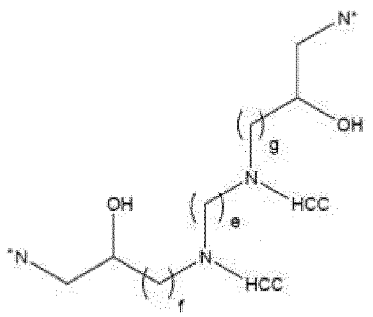

Formula (202),

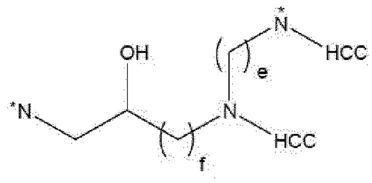

Formula (203);

wherein g, e or f is independently an integer of 1-6, "HCC" represents a hydrocarbon chain, and each *N represents a nitrogen atom shown in Formula (201).

In some embodiments, $R_{103}$ is a polyamine. In other embodiments, $R_{103}$ is a ketal. In some embodiments, each of $R_{101}$ and $R_{102}$ in the Formula (201) is independently any substituted or unsubstituted, branched or linear alkyl or alkenyl, wherein the alkyl or alkenyl has 3 to about 20 carbon atoms (such as 8 to about 18 carbon atoms) and 0 to 4 double bonds (such as 0 to 2 double bonds).

In some embodiments, if each of n and m is independently 1-3, $R_{103}$ may be any in the following Formulas (204)-(213):

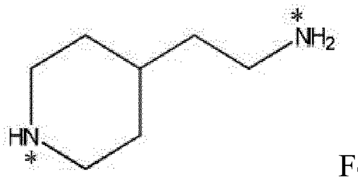 Formula (204), 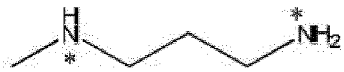 Formula (205),

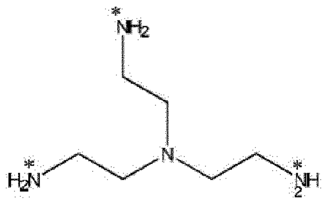 Formula (206), 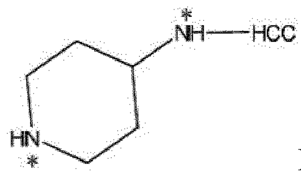 Formula (207),

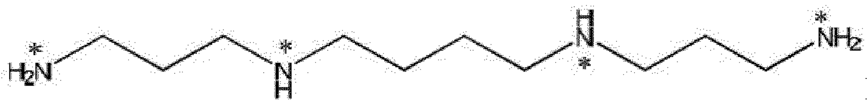 Formula (208),

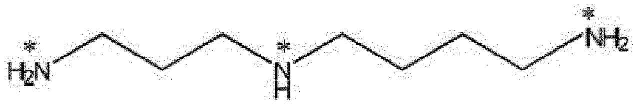 Formula (209),

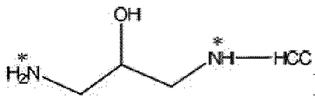 Formula (210), 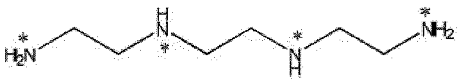 Formula (211),

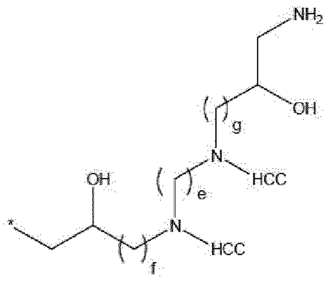 Formula (212) and 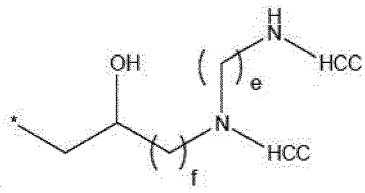 Formula (213);

wherein, in Formula (204) to Formula (213), each of g, e and f is independently an integer of 1-6; each "HCC" represents a hydrocarbon chain, and each * represents a potential attachment point of $R_{103}$ to the nitrogen atom in Formula (201), wherein each H at any * position may be replaced to realize the attachment to the nitrogen atom in Formula (201).

The compound as shown by (201) may be prepared as described in CN103380113A.

In some embodiments, the organic amine may be an organic amine as shown by Formula (214) and/or an organic amine as shown by Formula (215):

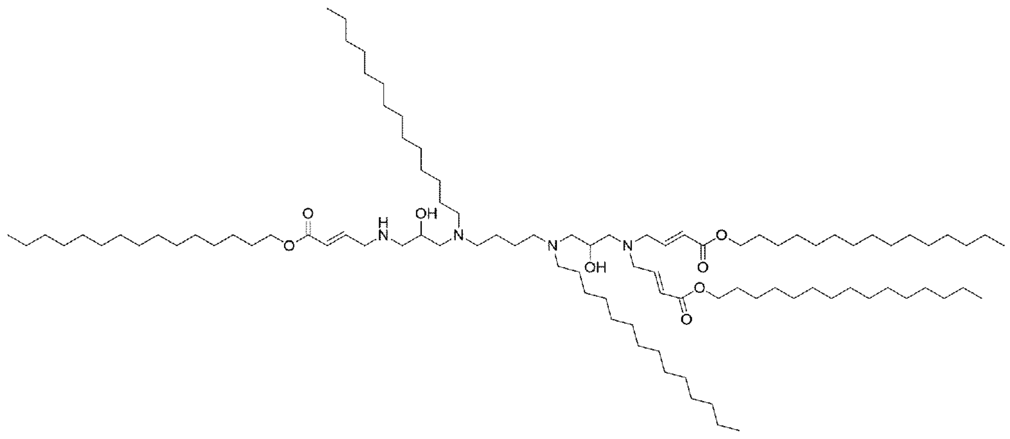

Formula (214),

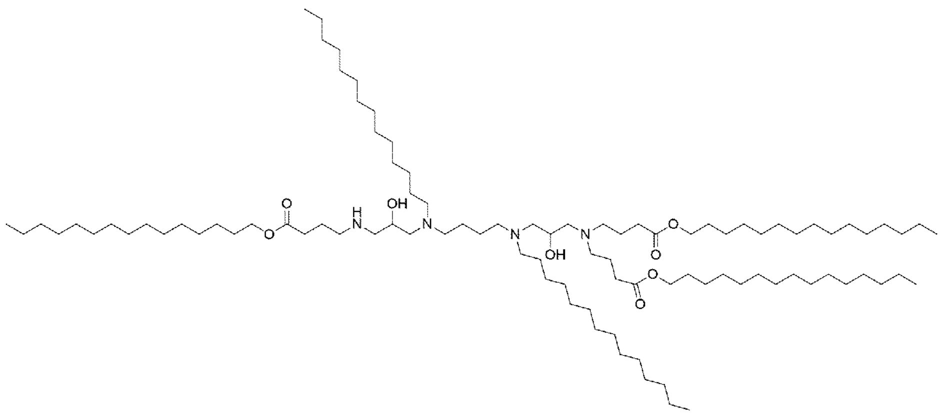

Formula (215);

The helper lipid is a cholesterol, a cholesterol analogue and/or a cholesterol derivative.

The pegylated lipid is 1,2-dipalmitoyl-sn-glycero-3-phosphatidylethanolamine-N-[methoxy(polyethyleneglycol)]-2000.

In some embodiments, the molar ratio among the organic amine, the helper lipid, and the pegylated lipid in the pharmaceutical composition is (19.7-80): (19.7-80): (0.3-50); for example, the molar ratio may be (50-70): (20-40): (3-20).

In some embodiments, the pharmaceutical compositions formed by the siRNA of the present disclosure and the above amine-containing transfection agent have an average diameter from about 30 nm to about 200 nm, typically from about 40 nm to about 135 nm, and more typically, the average diameter of the liposome particles is from about 50 nm to about 120 nm, from about 50 nm to about 100 nm, from about 60 nm to about 90 nm, or from about 70 nm to about 90 nm, for example, the average diameter of the liposome particles is about 30, 40, 50, 60, 70, 75, 80, 85, 90, 100, 110, 120, 130, 140, 150 or 160 nm.

In some embodiments, in the pharmaceutical composition formed by the siRNA of the present disclosure and the above amine-containing transfection agent, the weight ratio (weight/weight ratio) of the siRNA to total lipids (e.g., the organic amine, the helper lipid and/or the pegylated lipid), ranges from about 1: 1 to about 1: 50, from about 1: 1 to about 1: 30, from about 1: 3 to about 1: 20, from about 1: 4 to about 1: 18, from about 1: 5 to about 1: 17, from about 1: 5 to about 1: 15, from about 1: 5 to about 1: 12, from about 1: 6 to about 1: 12, or from about 1: 6 to about 1: 10. For example, the ratio of the siRNA of the present disclosure to the total lipids is about 1: 5, 1: 6, 1: 7, 1: 8, 1: 9, 1: 10, 1: 11, 1: 12, 1: 13, 1: 14, 1: 15, 1: 16, 1: 17 or 1: 18 by weight.

In some embodiments, the pharmaceutical composition may be marketed with each component being separate, and used in the form of a liquid formulation. In some embodiments, the pharmaceutical composition formed by the siRNA of the present disclosure and the above pharmaceutically acceptable carrier may be prepared by various known processes, except replacing the existing siRNA with the siRNA of the present disclosure. In some embodiments, the pharmaceutical composition may be prepared according to the following process.

The organic amines, helper lipids and pegylated lipids are suspended in alcohol at a molar ratio as described above and mixed homogeneously to yield a lipid solution; and the alcohol is used in an amount such that the resultant lipid solution is present at a total mass concentration of 2 to 25 mg/mL, e.g., 8 to 18 mg/mL. The alcohol is a pharmaceutically acceptable alcohol, such as an alcohol that is in liquid form at about room temperature, for example, one or

more of ethanol, propylene glycol, benzyl alcohol, glycerol, PEG 200, PEG 300, PEG 400, and for example, ethanol.

The siRNA provided by the present disclosure is dissolved in a buffered salt solution to produce an aqueous solution of the siRNA. The buffered salt solution has a concentration of 0.05-0.5 M, such as 0.1-0.2 M. The pH of the buffered salt solution is adjusted to 4.0-5.5, such as 5.0-5.2. The buffered salt solution is used in an amount such that the siRNA is present at a concentration of less than 0.6 mg/ml, such as 0.2-0.4 mg/mL. The buffered salt may be one or more selected from the group consisting of soluble acetate and soluble citrate, such as sodium acetate and/or potassium acetate.

The lipid solution and the aqueous solution of the siRNA are mixed. The product obtained after mixing is incubated at a temperature of 40-60°C for at least 2 minutes (e.g., 5-30 minutes) to produce an incubated lipid formulation. The volume ratio of the lipid solution to the aqueous solution of the siRNA is 1: (2-5).

The incubated liposome formulation is concentrated or diluted, purified to remove impurities, and then sterilized to obtain the pharmaceutical composition provided by the present disclosure, which has physicochemical parameters as follows: a pH of 6.5-8, an encapsulation percentage of more than 80%, a particle size of 40-200 nm, a polydispersity index of less than 0.30, and an osmotic pressure of 250-400 mOsm/kg; for example, the physicochemical parameters may be as follows: a pH of 7.2-7.6, an encapsulation percentage of more than 90%, a particle size of 60-100 nm, a polydispersity index of less than 0.20, and an osmotic pressure of 300-400 mOsm/kg.

The concentration or dilution step may be performed before, after or simultaneously with the step of impurity removal. The method for removing impurities may be any of various existing methods, for example, ultrafiltration using 100 KDa hollow fiber column and a phosphate buffer solution (PBS) at pH 7.4 as an ultrafiltration exchange solution and a tangential flow system. The method for sterilization may be any of various existing methods, such as filtration sterilization on a 0.22 μm filter.

siRNA conjugate

The present disclosure provides an siRNA conjugate, wherein the siRNA conjugate comprises the siRNA above and a conjugating group conjugatively linked to the siRNA.

The conjugating group typically comprises at least one pharmaceutically acceptable targeting group and an optional linker. Moreover, the siRNA, the linker and the targeting group are linked in succession. In some embodiments, there are 1-6 targeting groups. In some embodiments, there are 2-4 targeting groups. The siRNA molecule may be non-covalently or covalently conjugated to the conjugating group, for example, the siRNA molecule may be covalently conjugated to the conjugating group. The conjugating site between the siRNA and the conjugating group may be at 3'-terminal or 5'-terminal of the sense strand of the siRNA, or at 5'-terminal of the antisense strand, or within the internal sequence of the siRNA. In some embodiments, the conjugating site between the siRNA and the conjugating group is at 3' terminal of the sense strand of the siRNA.

In some embodiments, the conjugation group is linked to a phosphate group, the 2'-hydroxy or the base of a nucleotide. In some embodiments, the conjugation group may be linked to a 3'-hydroxy when the nucleotides are linked via a 2'-5'-phosphodiester bond. When the conjugating group is linked to a terminal of the siRNA, the conjugating group is typically linked to a phosphate group of a nucleotide; when the conjugating group is linked to an internal sequence of the siRNA, the conjugating group is typically linked to a ribose ring or a base. For specific linking modes, reference may be made to: siRNA conjugates carrying sequentially assembled trivalent N-acetylgalactosamine linked through nucleosides elicit robust gene silencing in vivo in hepatocytes. ACS Chemical biology, 2015,10(5):1181-7, written by Muthiah Manoharan et.al.

In some embodiments, the siRNA and the conjugating group may be linked by an acid labile or reducible chemical bond, and these chemical bonds may be degraded under the acidic environment of cell endosomes, thereby rendering the siRNA to be in free state. For non degradable conjugating modes, the conjugating group may be linked to the sense strand of the siRNA, thereby minimizing the effect of conjugating on the activity of the siRNA.

In some embodiments, the pharmaceutically acceptable targeting group may be a conventionally used ligand in the field of siRNA administration, for example, the various ligands as described in WO2009082607A2, which is incorporated herein by reference in its entirety.

In some embodiments, the pharmaceutically acceptable targeting group may be selected from one or more of the ligands formed by the following targeting molecules or derivatives thereof: lipophilic molecules, such as cholesterol, bile acids, vitamins (such as vitamin E), lipid molecules of different chain lengths; polymers, such as polyethylene glycol; polypeptides, such as cell-penetrating peptide; aptamers; antibodies; quantum dots; saccharides, such as lactose, polylactose, mannose, galactose, and N-acetylgalactosamine (GalNAc); folate; and receptor ligands expressed in hepatic parenchymal cells, such as asialoglycoprotein, asialo-sugar residue, lipoproteins (such as high density lipoprotein, low density lipoprotein), glucagon, neurotransmitters (such as adrenaline), growth factors, transferrin and the like.

In some embodiments, each ligand is independently a ligand capable of binding to a cell surface receptor. In some embodiments, at least one ligand is a ligand capable of binding to a hepatocyte surface receptor. In some embodiments, at least one ligand is a ligand capable of binding to a mammalian cell surface receptor. In some embodiments, at least one ligand is a ligand capable of binding to a human cell surface receptor. In some embodiments, at least one ligand is a ligand capable of binding to a hepatic surface asialoglycoprotein receptor (ASGPR). The types of these ligands are well-known to those skilled in the art and they typically serve the function of binding to specific receptors on the surface of the target cell, thereby mediating delivery of the siRNA linked to the ligand into the target cell.

In some embodiments, the pharmaceutically acceptable targeting group may be any ligand that binds to asialoglycoprotein receptors (ASGPR) on the surface of mammalian hepatocytes. In one embodiment, each ligand is independently an asialoglycoprotein, such as asialoorosomucoid (ASOR) or asialofetuin (ASF). In some embodiments, the ligand is a saccharide or a saccharide derivative.

In some embodiments, at least one ligand is a saccharide. In some embodiments, each ligand is a saccharide. In some embodiments, at least one ligand is a monosaccharide, polysaccharide, modified monosaccharide, modified polysaccharide, or saccharide derivative. In some embodiments, at least one ligand may be a monosaccharide, disaccharide or trisaccharide. In some embodiments, at least one ligand is a modified saccharide. In some embodiments, each ligand is a modified saccharide. In some embodiments, each ligand is independently selected from the group consisting of polysaccharides, modified polysaccharides, monosaccharides, modified monosaccharides, polysaccharide derivatives or monosaccharide derivatives. In some embodiments, each ligand or at least one ligand is selected from the group consisting of the following saccharides: glucose and derivative thereof, mannose and derivative thereof, galactose and derivative thereof, xylose and derivative thereof, ribose and derivative thereof, fucose and derivative thereof, lactose and derivative thereof, maltose and derivative thereof, arabinose and derivative thereof, fructose and derivative thereof, and sialic acid.

In some embodiments, each ligand may be independently selected from one of. D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucofuranose, methyl 2,3,4-tris-O-acetyl-1-thio-6-0-trityl-a-D-glucofuranose, 4-thio-β-D-galactopyranose, ethyl 3,4,6,7-tetra-O-acetyl-2-deoxy-1,5-dithio-a-D-glucoheptopyranoside, 2,5-anhydro-D-allononitrile, ribose, D-ribose, D-4-thioribose, L-ribose, or L-4-thioribose. Other ligand selections may be found, for example, in the disclosure of CN105378082A, which is incorporated herein by reference in its entirety.

In some embodiments, the pharmaceutically acceptable targeting group in the siRNA conjugate may be galactose or N-acetylgalactosamine, wherein the galactose or N-acetylgalactosamine molecules may be monovalent, bivalent, trivalent and tetravalent. It should be understood that the terms monovalent, bivalent, trivalent and tetravalent described herein respectively mean that the molar ratio of the siRNA molecule to the galactose or N-acetylgalactosamine molecule in the siRNA conjugate is 1: 1, 1: 2, 1: 3 or 1: 4, wherein the siRNA conjugate is formed from the siRNA molecule and the conjugating group containing galactose or N-acetylgalactosamine as the targeting group. In some embodiments, the pharmaceutically acceptable targeting group is N-acetylgalactosamine. In some embodiments, when the siRNA of the present disclosure is conjugated to a conjugation group comprising N-acetylgalactosamine, the N-acetylgalactosamine molecule is trivalent or tetravalent. In some embodiments, when the siRNA of the present disclosure is conjugated to a conjugating group containing N-acetylgalactosamine, the N-acetylgalactosamine molecule is trivalent.

The targeting group may be linked to the siRNA molecule via an appropriate linker, and the appropriate linker may be selected by those skilled in the art according to the specific type of the targeting group. The types of these linkers and targeting groups and the linking modes with the siRNA may be found in the disclosure of WO2015006740A2, which is incorporated herein by reference in its entirety.

In some embodiments, when the targeting group is N-acetylgalactosamine, an appropriate linker may be a structure as shown by Formula (301):

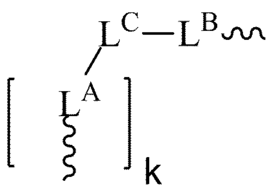

Formula (301)

wherein,

k is an integer of 1-3; and

$L^A$ is an amide bond-comprising chain moiety that has a structure as shown by Formula (302), each $L^A$ being respectively linked to the targeting group and the $L^C$ moiety through an ether bond at two terminals thereof:

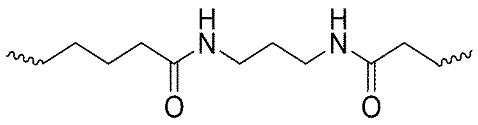

Formula (302);

$L^B$ is an N-acylpyrrolidine-comprising chain moiety that has a structure as shown by Formula (303), the chain moiety having a carbonyl at one terminal thereof and being linked to the $L^C$ moiety through an amide bond, and having an oxy-group at the other terminal thereof and being linked to the siRNA via a phosphoester bond:

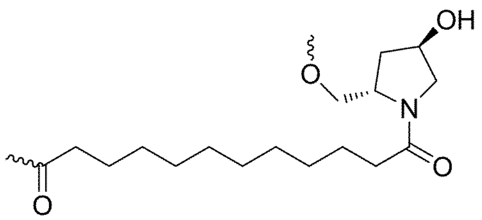

Formula (303);

$L^C$ is a bivalent to tetravalent linking group based on hydroxymethyl aminomethane, dihydroxymethyl aminomethane or trihydroxymethyl aminomethane, the $L^C$ being linked to each of the $L^A$ moieties through an ether bond via an oxygen atom, and being linked to the $L^B$ moiety through an amide bond via a nitrogen atom.

In some embodiments, when n=3 and $L^C$ is a tetravalent linking group based on trihydroxymethyl aminomethane, the siRNA conjugate formed by linking an N-acetylgalactosamine molecule with an siRNA molecule via $-(L^A)_3$-trihydroxymethyl aminomethane-$L^B$- as a linker has a structure as shown by Formula (304):

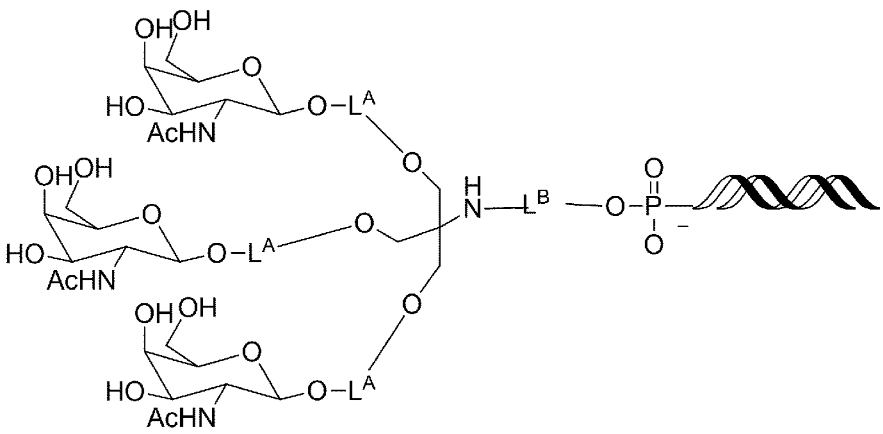

Formula (304),

wherein the double helix structure represents an siRNA.

Likewise, the conjugating site between the siRNA and the conjugating group nay be at the 3'-terminal or 5'-terminal of the sense strand of the siRNA, or at the 5'-terminal of the antisense strand, or within the internal sequence of the siRNA.

In some embodiments, the 3'-terminal of the sense strand of the siRNA of the present disclosure is covalently conjugated to three N-acetylgalactosamine (GalNAc) molecules via a linker $-(L^A)_3$-trihydroxymethyl aminomethane-$L^B$- to obtain an siRNA conjugate in which the molar ratio of the siRNA molecule to the GalNAc molecule is 1: 3, which may also be hereinafter referred to as $(GalNAc)_3$-siRNA), and the siRNA conjugate has a structure as shown by Formula (305):

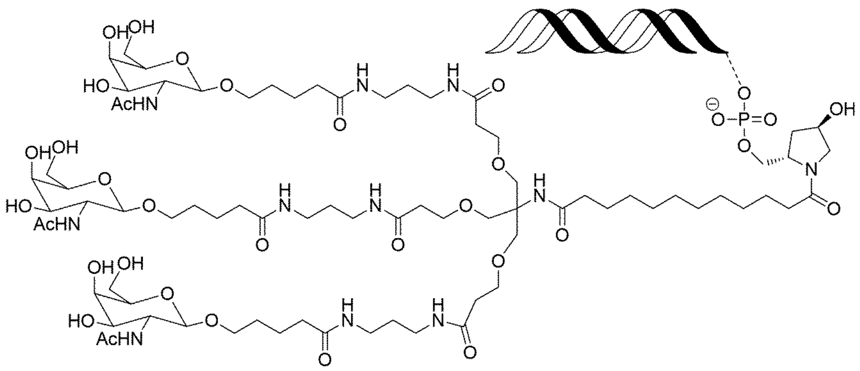

Formula (305),

wherein the double helix structure represents an siRNA; and the linker is linked to the 3' terminal of the sense strand of the siRNA.

In some embodiments, when the targeting group is N-acetylgalactosamine, an appropriate linker may be a structure as shown by Formula (306):

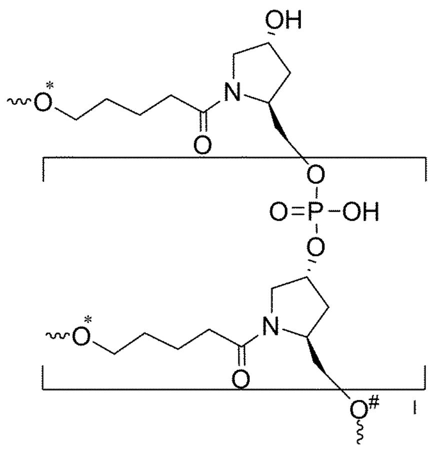

, Formula (306)

wherein,

1 is an integer of 0-3;

* represents a site linked to the targeting group via an ether bond on the linker; and

# represents a site linked to the siRNA via a phosphoester bond on the linker.

In some embodiments, when 1=2, the siRNA conjugate has a structure as shown by Formula (307):

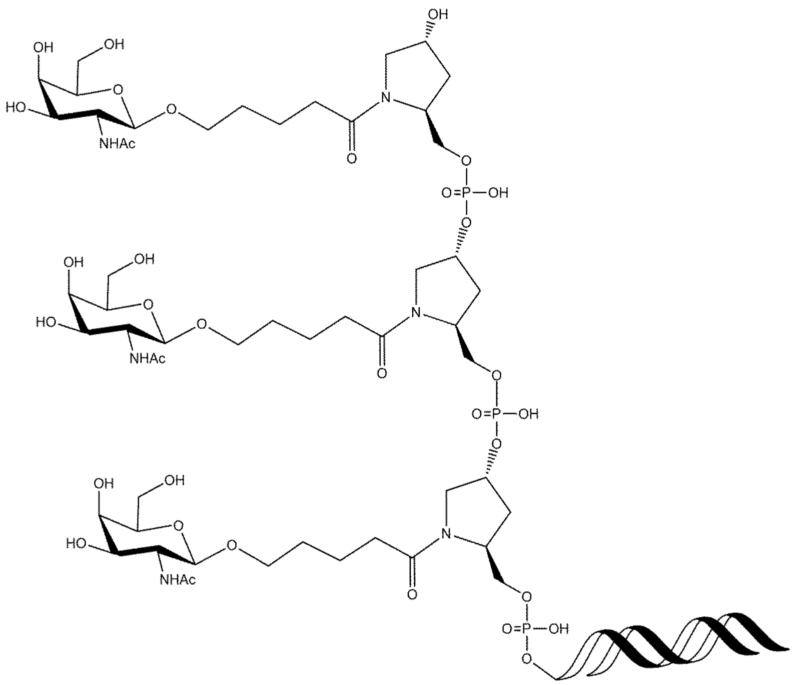

Formula (307),

wherein the double helix structure represents an siRNA; and the linker is linked to the 3' terminal of the sense strand of the siRNA.

The above conjugates may be synthesized according to the methods described in detail in the prior art. For example, WO2015006740A2 describes the method of preparing various conjugates in detail. The siRNA conjugate of the present disclosure may be obtained by methods well known to those skilled in the art. As another example, WO2014025805A1 describes the preparation method of the conjugate having a structure as shown by Formula (305). Rajeev et al., describes the preparation method of the conjugate having a structure as shown by Formula (307) in ChemBioChem 2015, 16, 903-908.

In some embodiments, the siRNA conjugate has a structure as shown by Formula (308):

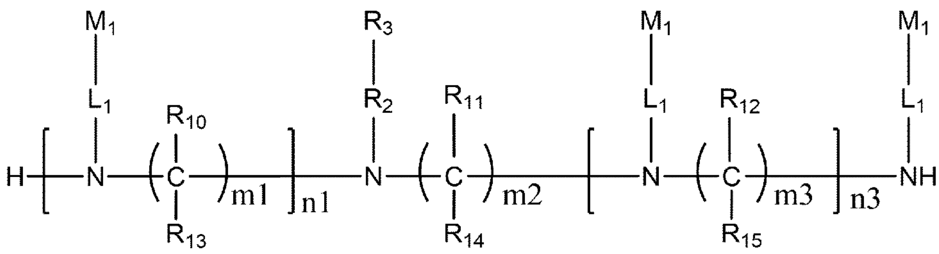

Formula (308),

wherein:

n1 is an integer of 1-3, and n3 is an integer of 0-4;

each of ml, m2, and m3 is independently an integer of 2-10;

each of $R_{10}$, $R_{11}$, $R_{12}$, $R_{13}$, $R_{14}$ or $R_{15}$ is independently H or selected from the group consisting of $C_1$-$C_{10}$ alkyl, $C_1$-$C_{10}$ haloalkyl and $C_1$-$C_{10}$ alkoxy; and

$R_3$ is a group having a structure as shown by Formula A59:

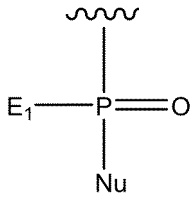

(A59)

wherein, $E_1$ is OH, SH or $BH_2$, and Nu is the siRNA of the present disclosure;

$R_2$ is a linear alkylene of 1-20 carbon atoms in length, wherein one or more carbon atoms are optionally replaced with any one or more of the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $R_2$ is optionally substituted by any one or more of the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -CO2H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2NH$($C_1$-$C_{10}$ alkyl), -$SO_2NH$(phenyl), -$NHSO_2$($C_1$-$C_{10}$ alkyl), -$NHSO_2$(phenyl), and -$NHSO_2$($C_1$-$C_{10}$ haloalkyl); and

each $L_1$ is independently a linear alkylene of 1-70 carbon atoms in length, wherein one or more carbon atoms are optionally replaced with any one or more of the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ is optionally substituted by any one or more of the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -$CO_2H$, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2NH$($C_1$-$C_{10}$ alkyl), -$SO_2NH$(phenyl), -$NHSO_2$($C_1$-$C_{10}$ alkyl), -$NHSO_2$(phenyl), and -$NHSO_2$($C_1$-$C_{10}$ haloalkyl).

In some embodiments, $L_1$ may be selected from the group consisting of groups (A1)-(A26) and any combination thereof, wherein the structures and definitions of (A1)-(A26) are as follows:

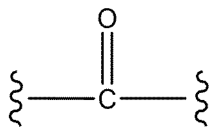 , 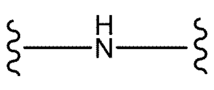 , 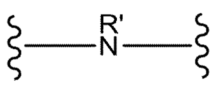 , 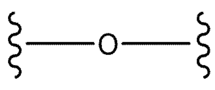 ,

(A1) (A2) (A3) (A4)

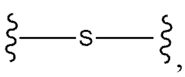 , 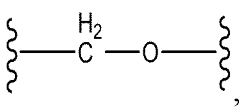 , 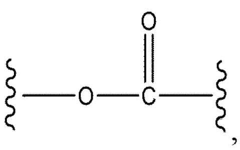 , 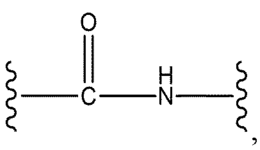 ,

(A5) (A6) (A7) (A8)

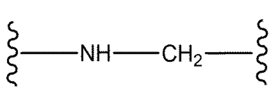, 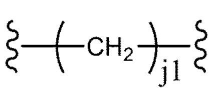, 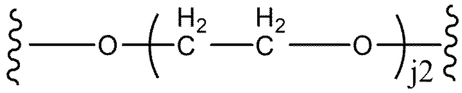,

(A9) (A10) (A11)

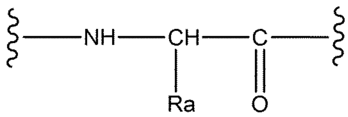, 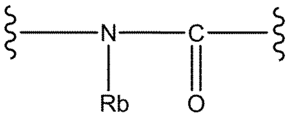, 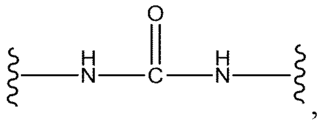,

(A12) (A13) (A14)

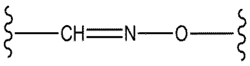, 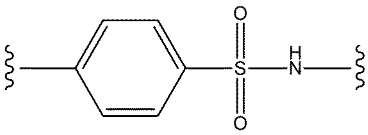, 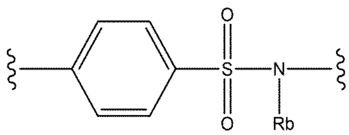,

(A15) (A16) (A17)

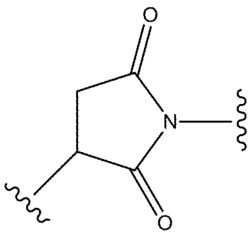, 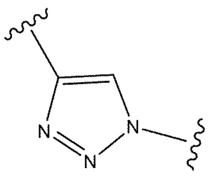, 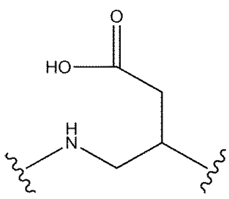, 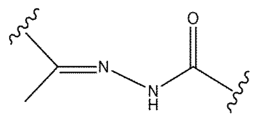,

(A18) (A19) (A20) (A21)

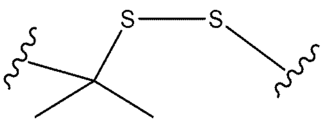, 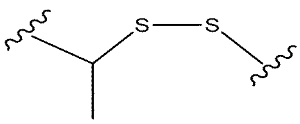, 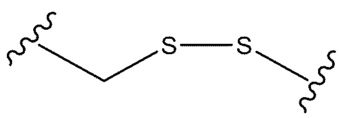,

(A22) (A23) (A24)

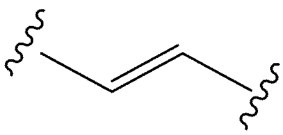, 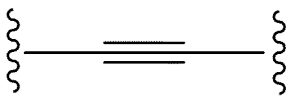;

(A25) (A26)

wherein, each j1 is independently an integer of 1-20; and each j2 is independently an integer of 1-20;

each R' is independently a $C_1$-$C_{10}$ alkyl; and

each Ra is independently selected from the group consisting of groups (A27)-(A45) and any connection combinations thereof:

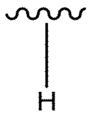 , 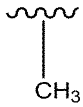 , 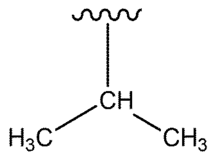 , 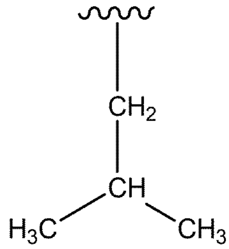 , 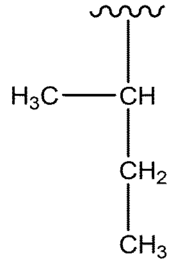 , 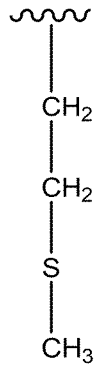 ,

(A27) (A28) (A29) (A30) (A31) (A32)

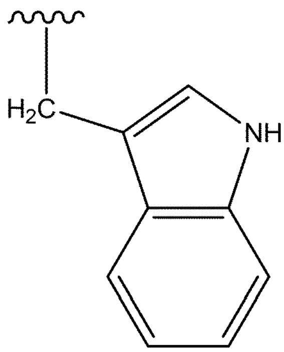 , 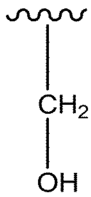 , 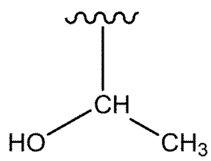 , 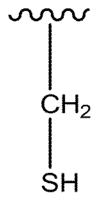 , 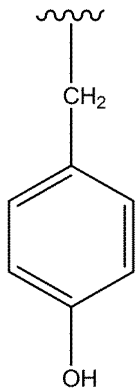 ,

(A33) (A34) (A35) (A36) (A37)

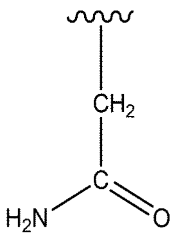 , 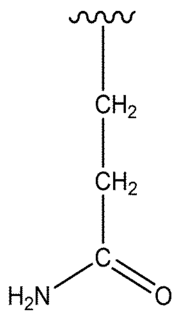 , 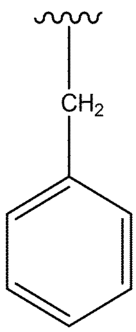 , 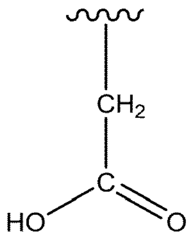 , 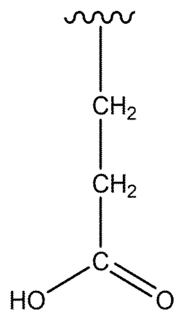 ,

(A38) (A39) (A40) (A41) (A42)

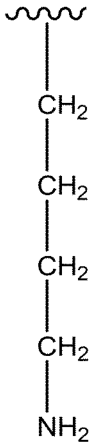, 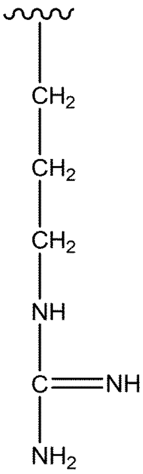 or 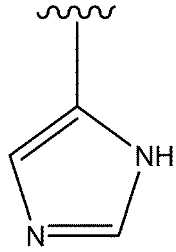 ;

(A43) (A44) (A45)

each Rb is independently a $C_1$-$C_{10}$ alkyl; and 



represents a site where the group is covalently linked.

Those skilled in the art would understand that, though $L_1$ is defined as a linear alkylene for convenience, but it may not be a linear group or be named differently, such as an amine or alkenyl produced by the above replacement and/or substitution. For the purpose of the present disclosure, the length of $L_1$ is the number of the atoms in the chain connecting the two attaching points. For this purpose, a ring obtained by replacement of a carbon atom of the linear alkylene, such as a heterocyclylene or heteroarylene, is counted as one atom.

$M_1$ represents a targeting group, of which the definitions and options are the same as those described above. In some embodiments, each $M_1$ is independently selected from one of the ligands that have affinity to the asialoglycoprotein receptor on the surface of mammalian hepatocytes.

When $M_1$ is a ligand that has affinity to the asialoglycoprotein receptor on the surface of mammalian hepatocytes, in some embodiments, n1 may be an integer of 1-3, and n3 may be an integer of 0-4 to ensure that the number of the $M_1$ targeting group in the siRNA conjugate may be at least 2. In some embodiments, n1+n3≥2, such that the number of the $M_1$ targeting group in the conjugate may be at least 3, thereby allowing the $M_1$ targeting group to more conveniently bind to the asialoglycoprotein receptor on the surface of hepatocytes, which may facilitate the endocytosis of the siRNA conjugate into cells. Experiments have shown that when the number of the $M_1$ targeting group is greater than 3, the ease of binding the $M_1$ targeting group to the asialoglycoprotein receptor on the surface of hepatocytes is not significantly increased. Therefore, in view of various aspects such as synthesis convenience, structure/process costs and delivery efficiency, in some embodiments, n1 is an integer of 1-2, n3 is an integer of 0-1, and n1+n3=2-3.

In some embodiments, when m1, m2, or m3 is each independently selected from selected from an integer of 2-10, the steric mutual positions among a plurality of $M_1$ targeting groups may be fit for binding the $M_1$ targeting groups to the asialoglycoprotein receptor on the surface of hepatocytes. In order to make the siRNA conjugate of the present disclosure have simpler structure, easier synthesis and/or reduced cost, in some embodiments, m1, m2 or m3 is independently an integer of 2-5, and in some embodiments, m1=m2=m3.

Those skilled in the art would understand that when $R_{10}$, $R_{11}$, $R_{12}$, $R_{13}$, $R_{14}$, or $R_{15}$ is each independently selected from one of H, $C_1$-$C_{10}$ alkyl, $C_1$-$C_{10}$ haloalkyl, and $C_1$-$C_{10}$ alkoxy, they would not change the properties of the siRNA conjugate of the present disclosure and could all achieve the purpose of the present disclosure. In some embodiments, $R_{10}$, $R_{11}$, $R_{12}$, $R_{13}$, $R_{14}$, or $R_{15}$ is each independently selected from selected from H, methyl or ethyl. In some embodiments, $R_{10}$, $R_{11}$, $R_{12}$, $R_{13}$, $R_{14}$, and $R_{15}$ are all H.

$R_3$ is a group having the structure as shown by Formula A59, wherein $E_1$ is OH, SH or $BH_2$, and considering the availability of starting materials, in some embodiments, $E_1$ is OH or SH.

$R_2$ is selected to achieve the linkage between the group as shown by Formula A59 and the N atom on a

nitrogenous backbone. In the context of the present disclosure, the "nitrogenous backbone" refers to a chain structure in which the carbon atoms attached to $R_{10}$, $R_{11}$, $R_{12}$, $R_{13}$, $R_{14}$, and $R_{15}$ and the N atoms are linked to each other. Therefore, $R_2$ may be any linking group capable of attaching the group as shown by Formula A59 to the N atom on a nitrogenous backbone by suitable means. In some embodiments, in the case where the siRNA conjugate as shown by Formula (308) of the present disclosure is prepared by a solid phase synthesis process, $R_2$ group needs to have both a site linking to the N atom on the nitrogenous backbone and a site linking to the P atom in $R_3$. In some embodiments, in $R_2$, the site linking to the N atom on the nitrogenous backbone forms an amide bond with the N atom, and the site linking to the P atom in $R_3$ forms a phosphoester bond with the P atom. In some embodiments, $R_2$ may be B5, B6, B5' or B6':

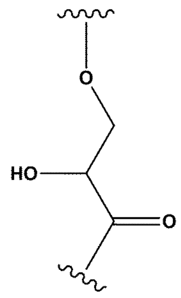
,
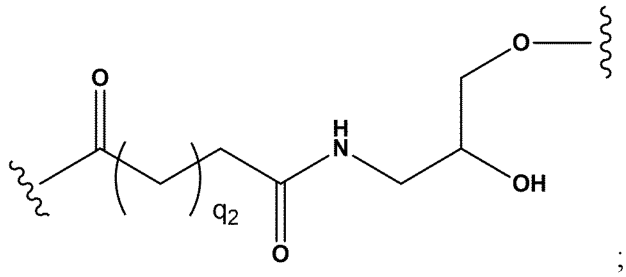
;

(B5) (B6),

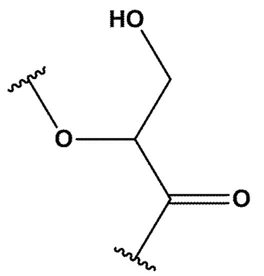
,
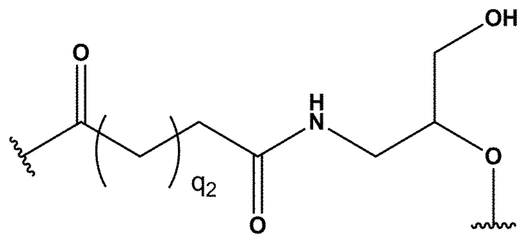
;

(B5') (B6'),

wherein, 



represents a site where the group is covalently linked.

A value range of $q_2$ may be an integer of 1-10; and in some embodiments, $q_2$ is an integer of 1-5.

$L_1$ is used to link the $M_1$ targeting group to the N atom on the nitrogenous backbone, thereby providing liver targeting function for the siRNA conjugate as shown by Formula (308). In some embodiments, $L_1$ is selected from the connection combinations of one or more of groups as shown by Formulae Al-A26. In some embodiments, $L_1$ is selected from the connection combinations of one or more of Al, A4, A5, A6, A8, A10, A11, and A13. In some embodiments, $L_1$ is selected from the connection combinations of at least two of A1, A4, A8, A10, and A11. In some embodiments, $L_1$ is selected from the connection combinations of at least two of A1, A8, and A10.

In some embodiments, the length of $L_1$ may be 3-25 atoms, 3-20 atoms, 4-15 atoms or 5-12 atoms. In some embodiments, the length of $L_1$ is 3, 4, 5, 6, 7, 8, 9, 10, 11, 12, 13, 14, 15, 16, 17, 18, 19, 20, 21, 22, 23, 24, 25, 30, 35, 40, 45, 50, 55, 60 atoms.

In some embodiments, j 1 is an integer of 2-10, and in some embodiments, j 1 is an integer of 3-5. In some embodiments, j2 is an integer of 2-10, and in some embodiments, j2 is an integer of 3-5. R' is a $C_1$-$C_4$ alkyl, and in some embodiments, R' is one of methyl, ethyl, and isopropyl. Ra is one of A27, A28, A29, A30, and A31, and in some embodiments, Ra is A27 or A28. Rb is a $C_1$-$C_5$ alkyl, and in some embodiments, Rb is one of methyl, ethyl, isopropyl, and butyl. In some embodiments, j1, j2, R', Ra, and Rb of Formulae A1-A26 are respectively selected to achieve the

linkage between the $M_1$ targeting group and the N atom on the nitrogenous backbone, and to make the steric mutual position among the $M_1$ targeting group more suitable for binding the $M_1$ targeting group to the asialoglycoprotein receptor on the surface of hepatocytes.

In some embodiments, the siRNA conjugate has a structure as shown by Formula (403), (404), (405), (406), (407), (408), (409), (410), (411), (412), (413), (414), (415), (416), (417), (418), (419), (420), (421) or (422):

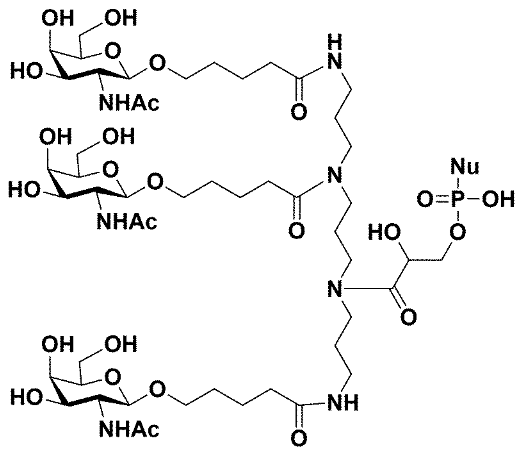

Formula (403)

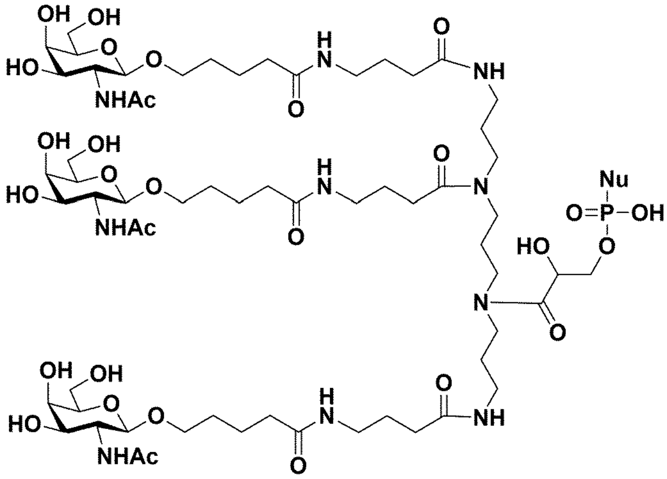

Formula (404)

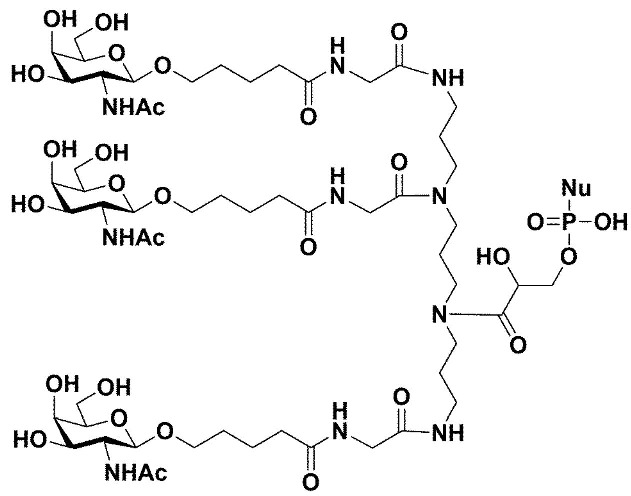

Formula (405)

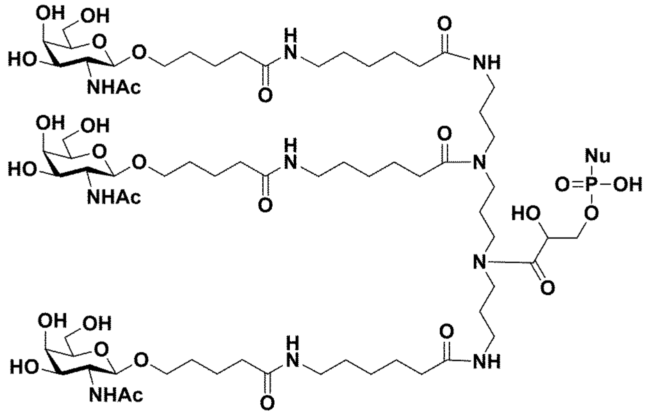

Formula (406)

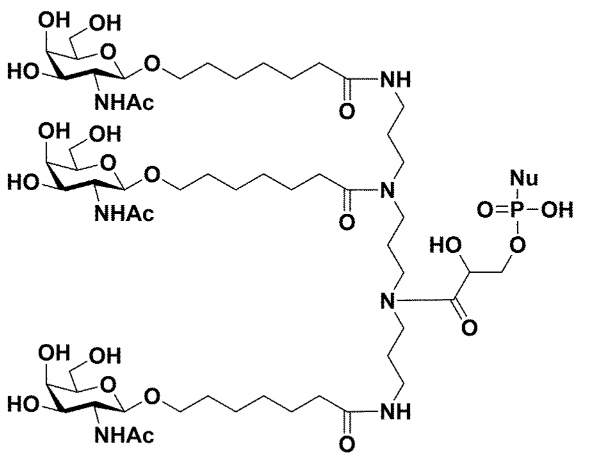

Formula (407)

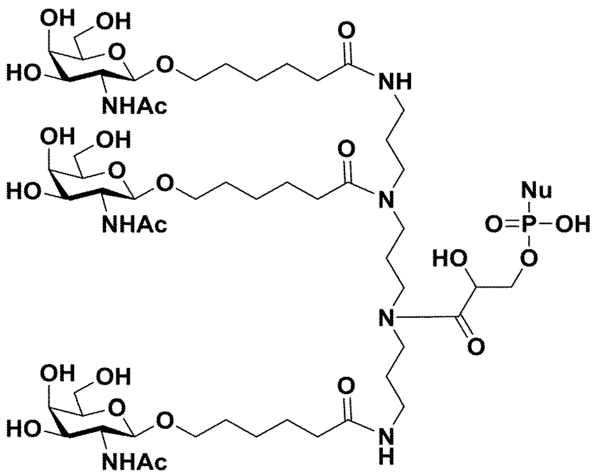

Formula (408)

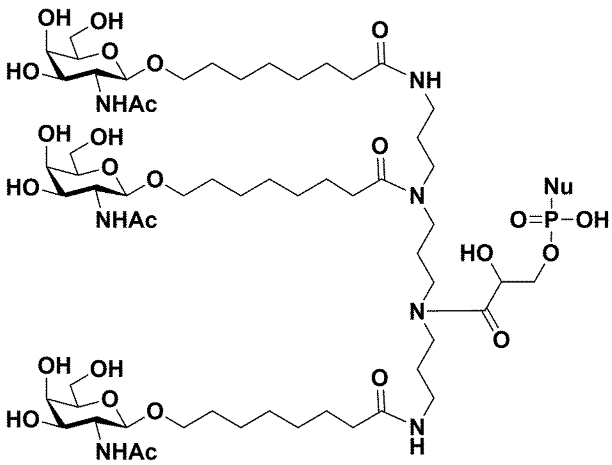

Formula (409)

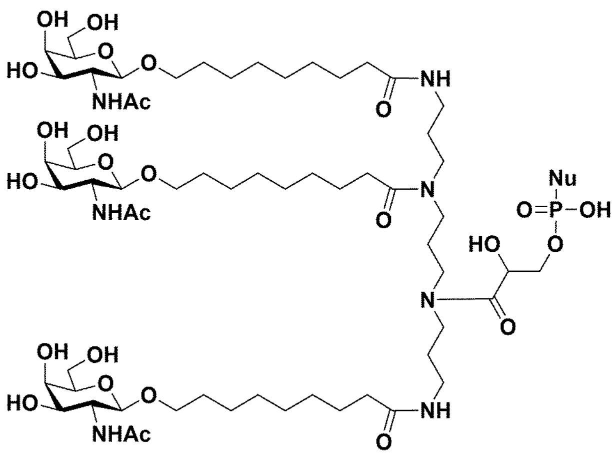

Formula (410)

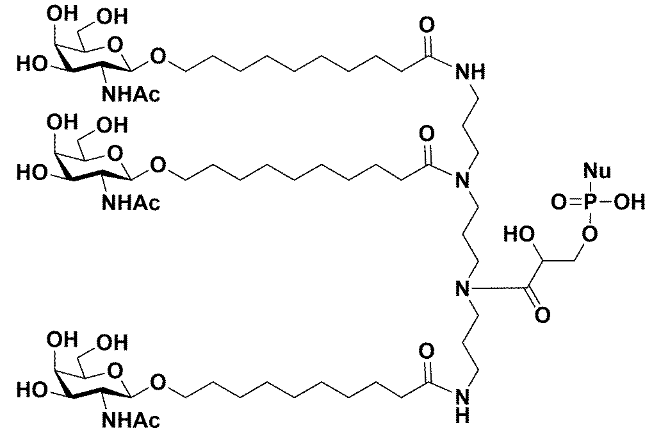

Formula (411)

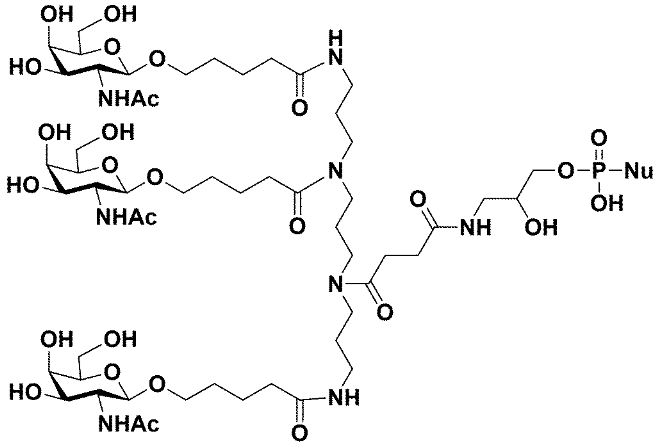

Formula (412)

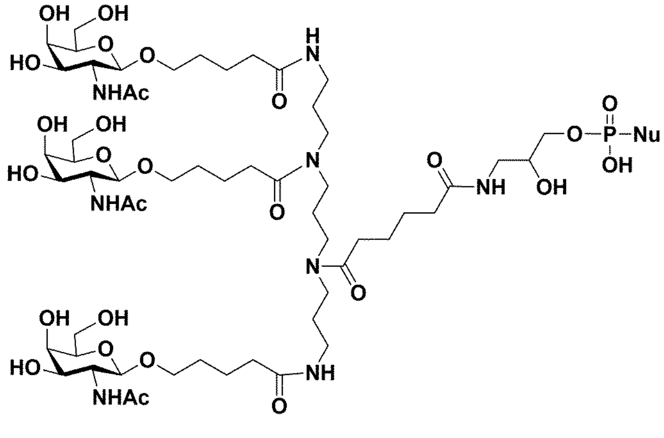

Formula (413)

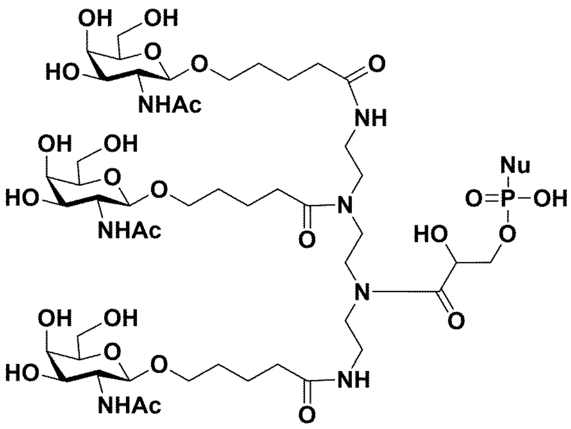

Formula (414)

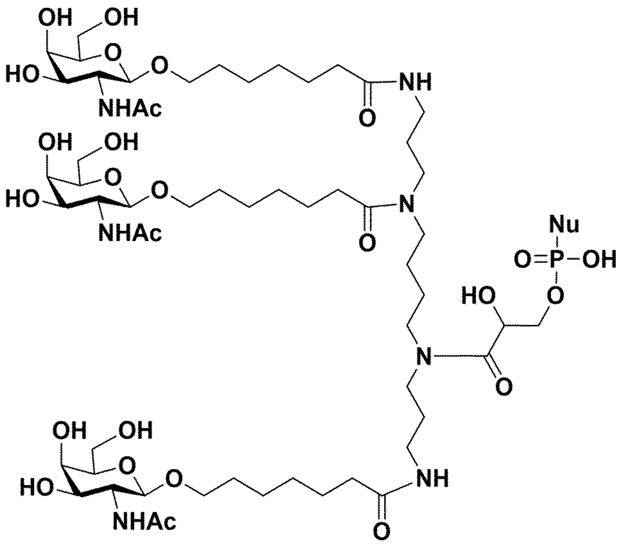

Formula (415)

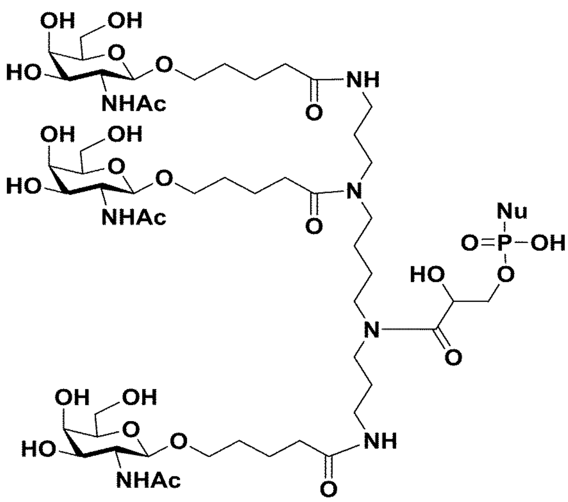

Formula (416)

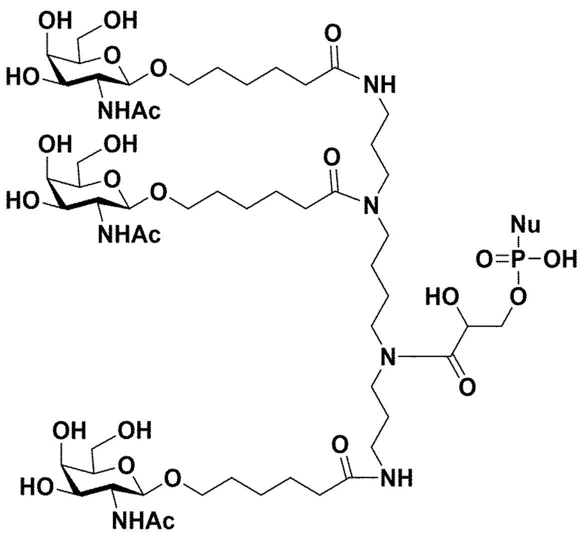

Formula (417)

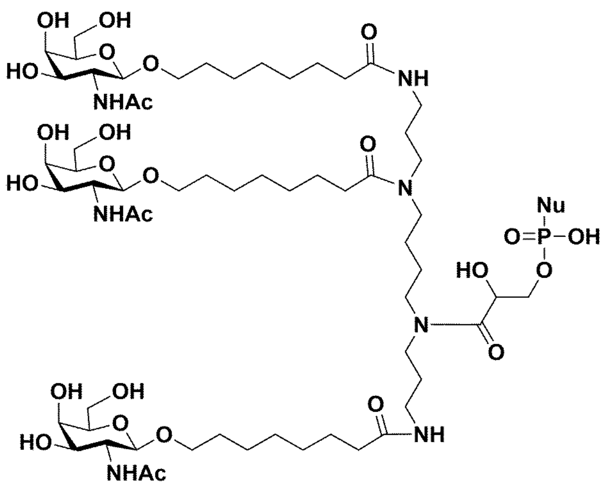

Formula (418)

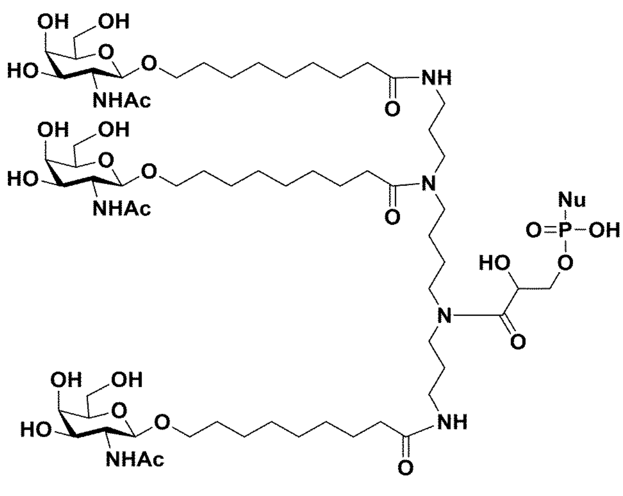

Formula (419)

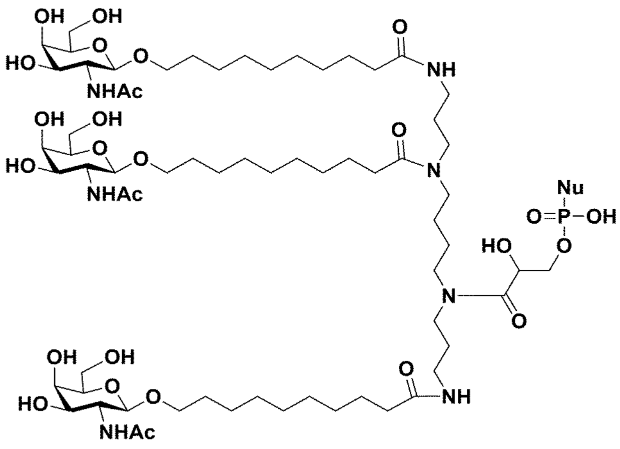

Formula (420)

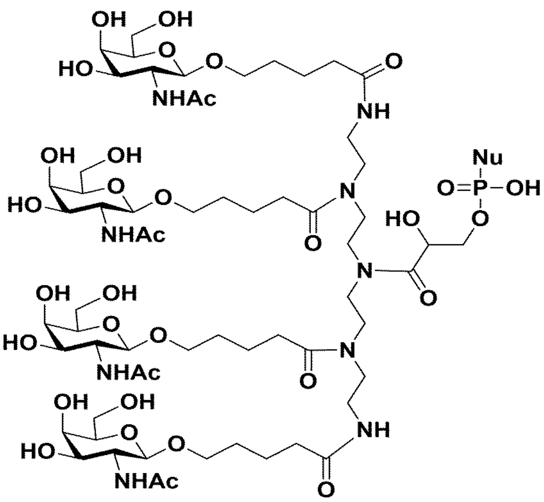

Formula (421)

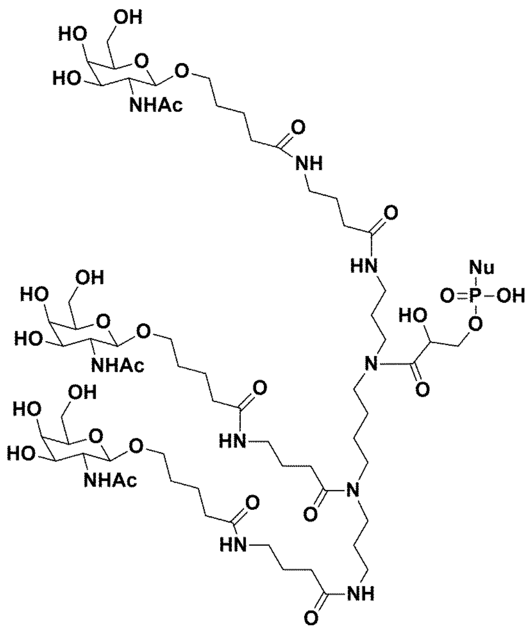

Formula (422).

In some embodiments, the P atom in Formula A59 may be linked to any possible position in the siRNA sequence, for example, the P atom in Formula A59 may be linked to any nucleotide in the sense strand or the antisense strand of the siRNA. In some embodiments, the P atom in Formula A59 is linked to any nucleotide in the sense strand of the siRNA. In some embodiments, the P atom in Formula A59 is linked to a terminal of the sense strand or the antisense strand of the siRNA. In some embodiments, the P atom in Formula A59 is linked to a terminal of the sense strand of the siRNA. The terminal refers to the first 4 nucleotides counted from one terminal of the sense strand or antisense strand. In some embodiments, the P atom in Formula A59 is linked to the terminal of the sense strand or the antisense strand of the siRNA. In some embodiments, the P atom in Formula A59 is linked to 3' terminal of the sense strand of the siRNA. In the case where the P atom in Formula A59 is linked to the above position in the sense strand of the siRNA, after entering into cells, the siRNA conjugate as shown by Formula (308) can release a separate antisense strand of the siRNA during unwinding, thereby blocking the translation of the C5 mRNA into protein and inhibiting the expression of complement protein C5 gene.

In some embodiments, the P atom in Formula A59 may be linked to any possible position of a nucleotide in the siRNA, for example, to position 5', 2' or 3', or to the base of the nucleotide. In some embodiments, the P atom in Formula A59 may be linked to position 2', 3', or 5' of a nucleotide in the siRNA by forming a phosphodiester bond. In some embodiments, the P atom in Formula A59 is linked to an oxygen atom formed by deprotonation of 3' hydroxy of the nucleotide at 3'terminal of the sense strand of the siRNA (in this time, the P atom in Formula A59 may also be regarded as a P atom in a phosphate group contained in the siRNA), or the P atom in Formula A59 is linked to a nucleotide by substituting the hydrogen atom in the 2'-hydroxy of the nucleotide of the sense strand of the siRNA, or the P atom in Formula A59 is linked to a nucleotide by substituting hydrogen in the 5'-hydroxy of the nucleotide at 5' terminal of the sense strand of the siRNA.

The inventors of the present disclosure have surprisingly found that the siRNA conjugate of the present disclosure has significantly improved stability in plasma and low off-target effect, and also shows higher silencing activity against C5 mRNA. In some embodiments, the siRNA of the present disclosure may be one of the siRNAs shown in Tables 1a-1f. The siRNA conjugates containing these siRNA show higher silencing activity against C5 mRNA.

Table 1a The first siRNA sequence of the present disclosure

| siRNA No. | SEQ ID NO: | Sequence direction 5'- 3' |
|---|---|---|
| siC5a1 | 9 | CUUCAUUCAUACAGACAAA |
|  | 10 | UUUGUCUGUAUGAAUGAAGAG |

(continued)

| siRNA No. | SEQ ID NO: | Sequence direction 5'- 3' |
|---|---|---|
| siC5a2 | 11 | CUCUUCAUUCAUACAGACAAA |
| | 12 | UUUGUCUGUAUGAAUGAAGAGAA |
| siC5a1-M1 | 13 | CmUmUmCmAmUmUfCfAfUmAmCmAmGmAmCmAmAmAm |
| | 14 | UmUfUmGmUmCfUmGmUmAmUmGmAmAfUmGfAmAmGmAmGm |
| siC5a1-M2 | 15 | CmUmUmCmAfUmUfCfAfUmAmCmAmGmAmCmAmAmAm |
| | 16 | UmUfUmGmUmCfUmGfUfAmUmGmAmAfUmGfAmAmGmAmGm |
| siC5a1-M3 | 17 | CmUmUmCmAfUmUfCfAfUmAmCmAmGmAmCmAmAmAm |
| | 18 | UmUfUmGmUmCfUmGmUmAmUmGmAmAfUmGfAmAmGmAmGm |
| siC5a2-M1 | 19 | CmUmCmUmUmCmAmUmUfCfAfUmAmCmAmGmAmCmAmAmAm |
| | 20 | UmUfUmGmUmCfUmGmUmAmUmGmAmAfUmGfAmAmGmAmGmAmAm |
| siC5a2-M2 | 21 | CmUmCmUmUmCmAfUmUfCfAfUmAmCmAmGmAmCmAmAmAm |
| | 22 | UmUfUmGmUmCfUmGfUfAmUmGmAmAfUmGfAmAmGmAmGmAmAm |
| siC5a2-M3 | 23 | CmUmCmUmUmCmAfUmUfCfAfUmAmCmAmGmAmCmAmAmAm |
| | 24 | UmUfUmGmUmCfUmGmUmAmUmGmAmAfUmGfAmAmGmAmGmAmAm |
| siC5a1-M1S | 25 | CmsUmsUmCmAmUmUfCfAfUmAmCmAmGmAmCmAmAmAm |
| | 26 | UmsUfsUmGmUmCfUmGmUmAmUmGmAmAfUmGfAmAmGmsAmsGm |
| siC5a1-M2S | 27 | CmsUmsUmCmAfUmUfCfAfUmAmCmAmGmAmCmAmAmAm |
| | 28 | UmsUfsUmGmUmCfUmGfUfAmUmGmAmAfUmGfAmAmGmsAmsGm |
| siC5a1-M3S | 29 | CmsUmsUmCmAfUmUfCfAfUmAmCmAmGmAmCmAmAmAm |
| | 30 | UmsUfsUmGmUmCfUmGmUmAmUmGmAmAfUmGfAmAmGmsAmsGm |
| siC5a2-M1S | 31 | CmsUmsCmUmUmCmAmUmUfCfAfUmAmCmAmGmAmCmAmAmAm |
| | 32 | UmsUfsUmGmUmCfUmGmUmAmUmGmAmAfUmGfAmAmGmAmGmsAmsAm |

(continued)

| siRNA No. | SEQ ID NO: | Sequence direction 5'- 3' |
|---|---|---|
| siC5a2-M2S | 33 | CmsUmsCmUmUmCmAfUmUfCfAfUmAmCmAmGmAmCmAmAmAm |
| | 34 | UmsUfsUmGmUmCfUmGfUfAmUmGmAmAfUmGfAmAmGmAmGmsAmsAm |
| siC5a2-M3S | 35 | CmsUmsCmUmUmCmAfUmUfCfAfUmAmCmAmGmAmCmAmAmAm |
| | 36 | UmsUfsUmGmUmCfUmGmUmAmUmGmAmAfUmGfAmAmGmAmGmsAmsAm |
| siC5a1-M1P1 | 37 | CmUmUmCmAmUmUfCfAfUmAmCmAmGmAmCmAmAmAm |
| | 38 | P1UmUfUmGmUmCfUmGmUmAmUmGmAmAfUmGfAmAmGmAmGm |
| siC5a1-M2P1 | 39 | CmUmUmCmAfUmUfCfAfUmAmCmAmGmAmCmAmAmAm |
| | 40 | P1UmUfUmGmUmCfUmGfUfAmUmGmAmAfUmGfAmAmGmAmGm |
| siC5a1-M3P1 | 41 | CmUmUmCmAfUmUfCfAfUmAmCmAmGmAmCmAmAmAm |
| | 42 | P1UmUfUmGmUmCfUmGmUmAmUmGmAmAfUmGfAmAmGmAmGm |
| siC5a2-M1P1 | 43 | CmUmCmUmUmCmAmUmUfCfAfUmAmCmAmGmAmCmAmAmAm |
| | 44 | P1UmUfUmGmUmCfUmGmUmAmUmGmAmAfUmGfAmAmGmAmGmAmAm |
| siC5a2-M2P1 | 45 | CmUmCmUmUmCmAfUmUfCfAfUmAmCmAmGmAmCmAmAmAm |
| | 46 | P1UmUfUmGmUmCfUmGfUfAmUmGmAmAfUmGfAmAmGmAmGmAmAm |
| siC5a2-M3P1 | 47 | CmUmCmUmUmCmAfUmUfCfAfUmAmCmAmGmAmCmAmAmAm |
| | 48 | P1UmUfUmGmUmCfUmGmUmAmUmGmAmAfUmGfAmAmGmAmGmAmAm |
| siC5a1-M1SP1 | 49 | CmsUmsUmCmAmUmUfCfAfUmAmCmAmGmAmCmAmAmAm |
| | 50 | P1UmsUfsUmGmUmCfUmGmUmAmUmGmAmAfUmGfAmAmGmsAmsGm |
| siC5a1-M2SP1 | 51 | CmsUmsUmCmAfUmUfCfAfUmAmCmAmGmAmCmAmAmAm |
| | 52 | P1UmsUfsUmGmUmCfUmGfUfAmUmGmAmAfUmGfAmAmGmsAmsGm |

(continued)

| siRNA No. | SEQ ID NO: | Sequence direction 5'- 3' |
|---|---|---|
| siC5a1-M3SP1 | 53 | CmsUmsUmCmAfUmUfCfAfUmAmCmAmGmAmCmAmAmAm |
| | 54 | P1UmsUfsUmGmUmCfUmGmUmAmUmGmAmAfUmGfAmAmGmsAmsGm |
| siC5a2-M1SP1 | 55 | CmsUmsCmUmUmCmAmUmUfCfAfUmAmCmAmGmAmCmAmAmAm |
| | 56 | P1UmsUfsUmGmUmCfUmGmUmAmUmGmAmAfUmGfAmAmGmA |
| | | mGmsAmsAm |
| siC5a2-M2SP1 | 57 | CmsUmsCmUmUmCmAfUmUfCfAfUmAmCmAmGmAmCmAmAmAm |
| | 58 | P1UmsUfsUmGmUmCfUmGfUfAmUmGmAmAfUmGfAmAmGmAmGmsAmsAm |
| siC5a2-M3SP1 | 59 | CmsUmsCmUmUmCmAfUmUfCfAfUmAmCmAmGmAmCmAmAmAm |
| | 60 | P1UmsUfsUmGmUmCfUmGmUmAmUmGmAmAfUmGfAmAmGmAmGmsAmsAm |

Table 1b The second siRNA sequence of the present disclosure

| siRNA No. | SEQ ID NO: | Sequence direction 5'- 3' |
|---|---|---|
| siC5b1 | 69 | CUACAGUUUAGAAGAUUUA |
| | 70 | UAAAUCUUCUAAACUGUAGUA |
| siC5b2 | 71 | UACUACAGUUUAGAAGAUUUA |
| | 72 | UAAAUCUUCUAAACUGUAGUAUG |
| siC5b1-M1 | 73 | CmUmAmCmAmGmUfUfUfAmGmAmAmGmAmUmUmUmAm |
| | 74 | UmAfAmAmUmCfUmUmCmUmAmAmAmCfUmGfUmAmGmUmAm |
| siC5b1-M2 | 75 | CmUmAmCmAfGmUfUfUfAmGmAmAmGmAmUmUmUmAm |
| | 76 | UmAfAmAmUmCfUmUfCfUmAmAmAmCfUmGfUmAmGmUmAm |
| siC5b1-M3 | 77 | CmUmAmCmAfGmUfUfUfAmGmAmAmGmAmUmUmUmAm |
| | 78 | UmAfAmAmUmCfUmUmCmUmAmAmAmCfUmGfUmAmGmUmAm |
| siC5b2-M1 | 79 | UmAmCmUmAmCmAmGmUfUfUfAmGmAmAmGmAmUmUmUmAm |
| | 80 | UmAfAmAmUmCfUmUmCmUmAmAmAmCfUmGfUmAmGmUmAmUmGm |

(continued)

| siRNA No. | SEQ ID NO: | Sequence direction 5'- 3' |
|---|---|---|
| siC5b2-M2 | 81 | UmAmCmUmAmCmAfGmUfUfUfAmGmAmAmGmAmUmUmUmAm |
| | 82 | UmAfAmAmUmCfUmUfCfUmAmAmAmCfUmGfUmAmGmUmAmUmGm |
| siC5b2-M3 | 83 | UmAmCmUmAmCmAfGmUfUfUfAmGmAmAmGmAmUmUmUmAm |
| | 84 | UmAfAmAmUmCfUmUmCmUmAmAmAmCfUmGfUmAmGmUmAmUmGm |
| siC5b1-M1S | 85 | CmsUmsAmCmAmGmUfUfUfAmGmAmAmGmAmUmUmUmAm |
| | 86 | UmsAfsAmAmUmCfUmUmCmUmAmAmAmCfUmGfUmAmGmsUmsAm |
| siC5b1-M2S | 87 | CmsUmsAmCmAfGmUfUfUfAmGmAmAmGmAmUmUmUmAm |
| | 88 | UmsAfsAmAmUmCfUmUfCfUmAmAmAmCfUmGfUmAmGmsUmsAm |
| siC5b1-M3S | 89 | CmsUmsAmCmAfGmUfUfUfAmGmAmAmGmAmUmUmUmAm |
| | 90 | UmsAfsAmAmUmCfUmUmCmUmAmAmAmCfUmGfUmAmGmsUmsAm |
| siC5b2-M1S | 91 | UmsAmsCmUmAmCmAmGmUfUfUfAmGmAmAmGmAmUmUmUmAm |
| | 92 | UmsAfsAmAmUmCfUmUmCmUmAmAmAmCfUmGfUmAmGmUmAmsUmsGm |
| siC5b2-M2S | 93 | UmsAmsCmUmAmCmAfGmUfUfUfAmGmAmAmGmAmUmUmUmAm |
| | 94 | UmsAfsAmAmUmCfUmUfCfUmAmAmAmCfUmGfUmAmGmUmAmsUmsGm |
| siC5b2-M3S | 95 | UmsAmsCmUmAmCmAfGmUfUfUfAmGmAmAmGmAmUmUmUmAm |
| | 96 | UmsAfsAmAmUmCfUmUmCmUmAmAmAmCfUmGfUmAmGmUmAmsUmsGm |
| siC5b1-M1P1 | 97 | CmUmAmCmAmGmUfUfUfAmGmAmAmGmAmUmUmUmAm |
| | 98 | P1UmAfAmAmUmCfUmUmCmUmAmAmAmCfUmGfUmAmGmUmAm |
| siC5b1-M2P1 | 99 | CmUmAmCmAfGmUfUfUfAmGmAmAmGmAmUmUmUmAm |
| | 100 | P1UmAfAmAmUmCfUmUfCfUmAmAmAmCfUmGfUmAmGmUmAm |

(continued)

| siRNA No. | SEQ ID NO: | Sequence direction 5'- 3' |
|---|---|---|
| siC5b1-M3P1 | 101 | CmUmAmCmAfGmUfUfUfAmGmAmAmGmAmUmUmUmAm |
| | 102 | P1UmAfAmAmUmCfUmUmCmUmAmAmAmCfUmGfUmAmGmUmAm |
| siC5b2-M1P1 | 103 | UmAmCmUmAmCmAmGmUfUfUfAmGmAmAmGmAmUmUmUmAm |
| | 104 | P1UmAfAmAmUmCfUmUmCmUmAmAmAmCfUmGfUmAmGmUmAmUmGm |
| siC5b2-M2P1 | 105 | UmAmCmUmAmCmAfGmUfUfUfAmGmAmAmGmAmUmUmUmAm |
| | 106 | P1UmAfAmAmUmCfUmUfCfUmAmAmAmCfUmGfUmAmGmUmAmUmGm |
| siC5b2-M3P1 | 107 | UmAmCmUmAmCmAfGmUfUfUfAmGmAmAmGmAmUmUmUmAm |
| | 108 | P1UmAfAmAmUmCfUmUmCmUmAmAmAmCfUmGfUmAmGmUmAmUmGm |
| siC5b1-M1SP 1 | 109 | CmsUmsAmCmAmGmUfUfUfAmGmAmAmGmAmUmUmUmAm |
| | 110 | P1UmsAfsAmAmUmCfUmUmCmUmAmAmAmCfUmGfUmAmGmsUmsAm |
| siC5b1-M2SP 1 | 111 | CmsUmsAmCmAfGmUfUfUfAmGmAmAmGmAmUmUmUmAm |
| | 112 | P1UmsAfsAmAmUmCfUmUfCfUmAmAmAmCfUmGfUmAmGmsUmsAm |
| siC5b1-M3SP 1 | 113 | CmsUmsAmCmAfGmUfUfUfAmGmAmAmGmAmUmUmUmAm |
| | 114 | P1UmsAfsAmAmUmCfUmUmCmUmAmAmAmCfUmGfUmAmGmsUmsAm |
| siC5b2-M1SP 1 | 115 | UmsAmsCmUmAmCmAmGmUfUfUfAmGmAmAmGmAmUmUmUmAm |
| | 116 | P1UmsAfsAmAmUmCfUmUmCmUmAmAmAmCfUmGfUmAmGmUmAmsUmsGm |
| siC5b2-M2SP 1 | 117 | UmsAmsCmUmAmCmAfGmUfUfUfAmGmAmAmGmAmUmUmUmAm |
| | 118 | P1UmsAfsAmAmUmCfUmUfCfUmAmAmAmCfUmGfUmAmGmUmAmsUmsGm |

(continued)

| siRNA No. | SEQ ID NO: | Sequence direction 5'- 3' |
|---|---|---|
| siC5b2-M3SP 1 | 119 | UmsAmsCmUmAmCmAfGmUfUfUfAmGmAmAmGmAmUmUmUmAm |
| | 120 | P1UmsAfsAmAmUmCfUmUmCmUmAmAmAmCfUmGfUmAmGmUmAmsUmsGm |

Table 1c The third siRNA sequence of the present disclosure

| siRNA No. | SEQ ID NO: | Sequence direction5' - 3' |
|---|---|---|
| siC5c1 | 129 | GGAAGGUUACCGAGCAAUA |
| | 130 | UAUUGCUCGGUAACCUUCCCU |
| siC5c2 | 131 | AGGGAAGGUUACCGAGCAAUA |
| | 132 | UAUUGCUCGGUAACCUUCCCUGG |
| siC5c1-M1 | 133 | GmGmAmAmGmGmUfUfAfCmCmGmAmGmCmAmAmUmAm |
| | 134 | UmAfUmUmGmCfUmCmGmGmUmAmAmCfCmUfUmCmCmCmUm |
| siC5c1-M2 | 135 | GmGmAmAmGfGmUfUfAfCmCmGmAmGmCmAmAmUmAm |
| | 136 | UmAfUmUmGmCfUmCfGfGmUmAmAmCfCmUfUmCmCmCmUm |
| siC5c1-M3 | 137 | GmGmAmAmGfGmUfUfAfCmCmGmAmGmCmAmAmUmAm |
| | 138 | UmAfUmUmGmCfUmCmGmGmUmAmAmCfCmUfUmCmCmCmUm |
| siC5c2-M1 | 139 | AmGmGmGmAmAmGmGmUfUfAfCmCmGmAmGmCmAmAmUmAm |
| | 140 | UmAfUmUmGmCfUmCmGmGmUmAmAmCfCmUfUmCmCmCmUmGmGm |
| siC5c2-M2 | 141 | AmGmGmGmAmAmGfGmUfUfAfCmCmGmAmGmCmAmAmUmAm |
| | 142 | UmAfUmUmGmCfUmCfGfGmUmAmAmCfCmUfUmCmCmCmUmGmGm |
| siC5c2-M3 | 143 | AmGmGmGmAmAmGfGmUfUfAfCmCmGmAmGmCmAmAmUmAm |
| | 144 | UmAfUmUmGmCfUmCmGmGmUmAmAmCfCmUfUmCmCmCmUmGmGm |
| siC5c1-M1S | 145 | GmsGmsAmAmGmGmUfUfAfCmCmGmAmGmCmAmAmUmAm |
| | 146 | UmsAfsUmUmGmCfUmCmGmGmUmAmAmCfCmUfUmCmCmsCmsUm |
| siC5c1-M2S | 147 | GmsGmsAmAmGfGmUfUfAfCmCmGmAmGmCmAmAmUmAm |
| | 148 | UmsAfsUmUmGmCfUmCfGfGmUmAmAmCfCmUfUmCmCmsCmsUm |

(continued)

| siRNA No. | SEQ ID NO: | Sequence direction5' - 3' |
|---|---|---|
| siC5c1-M3 S | 149 | GmsGmsAmAmGfGmUfUfAfCmCmGmAmGmCmAmAmUmAm |
| | 150 | UmsAfsUmUmGmCfUmCmGmGmUmAmAmCfCmUfUmCmCmsCmsUm |
| siC5c2-M1 S | 151 | AmsGmsGmGmAmAmGmGmUfUfAfCmCmGmAmGmCmAmAmUmAm |
| | 152 | UmsAfsUmUmGmCfUmCmGmGmUmAmAmCfCmUfUmCmCmCmUmsGmsGm |
| siC5c2-M2S | 153 | AmsGmsGmGmAmAmGfGmUfUfAfCmCmGmAmGmCmAmAmUmAm |
| | 154 | UmsAfsUmUmGmCfUmCfGfGmUmAmAmCfCmUfUmCmCmCmUmsGmsGm |
| siC5c2-M3S | 155 | AmsGmsGmGmAmAmGfGmUfUfAfCmCmGmAmGmCmAmAmUmAm |
| | 156 | UmsAfsUmUmGmCfUmCmGmGmUmAmAmCfCmUfUmCmCmCmUmsGmsGm |
| siC5c1-M1P1 | 157 | GmGmAmAmGmGmUfUfAfCmCmGmAmGmCmAmAmUmAm |
| | 158 | P1UmAfUmUmGmCfUmCmGmGmUmAmAmCfCmUfUmCmCmCmUm |
| siC5c1-M2P1 | 159 | GmGmAmAmGfGmUfUfAfCmCmGmAmGmCmAmAmUmAm |
| | 160 | P1UmAfUmUmGmCfUmCfGfGmUmAmAmCfCmUfUmCmCmCmUm |
| siC5c1-M3P1 | 161 | GmGmAmAmGfGmUfUfAfCmCmGmAmGmCmAmAmUmAm |
| | 162 | P1UmAfUmUmGmCfUmCmGmGmUmAmAmCfCmUfUmCmCmCmUm |
| siC5c2-M1P1 | 163 | AmGmGmGmAmAmGmGmUfUfAfCmCmGmAmGmCmAmAmUmAm |
| | 164 | P1UmAfUmUmGmCfUmCmGmGmUmAmAmCfCmUfUmCmCmCmUmGmGm |
| siC5c2-M2P1 | 165 | AmGmGmGmAmAmGfGmUfUfAfCmCmGmAmGmCmAmAmUmAm |
| | 166 | P1UmAfUmUmGmCfUmCfGfGmUmAmAmCfCmUfUmCmCmCmUmGmGm |
| siC5c2-M3P1 | 167 | AmGmGmGmAmAmGfGmUfUfAfCmCmGmAmGmCmAmAmUmAm |
| | 168 | P1UmAfUmUmGmCfUmCmGmGmUmAmAmCfCmUfUmCmCmCmUmGmGm |

(continued)

| siRNA No. | SEQ ID NO: | Sequence direction5' - 3' |
|---|---|---|
| siC5c1-M1SP 1 | 169 | GmsGmsAmAmGmGmUfUfAfCmCmGmAmGmCmAmAmUmAm |
| | 170 | P1UmsAfsUmUmGmCfUmCmGmGmUmAmAmCfCmUfUmCmCmsCmsUm |
| siC5c1-M2SP 1 | 171 | GmsGmsAmAmGfGmUfUfAfCmCmGmAmGmCmAmAmUmAm |
| | 172 | P1UmsAfsUmUmGmCfUmCfGfGmUmAmAmCfCmUfUmCmCmsCmsUm |
| siC5c1-M3SP 1 | 173 | GmsGmsAmAmGfGmUfUfAfCmCmGmAmGmCmAmAmUmAm |
| | 174 | P1UmsAfsUmUmGmCfUmCmGmGmUmAmAmCfCmUfUmCmCmsCmsUm |
| siC5c2-M1SP 1 | 175 | AmsGmsGmGmAmAmGmGmUfUfAfCmCmGmAmGmCmAmAmUmAm |
| | 176 | P1UmsAfsUmUmGmCfUmCmGmGmUmAmAmCfCmUfUmCmCmCmUmsGmsGm |
| siC5c2-M2SP 1 | 177 | AmsGmsGmGmAmAmGfGmUfUfAfCmCmGmAmGmCmAmAmUmAm |
| | 178 | P1UmsAfsUmUmGmCfUmCfGfGmUmAmAmCfCmUfUmCmCmCmUmsGmsGm |
| siC5c2-M3SP 1 | 179 | AmsGmsGmGmAmAmGfGmUfUfAfCmCmGmAmGmCmAmAmUmAm |
| | 180 | P1UmsAfsUmUmGmCfUmCmGmGmUmAmAmCfCmUfUmCmCmCmUmsGmsGm |

Table Id The fourth siRNA sequence of the present disclosure

| siRNA No. | SEQ ID NO: | Sequence direction5' - 3' |
|---|---|---|
| siC5d1 | 189 | AGAACAGACAGCAGAAUUA |
| | 190 | UAAUUCUGCUGUCUGUUCUCC |
| siC5d2 | 191 | GGAGAACAGACAGCAGAAUUA |
| | 192 | UAAUUCUGCUGUCUGUUCUCCUG |
| siC5d1-M1 | 193 | AmGmAmAmCmAmGfAfCfAmGmCmAmGmAmAmUmUmAm |
| | 194 | UmAfAmUmUmCfUmGmCmUmGmUmCmUfGmUfUmCmUmCmCm |
| siC5d1-M2 | 195 | AmGmAmAmCfAmGfAfCfAmGmCmAmGmAmAmUmUmAm |
| | 196 | UmAfAmUmUmCfUmGfCfUmGmUmCmUfGmUfUmCmUmCmCm |

(continued)

| siRNA No. | SEQ ID NO: | Sequence direction5' - 3' |
|---|---|---|
| siC5d1-M3 | 197 | AmGmAmAmCfAmGfAfCfAmGmCmAmGmAmAmUmUmAm |
| | 198 | UmAfAmUmUmCfUmGmCmUmGmUmCmUfGmUfUmCmUmCmCm |
| siC5d2-M1 | 199 | GmGmAmGmAmAmCmAmGfAfCfAmGmCmAmGmAmAmUmUmA |
| | | m |
| | 200 | UmAfAmUmUmCfUmGmCmUmGmUmCmUfGmUfUmCmUmCmCmUmGm |
| siC5d2-M2 | 201 | GmGmAmGmAmAmCfAmGfAfCfAmGmCmAmGmAmAmUmUmAm |
| | 202 | UmAfAmUmUmCfUmGfCfUmGmUmCmUfGmUfUmCmUmCmCmUmGm |
| siC5d2-M3 | 203 | GmGmAmGmAmAmCfAmGfAfCfAmGmCmAmGmAmAmUmUmAm |
| | 204 | UmAfAmUmUmCfUmGmCmUmGmUmCmUfGmUfUmCmUmCmCmUmGm |
| siC5d1-M1S | 205 | AmsGmsAmAmCmAmGfAfCfAmGmCmAmGmAmAmUmUmAm |
| | 206 | UmsAfsAmUmUmCfUmGmCmUmGmUmCmUfGmUfUmCmUmsCmsCm |
| siC5d1-M2S | 207 | AmsGmsAmAmCfAmGfAfCfAmGmCmAmGmAmAmUmUmAm |
| | 208 | UmsAfsAmUmUmCfUmGfCfUmGmUmCmUfGmUfUmCmUmsCmsCm |
| siC5d1-M3S | 209 | AmsGmsAmAmCfAmGfAfCfAmGmCmAmGmAmAmUmUmAm |
| | 210 | UmsAfsAmUmUmCfUmGmCmUmGmUmCmUfGmUfUmCmUmsCmsCm |
| siC5d2-M1 S | 211 | GmsGmsAmGmAmAmCmAmGfAfCfAmGmCmAmGmAmAmUmUmAm |
| | 212 | UmsAfsAmUmUmCfUmGmCmUmGmUmCmUfGmUfUmCmUmCmCmsUmsGm |
| siC5d2-M2S | 213 | GmsGmsAmGmAmAmCfAmGfAfCfAmGmCmAmGmAmAmUmUmAm |
| | 214 | UmsAfsAmUmUmCfUmGfCfUmGmUmCmUfGmUfUmCmUmCmCmsUmsGm |
| siC5d2-M3S | 215 | GmsGmsAmGmAmAmCfAmGfAfCfAmGmCmAmGmAmAmUmUmAm |

(continued)

| siRNA No. | SEQ ID NO: | Sequence direction5' - 3' |
|---|---|---|
| | 216 | UmsAfsAmUmUmCfUmGmCmUmGmUmCmUfGmUfUmCmUmCmCmsUmsGm |
| siC5d1-M1P1 | 217 | AmGmAmAmCmAmGfAfCfAmGmCmAmGmAmAmUmUmAm |
| | 218 | P1UmAfAmUmUmCfUmGmCmUmGmUmCmUfGmUfUmCmUmCmCm |
| siC5d1-M2P1 | 219 | AmGmAmAmCfAmGfAfCfAmGmCmAmGmAmAmUmUmAm |
| | 220 | P1UmAfAmUmUmCfUmGfCfUmGmUmCmUfGmUfUmCmUmCmCm |
| siC5d1-M3P1 | 221 | AmGmAmAmCfAmGfAfCfAmGmCmAmGmAmAmUmUmAm |
| | 222 | P1UmAfAmUmUmCfUmGmCmUmGmUmCmUfGmUfUmCmUmCmCm |
| siC5d2-M1P1 | 223 | GmGmAmGmAmAmCmAmGfAfCfAmGmCmAmGmAmAmUmUmAm |
| | 224 | P1UmAfAmUmUmCfUmGmCmUmGmUmCmUfGmUfUmCmUmCmCmUmGm |
| siC5d2-M2P1 | 225 | GmGmAmGmAmAmCfAmGfAfCfAmGmCmAmGmAmAmUmUmAm |
| | 226 | P1UmAfAmUmUmCfUmGfCfUmGmUmCmUfGmUfUmCmUmCmCmUmGm |
| siC5d2-M3P1 | 227 | GmGmAmGmAmAmCfAmGfAfCfAmGmCmAmGmAmAmUmUmAm |
| | 228 | P1UmAfAmUmUmCfUmGmCmUmGmUmCmUfGmUfUmCmUmCmCmUmGm |
| siC5d1-M1SP1 | 229 | AmsGmsAmAmCmAmGfAfCfAmGmCmAmGmAmAmUmUmAm |
| | 230 | P1UmsAfsAmUmUmCfUmGmCmUmGmUmCmUfGmUfUmCmUmsCmsCm |
| siC5d1-M2SP1 | 231 | AmsGmsAmAmCfAmGfAfCfAmGmCmAmGmAmAmUmUmAm |
| | 232 | P1UmsAfsAmUmUmCfUmGfCfUmGmUmCmUfGmUfUmCmUmsCmsCm |
| siC5d1-M3SP1 | 233 | AmsGmsAmAmCfAmGfAfCfAmGmCmAmGmAmAmUmUmAm |
| | 234 | P1UmsAfsAmUmUmCfUmGmCmUmGmUmCmUfGmUfUmCmUmsCmsCm |

(continued)

| siRNA No. | SEQ ID NO: | Sequence direction5' - 3' |
|---|---|---|
| siC5d2-M1SP1 | 235 | GmsGmsAmGmAmAmCmAmGfAfCfAmGmCmAmGmAmAmUmUmAm |
| | 236 | P1UmsAfsAmUmUmCfUmGmCmUmGmUmCmUfGmUfUmCmUmCmCmsUmsGm |
| siC5d2-M2SP1 | 237 | GmsGmsAmGmAmAmCfAmGfAfCfAmGmCmAmGmAmAmUmUmAm |
| | 238 | P1UmsAfsAmUmUmCfUmGfCfUmGmUmCmUfGmUfUmCmUmCmCmsUmsGm |
| siC5d2-M3SP1 | 239 | GmsGmsAmGmAmAmCfAmGfAfCfAmGmCmAmGmAmAmUmUmAm |
| | 240 | P1UmsAfsAmUmUmCfUmGmCmUmGmUmCmUfGmUfUmCmUmCmCmsUmsGm |

Table 1e The fifth siRNA sequence of the present disclosure

| siRNA No. | SEQ ID NO: | Sequence direction5' - 3' |
|---|---|---|
| siC5e1 | 249 | CCAAGAAGAACGCUGCAAA |
| | 250 | UUUGCAGCGUUCUUCUUGGCC |
| siC5e2 | 251 | GGCCAAGAAGAACGCUGCAAA |
| | 252 | UUUGCAGCGUUCUUCUUGGCCUG |
| siC5e1-M1 | 253 | CmCmAmAmGmAmAfGfAfAmCmGmCmUmGmCmAmAmAm |
| | 254 | UmUfUmGmCmAfGmCmGmUmUmCmUmUfCmUfUmGmGmCmCm |
| siC5e1-M2 | 255 | CmCmAmAmGfAmAfGfAfAmCmGmCmUmGmCmAmAmAm |
| | 256 | UmUfUmGmCmAfGmCfGfUmUmCmUmUfCmUfUmGmGmCmCm |
| siC5e1-M3 | 257 | CmCmAmAmGfAmAfGfAfAmCmGmCmUmGmCmAmAmAm |
| | 258 | UmUfUmGmCmAfGmCmGmUmUmCmUmUfCmUfUmGmGmCmCm |
| siC5e2-M1 | 259 | GmGmCmCmAmAmGmAmAfGfAfAmCmGmCmUmGmCmAmAmAm |
| | 260 | UmUfUmGmCmAfGmCmGmUmUmCmUmUfCmUfUmGmGmCmCmUmGm |
| siC5e2-M2 | 261 | GmGmCmCmAmAmGfAmAfGfAfAmCmGmCmUmGmCmAmAmAm |
| | 262 | UmUfUmGmCmAfGmCfGfUmUmCmUmUfCmUfUmGmGmCmCmUmGm |

(continued)

| siRNA No. | SEQ ID NO: | Sequence direction5' - 3' |
|---|---|---|
| siC5e2-M3 | 263 | GmGmCmCmAmAmGfAmAfGfAfAmCmGmCmUmGmCmAmAmAm |
| | 264 | UmUfUmGmCmAfGmCmGmUmUmCmUmUfCmUfUmGmGmCmCmUmGm |
| siC5e1-M1 S | 265 | CmsCmsAmAmGmAmAfGfAfAmCmGmCmUmGmCmAmAmAm |
| | 266 | UmsUfsUmGmCmAfGmCmGmUmUmCmUmUfCmUfUmGmGmsCmsCm |
| siC5e1-M2S | 267 | CmsCmsAmAmGfAmAfGfAfAmCmGmCmUmGmCmAmAmAm |
| | 268 | UmsUfsUmGmCmAfGmCfGfUmUmCmUmUfCmUfUmGmGmsCmsCm |
| siC5e1-M3S | 269 | CmsCmsAmAmGfAmAfGfAfAmCmGmCmUmGmCmAmAmAm |
| | 270 | UmsUfsUmGmCmAfGmCmGmUmUmCmUmUfCmUfUmGmGmsCmsCm |
| siC5e2-MI S | 271 | GmsGmsCmCmAmAmGmAmAfGfAfAmCmGmCmUmGmCmAmAmAm |
| | 272 | UmsUfsUmGmCmAfGmCmGmUmUmCmUmUfCmUfUmGmGmCmCmsUmsGm |
| siC5e2-M2S | 273 | GmsGmsCmCmAmAmGfAmAfGfAfAmCmGmCmUmGmCmAmAmAm |
| | 274 | UmsUfsUmGmCmAfGmCfGfUmUmCmUmUfCmUfUmGmGmCmCmsUmsGm |
| siC5e2-M3S | 275 | GmsGmsCmCmAmAmGfAmAfGfAfAmCmGmCmUmGmCmAmAmAm |
| | 276 | UmsUfsUmGmCmAfGmCmGmUmUmCmUmUfCmUfUmGmGmCmCmsUmsGm |
| siC5e1-M1P1 | 277 | CmCmAmAmGmAmAfGfAfAmCmGmCmUmGmCmAmAmAm |
| | 278 | P1UmUfUmGmCmAfGmCmGmUmUmCmUmUfCmUfUmGmGmCmCm |
| siC5e1-M2P1 | 279 | CmCmAmAmGfAmAfGfAfAmCmGmCmUmGmCmAmAmAm |
| | 280 | P1UmUfUmGmCmAfGmCfGfUmUmCmUmUfCmUfUmGmGmCmCm |
| siC5e1-M3P1 | 281 | CmCmAmAmGfAmAfGfAfAmCmGmCmUmGmCmAmAmAm |
| | 282 | P1UmUfUmGmCmAfGmCmGmUmUmCmUmUfCmUfUmGmGmCmCm |

(continued)

| siRNA No. | SEQ ID NO: | Sequence direction5' - 3' |
|---|---|---|
| siC5e2-M1P1 | 283 | GmGmCmCmAmAmGmAmAfGfAfAmCmGmCmUmGmCmAmAmAm |
| | 284 | P1UmUfUmGmCmAfGmCmGmUmUmCmUmUfCmUfUmGmGmCmCmUmGm |
| siC5e2-M2P1 | 285 | GmGmCmCmAmAmGfAmAfGfAfAmCmGmCmUmGmCmAmAmAm |
| | 286 | P1UmUfUmGmCmAfGmCfGfUmUmCmUmUfCmUfUmGmGmCmCmUmGm |
| siC5e2-M3P1 | 287 | GmGmCmCmAmAmGfAmAfGfAfAmCmGmCmUmGmCmAmAmAm |
| | 288 | P1UmUfUmGmCmAfGmCmGmUmUmCmUmUfCmUfUmGmGmCmCmUmGm |
| siC5e1-M1SP1 | 289 | CmsCmsAmAmGmAmAfGfAfAmCmGmCmUmGmCmAmAmAm |
| | 290 | P1UmsUfsUmGmCmAfGmCmGmUmUmCmUmUfCmUfUmGmGmsCmsCm |
| siC5e1-M2SP1 | 291 | CmsCmsAmAmGfAmAfGfAfAmCmGmCmUmGmCmAmAmAm |
| | 292 | P1UmsUfsUmGmCmAfGmCfGfUmUmCmUmUfCmUfUmGmGmsCmsCm |
| siC5e1-M3SP1 | 293 | CmsCmsAmAmGfAmAfGfAfAmCmGmCmUmGmCmAmAmAm |
| | 294 | P1UmsUfsUmGmCmAfGmCmGmUmUmCmUmUfCmUfUmGmGmsCmsCm |
| siC5e2-M1SP1 | 295 | GmsGmsCmCmAmAmGmAmAfGfAfAmCmGmCmUmGmCmAmAmAm |
| | 296 | P1UmsUfsUmGmCmAfGmCmGmUmUmCmUmUfCmUfUmGmGmCmCmsUmsGm |
| siC5e2-M2SP1 | 297 | GmsGmsCmCmAmAmGfAmAfGfAfAmCmGmCmUmGmCmAmAmAm |
| | 298 | P1UmsUfsUmGmCmAfGmCfGfUmUmCmUmUfCmUfUmGmGmCmCmsUmsGm |
| siC5e2-M3SP1 | 299 | GmsGmsCmCmAmAmGfAmAfGfAfAmCmGmCmUmGmCmAmAmAm |
| | 300 | P1UmsUfsUmGmCmAfGmCmGmUmUmCmUmUfCmUfUmGmGmCmCmsUmsGm |

Table If The sixth siRNA sequence of the present disclosure

| siRNA No. | SEQ ID NO: | Sequence direction5' - 3' |
|---|---|---|
| siC5f1 | 309 | CCAGUAAGCAAGCCAGAAA |
| | 310 | UUUCUGGCUUGCUUACUGGUA |
| siC5f2 | 311 | UACCAGUAAGCAAGCCAGAAA |
| | 312 | UUUCUGGCUUGCUUACUGGUAAC |
| siC5f1-M1 | 313 | CmCmAmGmUmAmAfGfCfAmAmGmCmCmAmGmAmAmAm |
| | 314 | UmUfUmCmUmGfGmCmUmUmGmCmUmUfAmCfUmGmGmUmAm |
| siC5f1-M2 | 315 | CmCmAmGmUfAmAfGfCfAmAmGmCmCmAmGmAmAmAm |
| | 316 | UmUfUmCmUmGfGmCfUfUmGmCmUmUfAmCfUmGmGmUmAm |
| siC5f1-M3 | 317 | CmCmAmGmUfAmAfGfCfAmAmGmCmCmAmGmAmAmAm |
| | 318 | UmUfUmCmUmGfGmCmUmUmGmCmUmUfAmCfUmGmGmUmAm |
| siC5f2-M1 | 319 | UmAmCmCmAmGmUmAmAfGfCfAmAmGmCmCmAmGmAmAmAm |
| | 320 | UmUfUmCmUmGfGmCmUmUmGmCmUmUfAmCfUmGmGmUmAmAmCm |
| siC5f2-M2 | 321 | UmAmCmCmAmGmUfAmAfGfCfAmAmGmCmCmAmGmAmAmAm |
| | 322 | UmUfUmCmUmGfGmCfUfUmGmCmUmUfAmCfUmGmGmUmAmAmCm |
| siC5f2-M3 | 323 | UmAmCmCmAmGmUfAmAfGfCfAmAmGmCmCmAmGmAmAmAm |
| | 324 | UmUfUmCmUmGfGmCmUmUmGmCmUmUfAmCfUmGmGmUmAmAmCm |
| siC5f1-M1S | 325 | CmsCmsAmGmUmAmAfGfCfAmAmGmCmCmAmGmAmAmAm |
| | 326 | UmsUfsUmCmUmGfGmCmUmUmGmCmUmUfAmCfUmGmGmsUmsAm |

(continued)

| siRNA No. | SEQ ID NO: | Sequence direction5' - 3' |
|---|---|---|
| siC5f1-M2S | 327 | CmsCmsAmGmUfAmAfGfCfAmAmGmCmCmAmGmAmAmAm |
| | 328 | UmsUfsUmCmUmGfGmCfUfUmGmCmUmUfAmCfUmGmGmsUmsAm |
| siC5f1-M3S | 329 | CmsCmsAmGmUfAmAfGfCfAmAmGmCmCmAmGmAmAmAm |
| | 330 | UmsUfsUmCmUmGfGmCmUmUmGmCmUmUfAmCfUmGmGmsUmsAm |
| siC5f2-M1S | 331 | UmsAmsCmCmAmGmUmAmAfGfCfAmAmGmCmCmAmGmAmAmAm |
| | 332 | UmsUfsUmCmUmGfGmCmUmUmGmCmUmUfAmCfUmGmGmUmAmsAmsCm |
| siC5f2M2S | 333 | UmsAmsCmCmAmGmUfAmAfGfCfAmAmGmCmCmAmGmAmAmAm |
| | 334 | UmsUfsUmCmUmGfGmCfUfUmGmCmUmUfAmCfUmGmGmUmAmsAmsCm |
| siC5f2-M3S | 335 | UmsAmsCmCmAmGmUfAmAfGfCfAmAmGmCmCmAmGmAmAmAm |
| | 336 | UmsUfsUmCmUmGfGmCmUmUmGmCmUmUfAmCfUmGmGmUmAmsAmsCm |
| siC5f1-M1P1 | 337 | CmCmAmGmUmAmAfGfCfAmAmGmCmCmAmGmAmAmAm |
| | 338 | P1UmUfUmCmUmGfGmCmUmUmGmCmUmUfAmCfUmGmGmUmAm |

(continued)

| siRNA No. | SEQ ID NO: | Sequence direction5' - 3' |
|---|---|---|
| siC5f1-M2P1 | 339 | CmCmAmGmUfAmAfGfCfAmAmGmCmCmAmGmAmAmAm |
| | 340 | P1UmUfUmCmUmGfGmCfUfUmGmCmUmUfAmCfUmGmGmUmAm |
| siC5f1-M3P1 | 341 | CmCmAmGmUfAmAfGfCfAmAmGmCmCmAmGmAmAmAm |
| | 342 | P1UmUfUmCmUmGfGmCmUmUmGmCmUmUfAmCfUmGmGmUmAm |
| siC5f2-M1P1 | 343 | UmAmCmCmAmGmUmAmAfGfCfAmAmGmCmCmAmGmAmAmAm |
| | 344 | P1UmUfUmCmUmGfGmCmUmUmGmCmUmUfAmCfUmGmGmUmAmAmCm |
| siC5f2-M2P1 | 345 | UmAmCmCmAmGmUfAmAfGfCfAmAmGmCmCmAmGmAmAmAm |
| | 346 | P1UmUfUmCmUmGfGmCfUfUmGmCmUmUfAmCfUmGmGmUmAmAmCm |
| siC5f2-M3P1 | 347 | UmAmCmCmAmGmUfAmAfGfCfAmAmGmCmCmAmGmAmAmAm |
| | 348 | P1UmUfUmCmUmGfGmCmUmUmGmCmUmUfAmCfUmGmGmUmAmAmCm |
| siC5f1-M1SP1 | 349 | CmsCmsAmGmUmAmAfGfCfAmAmGmCmCmAmGmAmAmAm |
| | 350 | P1UmsUfsUmCmUmGfGmCmUmUmGmCmUmUfAmCfUmGmGmsUmsAm |
| siC5f1-M2SP1 | 351 | CmsCmsAmGmUfAmAfGfCfAmAmGmCmCmAmGmAmAmAm |
| | 352 | P1UmsUfsUmCmUmGfGmCfUfUmGmCmUmUfAmCfUmGmGmsUmsAm |

(continued)

| siRNA No. | SEQ ID NO: | Sequence direction5' - 3' |
|---|---|---|
| siC5f1-M3SP1 | 353 | CmsCmsAmGmUfAmAfGfCfAmAmGmCmCmAmGmAmAmAm |
| | 354 | P1UmsUfsUmCmUmGfGmCmUmUmGmCmUmUfAmCfUmGmGmsUmsAm |
| siC5f2-M1SP1 | 355 | UmsAmsCmCmAmGmUmAmAfGfCfAmAmGmCmCmAmGmAmAmAm |
| | 356 | P1UmsUfsUmCmUmGfGmCmUmUmGmCmUmUfAmCfUmGmGmUmAmsAmsCm |
| siC5f2-M2SP1 | 357 | UmsAmsCmCmAmGmUfAmAfGfCfAmAmGmCmCmAmGmAmAmAm |
| | 358 | P1UmsUfsUmCmUmGfGmCfUfUmGmCmUmUfAmCfUmGmGmUmAmsAmsCm |
| siC5f2-M3SP1 | 359 | UmsAmsCmCmAmGmUfAmAfGfCfAmAmGmCmCmAmGmAmAmAm |
| | 360 | P1UmsUfsUmCmUmGfGmCmUmUmGmCmUmUfAmCfUmGmGmUmAmsAmsCm |

In the siRNA or the siRNA conjugate of the present disclosure, each pair of adjacent nucleotides is linked via a phosphodiester bond or phosphorothioate diester bond. The non-bridging oxygen atom or sulfur atom in the phosphodiester bond or phosphorothioate diester bond is negatively charged, and may be present in the form of hydroxy or sulfhydryl. Moreover, the hydrogen ion in the hydroxy or sulfhydryl may be partially or completely substituted with a cation. The cation may be any cation, such as a metal cation, an ammonium ion $NH4^+$ or an organic ammonium cation. In order to increase solubility, in some embodiments, the cation is selected from one or more of an alkali metal ion, an ammonium cation formed by a tertiary amine and a quaternary ammonium cation. The alkali metal ion may be $K^+$ and/or $Na^+$, and the cation formed by the tertiary amine may be an ammonium ion formed by triethylamine and/or an ammonium ion formed by N,N-diisopropylethylamine. Thus, the siRNA or siRNA conjugate of the present disclosure may be at least partially present in the form of salt. In one embodiment, the non-bridging oxygen atom or sulfur atom in the phosphodiester bond or phosphorothioate diester bond at least partly binds to a sodium ion, and thus the siRNA or the siRNA conjugate of the present disclosure is present or partially present in the form of sodium salt.

Those skilled in the art clearly know that a modified nucleotide group may be introduced into the siRNA of the present disclosure by a nucleoside monomer having a corresponding modification. The methods for preparing the nucleoside monomer having the corresponding modification and the methods for introducing the modified nucleotide group into the siRNA are also well-known to those skilled in the art. All the modified nucleoside monomers may be either commercially available or prepared by known methods.

Preparation of the siRNA conjugate as shown by Formula (308)

The siRNA conjugate as shown by Formula (308) may be prepared by any appropriate synthetic routes.

In some embodiments, the siRNA conjugate as shown by Formula (308) may be prepared by the following method. The method comprises: successively linking nucleoside monomers in the direction from 3' to 5' according to the nucleotide types and sequences in the sense strand and antisense strand respectively under the condition of solid phase phosphoramidite synthesis, wherein the step of linking each nucleoside monomer comprises a four-step reaction of deprotection, coupling, capping, and oxidation or sulfurization; isolating the sense strand and the antisense strand of the siRNA; and annealing; wherein the siRNA is the siRNA of the present disclosure mentioned above.

Moreover, the method further comprises: contacting the compound as shown by Formula (321) with a nucleoside monomer or a nucleotide sequence linked to a solid phase support under coupling reaction condition and in the presence of a coupling agent, thereby linking the compound as shown by Formula (321) to the nucleotide sequence through a coupling reaction. Hereinafter, the compound as shown by Formula (321) is also called a conjugating molecule.

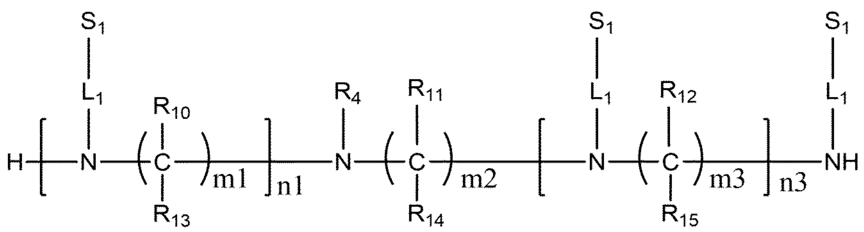

Formula (321)

wherein:

$R_4$ is a group capable of binding to the siRNA represented by Nu in the compound as shown by Formula (308). In some embodiments, $R_4$ is a group capable of binding to the siRNA represented by Nu via a covalent bond. In some embodiments, $R_4$ is a group capable of being conjugated to any functional group of the siRNA represented by Nu via a phosphodiester bond by reaction;

each Si is independently an Mi, which is a group formed by substituting all active hydroxy with a YCOO- group, wherein each Y is independently selected from one of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl. In some embodiments, Y is a methyl.

Definitions and options of n1, n3, m1, m2, m3, $R_{10}$, $R_{11}$, $R_{12}$, $R_{13}$, $R_{14}$, $R_{15}$, $L_1$, and $M_1$ are respectively as described above.

$R_4$ is selected to achieve the linkage to the N atom on a nitrogenous backbone and to provide a suitable reaction site for synthesizing the siRNA conjugate as shown by Formula (308). In some embodiments, $R_4$ comprises a $R_2$ linking group or a protected $R_2$ linking group, and can form a functional group as shown by Formula (A59) with an siRNA via reaction.

In some embodiments, R4 comprises a first functional group that can react with a group on an siRNA or a nucleoside monomer represented by Nu to form a phosphite ester, and a second functional group that can form a covalent bond with a hydroxy or an amino, or comprises a solid phase support linked via the covalent bond. In some embodiments, the first functional group is a phosphoramidite, a hydroxy or a protected hydroxy. In some embodiments, the second functional group is a phosphoramidite, a carboxyl or a carboxylate. In some embodiments, the second functional group is a solid phase support linked to the rest of the molecule via a covalent bond which is formed by a hydroxy or an amino. In some embodiments, the solid phase support is linked via a phosphoester bond, a carboxyl ester bond, or an amide bond. In some embodiments, the solid phase support is a resin.

In some embodiments, the first functional group comprises a hydroxy, $-OR_k$ or a group as shown by Formula (C3); and the second functional group comprises a group as shown by Formula (C1), (C2), (C3), (C1'), or (C3'):

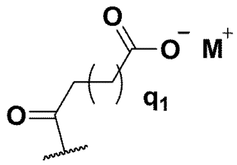
,
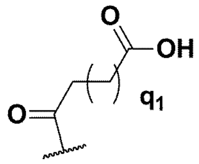
,
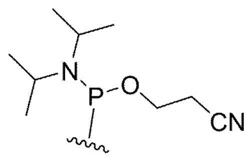
,

(C1) (C2) (C3)

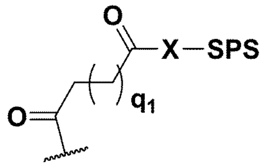
,
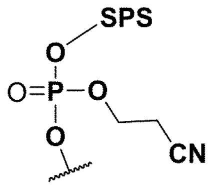
,

(C1') (C3')

wherein $q_1$ is an integer of 1-4, X is O or NH, $M^+$ is a cation, $R_k$ is a hydroxy protecting group, SPS represents a solid phase support, and 



represents the site where a group is covalently linked.

In some embodiments, the first functional group comprises a phosphoramidite group as shown by Formula (C3). The phosphoramidite group can form a phosphite ester with a hydroxy at any position on a nucleotide such as a 2' or 3' hydroxy by a coupling reaction, and the phosphite ester can form a phosphodiester bond or phosphorothioate ester bond as shown by Formula (A59) via oxidation or sulfurization, so as to conjugate the conjugating molecule to the siRNA. In this case, even if the second functional group does not exist, the compound as shown by Formula (321) will still be able to be conjugated to the nucleotide, without affecting the acquisition of the siRNA conjugate as shown by Formula (308). Under such circumstances, after obtaining a sense strand or an antisense strand of the siRNA by a method such as solid phase phosphoramidite synthesis, the compound as shown by Formula (321) is reacted with a hydroxy on the terminal nucleotide of the nucleotide sequence, and phosphodiester bonding or phosphorothioate bonding is formed by a subsequent oxidation or sulfurization process, thereby conjugating the compound as shown by Formula (321) to the siRNA.

In some embodiments, the first functional group comprises a protected hydroxy. In some embodiments, the second functional group comprises a group that can react with a solid phase support to provide a conjugating molecule comprising the solid phase support. In some embodiments, the second functional group comprises a carboxyl, a carboxylate or a phosphoramidite as shown by Formula (C1), (C2) or (C3). When the second functional group comprises

a carboxyl or a carboxylate, the compound as shown by Formula (321) reacts with a hydroxy or an amino on a solid phase support such as a resin via an esterification or an amidation reaction, to form a conjugating molecule comprising the solid phase support linked via a carboxyl ester bond. When the second functional group comprises a phosphoramidite functional group, the compound as shown by Formula (321) may be coupled with a hydroxy on a universal solid phase support, such as a resin, and form, by oxidation, a conjugating molecule comprising the solid phase support linked via a phosphodiester bond. Subsequently, starting from the above product linked to the solid phase support, the nucleoside monomers are linked sequentially by a solid phase phosphoramidite synthesis method, thereby obtaining a sense or strand or an antisense strand of the siRNA linked to the conjugation group. During the solid phase phosphoramidite synthesis, the first functional group is deprotected, and then coupled with a phosphoramidite group on a nucleoside monomer under coupling reaction condition.

In some embodiments, the first functional group comprises a hydroxy or a protected hydroxy; and the second functional group comprises a solid phase support linked via a carboxyl ester bond, a solid phase support linked via an amide bond or a solid phase support linked via a phosphoester bond, as shown by Formula (C1') or (C3'). In this case, starting from the compound as shown by Formula (321) in place of the solid phase support, the nucleoside monomers are linked sequentially by a solid phase phosphoramidite synthesis, thereby obtaining a sense strand or an antisense strand of the siRNA linked to a conjugating group.

In some embodiments, the carboxylate may be expressed as $-COO^-M^+$, wherein $M^+$ is a cation such as one of a metal cation, an ammonium cation $NH4^+$ and an organic ammonium cation. In one embodiment, the metal ion may be an alkali metal ion, such as $K^+$ or $Na^+$. In order to increase solubility and facilitate the reaction, in some embodiments, the organic ammonium ion is an ammonium cation formed by a tertiary amine, or a quaternary ammonium cation, such as an ammonium ion formed by triethylamine or an ammonium ion formed by N,N-diisopropylethylamine. In some embodiments, the carboxylate is a triethylamine carboxylate or an N,N-diisopropylethylamine carboxylate.

In some embodiments, $R_4$ comprises a structure as shown by Formula (B9), (B10), (B9'), (B10'), (B 11), (B12), (B11') or (B12'):

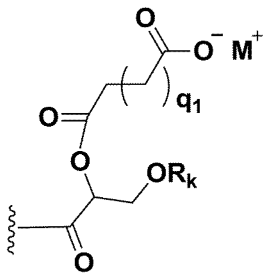
,
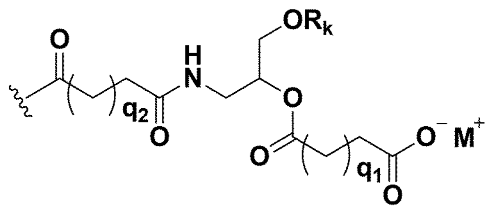
,

(B9) (B10)

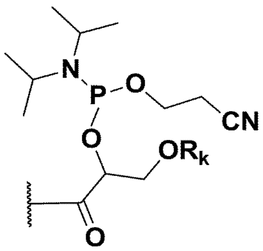
,
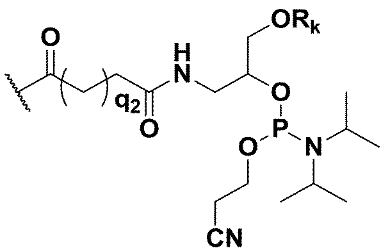
,

(B9') (B10')

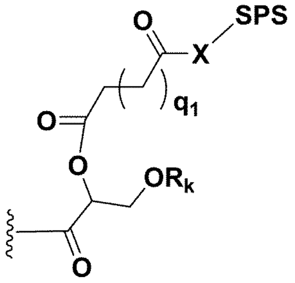
,
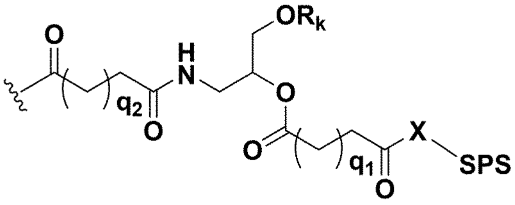
,

(B11) (B12)

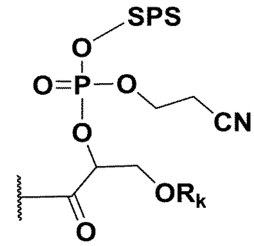
,
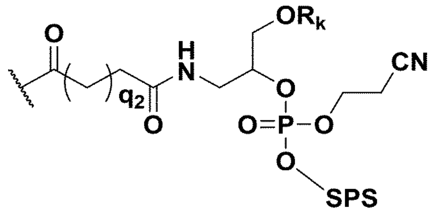
,

(B11') (B12')

wherein $q_1$ is an integer of 1-4, $q_2$ is an integer of 1-10, X is O or NH, $M^+$ is a cation, $R_k$ is a hydroxy protecting group, SPS represents a solid phase support, and 



represents a site where a group is covalently linked. In some embodiments, $q_1$ is 1 or 2. In some embodiments, $q_2$ is an integer of 1-5. In some embodiments, $R_4$ comprises a structure as shown by Formula (B9) or (B10). In some embodiments, $R_4$ comprises a structure as shown by Formula (B11) or (B12).

In some embodiments, $R_k$ is one or more of Tr (trityl), MMTr (4-methoxytrityl), DMTr (4,4'-dimethoxytrityl), and TMTr (4,4',4"-trimethoxytrityl). In some embodiments, $R_k$ may be DMTr, i.e., 4,4'-dimethoxytrityl.

The definition of $L_1$ is as described above.

In some embodiments, $L_1$ is used to link the $M_1$ targeting group to the N atom on the nitrogenous backbone, thereby providing liver targeting function for the siRNA conjugate as shown by Formula (308). In some embodiments, $L_1$ comprises any one of Formula (A1) to Formula (A26), or the combination thereof.

According to the description above, those skilled in the art would easily understand that as compared with the well-known solid phase phosphoramidite synthesis methods in the art, an siRNA conjugate in which a conjugating molecule is linked to any possible position of the nucleotide sequence can be obtained through the above first functional group and an optional second functional group. For example, the conjugating molecule is linked to a terminal of the nucleotide sequence or to either terminal of the nucleotide sequence. Correspondingly, unless otherwise specified, in the following description regarding siRNA conjugate and/or conjugating molecule preparation, when referring to the reactions such as "deprotection", "coupling", "capping", "oxidation", "sulfurization", it will be understood that the reaction conditions and agents involved in the well-known phosphoramidite nucleic acid solid phase synthesis methods in the art would also apply to these reactions. Exemplary reaction conditions and agents will be described in detail hereinafter.

In some embodiments, each Si is independently an $M_1$. In some embodiments, each Si is independently a group formed by protecting at least one active hydroxy in $M_1$ with a hydroxy protecting group. In some embodiments, each Si is independently a group formed by protecting all active hydroxys in $M_1$ with hydroxy protecting groups. In some embodiments, any hydroxy protecting group known to those skilled in the art may be used to protect the active hydroxy in $M_1$. In some embodiments, the protected hydroxy is expressed as the formula YCOO-, wherein each Y is independently selected from the group consisting of $C_1$-$C_{10}$ alkyl and $C_6$-$C_{10}$ aryl, wherein the $C_1$-$C_{10}$ alkyl and $C_6$-$C_{10}$ aryl are optionally substituted with one or more substituents selected from the group consisting of halo and $C_1$-$C_6$ alkyl. In some embodi-

ments, each Y is independently selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and $C_1$-$C_6$ alkylphenyl.

In some embodiments, each Si is independently selected from the group consisting of Formulae A46-A54:

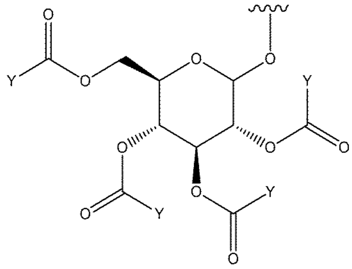
,
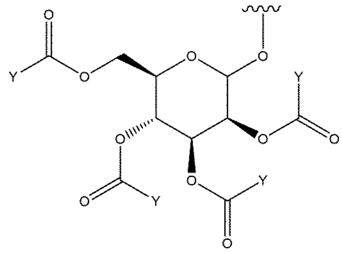
,
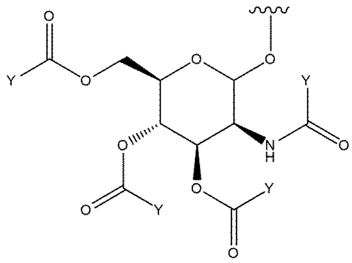
,

(A46) (A47) (A48)

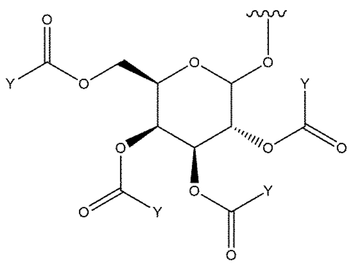
,
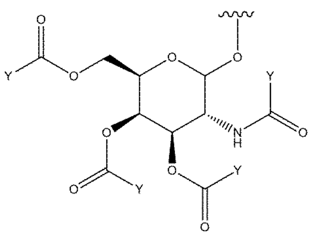
,
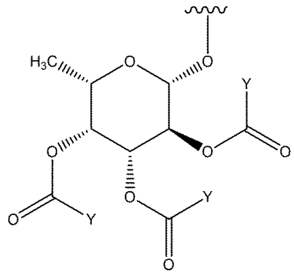
,

(A49) (A50) (A51)

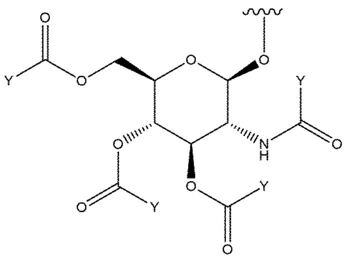
,
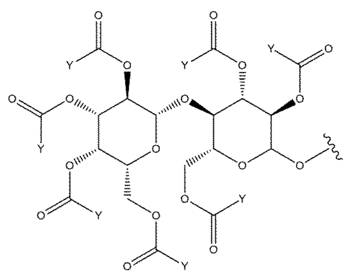
,
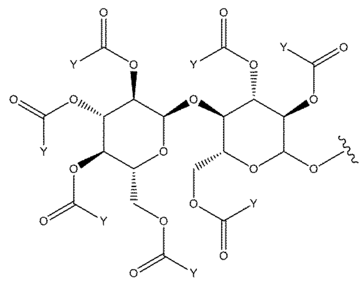
.

(A52) (A53) (A54)

In some embodiments, $S_1$ is Formula A49 or A50.

In some embodiments, each Y is independently selected from one of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl. In some embodiments, Y is a methyl.

As mentioned previously, the method for preparing the siRNA conjugate as shown by Formula (308) further comprises the following steps of: synthesizing the other strand of the siRNA (for example, when the sense strand of the siRNA linked to the conjugating molecule is synthesized in the above step, the method further comprises synthesizing the antisense strand of the siRNA by the solid phase synthesis method, and vice versa); isolating the sense strand and the antisense strand; and annealing. In particular, in the isolating step, the solid phase support linked to the nucleotide sequence and/or the conjugating molecule is cleaved and at the same time the necessary protecting group is removed (in this case, each Si group in the compound as shown by Formula (321) is converted to a corresponding $M_1$ targeting group), thereby providing the sense strand (or antisense strand) of the siRNA linked to the conjugating molecule and the corresponding antisense strand (or sense strand). The sense strand and the antisense strand are annealed to form

a double-stranded RNA structure, thereby obtaining the siRNA conjugate as shown by Formula (308).

In some embodiments, the method for preparing the siRNA conjugate as shown by Formula (308) further comprises the following steps of: contacting the compound as shown by Formula (321) with the first nucleoside monomer at 3'terminal of the sense strand or antisense strand under coupling reaction condition in the presence of a coupling agent, thereby linking the compound as shown by Formula (321) to the first nucleotide in the sequence; successively linking nucleoside monomers in the direction from 3' to 5' to synthesize the sense strand or the antisense strand of the siRNA according to the desired nucleotide type and sequence of the sense strand or antisense strand, under the condition of solid phase phosphoramidite synthesis; wherein the compound as shown by Formula (321) is a compound in which R4 comprises a first functional group and a second functional group, the first functional group comprises a protected hydroxy and the second functional group comprises a group as shown by Formula (C1') or (C3'), and the compound as shown by Formula (321) is deprotected before linked to the first nucleoside monomer; and the linking of each nucleoside monomer comprises a four-step reaction of deprotection, coupling, capping, and oxidation or sulfurization, thus obtaining a sense strand or an antisense strand of a nucleic acid linked to the conjugating molecule; successively linking the nucleoside monomers in the direction from 3' to 5' to synthesize the sense strand or antisense strand of the nucleic acid according to the nucleotide type and sequence of the sense strand or the antisense strand, under the condition of solid phase phosphoramidite synthesis; wherein the linking of each nucleoside monomer comprises a four-step reaction of deprotection, coupling, capping, and oxidation or sulfurization; removing the protecting groups and cleaving the solid phase support; isolating and purifying to obtain the sense strand and the antisense strand; and annealing.

In some embodiments, the method for preparing the siRNA conjugate as shown by Formula (308) further comprises the following steps of: successively linking nucleoside monomers in the direction from 3' to 5' to synthesize the sense strand or the antisense strand according to the nucleotide type and sequence of the sense strand or antisense strand in the double-stranded siRNA; wherein the linking of each nucleoside monomer comprises a four-step reaction of deprotection, coupling, capping, and oxidation or sulfurization, thus obtaining a sense strand linked to the solid phase support and an antisense strand linked to the solid phase support; contacting the compound as shown by Formula (321) with the sense strand linked to the solid phase support or the antisense strand linked to the solid phase support under coupling reaction condition in the presence of a coupling agent, thereby linking the compound as shown by Formula (321) to the sense strand or the antisense strand; wherein the compound as shown by Formula (321) is a compound in which $R_4$ comprises a phosphoramidite group as the first functional group; removing the protecting groups and cleaving the solid phase support; respectively isolating and purifying to obtain the sense strand or the antisense strand of the siRNA; and annealing; wherein the sense strand or the antisense strand of the siRNA is linked to a conjugating molecule.

In some embodiments, the P atom in Formula A59 is linked to the 3' terminal of the sense strand of the siRNA, and the method for preparing the siRNA conjugate as shown by Formula (308) comprises:

(1) removing the hydroxy protecting group $R_k$ in the compound as shown by Formula (321) (wherein the compound as shown by Formula (321) is a compound in which $R_4$ comprises a first functional group and a second function group, the first functional group comprises a protected hydroxy $OR_k$, and the second function group has a structure as shown by Formula (C1') or (C3')); and contacting the deprotected product with a nucleoside monomer to obtain a nucleoside monomer linked to a solid phase support via the conjugating molecule under a coupling reaction condition in the presence of a coupling agent;

(2) starting from the nucleoside monomer linked to the solid phase support via the conjugating molecule, synthesizing the sense strand of the siRNA in the direction from 3' to 5' by a solid phase phosphoramidite synthesis;

(3) synthesizing the antisense strand of the siRNA by a solid phase phosphoramidite synthesis method; and

(4) isolating the sense strand and the antisense strand of the siRNA, and annealing the same to obtain the siRNA conjugate as shown by Formula (308).

In step (1), the method for removing the protecting group $R_k$ in the compound as shown by Formula (321) comprises contacting the compound as shown by Formula (321) with a deprotection agent under a deprotection condition. The deprotection condition comprises a temperature of 0-50°C, and in some embodiments, 15-35°C, and a reaction time of 30-300 seconds, and in some embodiments, 50-150 seconds. The deprotection agent may be selected from one or more of trifluoroacetic acid, trichloroacetic acid, dichloroacetic acid, and monochloroacetic acid, and in some embodiments, the deprotection agent is dichloroacetic acid. The molar ratio of the deprotection agent to the compound as shown by Formula (321) may be 10: 1 to 1000: 1, and in some embodiments, 50: 1 to 500: 1.

The coupling reaction condition and the coupling agent may be any conditions and agents suitable for the above coupling reaction. In some embodiments, the same condition and agent as those of the coupling reaction in the solid phase synthesis method may be used.

In some embodiments, the coupling reaction condition comprises a reaction temperature of 0-50°C, and in some embodiments, 15-35°C. The molar ratio of the compound as shown by Formula (321) to the nucleoside monomer may be 1: 1 to 1: 50, and in some embodiments, 1: 2 to 1:5. The molar ratio of the compound as shown by Formula (321) to the coupling agent may be 1: 1 to 1: 50, and in some embodiments, 1: 3 to 1: 10. The reaction time may be 200-3000 seconds, and in some embodiments, 500-1500 seconds. The coupling agent may be selected from one or more of 1H-tetrazole, 5-ethylthio-1H-tetrazole and 5-benzylthio-1H-tetrazole, and in some embodiments, is 5-ethylthio-1H-tetrazole. The organic solvent may be selected from one or more of anhydrous acetonitrile, anhydrous DMF and anhydrous dichloromethane, and in some embodiments, is anhydrous acetonitrile. The amount of the organic solvent may be 3-50 L/mol, and in some embodiments, 5-20 L/mol, with respect to the compound as shown by Formula (321).

In step (2), a sense strand SS of the siRNA conjugate is synthesized in the direction from 3' to 5' by the phosphoramidite nucleic acid solid phase synthesis method, starting from the nucleoside monomer linked to the solid phase support via the conjugating molecule prepared in the above steps. In this case, the conjugating molecule is linked to 3'terminal of the resultant sense strand.

Other conditions for the solid phase synthesis in steps (2) and (3), comprising the deprotection condition for the nucleoside monomer, the type and amount of the deprotection agent, the coupling reaction condition, the type and amount of the coupling agent, the capping reaction condition, the type and amount of the capping agent, the oxidation reaction condition, the type and amount of the oxidation agent, the sulfurization reaction condition, and the type and amount of the sulfurization agent, adopt various conventional agents, amounts, and conditions in the art.

For instance, in some embodiments, the solid phase synthesis in steps (2) and (3) may use the following conditions:

The deprotection condition for the nucleoside monomer comprises a temperature of 0-50°C, and in some embodiments, 15-35°C, and a reaction time of 30-300 seconds, and in some embodiments, 50-150 seconds. The deprotection agent may be selected from one or more of trifluoroacetic acid, trichloroacetic acid, dichloroacetic acid, and monochloroacetic acid, and in some embodiments, the deprotection agent is dichloroacetic acid. The molar ratio of the deprotection agent to the protecting group 4,4'-dimethoxytrityl on the solid phase support is 2: 1 to 100: 1, and in some embodiments, is 3: 1 to 50: 1.

The coupling reaction condition comprises a reaction temperature of 0-50°C, and in some embodiments, 15-35°C. The molar ratio of the nucleic acid sequence linked to the solid phase support to the nucleoside monomer is 1: 1 to 1: 50, and in some embodiments, is 1: 5 to 1: 15. The molar ratio of the nucleic acid sequence linked to the solid phase support to the coupling agent is 1: 1 to 1: 100, and in some embodiments, is 1: 50 to 1: 80. The selection of the reaction time and the coupling agent can be same as above.

The capping reaction condition comprises a reaction temperature of 0-50°C, and in some embodiments, 15-35°C, and a reaction time of 5-500 seconds, and in some embodiments, 10-100 seconds. The selection of the capping agent can be same as above. The molar ratio of the total amount of the capping agent to the nucleic acid sequence linked to the solid phase support may be 1: 100 to 100: 1, and in some embodiments, is 1: 10 to 10: 1. In the case where the capping agent uses equimolar acetic anhydride and N-methylimidazole, the molar ratio of the acetic anhydride to the N-methylimidazole and the nucleic acid sequence linked to the solid phase support may be 1: 1: 10 to 10: 10: 1, and in some embodiments, is 1: 1: 2 to 2: 2: 1.

The oxidation reaction condition comprises a reaction temperature of 0-50°C, and in some embodiments, 15-35°C, and a reaction time of 1-100 seconds, and in some embodiments, 5-50 seconds. In some embodiments, the oxidation agent is iodine (in some embodiments, provided as iodine water). The molar ratio of the oxidation agent to the nucleic acid sequence linked to the solid phase support in the coupling step may be 1: 1 to 100: 1, and in some embodiments, is 5: 1 to 50: 1. In some embodiments, the oxidation reaction is performed in a mixed solvent in which the ratio of tetrahydrofuran: water: pyridine is 3: 1: 1 to 1: 1: 3. The sulfurization reaction condition comprises a reaction temperature of 0-50°C, and in some embodiments, 15-35°C, and a reaction time of 50-2000 seconds, and in some embodiments, 100-1000 seconds. In some embodiments, the sulfurization agent is xanthane hydride. The molar ratio of the sulfurization agent to the nucleic acid sequence linked to the solid phase support in the coupling step is 10: 1 to 1000: 1, and in some embodiments, is 10: 1 to 500: 1. In some embodiments, the sulfurization reaction is performed in a mixed solvent in which the ratio of acetonitrile: pyridine is 1: 3 to 3: 1.

The method further comprises isolating the sense strand and the antisense strand of the siRNA after linking all nucleoside monomers and before the annealing. Methods for isolation are well-known to those skilled in the art and generally comprise cleaving the synthesized nucleotide sequence from the solid phase support, removing protecting groups on the bases, phosphate groups and ligands, purifying and desalting.

The conventional cleavage and deprotection methods in the synthesis of siRNAs can be used to cleave the synthesized nucleotide sequence from the solid phase support, and remove the protecting groups on the bases, phosphate groups and ligands. For example, contacting the resultant nucleotide sequence linked to the solid phase support with strong aqua; during deprotection, the protecting group YCOO- in groups A46-A54 is converted to a hydroxy, and thus the Si groups is converted to a corresponding $M_1$ group, providing the conjugate as shown by Formula (308);

wherein the strong aqua may be aqueous ammonia of a concentration of 25-30% by weight. The amount of the strong aqua may be 0.2 ml/μmol-0.8 ml/μmol with respect to the target siRNA.

When there is at least one 2'-TBDMS protection on the synthesized nucleotide sequence, the method further comprises contacting the nucleotide sequence removed from the solid phase support with triethylamine trihydrofluoride to remove the 2'-TBDMS protection. In this case, the resultant target siRNA sequence comprises the corresponding nucleoside having free 2'-hydroxy. The amount of pure triethylamine trihydrofluoride is 0.4 ml/μmol-1.0 ml/μmol with respect to the target siRNA sequence. As such, the siRNA conjugate as shown by Formula (308) may be obtained.

Methods for purification and desalination are well-known to those skilled in the art. For example, nucleic acid purification may be performed using a preparative ion chromatography purification column with a gradient elution of NaBr or NaCl; after collection and combination of the product, the desalination may be performed using a reverse phase chromatography purification column.

The non-bridging oxygen atom or sulfur atom in the phosphodiester bond or phosphorothioate diester bond between the nucleotides in the resultant siRNA conjugate as shown by Formula (308) substantially binds to a sodium ion, and the siRNA conjugate as shown by Formula (308) is substantially present in the form of a sodium salt. The well-known ion-exchange methods may be used, in which the sodium ion may be replaced with hydrogen ion and/or other cations, thereby providing other forms of siRNA conjugates as shown by Formula (308). The cations are as described above.

During synthesis, the purity and molecular weight of the nucleic acid sequence may be determined at any time. In order to better control the synthesis quality, such detection methods are well-known to those skilled in the art. For example, the purity of the nucleic acid may be detected by ion exchange chromatography, and the molecular weight may be determined by liquid chromatography-mass spectrometry (LC-MS).

Methods for annealing are also well-known to those skilled in the art. For example, the synthesized sense strand (SS strand) and antisense strand (AS strand) may be simply mixed in water for injection at an equimolar ratio, heated to 70-95°C, and then cooled at room temperature to form a double-stranded structure via hydrogen bond. As such, the siRNA conjugate as shown by Formula (308) may be obtained.

After obtaining the conjugate, in some embodiments, the siRNA conjugate as shown by Formula (308) thus synthesized can also be characterized by the means such as molecular weight detection using the methods such as liquid chromatography-mass spectrometry, to confirm that the synthesized siRNA conjugate is the designed siRNA conjugate as shown by Formula (308) of interest, and the sequence of the synthesized siRNA is the sequence of the siRNA sequence desired to be synthesized, for example, is one of the sequences listed in Tables 1a-1f.

The compound as shown by Formula (321) may be prepared by the following method comprising: contacting a compound as shown by Formula (313) with a cyclic anhydride in an organic solvent under esterification reaction condition in the presence of a base and an esterification catalyst; and isolating the compound as shown by Formula (321) by ion exchange:

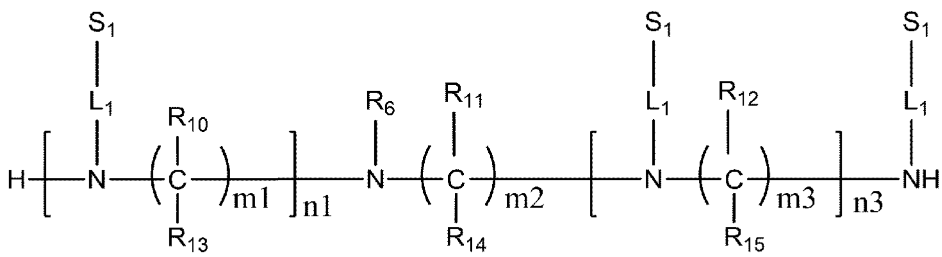

Formula (313)

wherein the definitions and options of n1, n3, m1, m2, m3, $R_{10}$, $R_{11}$, $R_{12}$, $R_{13}$, $R_{14}$, $R_{15}$, $L_1$, and $S_1$ are respectively as described above;

$R_6$ is a group for providing $R_4$ of Formula (321). In some embodiments, $R_6$ comprises a structure as shown by Formula (A61):

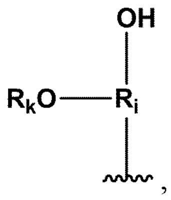

(A61)

wherein, $R_i$ is any group capable of linking to the N atom on the nitrogenous backbone, linking to $R_kO$ and linking to a free hydroxy; and $R_k$ is a hydroxy protecting group. In this case, the compound as shown by Formula (321) is obtained, wherein $R_4$ comprises a first functional group as a hydroxy protecting group and a second functional group comprising a group as shown by Formula (C1) or (C2).

The esterification reaction condition comprises a reaction temperature of 0-100°C and a reaction time of 8-48 hours. In some embodiments, the esterification reaction condition comprises a reaction temperature of 10-40°C and a reaction time of 20-30 hours.

In some embodiments, the organic solvent comprises one or more of an epoxy solvent, an ether solvent, a haloalkane solvent, dimethyl sulfoxide, N,N-dimethylformamide, and N,N-diisopropylethylamine. In some embodiments, the epoxy solvent is dioxane and/or tetrahydrofuran, the ether solvent is diethyl ether and/or methyl tertbutyl ether, and the haloalkane solvent is one or more of dichloromethane, trichloromethane and 1,2-dichloroethane. In some embodiments, the organic solvent is dichloromethane. The amount of the organic solvent is 3-50 L/mol, and in some embodiments, 5-20 L/mol, with respect to the compound as shown by Formula (313).

In some embodiments, the cyclic anhydride is one of succinic anhydride, glutaric anhydride, adipic anhydride or pimelic anhydride, and in some embodiments, the cyclic anhydride is succinic anhydride. The molar ratio of the cyclic anhydride to the compound as shown by Formula (313) is 1: 1 to 10: 1, and in some embodiments, 2: 1 to 5: 1.

The esterification catalyst may be any catalyst capable of catalyzing esterification, for example, the catalyst may be 4-dimethylaminopyridine. The molar ratio of the catalyst to the compound as shown by Formula (313) is 1: 1 to 10: 1, and in some embodiments, 2: 1 to 5: 1.

In some embodiments, the base may be any inorganic base, organic base or a combination thereof. Considering solubility and product stability, the base may be, for example, tertiary amine. In some embodiments, the tertiary amine is triethylamine or N,N-diisopropylethylamine. The molar ratio of the tertiary amine to the compound as shown by Formula (313) is 1: 1 to 20: 1, and in some embodiments, is 3: 1 to 10: 1.

The ion exchange serves the function of converting the compound as shown by Formula (321) into a desired form of carboxylic acid or carboxylic salt and the methods of ion exchange are well-known to those skilled in the art. The above conjugating molecule in which the cation is $M^+$ may be obtained by using suitable ion exchange solution and ion exchange condition, which is not described here in detail. In some embodiments, a triethylamine phosphate solution is used in the ion exchange reaction, and the concentration of the triethylamine phosphate solution is 0.2-0.8 M. In some embodiments, the concentration of the triethylamine phosphate solution is 0.4-0.6 M. In some embodiments, the amount of the triethylamine phosphate solution is 3-6 L/mol, and in further embodiment, 4-5 L/mol, with respect to the compound as shown by Formula (313).

The compound as shown by Formula (321) may be isolated from the reaction mixture using any suitable isolation methods. In some embodiments, the compound as shown by Formula (321) may be isolated by removal of solvent via evaporation followed by chromatography, for example, using the following two chromatographic conditions for the isolation: (1) normal phase purification of 200-300 mesh silica gel filler, and gradient elution of 1wt‰ triethylamine in dichloromethane: methanol = 100: 18 to 100: 20; or (2) reverse phase purification of C18 and C8 reverse phase filler, and gradient elution of methanol: acetonitrile = 0.1: 1 to 1: 0.1. In some embodiments, the solvent may be directly removed to obtain a crude product of the compound as shown by Formula (321), which may be directly used in subsequent reactions.

In some embodiments, the method for preparing the compound as shown by Formula (321) further comprises: contacting the product obtained from the above ion exchanging reaction with a solid phase support containing amino or hydroxy in an organic solvent under condensation reaction condition in the presence of a condensing agent, a condensing catalyst and tertiary amine. In this case, the compound as shown by Formula (321) is obtained, wherein $R_4$ comprises a first functional group comprising a hydroxy protecting group and a second functional group having a structure as shown by Formula (C1').

The solid phase support is one of the carriers used in solid phase synthesis of siRNA, some of which are well-known to those skilled in the art. For example, the solid phase support may be selected from the solid phase supports containing an active hydroxy or amino functional group. In some embodiments, the solid phase support is an amino

resin or hydroxy resin. In some embodiments, the amino or hydroxy resin has the following parameters: particle size of 100-400 mesh, and surface amino or hydroxy loading of 0.2-0.5 mmol/g. The ratio of the compound as shown by Formula (321) to the solid phase support is 10-400 μmol compound per gram of solid phase support (μmol/g). In some embodiments, the ratio of the compound of Formula (321) to the solid phase support is 50-200 μmol/g.

The organic solvent may be any suitable solvent or mixed solvents known to those skilled in the art. In some embodiments, the organic solvent comprises one or more of acetonitrile, an epoxy solvent, an ether solvent, a haloalkane solvent, dimethyl sulfoxide, N,N-dimethylformamide, and N,N-diisopropylethylamine. In some embodiments, the epoxy solvent is dioxane and/or tetrahydrofuran, the ether solvent is diethyl ether and/or methyl tertbutyl ether, and the haloalkane solvent is one or more of dichloromethane, trichloromethane and 1,2-dichloroethane. In some embodiments, the organic solvent is acetonitrile. The amount of the organic solvent may be 20-200 L/mol, and in some embodiments, 50-100 L/mol, with respect to the compound as shown by Formula (321).

In some embodiments, the condensing agent may be benzotriazol-1-yl-oxytripyrrolidino phosphonium hexafluorophosphate (PyBop), 3-(Diethoxyphosphoryloxy)-1,2,3-benzotriazin-4(3H)-one (DEPBT) and/or O-benzotriazol-1-yl-tetramethyluronium hexafluorophosphate. In some embodiments, the condensing agent is O-benzotriazol-1-yl-tetramethyluronium hexafluorophosphate. The molar ratio of the condensing agent to the compound as shown by Formula (321) is 1: 1 to 20: 1, and in some embodiments, 1: 1 to 5: 1.

In some embodiments, the tertiary amine is triethylamine and/or N,N-diisopropylethylamine, and in some embodiments, N,N-diisopropylethylamine. The molar ratio of the tertiary amine to the compound as shown by Formula (321) is 1: 1 to 20: 1, and in some embodiments, 1: 1 to 5: 1.

In some embodiments, the method for preparing the compound as shown by Formula (321) further comprises: contacting the resultant condensation product with a capping agent and an acylation catalyst in an organic solvent under capping reaction condition, and isolating the compound as shown by Formula (321). The capping reaction is used to remove any active functional group that does not completely react, so as to avoid producing unnecessary by products in subsequent reactions. The capping reaction condition comprises a reaction temperature of 0-50°C, and in some embodiments, 15-35°C, and a reaction time of 1-10 hours, and in some embodiments, 3-6 hours. The capping agent may be a capping agent used in solid phase synthesis of siRNA, and the capping agent used in solid phase synthesis of siRNA is well known to those skilled in the art.

In some embodiments, the capping agent is composed of a capping agent 1 (cap1) and a capping agent 2 (cap2). The cap1 is N-methylimidazole, and in some embodiments, provided as a mixed solution of N-methylimidazole in pyridine/acetonitrile, wherein the volume ratio of the pyridine to the acetonitrile is 1: 10 to 1: 1, and in some embodiments, 1: 3 to 1: 1. In some embodiments, the ratio of the total volume of the pyridine and acetonitrile to the volume of the N-methylimidazole is 1: 1 to 10: 1, and in some embodiments, 3: 1 to 7: 1. The capping agent 2 is acetic anhydride. In some embodiments, the capping agent 2 is provided as a solution of acetic anhydride in acetonitrile, wherein the volume ratio of the acetic anhydride to the acetonitrile is 1: 1 to 1: 10, and in some embodiments, 1: 2 to 1: 6.

In some embodiments, the ratio of the volume of the mixed solution of N-methylimidazole in pyridine/acetonitrile to the mass of the compound as shown by Formula (321) is 5 ml/g to 50 ml/g, and in some embodiments, 15 ml/g to 30 ml/g. The ratio of the volume of the solution of acetic anhydride in acetonitrile to the mass of the compound as shown by Formula (321) is 0.5 ml/g to 10 ml/g, and in some embodiments, 1 ml/g to 5 ml/g.

In some embodiments, the capping agent comprises equimolar acetic anhydride and N-methylimidazole. In some embodiments, the organic solvent comprises one or more of acetonitrile, an epoxy solvent, an ether solvent, a haloalkane solvent, dimethyl sulfoxide, N,N-dimethylformamide, and N,N-diisopropylethylamine. In some embodiments, the organic solvent is acetonitrile. The amount of the organic solvent may be 10-50 L/mol, and in some embodiments, 5-30 L/mol, with respect to the compound as shown by Formula (321).

In some embodiments, the acylation catalyst may be selected from any catalyst that may be used for esterification condensation or amidation condensation, such as alkaline heterocyclic compounds. In some embodiments, the acylation catalyst is 4-dimethylaminopyridine. The mass ratio of the catalyst to the compound as shown by Formula (321) may be 0.001: 1 to 1: 1, and in some embodiments, 0.01: 1 to 0.1: 1.

In some embodiments, the compound as shown by Formula (321) may be isolated from the reaction mixture using any suitable isolation methods. In some embodiments, the compound as shown by Formula (321) may be obtained by thoroughly washing with an organic solvent and filtering to remove unreacted reactants, excess capping agent and other impurities, wherein the organic solvent is selected from acetonitrile, dichloromethane, or methanol. In some embodiments, the organic solvent is acetonitrile.

In some embodiments, the preparation of the conjugating molecule as shown by Formula (321) comprises contacting a compound as shown by Formula (313) with a phosphorodiamidite in an organic solvent under coupling reaction condition in the presence of a coupling agent, and isolating the compound as shown by Formula (321). In this case, the compound as shown by Formula (321) is obtained, where $R_4$ comprises a first functional group comprising a hydroxy protecting group and a second functional group having a structure as shown by Formula (C3).

In some embodiments, the coupling reaction condition comprises a reaction temperature of 0-50°C, such as

15-35°C. The molar ratio of the compound as shown by Formula (313) to the phosphorodiamidite may be 1: 1 to 1: 50, such as 1: 5 to 1: 15.The molar ratio of the compound as shown by Formula (313) to the coupling agent may be 1: 1 to 1: 100, such as 1: 50 to 80. The reaction time may be 200-3000 seconds, such as 500-1500 seconds. The phosphorodiamidite may be, for example, bis(diisopropylamino)(2-cyanoethoxy)phosphine, which may be commercially available or synthesized according to well-known methods in the art. The coupling agent is selected from one or more of 1H-tetrazole, 5-ethylthio-1H-tetrazole and 5-benzylthio-1H tetrazole, such as 5-ethylthio-1H-tetrazole. The coupling reaction may be performed in an organic solvent, and the organic solvent is selected from one or more of anhydrous acetonitrile, anhydrous DMF and anhydrous dichloromethane, such as anhydrous acetonitrile. The amount of the organic solvent may be 3-50 L/mol, such as 5-20 L/mol, with respect to the compound as shown by Formula (313). By performing the coupling reaction, the hydroxy in the compound as shown by Formula (313) reacts with the phosphorodiamidite to form a phosphoramidite group. In some embodiments, the solvent may be directly removed to obtain a crude product of the compound as shown by Formula (321), which may be directly used in subsequent reactions.

In some embodiments, the method for preparing the compound as shown by Formula (321) further comprises: contacting the isolated product with a solid phase support containing hydroxy in an organic solvent under coupling reaction condition in the presence of a coupling agent, followed by capping, oxidation, and isolation, to obtain the compound as shown by Formula (321). In this case, the compound as shown by Formula (321) is obtained, where $R_4$ comprises a first functional group comprising a hydroxy protecting group and a second functional group having a structure as shown by Formula (C3').

In some embodiments, the solid phase support is a well-known solid phase support in the art for solid phase synthesis of a nucleic acid, such as a deprotected commercially available universal solid phase support (NittoPhase®HL UnyLinker™ 300 Oligonucleotide Synthesis Support, Kinovate Life Sciences, as shown by Formula B80):

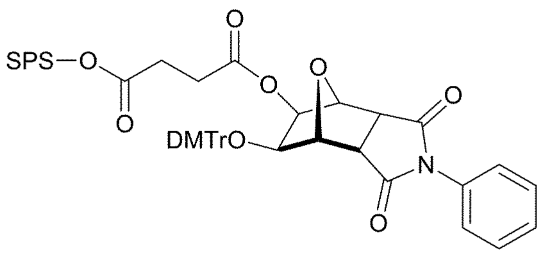

(B80)

A deprotection reaction is well-known in the art. In some embodiments, the deprotection condition comprises a temperature of 0-50°C, such as 15-35°C; and a reaction time of 30-300 seconds, such as 50-150 seconds. The deprotection agent may be selected from one or more of trifluoroacetic acid, trichloroacetic acid, dichloroacetic acid, and monochloroacetic acid. In some embodiments, the deprotection agent is dichloroacetic acid. The molar ratio of the deprotection agent to the protecting group -DMTr(4,4'-dimethoxytrityl) on the solid phase may be 2: 1 to 100: 1, such as 3: 1 to 50: 1. By such deprotection, hydroxys with reactivity are obtained on the surface of the solid phase support, for facilitating the subsequent coupling reaction.

The coupling reaction condition and the coupling agent may be selected as above. By performing coupling reaction, the free hydroxys formed in the deprotection reaction reacts with the phosphoramidite groups, so as to form a phosphite ester linkage.

In some embodiments, the capping reaction condition comprises a reaction temperature of 0-50°C, such as 15-35°C, and a reaction time of 5-500 seconds, such as 10-100 seconds. The capping reaction is performed in the presence of a capping agent. The selection and amount of the capping agent are as above.

The oxidation reaction condition may comprise a temperature of 0-50°C, such as 15 35°C, and a reaction time of 1-100 seconds, such as 5-50 seconds. The oxidation agent may be, for example, iodine (in some embodiments, provided as iodine water). In some embodiments, the molar ratio of the oxidation agent to the nucleic acid sequence linked to the solid phase support is 1: 1 to 100: 1, such as 5: 1 to 50: 1. In some embodiments, the oxidation reaction is performed in a mixed solvent in which the ratio of tetrahydrofuran: water: pyridine is 3: 1: 1 to 1: 1: 3.

In some embodiments, $R_6$ is a group as shown by Formula B7 or B8:

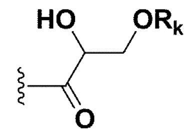, 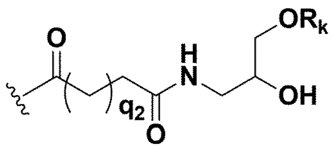,

(B7) (B8)

wherein the definitions of $q_2$ and $R_k$ are as described above.

In this case, the compound as shown by Formula (313) may be prepared by the following method: contacting a compound as shown by Formula (314) with a compound as shown by Formula (A-1) or a compound as shown by Formula (A-2) in an organic solvent under amidation reaction condition in the presence of an agent for amidation condensation and tertiary amine, and isolating:

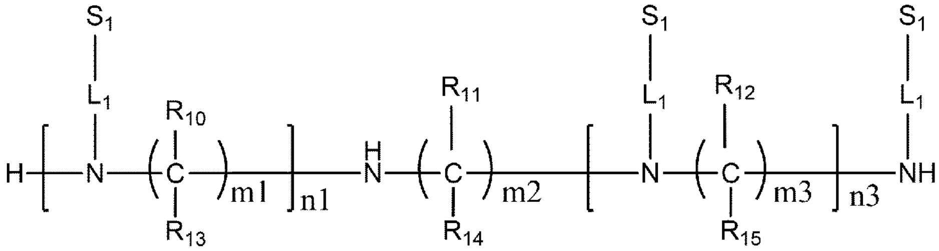

Formula (314)

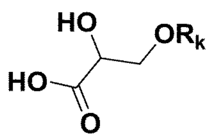, 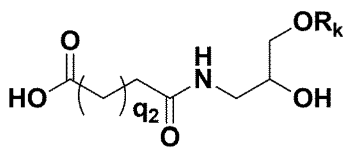,

(A-1) (A-2)

wherein the definitions and options of n1, n3, m1, m2, m3, $R_{10}$, $R_{11}$, $R_{12}$, $R_{13}$, $R_{14}$, $R_{15}$, Li, Si, $q_2$ and $R_k$ are respectively as described above.

The amidation reaction condition may comprise a reaction temperature of 0-100°C and a reaction time of 1-48 hours. In some embodiments, the amidation reaction condition comprises a reaction temperature of 10-40°C and a reaction time of 2-16 hours.

In some embodiments, the organic solvent is one or more of an alcohol solvent, an epoxy solvent, an ether solvent, a haloalkane solvent, dimethyl sulfoxide, N,N-dimethylformamide, and N,N-diisopropylethylamine. In some embodiments, the alcohol solvent is one or more of methanol, ethanol and propanol, and in some embodiments, ethanol. In some embodiments, the epoxy solvent is dioxane and/or tetrahydrofuran. In some embodiments, the ether solvent is diethyl ether and/or methyl tertbutyl ether. In some embodiments, the haloalkane solvent is one or more of dichloromethane, trichloromethane and 1,2-dichloroethane. In some embodiments, the organic solvent is dichloromethane. The amount of the organic solvent is 3-50 L/mol, and in further embodiments, 3-20 L/mol, with respect to the compound as shown by Formula (314).

In some embodiments, the agent for amidation condensation is benzotriazol-1-yl-oxytripyrrolidinophosphonium hexafluorophosphate, 3-(Diethoxyphosphoryloxy)-1,2,3-benzotriazin-4(3H)-one, 4-(4,6-dimethoxytriazin-2-yl)-4-methylmorpholine hydrochloride, 2 ethoxy-1-ethoxycarbonyl-1,2-dihydroquinoline (EEDQ) or O-benzotriazol-1-yl-tetramethyluronium hexafluorophosphate, and in further embodiments, 3-(Diethoxyphosphoryloxy)-1,2,3-benzotriazin-4(3H)-one. The molar ratio of the agent for amidation condensation to the compound as shown by Formula (314) may be 1: 1 to 10: 1, and in some embodiments, 2.5: 1 to 5: 1.

In some embodiments, the tertiary amine is triethylamine and/or N,N-diisopropylethylamine, and in further

embodiments, N,N-diisopropylethylamine. The molar ratio of the tertiary to the compound as shown by Formula (314) is 3: 1 to 20: 1, and in some embodiments, is 5: 1 to 10: 1.

The compounds as shown by Formula (A-1) and Formula (A-2) may be prepared by any suitable methods. For example, when $R_k$ is a DMTr group, the compound as shown by Formula (A-1) may be prepared by reacting calcium glycerate with DMTrCl. Similarly, the compound as shown by Formula (A-2) may be prepared by contacting 3-amino-1,2-propanediol with a cyclic anhydride and then reacting with DMTrCl, wherein the cyclic anhydride may have 4-13 carbon atoms, and in some embodiments, 4-8 carbon atoms. Those skilled in the art would readily understand that the selections of the cyclic anhydride correspond to different values for $q_2$ in the compound as shown by Formula (A-2). For example, when the cyclic anhydride is succinic anhydride, $q_2$=1; when the cyclic anhydride is glutaric anhydride, $q_2$=2, and so on.

In some variants, the compound as shown by Formula (313) can also be prepared by successively reacting the compound as shown by Formula (314) with the cyclic anhydride, 3-amino-1,2 propanediol, and DMTrCl. Those skilled in the art would readily understand that these variants would not affect the structure and function of the compound as shown by Formula (313), and these variants can be readily achieved by those skilled in the art on the basis of the above methods.

Similarly, the compound as shown by Formula (313) may be isolated from the reaction mixture by any suitable isolation methods. In some embodiments, the compound as shown by Formula (313) may be isolated by removal of solvent via evaporation followed by chromatography, for example, using the following two chromatographic conditions for isolation: (1) normal phase purification of 200-300 mesh silica gel filler, and gradient elution of petroleum ether: ethyl acetate: dichloromethane: N,N-dimethylformamide = 1: 1: 1: 0.5 1: 1: 1: 0.6; and (2) reverse phase purification of C18 and C8 reverse phase fillers, and gradient elution of methanol: acetonitrile = 0.1: 1 to 1: 0.1. In some embodiments, the solvent may be directly removed to obtain a crude product of the compound as shown by Formula (313), which may be directly used in subsequent reactions.

In some embodiments, the compound as shown by Formula (314) may be prepared by the following method comprising: contacting a compound as shown by Formula (320) with a compound as shown by Formula (316) in an organic solvent under under condensation reaction condition in the presence of an agent for amidation condensation and tertiary amine, and isolating:

$S_1$-$L_1$-OH Formula (316)

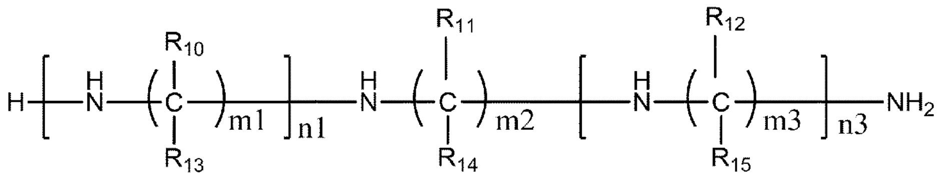

Formula (320)

wherein the definitions and options of n1, n3, m1, m2, m3, $R_{10}$, $R_{11}$, $R_{12}$, $R_{13}$, $R_{14}$, and $R_{15}$ are respectively as described above.

The compund as shown by Formula (316) can be, such as, those disclosed in J. Am. Chem. Soc. 2014, 136, 16958-16961, or, the compound as shown by Formula (316) may be prepared by those skilled in the art via various methods. For example, some compound as shown by Formula (316) may be prepared according to the methods as disclosed in Example 1 of US patent 8,106,022 B2, which is incorporated herein by reference in its entirety.

In some embodiments, the condensation reaction condition comprises a reaction temperature of 0-100°C and a reaction time of 0.1-24 hours. In some embodiments, the condensation reaction condition comprises a reaction temperature is 10-40°C and a reaction time is 0.5-16 hours.

Considering the structure of the desired compound as shown by Formula (314), the molar ratio of the compound as shown by Formula (316) to the compound as shown by Formula (320) should be determined based on the sum of n1 and n3 in Formula (320). In some embodiments, for example, when n1+n3=3, in order to ensure that the reaction is complete and not excessive, the molar ratio of the compound as shown by Formula (316) to the compound as shown by Formula (320) may be 3: 1 to 3.5: 1, and in some embodiments, is 3.01: 1 to 3.15: 1.

In some embodiments, the organic solvent is one or more of acetonitrile, an epoxy solvent, an ether solvent, a haloalkane solvent, dimethyl sulfoxide, N,N-dimethylformamide, and N,N-diisopropylethylamine. In some embodiments, the epoxy solvent is dioxane and/or tetrahydrofuran. In some embodiments, the ether solvent is diethyl ether

and/or methyl tertbutyl ether. In some embodiments, the haloalkane solvent is one or more of dichloromethane, trichloromethane and 1,2-dichloroethane. In some embodiments, the organic solvent is acetonitrile. The amount of the organic solvent is 3-50 L/mol, and in some embodiments, 5-20 L/mol, with respect to the compound as shown by Formula (320).

In some embodiments, the agent for amidation condensation is benzotriazol-1-yl-oxytripyrrolidinophosphonium hexafluorophosphate, 3-(Diethoxyphosphoryloxy)-1,2,3-benzotriazin-4(3H)-one (DEPBT), O-benzotriazol-1-yl-tetramethyluronium hexafluorophosphate, 4-(4,6-dimethoxytriazin-2-yl)-4-methylmorpholine hydrochloride or 1-hydroxybenzotriazole, and in further embodiments, is a mixture of the benzotriazol-1-yl-oxytripyrrolidinophosphonium hexafluorophosphate and the 1-hydroxybenzotriazole, wherein the benzotriazol-1-yl-oxytripyrrolidino phosphonium hexafluorophosphate (PyBop) and the 1-hydroxybenzotriazole are equimolar. The molar ratio of the total agent for amidation condensation to the compound as shown by Formula (316) may be 1: 1 to 3: 1, and in some embodiments, is 1.05: 1 to 1.5: 1.

The tertiary amine may be N-methylmorpholine, triethylamine or N,N-diisopropylethylamine, and in some embodiments, N-methylmorpholine. The molar ratio of the tertiary amine to the compound as shown by Formula (316) may be 2: 1 to 10: 1, and in some embodiments, is 2: 1 to 5: 1.

Similarly, the compound as shown by Formula (314) may be isolated from the reaction mixture by any suitable isolation methods. In some embodiments, the compound as shown by Formula (314) is isolated by removal of solvent via evaporation followed by chromatography, for example, using the following two chromatographic conditions for isolation: (1) normal phase purification of 200-300 mesh silica gel filler, and gradient elution of dichloromethane: methanol = 100: 5 to 100: 7; and (2) reverse phase purification of C18 and C8 reverse phase fillers, and gradient elution of methanol: acetonitrile = 0.1: 1 to 1: 0.1. In some embodiments, the solvent is directly removed to obtain a crude product of the compound as shown by Formula (314), and the crude product can be directly used in subsequent reactions.

The compound as shown by Formula (320) may be commercially available, or obtained by those skilled in the art via the known methods. For example, in the case that ml=m2=m3=3, nl=1, n3=2, and $R_{10}$, $R_{11}$, $R_{12}$, $R_{13}$, $R_{14}$, and $R_{15}$ are all H, the compound as shown by Formula (320) is commercially available from Alfa Aesar Inc.

The siRNA conjugate of the present disclosure may also be used in combination with other pharmaceutically acceptable excipients, which may be one or more of the various conventional formulations or compounds in the art. For details, please refer to the above description of the pharmaceutical compositions of the present disclosure.

Use of the siRNA, the pharmaceutical composition and the siRNA conjugate of the present disclosure

In some embodiments, the present disclosure provides the use of the siRNA, and/or the pharmaceutical composition, and/or the siRNA of the present disclosure in the manufacture of a medicament for treating and/or preventing myasthenia gravis.

According to some embodiments, the present disclosure provides a method for preventing and/or treating myasthenia gravis, comprising administering an effective amount of the siRNA and/or the pharmaceutical composition and/or the siRNA conjugate of the present disclosure to a subject in need.

It is possible to achieve the purpose of preventing and/or treating myasthenia gravis based on a mechanism of RNA interference by administering the active ingredients of the siRNA of the present disclosure to the subject in need. Thus, the siRNA and/or the pharmaceutical composition and/or the siRNA conjugate of the present disclosure may be used for preventing and/or treating myasthenia gravis, or for the manufacture of a medicament for preventing and/or treating myasthenia gravis.

As used herein, the term "administration/administer" refers to the delivery of the siRNA, the pharmaceutical composition, and/or the siRNA conjugate of the present disclosure into a body of a subject by a method or a route that at least partly locates the siRNA, the pharmaceutical composition, and/or the siRNA conjugate of the present disclosure at a desired site to produce a desired effect. Suitable administration routes for the methods of the present disclosure comprise topical administration and systemic administration. In general, the topical administration results in the delivery of more siRNA conjugate to a particular site compared with the systemic circulation of the subject; whereas the systemic administration results in the delivery of the siRNA, the pharmaceutical composition, and/or the siRNA conjugate of the present disclosure to the substantial systemic circulation of the subject. Considering that the present disclosure can provide a means for preventing and/or treating myasthenia gravis, in some embodiments, an administration mode capable of delivering drugs to liver is used.

The administration to a subject may be achieved by any suitable routes known in the art, including but not limited to, oral or parenteral route, such as intravenous administration, intramuscular administration, subcutaneous administration, transdermal administration, intratracheal administration (aerosol), pulmonary administration, nasal administration, rectal administration and topical administration (including buccal administration and sublingual administration). The administration frequency may be once or more times daily, weekly, biweekly, triweekly, monthly, bimonthly, trimonthly, semiannually or annually.

The dose of the siRNA, the pharmaceutical composition, or the second siRNA conjugate of the present disclosure may be a conventional dose in the art, and the dose may be determined according to various parameters, especially

age, weight and gender of a subject. Toxicity and efficacy may be measured in cell cultures or experimental animals by standard pharmaceutical procedures, for example, by determining $LD_{50}$ (the lethal dose that causes 50% population death), $ED_{50}$ (the dose that can cause 50% of the maximum response intensity in a quantitative response, and that causes 50% of the experimental subjects to have a positive response in a qualitative response), or $IC_{50}$ (the concentration of inhibitor/medicament when a quantitative reaction is suppressed by half). The dose range for human may be derived based on the data obtained from cell culture analysis and animal studies.

When administrating the siRNA, the pharmaceutical composition or the siRNA conjugate of the present disclosure, for example, to male or female C57BL/6J mice of 6-12 weeks old and 18-25 g body weight, and calculating based on the amount of the siRNA: (i) for the siRNA conjugate, the dosage of the siRNA thereof may be 0.001-100 mg/kg body weight, and in further embodiments, is 0.01-50 mg/kg body weight, and in some embodiments, is 0.05-20 mg/kg body weight, in some another embodiments is 0.1-15 mg/kg body weight, and in some another embodiments, is 0.1-10 mg/kg body weight; and (ii) for a pharmaceutical composition formed by an siRNA and a pharmaceutically acceptable carrier, the dosage of the siRNA thereof may be 0.001-50 mg/kg body weight, in some embodiments, is 0.01-10 mg/kg body weight, in some embodiments, is 0.05-5 mg/kg body weight, and in some embodiments, is 0.1-3 mg/kg body weight.

In some embodiments, the present disclosure provides a method for inhibiting expression of a C5 gene in a hepatocyte. The method comprises contacting an effective amount of the siRNA and/or the pharmaceutical composition and/or the siRNA conjugate of the present disclosure with the hepatocyte, introducing the siRNA and/or the pharmaceutical composition and/or the siRNA conjugate of the present disclosure into the hepatocyte, and achieving the purpose of inhibiting the expression of the C5 gene in the hepatocyte through a mechanism of RNA interference. The hepatocyte may be selected from SMMC-7721, HepG2, Huh7 and other hepatoma cell lines or isolated primary hepatocytes. In some embodiments, the cells are HepG2 hepatoma cells.

In the case where the expression of the C5 in the cell is inhibited by using the method provided by the present disclosure, the amount of the siRNA in the modified siRNA, the pharmaceutical composition, and/or the siRNA conjugate provided is typically: an amount sufficient to reduce the expression of the target gene and result in an extracellular concentration of 1 pM to 1 μM, or 0.01 nM to 100 nM, or 0.05 nM to 50 nM or 0.05 nM to about 5 nM on the surface of the target cell. The amount required to achieve this local concentration will vary with various factors, including the delivery method, the delivery site, the number of cell layers between the delivery site and the target cells or tissues, the delivery route (topical or systemic), etc. The concentration at the delivery site may be significantly higher than that on the surface of the target cells or tissues.

Kit

The present disclosure provides a kit, wherein the kit comprises an effective amount of at least one of the modified siRNA, the pharmaceutical composition, and the siRNA conjugate of the present disclosure.

In some embodiments, the kit disclosed herein may provide a modified siRNA in one container. In some embodiments, the kit of the present disclosure may comprise a container providing pharmaceutically acceptable excipients. In some embodiments, the kit may further comprise additional ingredients, such as stabilizers or preservatives. In some embodiments, the kit herein may comprise at least one additional therapeutic agent in other container than the container providing the modified siRNA herein. In some embodiments, the kit may comprise an instruction for mixing the modified siRNA with the pharmaceutically acceptable carrier and/or adjuvants or other ingredients (if any).

In the kit of the present disclosure, the modified siRNA and the pharmaceutically acceptable carrier and/or the adjuvants as well as the modified siRNA, the pharmaceutical composition, and/or the siRNA conjugate and/or the pharmaceutically acceptable adjuvants may be provided in any form, e.g., in a liquid form, a dry form, or a lyophilized form. In some embodiments, the modified siRNA and the pharmaceutically acceptable carrier and/or the adjuvants as well as the pharmaceutical composition and/or the siRNA conjugate and optional pharmaceutically acceptable adjuvants are substantially pure and/or sterile. In some embodiments, sterile water may be provided in the kit of the present disclosure.

Hereinafter, the present disclosure will be further described by examples, but is not limited thereto in any respect.

## Examples

Unless otherwise specified, the agents and culture media used in following examples are all commercially available, and the procedures used such as nucleic acid electrophoresis and real-time PCR are all performed according to methods described in Molecular Cloning (Cold Spring Harbor Laboratory Press (1989)).

The Lipofectamine™2000(Invitrogen) is used as the transfection reagent when the siRNA and the siRNA conjugate for C5 gene synthesized in the present disclosure or the siRNA and the siRNA conjugate as negative control transfect cells, and the specific operation refers to the instructions provided by the manufacturer.

Unless otherwise specified, ratios of reagents provided below are all calculated by volume ratio (v/v).

The experimental data are all expressed as $\overline{X}\pm SD$, and the data analysis is carried out by using Graphpad prism5.0 statistical analysis software.

Preparation Example 1 Preparation of conjugate 1

In this preparation example, conjugate 1 (i.e., L10-siC5a1M1SP) was synthesized. An siRNA conjugated in the conjugate has sense strand and antisense strand sequences corresponding to the conjugate 1 in Table 3.

(1-1) Synthesis of compound L-10

The Compound L-10 was synthesized according to the following method:

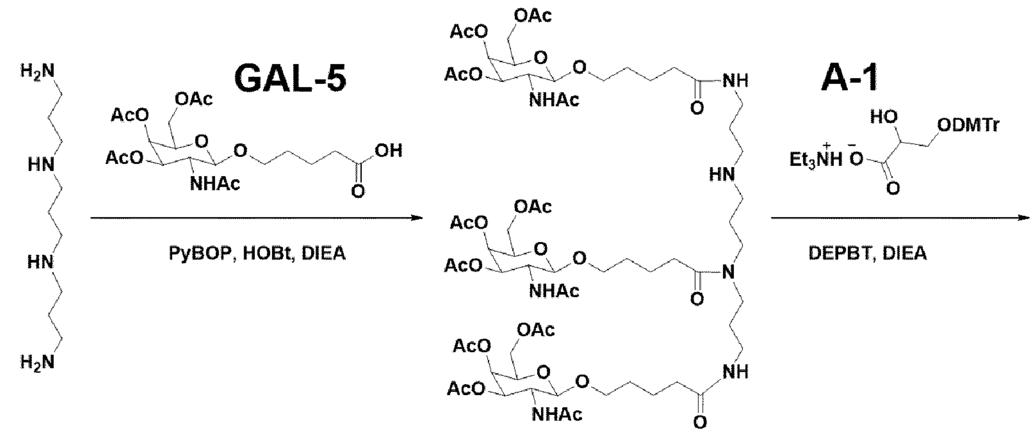


J-0 L-8

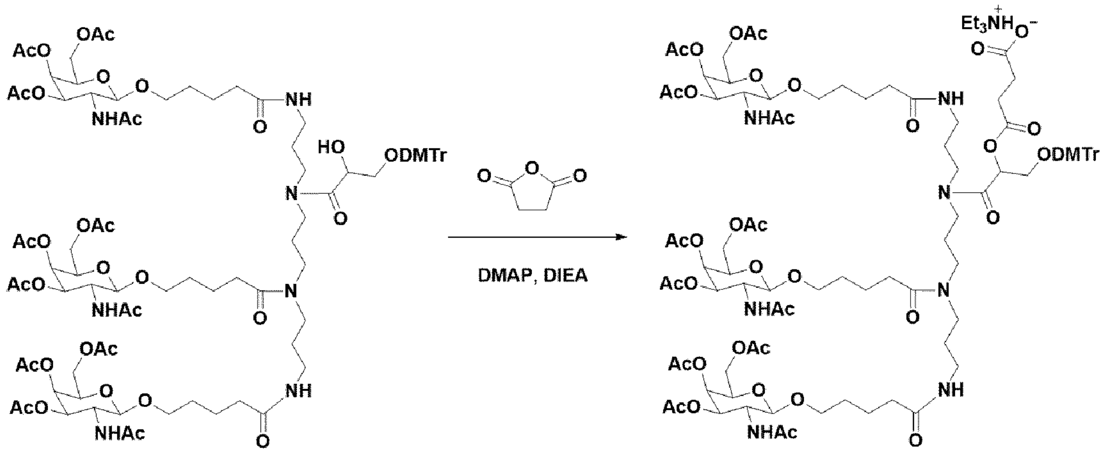


L-7 L-9

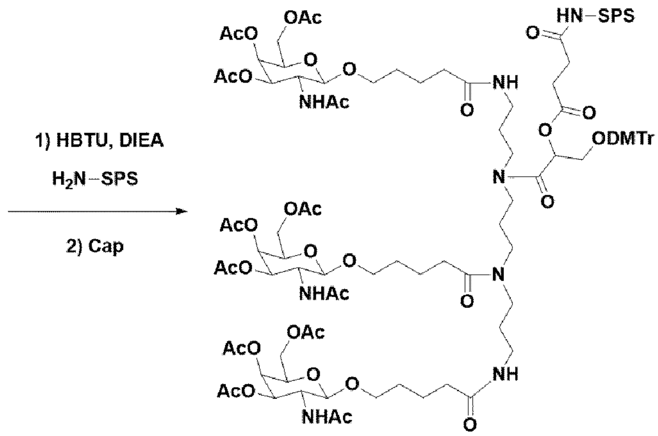


L-10

(1-1-1) Synthesis of GAL-5 (a terminal the conjugating molecule)

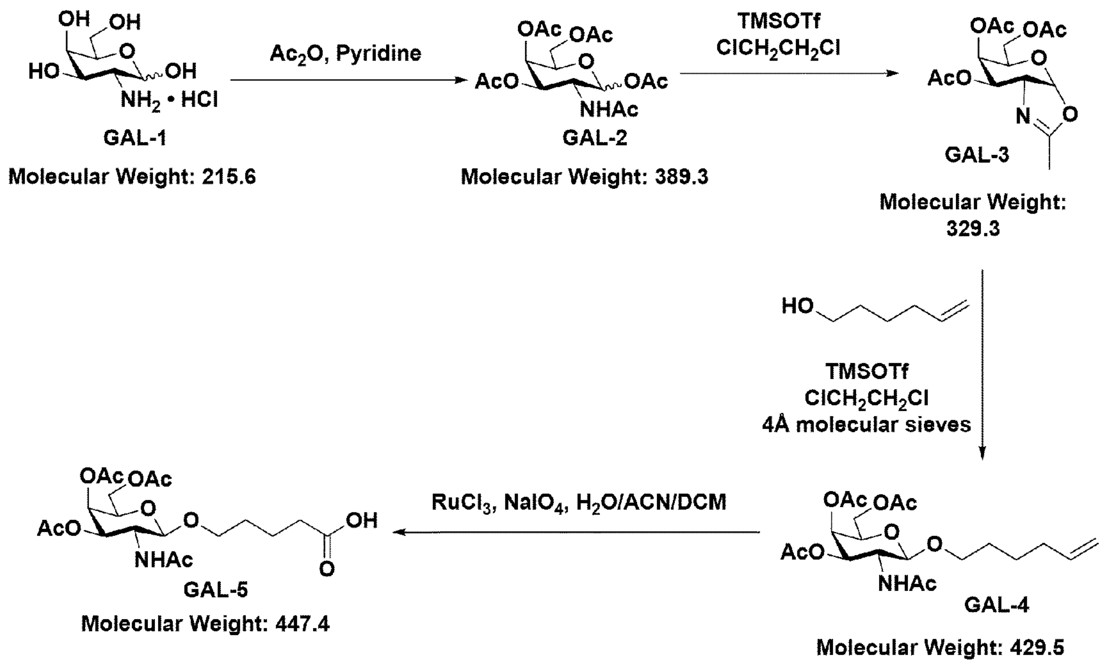


(1-1-1a) Synthesis of GAL-2

100.0 g of **GAL-1** (N-acetyl-D-galactosamine hydrochloride, CAS No.: 1772-03-8, purchased from Ningbo Hongxiang Bio-Chem Co., Ltd., 463.8 mmol) was dissolved in 1000 ml of anhydrous pyridine, to which 540 ml of acetic anhydride (purchased from Enox Inc., 5565.6 mmol) was added in an ice water bath to react under stirring at room temperature for 1.5 hours. The resultant reaction solution was poured into 10 L of ice water and subjected to suction filtration under reduced pressure. The residue was washed with 2 L of ice water, and then added with a mixed solvent of acetonitrile/toluene (v/v ratio = 1: 1) until completely dissolved. The solvent was removed by evaporation to give 130.0 g of product **GAL-2** as a white solid.

(1-1-1b) Synthesis of GAL-3

**GAL-2** (35.1 g, 90.0 mmol) obtained in step (1-1-la) was dissolved in 213 ml of anhydrous 1,2-dichloroethane, to which 24.0 g of trimethylsilyl trifluoromethanesulfonate (TMSOTf, CAS No.: 27607-77-8, purchased from Macklin Inc., 108.0 mmol) was added under an ice water bath and nitrogen protection to react at room temperature overnight.

400 ml of dichloromethane was added to the reaction solution for dilution, filtered with diatomite, and then added with 1 L of saturated aqueous sodium bicarbonate solution and stirred evenly. An organic phase was isolated. An aqueous phase remained was extracted twice, each with 300 ml of dichloroethane, and all organic phases were combined and washed with 300 ml of saturated aqueous sodium bicarbonate solution and 300 ml of saturated brine, respectively. The organic phase resulted from washing was isolated and dried with anhydrous sodium sulfate. The solvent was removed by evaporation under reduced pressure to give 26.9 g of product **GAL-3** as a light yellow viscous syrup.

(1-1-1c) Synthesis of GAL-4

**GAL-3** (26.9 g, 81.7 mmol) obtained in step (1-1-1b) was dissolved in 136 ml of anhydrous 1,2-dichloroethane, added with 30 g of dry 4Å molecular sieve powder followed by 9.0 g of 5-hexen-1-ol (CAS No.: 821-41-0, purchased from Adamas-beta Inc., 89.9 mmol), and stirred at room temperature for 30 minutes. 9.08 ml of TMSOTf (40.9 mmol) was added in an ice bath and nitrogen protection to react under stirring at room temperature overnight. The 4Å molecular sieve powder was removed by filtration. The filtrate was added with 300 ml of dichloroethane for dilution, filtered with diatomite, and then added with 500 ml of saturated aqueous sodium bicarbonate solution and stirred for 10 minutes for washing. An organic phase was isolated. An aqueous phase was extracted once with 300 ml of dichloroethane. All organic phases were combined and washed with 300 ml of saturated aqueous sodium bicarbonate solution and 300 ml of saturated brine respectively. The organic phase resulted from the washing was isolated and dried with anhydrous sodium sulfate. The solvent was removed by evaporation under reduced pressure to give 41.3 g of product **GAL-4** as

a yellow syrup, which was directly used in the next oxidation reaction without purification.

(1-1-1d) Synthesis of GAL-5

**GAL-4** (14.9 g, 34.7 mmol) obtained according to the method described in step (1-1 1c) was dissolved in a mixed solvent of 77 ml of dichloromethane and 77 ml of acetonitrile, added with 103 ml of deionized water and 29.7 g of sodium periodate (CAS No.: 7790-28-5, purchased from Aladdin Inc., 138.8 mmol) respectively, and stirred in an ice bath for 10 minutes. Ruthenium trichloride (CAS No.: 14898-67-0, purchased from Energy Chemical, 238 mg, 1.145 mmol) was added to react at room temperature overnight. The resultant reaction solution was diluted by adding 300 ml of water under stirring, and adjusted to a pH of about 7.5 by adding saturated sodium bicarbonate. An organic phase was isolated and discarded. An aqueous phase was extracted three times, each with 200 ml of dichloromethane, and the organic phase resulted from the extraction was discarded. The aqueous phase resulted from the extraction was adjusted to a pH of about 3 with citric acid solids and extracted three times, each with 200 ml of dichloromethane, and the resultant organic phases were combined and dried with anhydrous sodium sulfate. The solvent is removed by evaporation under reduced pressure to give 6.85 g of product **GAL-5** as a white foamy solid. $^1$H NMR (400 MHz, DMSO) $\delta$ 12.01 (br, 1H), 7.83 (d, *J* = 9.2 Hz, 1H), 5.21 (d, *J* = 3.2 Hz, 1H), 4.96 (dd, *J* = 11.2, 3.2 Hz, 1H), 4.49 (d, *J* = 8.4 Hz, 1H), 4.07 - 3.95 (m, 3H), 3.92 - 3.85 (m, 1H), 3.74 - 3.67 (m, 1H), 3.48 - 3.39 (m, 1H), 2.20 (t, *J*= 6.8 Hz, 2H), 2.11 (s, 3H), 2.00 (s, 3H), 1.90 (s, 3H), 1.77 (s, 3H), 1.55 - 1.45 (m, 4H).

(1-1-2) Synthesis of L-8

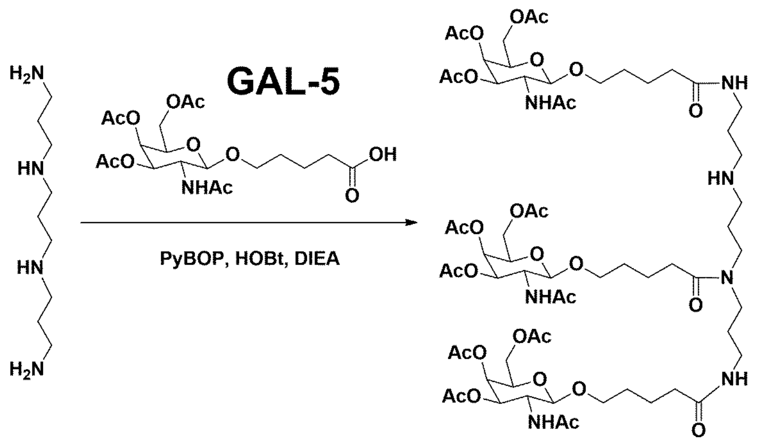


J-0 L-8

J-0 (9.886 g, 52.5 mmol, purchased from AlfaAesar) and GAL-5 (72.819 g, 162.75 mmol, obtained by combining the products of multiple batches) obtained in step (1-1-1) were dissolved in 525 ml of dichloromethane, added with diisopropylethylamine (DIEA, 44.782 g, 346.50 mmol), benzotriazol-1-yl-oxytripyrrolidino phosphonium hexafluorophosphate (PyBop, 90.158 g, 173.25 mmol) and hydroxybenzotriazole (HOBt, 23.410 g, 173.25 mmol) to react at room temperature for 4 hours, and then added with 20 ml of saturated sodium bicarbonate and 200 ml of saturated brine for washing. An aqueous phase was extracted twice, each with 100 ml of dichloromethane, and the resultant organic phases were combined and dried with anhydrous sodium sulfate. The solvent was removed by evaporation under reduced pressure to give a crude product. The crude product was purified by using a normal phase silica gel column (200-300 mesh). The column was added with 10wt% triethylamine for neutralizing the acidity of silica gel and equilibrated with 1wt‰ triethylamine, and eluted with a gradient elution of dichloromethane: methanol = 100: 30 to 100: 40. The eluate was collected, and the solvent was removed by evaporation under reduced pressure to give 38.8 g of pure product L-8. $^1$H NMR (400 MHz, DMSO) $\delta$ 7.84 (d, *J* = 9.0 Hz, 3H), 7.27 - 7.23 (m, 1H), 7.13 - 7.18 (m, 1H), 5.22 (d, *J* = 3.1 Hz, 3H), 4.97 (dd, *J* = 11.3, 3.1 Hz, 3H), 4.48 (d, *J* = 8.4 Hz, 3H), 4.09 - 3.98 (m, 9H), 3.88 (dd, *J* = 19.3, 9.3 Hz, 3H), 3.75 - 3.66 (m, 3H), 3.44 - 3.38 (m, 3H), 3.17 - 3.30 (m, 4H), 3.10 - 2.97 (m, 4H), 2.35 - 2.20 (m, 6H), 2.15 - 2.08 (m, 9H), 2.07 - 1.98 (m, 13H), 1.94 - 1.87 (m, 9H), 1.81 - 1.74 (m, 9H), 1.65 - 1.42 (m, 18H). MS m/z: $C_{85}H_{119}N_7O_{30}$, $[M+H]^+$, called: 1477.59,meaasured: 1477.23.

(1-1-3a) Synthesis of A-1

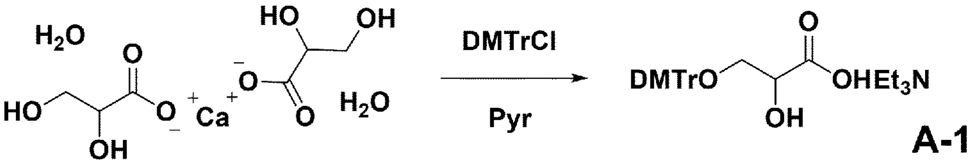


Molecular Weight: 286.25 Molecular Weight: 509.64

DMTrCl (4,4'-dimethoxytrityl chloride, 101.65 g, 300 mmol) was dissolved in 1000 ml of anhydrous pyridine, and added with calcium DL-glycerate hydrate (28.63 g, 100 mmol) to react at 45°C for 20 hours. The reaction solution was filtered. The residue was rinsed with 200 ml of DCM, and the filtrate was concentrated to dryness under reduced pressure. The residue was redissolved in 500 ml of dichloromethane and washed twice, each with 200 ml of 0.5 M triethylamine phosphate (pH = 7-8). An aqueous phase isolated was extracted twice, each with 200 ml of dichloromethane. All organic phases were combined, dried with anhydrous sodium sulfate, and filtered. The solvent was removed by evaporation under reduced pressure, and the residue was purified by using a normal phase silica gel column (200-300 mesh) which was eluted with a gradient elution of petroleum ether: ethyl acetate: dichloromethane: methanol =1: 1: 1: 0.35 to 1: 1: 1: 0.55. The eluate was collected, and the solvent was removed by evaporation under reduced pressure. The residue was redissolved in 600 ml of dichloromethane, and washed once with 200 ml of 0.5 M triethylamine phosphate. The aqueous phase isolated was extracted once with 200 ml of dichloromethane. All organic phases were combined, dried with anhydrous sodium sulfate, and filtered. The solvent was removed by evaporation under reduced pressure and overnight under reduced pressure in a vacuum oil pump to give 50.7 g of product A-1 as a white solid. $^1$H NMR (400 MHz, DMSO-d6) δ 7.46 (ddd, J = 6.5, 2.3, 1.1 Hz, 1H), 7.40 - 7.28 (m, 7H), 6.89 - 6.81 (m, 4H), 4.84 (d, J = 5.0 Hz, 1H), 4.36 - 4.24 (m, 1H), 4.29 (s, 6H), 3.92 (dd, J = 12.4, 7.0 Hz, 1H), 3.67 (dd, J = 12.3, 7.0 Hz, 1H), 2.52 (q, J = 6.3 Hz, 6H), 1.03 (t, J = 6.3 Hz, 9H). MS m/z: $C_{24}H_{23}O_6$, $[M-H]^-$, called: 407.15, measured: 406.92.

(1-1-3b) Synthesis of L-7

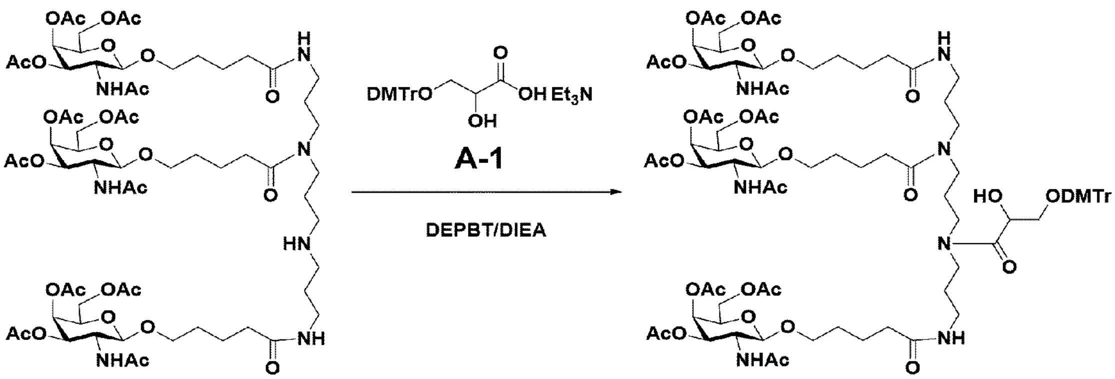


L-8 L-7

L-8 (40 g, 27.09 mmol, obtained by combining the products of multiple batches) obtained in step (1-1-2) and A-1 (41.418 g, 81.27 mmol) obtained in step (1-1-3a) were mixed and dissolved in 271 ml of dichloromethane, added with 3-(Diethoxyphosphoryloxy)-1,2,3-benzotriazin-4(3H)-one (DEPBT, 24.318 g, 81.37 mmol), and further added with diisopropylethylamine (21.007 g, 162.54 mmol) to react under stirring at 25°C for 1.5 hours. An organic phase was washed with 800 ml of saturated sodium bicarbonate. An aqueous phase isolated was extracted three times, each with 50 ml of dichloromethane. The organic phase was washed with 150 ml of saturated brine, and the aqueous phase was extracted once with 50 ml of dichloromethane. The resultant organic phases were combined and dried with anhydrous sodium sulfate. The solvent was removed by evaporation under reduced pressure and the residue was foam-dried in a vacuum oil pump overnight to give a crude product. The crude product was subjected to a column purification. The column was filled with 2 kg of normal phase silica gel (200-300 mesh), added with 200 ml of triethylamine for neutralizing

the acidity of the silica gel, equilibrated with petroleum ether containing 1wt% triethylamine, and eluted with a gradient elution of petroleum ether: ethyl acetate: dichloromethane: N,N-dimethylformamide=1: 1: 1: 0.5 to 1: 1: 1: 0.6. The eluate was collected, and the solvent was removed by evaporation under reduced pressure to give 40.4 g of pure product L-7. $^{1}$H NMR (400 MHz, DMSO) δ7.90 - 7.78 (m, 4H), 7.75 - 7.64 (m, 1H), 7.38 - 7.18 (m, 9H), 6.91 - 6.83 (m, 4H), 5.25 - 5.10 (m, 4H), 4.97 (dd, *J* = 11.2, 3.2 Hz, 3H), 4.48 - 4.30 (m, 4H), 4.02 (s, 9H), 3.93 - 3.84 (m, 3H), 3.76 - 3.66 (m, 9H), 3.45 - 3.35 (m, 3H), 3.24 - 2.98 (m, 10H), 2.30 - 2.20 (m, 2H), 2.11 - 1.88 (m, 31H), 1.80 - 1.40 (m, 28H). MS m/z: $C_{90}H_{128}N_7O_{35}$, [M-DMTr]$^+$, called: 1564.65, measured: 1564.88.

(1-1-4) Synthesis of L-9

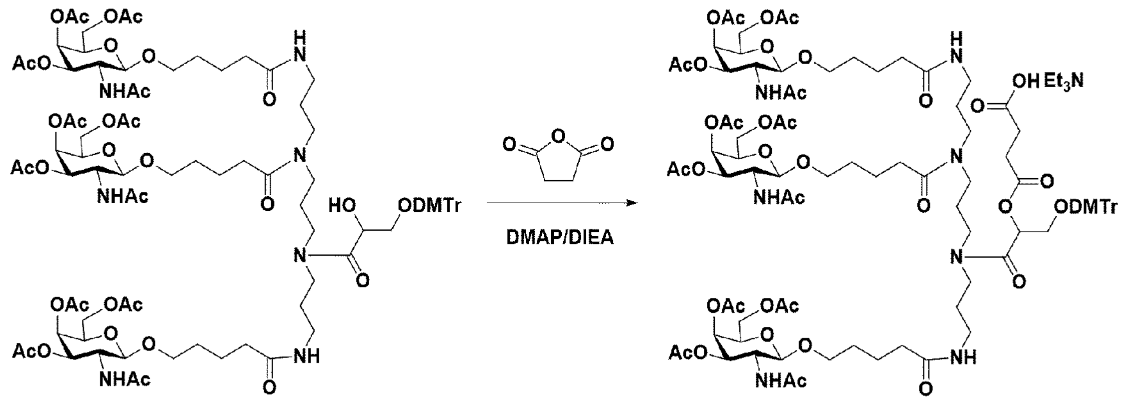


L-7 L-9

L-7 (40 g, 21.4247 mmol) obtained in step (1-1-3b), succinic anhydride (4.288 g, 42.8494 mmol) and 4-dimethylaminopyridine (DMAP, 5.235 g, 42.8494 mmol) were mixed and dissolved in 215 ml of dichloromethane, further added with diisopropylethylamine (DIPEA, 13.845 g, 107.1235 mmol), and stirred at 25°C for 24 hours. The reaction solution was washed with 800 ml of 0.5 M triethylamine phosphate. An aqueous phase was extracted three times, each with 5 ml of dichloromethane. All organic phases were combined, and the solvent was evaporated under reduced pressure to give a crude product. The crude product was subjected to a column purification. The column was filled with 1 kg normal phase silica gel (200-300 mesh), added with 1 wt% triethylamine for neutralizing the acidity of the silica gel, equilibrated with dichloromethane and eluted with a gradient elution of 1wt‰ triethylamine-containing dichloromethane: methanol=100: 18 to 100: 20. The eluate was collected, and the solvent was evaporated under reduced pressure to give 31.0 g of pure product of L-9 conjugating molecule. $^{1}$H NMR (400 MHz, DMSO) δ 8.58 (d, *J*= 4.2 Hz, 1H), 7.94 - 7.82 (m, 3H), 7.41 - 7.29 (m, 5H), 7.22 (d, *J* = 8.1 Hz, 5H), 6.89 (d, *J*= 8.3 Hz, 4H), 5.49 - 5.37 (m, 1H), 5.21 (d, *J*= 3.0 Hz, 3H), 4.97 (d, *J*= 11.1 Hz, 3H), 4.49 (d, *J*= 8.2 Hz, 3H), 4.02 (s, 9H), 3.88 (dd, *J*= 19.4, 9.4 Hz, 3H), 3.77 - 3.65 (m, 9H), 3.50 - 3.39 (m, 6H), 3.11 - 2.90 (m, 5H), 2.61 - 2.54 (m, 4H), 2.47 - 2.41 (m, 2H), 2.26 - 2.17 (m, 2H), 2.15 - 1.95 (m, 22H), 1.92 - 1.84 (m, 9H), 1.80 - 1.70 (m, 10H), 1.65 - 1.35 (m, 17H), 1.31 - 1.19 (m, 4H), 0.96 (t, *J* = 7.1 Hz, 9H). MS m/z: $C_{94}H_{132}N_7O_{38}$, [M-DMTr]$^+$, called: 1664.72, measured: 1665.03.

(1-1-5) Synthesis of compound L-10

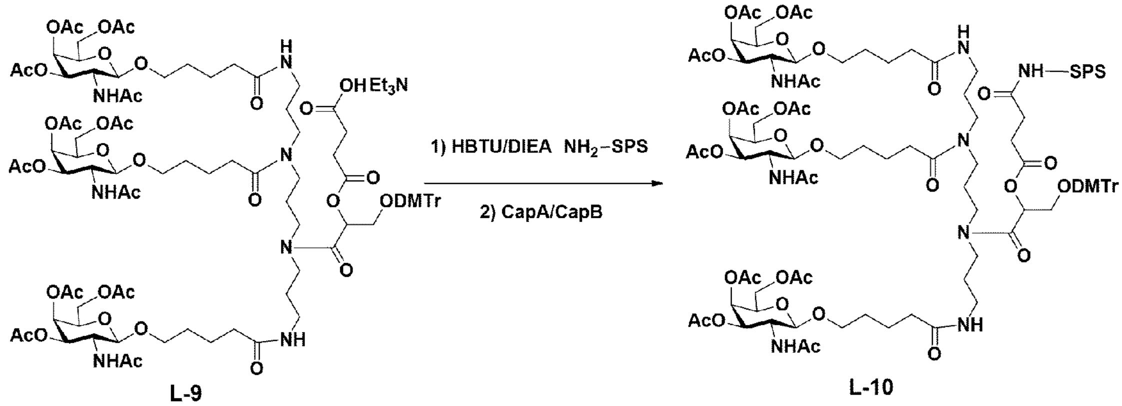


L-9

L-10

In this step, the compound L-10 was prepared by linking the L-9 conjugating molecule to a solid phase support.

The L-9 conjugating molecule (22.751 g, 11 mmol) obtained in step (1-1-4), O-benzotriazol-1-yl-tetramethyluronium hexafluorophosphate (HBTU, 6.257 g, 16.5 mmol) and diisopropylethylamine (DIEA, 2.843 g, 22 mmol) were mixed and dissolved in 900 ml of acetonitrile, and stirred at room temperature for 5 minutes. Aminomethyl resin (88 g, 100-200 mesh, amino loading: 400 μmol/g, purchased from Tianjin Nankai HECHENG S&T Co., Ltd.) was added into the reaction liquid. A reaction was performed on a shaker at 25°C and 150 rpm/min for 18 hours, followed by filtration. The residue was rinsed twice, each with 300 ml of DCM, and rinsed three times, each with 300 ml of acetonitrile, and dried for 18 hours with a vacuum oil pump. Then a capping reaction was performed by adding starting materials (CapA, CapB, 4-dimethylaminopyridine (DMAP) and acetonitrile) according to the charge ratio shown in Table 2. A reaction was performed on a shaker at 25°C and 150 rpm/min for 5 hours. The reaction liquid was filtrated. The residue was rinsed three times, each with 300 ml of acetonitrile, the solvent was evaporated to dryness, and the mixture was dried overnight under a reduced pressure with a vacuum oil pump to give 102 g of compound L-10 (i.e., the L-9 conjugating molecule linked to the solid phase support), with a loading of 90.8 μmol/g.

Table 2 The charge ratio of capping reaction

| Starting materials | Amount | Grade | Lot No. | Manufacturer |
|---|---|---|---|---|
| CapA | 1980 ml | - | - | - |
| CapB | 220 ml | - | - | - |
| DMAP | 1.100 g | Analytical pure | 11422139 | Aladdin |
| Acetonitrile | 220 ml | Spectroscopic pure | 015161001 | CINC (Shanghai) Co., Ltd |

In the above table, CapA and CapB are solutions of capping agents. CapA is a solution of 20% by volume of N-methylimidazole in a mixture of pyridine/acetonitrile, wherein the volume ratio of the pyridine to the acetonitrile is 3: 5. CapB is a solution of 20% by volume of acetic anhydride in acetonitrile.

(1-2) Synthesis of sense strand of conjugate 1

Nucleoside monomers were linked one by one in the direction from 3' to 5' according to the arrangement sequence of nucleotides in the sense strand by the solid phase phosphoramidite method, starting the cycles from the Compound L-10 prepared in the above step, to synthesize the sense strand SS of the conjugate 1 in Table 3. The linking of each nucleoside monomer comprised a four-step reaction of deprotection, coupling, capping, and oxidation or sulfurization. When two nucleotides are linked via a phosphoester, a four-step reaction of deprotection, coupling, capping, and oxidation was comprised during linking of the later nucleoside monomer. When two nucleotides are linked via a phosphorothioate, a four-step reaction of deprotection, coupling, capping, and sulfurization was comprised during linking of the later nucleoside monomer. The synthesis condition was given as follows.

The nucleoside monomers were provided in a 0.1 M acetonitrile solution. The condition for deprotection reaction in each step was identical, i.e., a temperature of 25°C, a reaction time of 70 seconds, a solution of dichloroacetic acid in dichloromethane (3% v/v) as a deprotection agent, and a molar ratio of the dichloroacetic acid to the protecting group 4,4'-dimethoxytrityl on the solid phase support of 5: 1.

The condition for coupling reaction in each step was identical, comprising a temperature of 25°C, a molar ratio of the nucleic acid sequence linked to the solid phase support to the nucleoside monomers of 1: 10, a molar ratio of the

nucleic acid sequence linked to the solid phase support to a coupling agent of 1: 65, a reaction time of 600 seconds, and 0.5 M acetonitrile solution of 5-ethylthio-1H-tetrazole (ETT) as a coupling agent.

The condition for capping reaction in each step was identical, comprising a temperature of 25°C and a reaction time of 15 seconds. A capping agent was a mixed solution of Cap A and Cap B in a molar ratio of 1: 1, and a molar ratio of the capping agent to the nucleic acid sequence linked to the solid phase support was 1: 1: 1 (anhydride: N-methylimidazole: the nucleic acid sequence linked to the solid phase support).

The condition for oxidation reaction in each step was identical, comprising a temperature of 25°C, a reaction time of 15 seconds, and 0.05 M iodine water as an oxidation agent. A molar ratio of iodine to the nucleic acid sequence linked to the solid phase support in the coupling step was 30: 1. The reaction was carried out in a mixed solvent in which the ratio of tetrahydrofuran: water: pyridine was 3: 1: 1.

The condition for sulfurization reaction in each step was identical, comprising a temperature of 25°C, a reaction time of 300 seconds, and xanthane hydride as a sulfurization agent. A molar ratio of the sulfurization agent to the nucleic acid sequence linked to the solid phase support in the coupling step was 120: 1. The reaction was carried out in a mixed solvent in which the ratio of acetonitrile: pyridine was 1:1.

After the last nucleoside monomer was linked, the nucleic acid sequence linked to the solid phase support was cleaved, deprotected, purified and desalted in turn, and then freeze-dried to obtain the sense strand, wherein,

The conditions for cleavage and deprotection were as follows: adding the synthesized nucleotide sequence linked to the support into 25 wt% aqueous ammonia to react for 16 hours at 55°C, wherein the aqueous ammonia was in an amount of 0.5 ml/μmol; filtering to remove the support, and concentrating the supernatant in vacuum to dryness.

The conditions for purification and desalination were as follows: purifying the nucleic acid by using a preparative ion chromatography column (Source 15Q) with a gradient elution of NaCl. Specifically, eluent A: 20 mM sodium phosphate (pH 8.1), solvent: water/acetonitrile = 9: 1 (v/v); eluent B: 1.5 M sodium chloride, 20 mM sodium phosphate (pH 8.1), solvent: water/acetonitrile = 9: 1 (v/v); elution gradient: eluent A: eluent B = 100: 0 to 50: 50. The eluate was collected, combined and desalted by using a reverse phase chromatography purification column. The specific conditions comprised using a Sephadex column (filler: Sephadex-G25) for desalination and deionized water for eluting.

The detection method was as follows: determining the purity of the sense strand above by ion exchange chromatography (IEX-HPLC); and analyzing the molecular weight by Liquid Chromatography-Mass Spectrometry (LC-MS). The measured value was in conformity with the called value, indicating that a sense strand SS conjugated with L-9 conjugating molecule at 3' terminal was synthesized.

(1-3) Synthesis of antisense strand of conjugate 1

The antisense strand AS of the conjugate 1 in Table 3 was synthesized by starting the cycles using a universal solid phase support (UnyLinker™ loaded NittoPhase®HL Solid Supports, Kinovate Life Sciences Inc.) according to the solid phase phosphoramidite method. The deprotection, coupling, capping, oxidation or sulfurization reaction conditions, cleavage and deprotection, purification and desalting conditions in the solid phase synthesis method were conducted under the same conditions as those in the synthesis of the sense strand. The difference was that antisense strand had 5'-phosphoric acid at the first nucleotide of the 5'-terminal. Therefore, in the process of preparing the antisense strand according to the solid phase phosphoramidite method, a CPR-I monomer (Suzhou GenePharma, Article No. Cat#13-2601-XX) was linked to the 5' terminal of the antisense strand to form 5'-phosphoester modification by four steps of deprotection, coupling, capping and oxidation after the last nucleoside monomer of the antisense strand was linked.

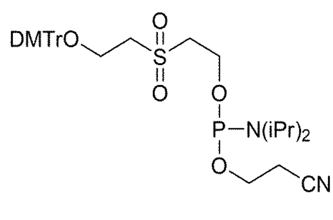

(CPR-I)

In this linkage, the deprotection, coupling, capping and oxidation reaction conditions, cleavage and deprotection, purification and desalting conditions used were the same as those in the synthesis of the sense strand. The residue was freeze-dried to obtain the antisense strand subsequently. The purity of the antisense strand was detected by ion exchange chromatography (IEX-HPLC), and the molecular weight was analyzed by liquid chromatography-mass spectrometry (LC-MS). The measured value was in conformity with the called value, indicating that an antisense strand AS having a

target sequence was synthesized.

(1-4) Synthesis of conjugate 1

The sense strand and the antisense strand were respectively dissolved in water for injection to give a solution of 40 mg/mL, mixed at an equimolar ratio, heated at 50°C for 15 minutes, and then cooled at room temperature, such that an annealed product was obtained and then freeze-dried to obtain lyophilized powder. The conjugate was diluted to a concentration of 0.2 mg/mL with ultra-pure water (prepared by Milli-Q ultra-pure water instrument, with resistivity of 18.2 MΩ*cm (25°C)). The molecular weight was measured by Liquid Chromatography-Mass Spectrometry (LC-MS, purchased from Waters Corp., model: LCT Premier). Since the measured value was in conformity with the called value, it was confirmed that the synthesized conjugate 1 was the designed double stranded nucleic acid sequence of interest with the L-9 conjugating molecule. The structure of the conjugate 1 was as shown in Formula (403), the conjugated siRNA sequence corresponds to the sequence of the conjugate 1 (i.e., L10-siC5a1M1SP) as shown in Table 3.

Preparation Example 2 Preparation of conjugates 2-8

The conjugates 2-6 shown in Table 3 were synthesized by the same method as that in Preparation Example 1, and the molecular weights were detected. The difference was that the sequences of the sense strands and antisense strands used in the synthesis were the sequences shown in Table 3 corresponding to the sense strands and antisense strands of the siRNAs conjugated in the conjugate 2, the conjugate 3, the conjugate 4, the conjugate 5 or the conjugate 6, respectively, thereby obtaining the conjugates 2-6 respectively.

The conjugates 7-8 shown in Table 3 were synthesized by the same method as that in Preparation Example 1, and the molecular weights were detected. The difference was that the sequence of the sequences of the sense strands and antisense strands used in the synthesis were the sequences shown in Table 3 corresponding to the sense strands and antisense strands of the siRNAs conjugated in the conjugate 7 or the conjugate 8, respectively, wherein the antisense strands of the conjugate 7 and the conjugate 8 do not have the 5'-phosphoric acid in the first nucleotides at the 5'-terminals thereof in comparison to the antisense strand of the antisense strand. Therefore, in the process of preparing the antisense strands according to the solid phase phosphoramidite method, it is not necessary to link the CPR-I monomer after linking the last nucleoside monomer of the antisense strand, thereby obtaining the conjugates 7-8 respectively.

Table 3 lists the conjugate numbers and the sequence compositions of the siRNAs.

Table 3 siRNA conjugates

| Conjugate | No. | Sequence direction5'-3' | | SEQ ID NO |
|---|---|---|---|---|
| Conjugate 1 | L10-siC5a1 M1SP | Sense strand | CmsUmsUmCmAmUmUfCfAfUmAmCmA mGmAmCmAmAmAm | 361 |
| | | Antisense strand | PUmsUfsUmGmUmCfUmGmUmAmUmG mAmAfUmGfAmAmGmsAmsGm | 362 |
| Conjugate 2 | L10-siC5b1 M1SP | Sense strand | CmsUmsAmCmAmGmUfUfUfAmGmAm AmGmAmUmUmUmAm | 363 |
| | | Antisense strand | PUmsAfsAmAmUmCfUmUmCmUmAmA mAmCfUmGfUmAmGmsUmsAm | 364 |

(continued)

| Conjugate | No. | Sequence direction5'-3' | | SEQ ID NO |
|---|---|---|---|---|
| Conjugate 3 | L10-siC5c1 M1SP | Sense strand | GmsGmsAmAmGmGmUfUfAfCmCmGm AmGmCmAmAmUmAm | 365 |
| | | Antisense strand | PUmsAfsUmUmGmCfUmCmGmGmUmA mAmCfCmUfUmCmCmsCmsUm | 366 |
| Conjugate 4 | L10-siC5d1 M1SP | Sense strand | AmsGmsAmAmCmAmGfAfCfAmGmCmA mGmAmAmUmUmAm | 367 |
| | | Antisense strand | PUmsAfsAmUmUmCfUmGmCmUmGmU mCmUfGmUfUmCmUmsCmsCm | 368 |
| Conjugate 5 | L10-siC5e1 M1SP | Sense strand | CmsCmsAmAmGmAmAfGfAfAmCmGmC mUmGmCmAmAmAm | 369 |
| | | Antisense strand | PUmsUfsUmGmCmAfGmCmGmUmUmC mUmUfCmUfUmGmGmsCmsCm | 370 |
| Conjugate 6 | L10-siC5f1 M1SP | Sense strand | CmsCmsAmGmUmAmAfGfCfAmAmGmC mCmAmGmAmAmAm | 371 |
| | | Antisense strand | PUmsUfsUmCmUmGfGmCmUmUmGmC mUmUfAmCfUmGmGmsUmsAm | 372 |
| Conjugate 7 | L10-siC5c1 MIS | Sense strand | GmsGmsAmAmGmGmUfUfAfCmCmGm AmGmCmAmAmUmAm | 377 |
| | | Antisense strand | UmsAfsUmUmGmCfUmCmGmGmUmAm AmCfCmUfUmCmCmsCmsUm | 378 |
| Conjugate 8 | L10-siC5d1 MIS | Sense strand | AmsGmsAmAmCmAmGfAfCfAmGmCmA mGmAmAmUmUmAm | 379 |
| | | Anti sense strand | UmsAfsAmUmUmCfUmGmCmUmGmUm CmUfGmUfUmCmUmsCmsCm | 380 |

Preparation Example 3 Synthesis of siRNA sequence

The siRNA 1 shown in Table 4a was synthesized by the same method as that in Preparation Example 1, and the difference was that:

1) for the sense strand, the cycles were started using a universal solid phase support (UnyLinker™ loaded NittoPhase®HL Solid Supports, Kinovate Life Sciences Inc.); and

2) for the antisense strand, compared with the antisense strand sequence of the siRNA conjugated in the conjugate 1, the first nucleotide at the 5'-terminal of the siRNA 1 had no 5'-phosphoric acid. Therefore, in the process of preparing the antisense strands according to the solid phase phosphoramidite method, it was not necessary to link the CPR-I monomer after linking the last nucleoside monomer of the antisense strand.

In this way, the siRNA 1 was prepared.

The siRNAs 2-6 were synthesized by the same method as that in preparing the siRNA 1, and the difference was that: the sequences of the sense strands and the antisense strands of the siRNAs used in the synthesis were the sequences as shown in Table 4 corresponding to the sense strands and the antisense strands of the siRNA 2, the siRNA 3, the siRNA 4, the siRNA 5, or the siRNA 6 respectively, thus obtaining the siRNAs 2-6 respectively.

Table 4a lists the siRNA numbers and siRNA sequence compositions.

Table 4a siRNA sequences

| siRNA | No. | Sequence direction 5'-3' | | SEQ ID NO |
|---|---|---|---|---|
| siRNA 1 | siC5a1M1S | Sense strand | CmsUmsUmCmAmUmUfCfAfUmAmCmAmGmAmCmAmAmAm | 373 |
| | | Antisense strand | UmsUfsUmGmUmCfUmGmUmAmUmGmAmAfUmGfAmAmGmsAmsGm | 374 |
| siRNA 2 | siC5b1M1S | Sense strand | CmsUmsAmCmAmGmUfUfUfAmGmAmAmGmAmUmUmUmAm | 375 |
| | | Antisense strand | UmsAfsAmAmUmCfUmUmCmUmAmAmAmCfUmGfUmAmGmsUmsAm | 376 |
| siRNA 3 | siC5c1M1S | Sense strand | GmsGmsAmAmGmGmUfUfAfCmCmGmAmGmCmAmAmUmAm | 377 |
| | | Anti sense strand | UmsAfsUmUmGmCfUmCmGmGmUmAmAmCfCmUfUmCmCmsCmsUm | 378 |
| siRNA 4 | siC5d1M1S | Sense strand | AmsGmsAmAmCmAmGfAfCfAmGmCmAmGmAmAmUmUmAm | 379 |
| | | Antisense strand | UmsAfsAmUmUmCfUmGmCmUmGmUmCmUfGmUfUmCmUmsCmsCm | 380 |
| siRNA 5 | siC5e1M1S | Sense strand | CmsCmsAmAmGmAmAfGfAfAmCmGmCmUmGmCmAmAmAm | 381 |
| | | Antisense strand | UmsUfsUmGmCmAfGmCmGmUmUmCmUmUfCmUfUmGmGmsCmsCm | 382 |

(continued)

| siRNA | No. | Sequence direction 5'-3' | | SEQ ID NO |
|---|---|---|---|---|
| siRNA 6 | siC5f1M1S | Sense strand | CmsCmsAmGmUmAmAfGfCfAmAmGmCmCmAmGmAmAmAm | 383 |
| | | Antisense strand | UmsUfsUmCmUmGfGmCmUmUmGmCmUmUfAmCfUmGmGmsUmsAm | 384 |

Preparation Example 4 Synthesis of Cy5-labeled siRNA conjugates and Cy5-labeled siRNAs (4-1) Synthesis of Cy5-conjugate 1 and Cy5-conjugate 2

The Cy5-conjugate 1 shown in Table 4b was synthesized by the same method as that in Preparation Example 1, and the molecular weight was detected. The difference was that the sequences of the sense strand and the antisense strand used in the synthesis were the sequences shown in Table 4b corresponding to the sense strand and the antisense strand of the siRNA conjugated in the Cy5-conjugate 1, wherein (1): a Cy5 fluorescent group was covalently linked to the 5' terminal of the sense strand of the siRNA conjugated in the Cy5-conjugate 1. Therefore, in the process of preparing the sense strand according to the solid phase phosphoramidite method described in step (1-2) of the Preparation Example 1, after linking the last nucleoside monomer of the sense strand, a Cy5 phosphoramidite monomer (purchased from Shanghai HonGene Biotech, with a article number of OP-057) needed to be linked to the 5' terminal of the sense strand through the four-step reaction of deprotection, coupling, capping, and oxidation; and (2) the first nucleotide at the 5'-terminal of the siRNA conjugated in the Cy5-conjugate 1 had no 5'-phosphoric acid; therefore, in the process of preparing the antisense strands according to the solid phase phosphoramidite method described in step (1-3) of the Preparation Example 1, it was not necessary to link the CPR-I monomer after linking the last nucleoside monomer of the antisense strand.

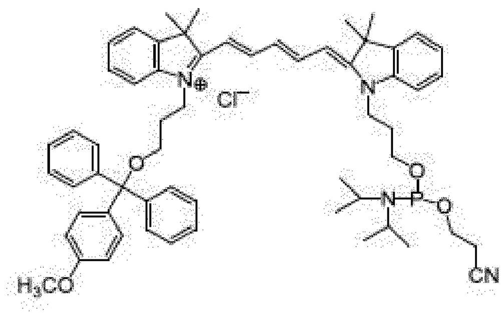

(Cy5 phosphoramidite monomer)

In the process of linking the Cy5 phosphoramidite monomer to the 5' terminal of the sense strand, the deprotection, coupling, capping and oxidation reaction conditions used were the same as those described in the synthesis of the sense strand in step (1-2) of the Preparation Example 1, and the differences were that: 1) the deprotection reaction time was extended to 300 seconds; and 2) the Cy5 coupling reaction time was extended to 900 seconds.

Then, the conditions for cleavage and deprotection of the sense strand were as follows: adding the synthesized nucleotide sequence linked with a support into an AMA solution (mixed solution of 40wt% methylamine aqueous solution and 25wt% ammonia water with a volume ratio of 1: 1), wherein the amount of the AMA solution was 0.5 ml/μmol, reacting for 2 hours in a water bath at 25°C, removing the remaining support by filtration, and concentrating the supernatant to dryness in vacuum. The conditions for purification and desalination of the sense strand were the same as those of the synthesis of the sense strand in step (1-2) in the Preparation Example 1. Then the residue was freeze-dried to obtain the sense strand of the Cy5-conjugate 1.

Thereby, the Cy5-conjugate 1 was obtained, and a Cy5 fluorescent group was covalently linked to the 5' terminal of the sense strand of the siRNA of the siRNA conjugate, which had the sequences of the sense strand and the antisense strand shown in Table 4b corresponding to the Cy5-conjugate 1.

The Cy5-conjugate 2 was prepared by the same method as that in preparing the Cy5-conjugate 1, and the

molecular weight was detected. The difference was that the sequences of the sense strands and antisense strands used in the synthesis were the sequences shown in Table 4b corresponding to the sense strand and antisense strand of the siRNAs conjugated in the Cy5-conjugate 2 respectively, thereby obtaining the Cy5-conjugate 2.

(4-2) Synthesis of Cy5-siRNA 1 and Cy5-siRNA 2

The Cy5-siRNA 1 was prepared by the same method as that in preparing the Cy5-conjugate 1, and the molecular weight was detected. The difference was that the cycles were started using a universal solid phase support (UnyLinker™ loaded NittoPhase®HL Solid Supports, Kinovate Life Sciences Inc.), thus obtaining the Cy5-siRNA 1.

The Cy5-siRNA 2 was prepared by the same method as that in preparing the Cy5-siRNA 1, and the molecular weight was detected. The difference was that the sequences of the sense strands and antisense strands used in the synthesis were the sequences shown in Table 4b corresponding to the sense strand and antisense strand of the Cy5-siRNA respectively, thus obtaining the Cy5-siRNA 2.

Table 4b lists the numbers and siRNA sequences of the Cy5-conjugate 1, the Cy5-conjugate 2, the Cy5-siRNA 1 and the Cy5-siRNA 2.

Table 4b siRNA sequences in Cy5-labeled siRNA conjugates and Cy5-labeled siRNAs

| SiRNA or conjugate | No. | Sequence direction 5'-3' | | SEQ ID NO |
|---|---|---|---|---|
| Cy5-siRNA 1 | Cy5-siC5c 1M1S | Sense strand | GmsGmsAmAmGmGmUfUfAfCmCmGm AmGmCmAmAmUmAm | 377 |
| | | Antisense strand | UmsAfsUmUmGmCfUmCmGmGmUmA mAmCfCmUfUmCmCmsCmsUm | 378 |
| Cy5-siRNA 2 | Cy5-siC5f 1M1S | Sense strand | CmsCmsAmGmUmAmAfGfCfAmAmGm CmCmAmGmAmAmAm | 383 |
| | | Antisense strand | UmsUfsUmCmUmGfGmCmUmUmGmC mUmUfAmCfUmGmGmsUmsAm | 384 |
| Cy5-conjugate 1 | Cy5-L10-s iC5c1M1S | Sense strand | GmsGmsAmAmGmGmUfUfAfCmCmGm AmGmCmAmAmUmAm | 377 |
| | | Antisense strand | UmsAfsUmUmGmCfUmCmGmGmUmA mAmCfCmUfUmCmCmsCmsUm | 378 |
| Cy5-conjugate 2 | Cy5-L10-s iC5f1M1S | Sense strand | CmsCmsAmGmUmAmAfGfCfAmAmGm CmCmAmGmAmAmAm | 383 |
| | | Antisense strand | UmsUfsUmCmUmGfGmCmUmUmGmC mUmUfAmCfUmGmGmsUmsAm | 384 |

Experimental Example 1 $IC_{50}$ determination of C5 mRNA in HepG2 cells by siRNA conjugate

HepG2 cells (purchased from Nanjing COBIOER Biotechnology Co., Ltd.) were cultured in H-DMEM complete media (HyClone company) containing 10% fetal bovine serum (FBS, RMBIO company) and 0.2v% Penicillin-Streptomycin (HyClone company) at 37°C in an incubator containing 5% $CO_2$/95% air.

HepG2 cells were seeded in a 24-well plate with $7\times10^4$ cells/well. After 16 hours, when the growth density of

the cells reached 70-80%, the H-DMEM complete media in the culture wells were sucked up, and 500 μl of Opti-MEM medium (GIBCO company) was added to each well to continue the culture for 1.5 hours.

The conjugate 1, the conjugate 2, the conjugate 3, the conjugate 4, the conjugate 5, the conjugate 6, the conjugate 7 and the conjugate 8 were each prepared to be conjugate working solution with 7 different concentrations comprising 20 μM, 5 μM, 1.25 μM, 0.313 μM, 0.0781 μM, 0.0195 μM and 0.0049 μM (based on the amount of siRNA) with DEPC water respectively.

For each conjugate, A1-A7 solution was prepared, and each solution contained 3 μl of the conjugate working solution with 7 concentrations as demonstrated above in turn and 50 μl of Opti-MEM medium.

For each conjugate, B solution was prepared separately, and each B solution contained 1 μl of Lipofectamine™ 2000 and 50 μl of Opti-MEM medium.

For each conjugate, one portion of the B solution was mixed with one portion of the A1-A7 solution in turn, and then incubated for 20 minutes at room temperature to obtain transfection complexes X1-X7. Two portions were prepared for each transfection complex.

One portion of the B solution was mixed with 50 μl of Opti-MEM medium, and incubated for 20 minutes at room temperature to obtain a transfection complex X8. Four portions of the transfection complex were prepared.

Transfection complexes X1-X7 corresponding to each conjugate were added into the above culture wells for culturing HepG2 cells, and evenly mixed with the addition amount of 100 μl/ well to obtain transfection mixtures with final concentrations (based on the siRNA) of 100 nM, 25 nM, 6.25 nM, 1.56 nM, 0.391 nM, 0.098 nM and 0.024 nM respectively. Two transfection complexes X1-X7 with the same concentration were added into two different culture wells to obtain transfection mixtures containing conjugates, which were labeled as test groups 1-7.

In the other four culture wells, one portion of transfection complex X8 was added in 100 μl/ well to obtain transfection mixture without conjugate, which was labeled as a control group.

After incubating the transfection mixture containing conjugate and the transfection mixture without conjugate with cells in the culture wells for 4 hours, each well was supplemented with 1 ml of H-DMEM complete media containing 20% FBS. The 24-well plate was placed in an incubator containing 5% $CO_2$/95% air for 24 hours.

Then, RNAVzol (purchased from Vigorous Biotechnology Beijing Co., Ltd.,article No. N002) was used to extract the total RNA of the cells in each well according to the operation steps of total RNA extraction in the specification.

1 μg of total RNA was taken as the template for reverse transcription for the cells in each well, and the reagent provided by the reverse transcription kit Goldenstar™ RT6 cDNA Synthesis Kit (purchased from Beijing Tsingke Biotechnology Co., Ltd., article No. TSK301M) was used, wherein Goldenstar™ Oligo $(dT)_{17}$ was selected as the primer, and 20 μl of reverse transcription reaction system was configured according to the reverse transcription operation steps in the kit manual, so as to reverse the total RNA of the cells in each well. The conditions for reverse transcription were as follows: the reverse transcription reaction system was incubated at 50°C for 50 minutes, then incubated at 85°C for 5 minutes, and finally incubated at 4°C for 30 seconds. After the reaction, 80 μl of DEPC water was added to the reverse transcription reaction system to obtain a solution containing cDNA.

For each reverse transcription reaction system, 5 μl of the solution containing cDNA was taken as the template of qPCR, and 20 μl of qPCR reaction system was prepared by using the reagent provided by NovoStart® SYBR qPCR SuperMix Plus (purchased from Novoprotein Science and Technology Co., Ltd., article No. E096-01B), wherein the seqeces of the PCR primers for amplifying the target gene C5 and internal reference gene GAPDH were shown in Table 5, and the final concentration of each primer was 0.25 μM. The qPCR reaction system was placed on ABI StepOnePlus Real-Time PCR instrument, and amplified by three-step method. The amplification procedure was pre-denatured at 95°C for 10 minutes, then denatured at 95°C for 30 seconds, annealed at 60°C for 30 seconds, and extended at 72°C for 30 seconds. After repeating the above denaturation, annealing and extension processes for 40 times, the product W containing amplified target gene C5 and internal reference gene GAPDH was obtained. The product W was then incubated at 95°C for 15 seconds, 60°C for 1 minute and 95°C for 15 seconds in turn. The dissolution curves of the target gene C5 and the internal reference gene GAPDH in the product W were collected by real-time fluorescence quantitative PCR, and the Ct values of the target gene C5 and the internal reference gene GAPDH were obtained.

Table 5: Sequences of the Detection Primers

| Gene | Upstream primer (5'-3' direction) | Downstream primer (5'-3' direction) |
|---|---|---|
| Human C5 | ATCAGGCCAGGGAAGGTTAC(SEQ ID NO: 385) | TCGGGATGAAGGAACCATGT(SEQ ID NO: 386) |
| Human GAPDH | GGTCGGAGTCAACGGATTT (SEQ ID NO: 387) | CCAGCATCGCCCCACTTGA (SEQ ID NO: 388) |

Comparative Ct (ΔΔCt) method was used to calculate relative quantitative expression of the target gene C5 in

each test group and the control group. The calculation method was as follows:

ΔCt (test group) = Ct (target gene of test group) - Ct (internal reference gene of test group)

ΔCt (control group) = Ct (target gene of control group) - Ct (internal reference gene of control group)

ΔΔCt (test group) = ΔCt (test group) - ΔCt (control group)

ΔΔCt (control group) = ΔCt (control group) - ΔCt (mean value of control group)

wherein, ΔCt (mean value of control group) was the arithmetic mean value of ΔCt (control group) of each of the four culture wells of the control group. Therefore, each culture well of each test group corresponded to one ΔΔCt (test group), and each culture well of the control group corresponded to one ΔΔCt (control group).

On the basis of the control group, the expression level of C5 mRNA in the test group was normalized, and the expression level of C5 mRNA in the control group was defined as 100%.

The relative expression level of C5 mRNA in the test group = 2^ (-ΔΔCt (test group)) × 100%.

According to the relative expression level of C5 mRNA in HepG2 cells transfected with different conjugates (conjugates 1-8), dose-response curves of the conjugates 1-8 as shown in FIGs. 1A-1H were obtained by fitting the log(inhibitor) vs. response (three parameters of Graphpad 5.0 software, wherein the logarithmic value (lg nM) of the final concentration of the siRNA conjugate was taken as abscissa and the relative expression level (%) of C5 mRNA was taken as ordinate, and each dot represented the mean value of the relative expression level of C5 mRNA in two culture wells of the test group compared with the control group.

The $IC_{50}$ of each conjugate towards C5 mRNA was calculated according to a function corresponding to the fitted dose-effect curve, wherein the function was as follows:

$$Y = \mathrm{Bot} + \frac{\mathrm{Top}-\mathrm{Bot}}{1+10^{(X'-X)\times \mathrm{HillSlope}}}$$

wherein:

Y is the relative expression level of C5 mRNA of the test group,

X is the logarithmic value of the final concentration of the siRNA conjugate,

Bot is the Y value at the bottom of the steady stage,

Top is the Y value at the top of the steady stage, and

X' is the X value at which Y is median value between the bottom and the top of the asymptote, and HillSlope is the slope of the curve at X', which is defined as -1 here.

According to the dose-effect curve and the corresponding function, the corresponding $X_{50}$ value when Y=50% was determined, and the $IC_{50}$ value of each conjugate was calculated to be $10^{X_{50}}$ (nM).

The $IC_{50}$ value towards C5 mRNA and $R^2$ value of the fitted curve of each conjugate are summarized in Table 6.

Table 6 $IC_{50}$ value towards C5 mRNA and $R^2$ value of the fitted dose-response curve of siRNA conjugate

| Conjugate | No. | $IC_{50}$ | $R^2$ |
|---|---|---|---|
| Conjugate 1 | L10-siC5a1M1SP | 9.688 nM | 0.9513 |
| Conjugate 2 | L10-siC5b1M1SP | 9.566 nM | 0.9609 |
| Conjugate 3 | L10-siC5c1M1SP | 2.861 nM | 0.9958 |
| Conjugate 4 | L10-siC5d1M1SP | 1.494 nM | 0.9418 |
| Conjugate 5 | L10-siC5e1M1SP | 4.077 nM | 0.9942 |

(continued)

| Conjugate | No. | $IC_{50}$ | $R^2$ |
|---|---|---|---|
| Conjugate 6 | L10-siC5f1M1SP | 3.795 nM | 0.9817 |
| Conjugate 7 | L10-siC5c1M1S | 3.023 nM | 0.9975 |
| Conjugate 8 | L10-siC5d1M1S | 1.629 nM | 0.9961 |

It can be seen from the results in FIGs. 1A-1H and the Table 6 above that the siRNA conjugate provided by the present disclosure has higher inhibitory activity in HepG2 hepatoma cells *in vitro,* and the $IC_{50}$ is between 1.494 nM and 9.688 nM.

Experimental Example 2 Distribution of siRNA conjugates in various organs of C57 mice 1 × PBS solution was used to respectively dissolve Cy5-siRNA 1, Cy5-conjugate 1, Cy5-siRNA 2 or Cy5-conjugate 2 into 0.6 mg/ml solution (based on siRNA).

Ten C57BL/6J female mice of 6-7 weeks (purchased from Beijing Charles River Laboratory Animal Technology Co., Ltd., referred to as C57 mice for short) were randomly divided into groups with two mice in each group, and subcutaneously injected with 1 × PBS and the Cy5-siRNA 1, the Cy5-conjugate 1, the Cy5-siRNA 2 or the Cy5-conjugate 2 solution prepared above, respectively. The administration doses of all animals were calculated according to their body weights, and the administration volume was 5 ml/kg body weight. Based on the amount of siRNA, the administration dose of each animal was 3 mg/kg body weight.

After the administration for 2 hours, 6 hours, 24 hours and 48 hours, the mice of each group were placed in a small animal *in vivo* optical imaging system IVIS Lumina Series III. The mice were anesthetized with isoflurane gas, and the anesthetized mice were placed with the abdomens facing up for living imaging in the small animal *in vivo* optical imaging system to dynamically detect Cy5 fluorescence signals, and track the distribution of Cy5-labeled siRNAs or Cy5-labeled conjugates in living animals. Only the mice administered with the Cy5-labeled conjugates had significantly enhanced fluorescence signals in the liver areas thereof, and the mice administered with the Cy5-labeled siRNAs or 1 × PBS did not have any fluorescence signal in the liver areas thereof.

After 48 hours, all the mice were sacrificed, and five organs of each mouse, comprising heart, lung, liver, spleen and kidney, were taken out for fluorescence imaging in the IVIS Lumina Series III. One animal was selected from each group of mice, and the above-mentioned five organs thereof were arranged longitudinally in turn. The organs of the mice administered with 1 × PBS or the Cy5-siRNA 1 or Cy5-conjugate 1 were photographed under the same visual field, and the results were shown in FIG. 2A. The organs of the mice administered with 1 × PBS, and the Cy5-siRNA 2 or Cy5-conjugate 2 were photographed under the same visual field, and the results were shown in FIG. 2B.

It can be seen from FIGs. 2A-2B that only the Cy5-conjugate 1 and the Cy5-conjugate 2 can be aggregated in a large amount in the liver, and the Cy5-conjugate 1 and the Cy5-conjugate 2 are aggregated in a small amount in the kidney, but are not aggregated in other organs, indicating that the conjugate of the present disclosure can effectively deliver siRNA specifically to the liver. Furthermore, combined with the results of FIG. 1G (showing that the conjugate 7 has higher inhibitory activity *in vitro*) and FIG. IF (showing that the conjugate 6 has higher inhibitory activity *in vitro*), it indicates that the conjugate provided by the present disclosure can specifically inhibit the expression of the target gene in the liver.

Some embodiments of the present disclosure are described in detail above, but the present disclosure is not limited to the specific details of the above-described embodiments. Various simple variations of the technical solution of the present disclosure can be made within the scope of the technical concept of the present disclosure, and these simple variations are within the scope of the present disclosure.

In addition, it is to be noted that each of the specific technical features described in the above embodiments can be combined in any suitable manner as long as no contradiction is caused. In order to avoid unnecessary repetition, the various possible combination manners are no longer described in the present disclosure.

In addition, the various different embodiments of the present disclosure may also be carried out in any combination as long as it does not contravene the idea of the present disclosure, which should also be regarded as the disclosure of the present disclosure.

## Claims

**1.** An siRNA conjugate, wherein the conjugate has a structure as shown by Formula (308):

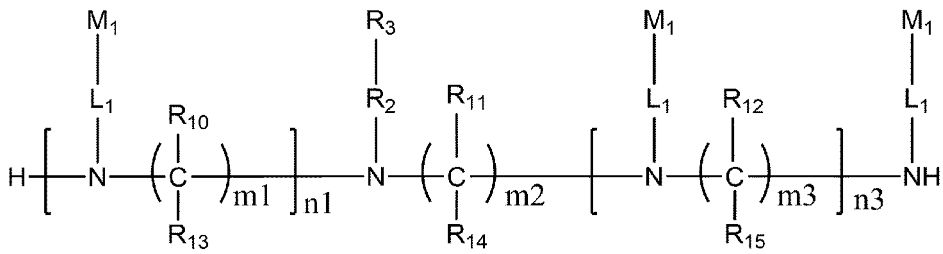

Formula (308),

wherein,

n1 is an integer of 1-3, and n3 is an integer of 0-4; each of m1, m2, and m3 is independently an integer of 2-10; each of $R_{10}$, $R_{11}$, $R_{12}$, $R_{13}$, $R_{14}$ or $R_{15}$ is independently H or selected from the group consisting of $C_1$-$C_{10}$ alkyl, $C_1$-$C_{10}$ haloalkyl and $C_1$-$C_{10}$ alkoxy;
$R_3$ is a group having a structure as shown by Formula A59:

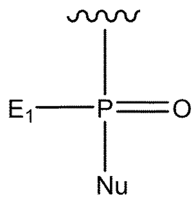

(A59)

wherein, $E_1$ is OH, SH or $BH_2$; and
Nu is siRNA; the siRNA comprises a sense strand and an antisense strand, each nucleotide in the siRNA is independently a modified or unmodified nucleotide, wherein the sense strand comprises a nucleotide sequence I, and the antisense strand comprises a nucleotide sequence II; the nucleotide sequence I and the nucleotide sequence II are at least partly reverse complementary to form a double-stranded region; and the nucleotide sequence I and the nucleotide sequence II are selected from a group of sequences shown in the following i) - vi):

i) the nucleotide sequence I has the same length and no more than three nucleotide differences from the nucleotide sequence shown in SEQ ID NO: 1; and the nucleotide sequence II has the same length and no more than three nucleotide differences from the nucleotide sequence shown in SEQ ID NO: 2:

5'-CUUCAUUCAUACAGACAA$Z_1$-3' (SEQ ID NO: 1);
5'-$Z_2$UUGUCUGUAUGAAUGAAG-3' (SEQ ID NO: 2),

wherein, $Z_1$ is A, $Z_2$ is U, the nucleotide sequence I comprises a nucleotide $Z_3$ at a corresponding site to $Z_1$, the nucleotide sequence II comprises a nucleotide $Z_4$ at a corresponding site to $Z_2$, and $Z_4$ is the first nucleotide from the 5' terminal of the antisense strand ;
ii) the nucleotide sequence I has the same length and no more than three nucleotide differences from the nucleotide sequence shown in SEQ ID NO: 61; and the nucleotide sequence II has the same length and no more than three nucleotide differences from the nucleotide sequence shown in SEQ ID NO: 62:

5'-CUACAGUUUAGAAGAUUU$Z_5$-3' (SEQ ID NO: 61);
5'-$Z_6$AAAUCUUCUAAACUGUAG-3' (SEQ ID NO: 62),

wherein, $Z_5$ is A, $Z_6$ is U, the nucleotide sequence I comprises a nucleotide $Z_7$ at a corresponding site to $Z_5$, the nucleotide sequence II comprises a nucleotide $Z_8$ at a corresponding site to $Z_6$, and $Z_8$ is the first nucleotide from the 5' terminal of the antisense strand;
iii) the nucleotide sequence I has the same length and no more than three nucleotide differences from the nucleotide sequence shown in SEQ ID NO: 121; and the nucleotide sequence II has the same length and no more than three nucleotide differences from the nucleotide sequence shown in SEQ ID NO: 122:

5'-GGAAGGUUACCGAGCAAU$Z_9$-3' (SEQ ID NO: 121);
5'-$Z_{10}$AUUGCUCGGUAACCUUCC-3' (SEQ ID NO: 122),

wherein, $Z_9$ is A, $Z_{10}$ is U, the nucleotide sequence I comprises a nucleotide $Z_{11}$ at a corresponding site to $Z_9$, the nucleotide sequence II comprises a nucleotide $Z_{12}$ at a corresponding site to $Z_{10}$, and $Z_{12}$ is the first nucleotide from the 5' terminal of the antisense strand;
iv) the nucleotide sequence I has the same length and no more than three nucleotide differences from the nucleotide sequence shown in SEQ ID NO: 181; and the nucleotide sequence II has the same length and no more than three nucleotide differences from the nucleotide sequence shown in SEQ ID NO: 182:

5'-AGAACAGACAGCAGAAUU$Z_{13}$-3' (SEQ ID NO: 181);
5'-$Z_{14}$AAUUCUGCUGUCUGUUCU-3' (SEQ ID NO: 182),

wherein, $Z_{13}$ is A, $Z_{14}$ is U, the nucleotide sequence I comprises a nucleotide $Z_{15}$ at a corresponding site to $Z_{13}$, the nucleotide sequence II comprises a nucleotide $Z_{16}$ at a corresponding site to $Z_{14}$, and $Z_{16}$ is the first nucleotide from the 5' terminal of the antisense strand;
v) the nucleotide sequence I has the same length and no more than three nucleotide differences from the nucleotide sequence shown in SEQ ID NO: 241; and the nucleotide sequence II has the same length and no more than three nucleotide differences from the nucleotide sequence shown in SEQ ID NO: 242:

5'-CCAAGAAGAACGCUGCAA$Z_{17}$-3' (SEQ ID NO: 241);
5'-$Z_{18}$UUGCAGCGUUCUUCUUGG-3' (SEQ ID NO: 242),

wherein, $Z_{17}$ is A, $Z_{18}$ is U, the nucleotide sequence I comprises a nucleotide $Z_{19}$ at a corresponding site to $Z_{17}$, the nucleotide sequence II comprises a nucleotide $Z_{20}$ at a corresponding site to $Z_{18}$, and $Z_{20}$ is the first nucleotide from the 5' terminal of the antisense strand; and
vi) the nucleotide sequence I has the same length and no more than three nucleotide differences from the nucleotide sequence shown in SEQ ID NO: 301; and the nucleotide sequence II has the same length and no more than three nucleotide differences from the nucleotide sequence shown in SEQ ID NO: 302:

5'-CCAGUAAGCAAGCCAGAA$Z_{21}$-3' (SEQ ID NO: 301);
5'-$Z_{22}$UUCUGGCUUGCUUACUGG-3' (SEQ ID NO: 302),

wherein, $Z_{21}$ is A, $Z_{22}$ is U, the nucleotide sequence I comprises a nucleotide $Z_{23}$ at a corresponding site to $Z_{21}$, the nucleotide sequence II comprises a nucleotide $Z_{24}$ at a corresponding site to $Z_{22}$, and $Z_{24}$ is the first nucleotide from the 5' terminal of the antisense strand;

$R_2$ is a linear alkylene of 1-20 carbon atoms in length, wherein one or more carbon atoms are optionally replaced

with any one or more of the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $R_2$ is optionally substituted by any one or more of the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -CO2H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2NH$($C_1$-$C_{10}$ alkyl), -$SO_2NH$(phenyl), -$NHSO_2$($C_1$-$C_{10}$ alkyl), -$NHSO_2$(phenyl), and -$NHSO_2$($C_1$-$C_{10}$ haloalkyl);

each $L_1$ is independently a linear alkylene of 1-70 carbon atoms in length, wherein one or more carbon atoms are optionally replaced with any one or more of the group consisting of: C(O), NH, O, S, CH=N, $S(O)_2$, $C_2$-$C_{10}$ alkeylene, $C_2$-$C_{10}$ alkynylene, $C_2$-$C_{10}$, $C_3$-$C_{18}$ heterocyclylene, and $C_5$-$C_{10}$ heteroarylene; and wherein $L_1$ is optionally substituted by any one or more of the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -CO2H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2NH$($C_1$-$C_{10}$ alkyl), -$SO_2NH$(phenyl), -$NHSO_2$($C_1$-$C_{10}$ alkyl), -$NHSO_2$(phenyl), and -$NHSO_2$($C_1$-$C_{10}$ haloalkyl); and

represents a site where the group is covalently linked; and $M_1$ represents a targeting group.

2. The siRNA conjugate according to claim 1, wherein each $L_1$ is independently selected from the group consisting of groups (A1)-(A26) and any connection combinations thereof:

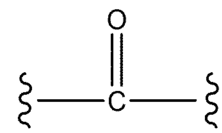, 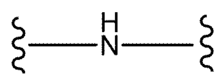, 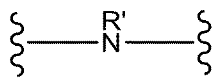, 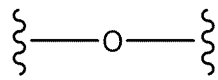,

(A1) (A2) (A3) (A4)

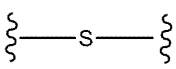, 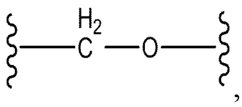, 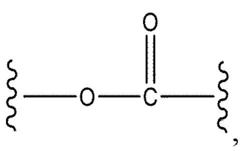, 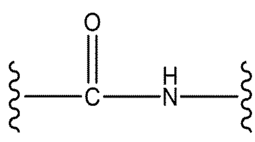,

(A5) (A6) (A7) (A8)

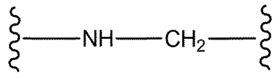, 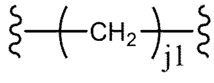, 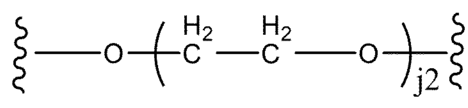,

(A9) (A10) (A11)

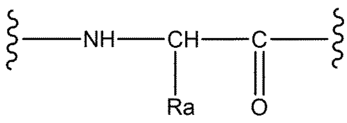, 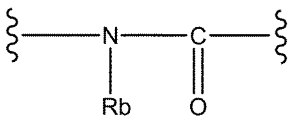, 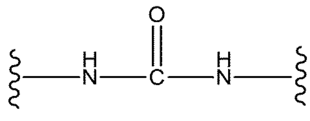,

(A12) (A13) (A14)

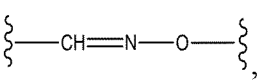, 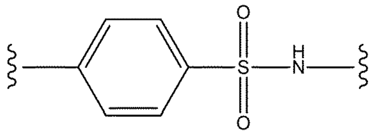, 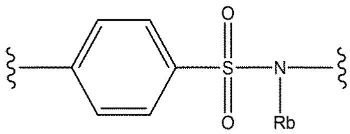,

(A15) (A16) (A17)

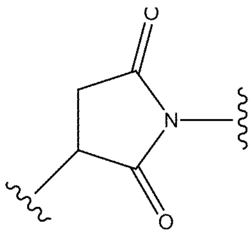, 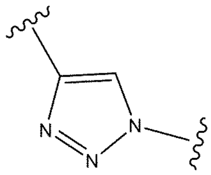, 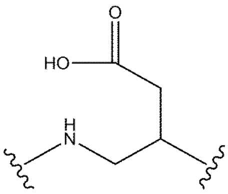, 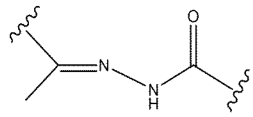,

(A18) (A19) (A20) (A21)

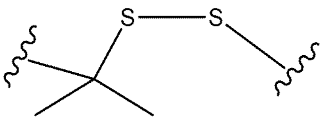, 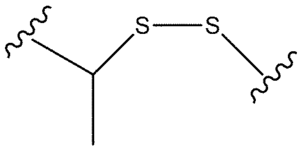, 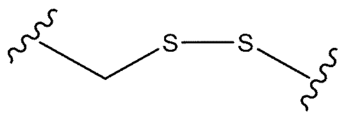,

(A22) (A23) (A24)

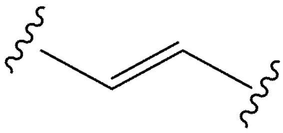, 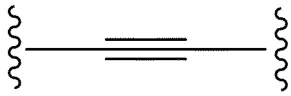;

(A25) (A26)

wherein, each j1 is independently an integer of 1-20; and each j2 is independently an integer of 1-20;
each R' is independently a $C_1$-$C_{10}$ alkyl; and
each Ra is independently selected from the group consisting of groups (A27)-(A45) and any connection combinations thereof:

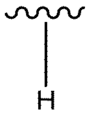 , 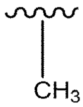 , 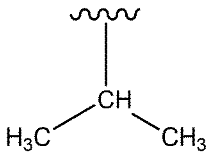 , 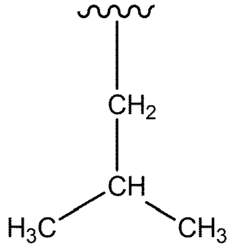 , 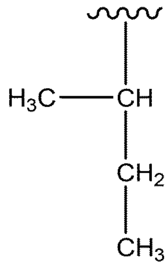 , 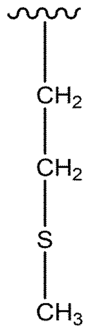 ,

(A27) (A28) (A29) (A30) (A31) (A32)

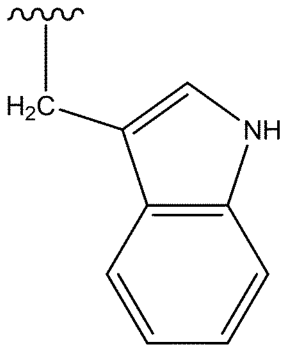 , 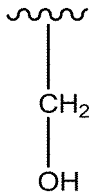 , 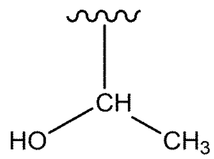 , 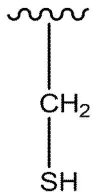 , 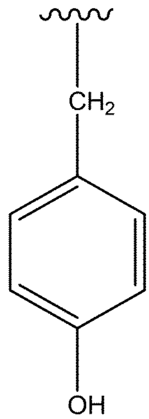 ,

(A33) (A34) (A35) (A36) (A37)

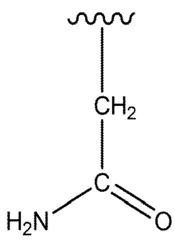 , 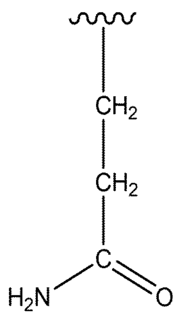 , 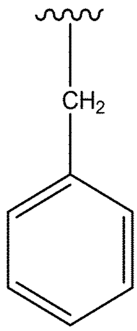 , 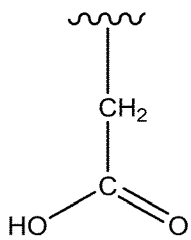 , 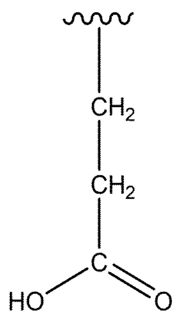 ,

(A38) (A39) (A40) (A41) (A42)

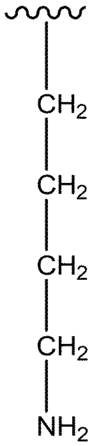, 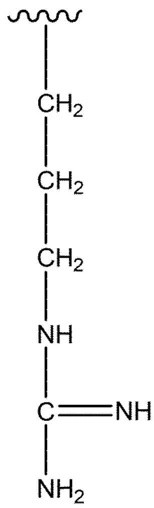 or 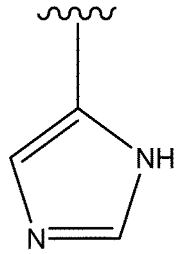 ;

(A43) (A44) (A45)

each Rb is independently a $C_1$-$C_{10}$ alkyl;

or, $L_1$ is selected from the connection combinations of one or more of A1, A4, A5, A6, A8, A10, A11 and A13;

or, $L_1$ is selected from the connection combinations of at least two of A1, A4, A8, A10 and A11;

or, $L_1$ is selected from the connection combinations of at least two of A1, A18 and A10;

or, the length of $L_1$ is 3-25 atoms;

or, the length of $L_1$ is 4-15 atoms;





represents the site where the group is covalently linked.

**3.** The siRNA conjugate according to claim 2, wherein j 1 is an integer of 2-10, j2 is an integer of 2-10, R' is a $C_1$-$C_4$ alkyl, Ra is selected from one of A27, A28, A29, A30 and A31, and Rb is a $C_1$-$C_5$ alkyl;
or, j 1 is an integer of 3-5, j2 is an integer of 3-5, R' is one of methyl, ethyl and isopropyl, Ra is a group as shown by Formula (A27) or a group as shown by Formula (A28), and Rb is one of a methyl, ethyl, isopropyl and butyl.

**4.** The siRNA conjugate according to any one of claims 1-3, wherein n1 is an integer of 1-2, n3 is an integer of 0-1, and n1+n3=2-3.

**5.** The siRNA conjugate according to any one of claims 1-4, wherein each of m1, m2 and m3 is independently an integer of 2-5, and/or m1=m2=m3.

**6.** The siRNA conjugate according to any one of claims 1-5, wherein each targeting group is independently a ligand that binds to an asialoglycoprotein receptor on a surface of a mammalian hepatocyte;

or, each targeting group is independently an asialoglycoprotein or saccharide;

or, each targeting group is independently selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycoly1-α-neuraminic acid, 5-thio-β-D-glucofuranose, methyl 2,3,4-tris-O-acetyl-1-thio-6-0-trityl-α-D-glucofuranose, 4-thio-β-D-galactopyranose, ethyl 3,4,6,7-tetra-O-acetyl-2-deoxy-1,5-dithio-a-D-glucoheptopyranoside, 2,5-anhydro-D-allononitrile, ribose, D-ribose, D-4-thioribose, L-ribose, and L-4-thioribose; or, at least one or each targeting group is galactose or N-acetylgalactosamine.

7. The siRNA conjugate according to any one of claims 1-6, wherein each of Rio, $R_{11}$, $R_{12}$, $R_{13}$, $R_{14}$ and $R_{15}$ is independently H, methyl or ethyl;
or, each of $R_{10}$, $R_{11}$, $R_{12}$, $R_{13}$, $R_{14}$ and $R_{15}$ is H.

8. The siRNA conjugate according to any one of claims 1-7, wherein $R_2$ has both a site linking to an N atom on a nitrogenous backbone and a site linking to a P atom in $R_{3;}$

or, on $R_2$, the site linking to the N atom on the nitrogenous backbone forms an amide bond with the N atom, and the site linking to the P atom in $R_3$ forms a phosphoester bond with the P atom;
or, $R_2$ is selected from groups as shown by Formulas (B5), (B6), (B5') or (B6').

9. The siRNA conjugate according to any one of claims 1-8, wherein the conjugate has a structure as shown by Formula (403), (404), (405), (406), (407), (408), (409), (410), (411), (412), (413), (414), (415), (416), (417), (418), (419), (420), (421) or (422).

10. The siRNA conjugate according to any one of claims 1-9, wherein the P atom in Formula A59 is linked to a terminal region of the sense strand or antisense strand of the siRNA, and the terminal region refers to the first four nucleotides closest to either end terminal of the sense strand or antisense strand;

or, the P atom in Formula A59 is linked to the terminal of the sense strand or antisense strand of the siRNA;
or, the P atom in Formula A59 is linked to a 3' terminal of the sense strand of the siRNA;
or, the P atom in Formula A59 is linked to position 2', 3', or 5' of the nucleotide in the siRNA by a phosphodiester bond.

11. The siRNA conjugate according to claim 1, wherein, i) the nucleotide sequence I has no more than one nucleotide difference from the nucleotide sequence shown in SEQ ID NO: 1; and/or the nucleotide sequence II has no more than one nucleotide difference from the nucleotide sequence shown in SEQ ID NO: 2; or, ii) the nucleotide sequence I has no more than one nucleotide difference from the nucleotide sequence shown in SEQ ID NO: 61, and/or the nucleotide sequence II has no more than one nucleotide difference from the nucleotide sequence shown in SEQ ID NO: 62; or, iii) the nucleotide sequence I has no more than one nucleotide difference from the nucleotide sequence shown in SEQ ID NO: 121, and/or the nucleotide sequence II has no more than one nucleotide difference from the nucleotide sequence shown in SEQ ID NO: 122; or, iv) the nucleotide sequence I has no more than one nucleotide difference from the nucleotide sequence shown in SEQ ID NO: 181, and/or the nucleotide sequence II has no more than one nucleotide difference from the nucleotide sequence shown in SEQ ID NO: 182; or, v) the nucleotide sequence I has no more than one nucleotide difference from the nucleotide sequence shown in SEQ ID NO: 241, and/or the nucleotide sequence II has no more than one nucleotide difference from the nucleotide sequence shown in SEQ ID NO: 242; or, vi), the nucleotide sequence I has no more than one nucleotide difference from the nucleotide sequence shown in SEQ ID NO: 301, and/or the nucleotide sequence II has no more than one nucleotide difference from the nucleotide sequence shown in SEQ ID NO: 302.

12. The siRNA conjugate according to claim 1 or 11, wherein, i) the nucleotide difference between the nucleotide sequence II and the nucleotide sequence shown in SEQ ID NO: 2 comprises a difference at the site of $Z_4$, and $Z_4$ is selected from A, C or G; or ii) the nucleotide difference between the nucleotide sequence II and the nucleotide sequence shown in SEQ ID NO: 62 comprises a difference at the site of $Z_8$, and $Z_8$ is selected from A, C or G; or iii) the nucleotide difference between the nucleotide sequence II and the nucleotide sequence shown in SEQ ID NO: 122 comprises a difference at the site of $Z_{12}$, and $Z_{12}$ is selected from A, C or G; or iv) the nucleotide difference between the nucleotide sequence II and the nucleotide sequence shown in SEQ ID NO: 182 comprises a difference at the site of $Z_{16}$, and $Z_{16}$ is selected from A, C or G; or v) the nucleotide difference between the nucleotide sequence II and the nucleotide sequence shown in SEQ ID NO: 242 comprises a difference at the site of $Z_{20}$, and $Z_{20}$ is selected from A, C or G; or vi) the nucleotide difference between the nucleotide sequence II and the nucleotide sequence shown in SEQ ID NO: 302 comprises a difference at the site of $Z_{24}$, and $Z_{24}$ is selected from A, C or G.

13. The siRNA conjugate according to claim 12, wherein $Z_3$ is a nucleotide complementary to $Z_4$; or $Z_7$ is a nucleotide complementary to $Z_8$; or $Z_{11}$ is a nucleotide complementary to $Z_{12}$; or $Z_{15}$ is a nucleotide complementary to $Z_{16}$; or $Z_{19}$ is a nucleotide complementary to $Z_{20}$; or $Z_{23}$ is a nucleotide complementary to $Z_{24}$.

**14.** The siRNA conjugate according to any one of claims 1-13, wherein the nucleotide sequence I is basically reverse complementary, substantially reverse complementary, or completely reverse complementary to the nucleotide sequence II; the basically reverse complementary refers to no more than three base mispairings between two nucleotide sequences; the substantially reverse complementary refers to no more than one base mispairing between two nucleotide sequences; and the completely reverse complementary refers to no mispairing between two nucleotide sequences.

**15.** The siRNA conjugate according to any one of claims 11-14, wherein the sense strand further comprises a nucleotide sequence III, the antisense strand further comprises a nucleotide sequence IV, the nucleotide sequence III and the nucleotide sequence IV each independently has a length of 1-4 nucleotides, the nucleotide sequence III is linked to the 5' terminal of the nucleotide sequence I, the nucleotide sequence IV is linked to the 3' terminal of the nucleotide sequence II, and the nucleotide sequence III has the same length and is substantially reverse complementary or completely reverse complementary to the nucleotide sequence IV; the substantially reverse complementary refers to no more than one base mispairing between two nucleotide sequences; and the completely reverse complementary refers to no mispairing between two nucleotide sequences.

**16.** The siRNA conjugate according to claim 15, wherein,

i) the nucleotide sequence I is the nucleotide sequence shown in SEQ ID NO: 3, and the nucleotide sequence II is the nucleotide sequence shown in SEQ ID NO: 4; and the nucleotide sequences III and IV both have a length of one nucleotide, and a base of the nucleotide sequence III is U; or, the nucleotide sequences III and IV both have a length of two nucleotides, and in a direction from the 5' terminal to the 3' terminal, the base composition of the nucleotide sequence III is CU; or, the nucleotide sequences III and IV both have a length of three nucleotides, and in the direction from the 5' terminal to the 3' terminal, the base composition of the nucleotide sequence III is UCU; or, the nucleotide sequences III and IV both have a length of four nucleotides, and in the direction from the 5' terminal to the 3' terminal, the base composition of the nucleotide sequence III is UUCU; or
ii) the nucleotide sequence I is the nucleotide sequence shown in SEQ ID NO: 63, and the nucleotide sequence II is the nucleotide sequence shown in SEQ ID NO: 64; and the nucleotide sequences III and IV both have a length of one nucleotide, and the base of the nucleotide sequence III is A; or, the nucleotide sequences III and IV both have a length of two nucleotides, and in the direction from the 5' terminal to the 3' terminal, the base composition of the nucleotide sequence III is UA; or, the nucleotide sequences III and IV both have a length of three nucleotides, and in the direction from the 5' terminal to the 3' terminal, the base composition of the nucleotide sequence III is AUA; or, the nucleotide sequences III and IV both have a length of four nucleotides, and in the direction from the 5' terminal to the 3' terminal, the base composition of the nucleotide sequence III is CAUA; or
iii) the nucleotide sequence I is the nucleotide sequence shown in SEQ ID NO: 123, and the nucleotide sequence II is the nucleotide sequence shown in SEQ ID NO: 124; and the nucleotide sequences III and IV both have a length of one nucleotide, and the base of the nucleotide sequence III is G; or, the nucleotide sequences III and IV both have a length of two nucleotides, and in the direction from the 5' terminal to the 3' terminal, the base composition of the nucleotide sequence III is AG; or, the nucleotide sequences III and IV both have a length of three nucleotides, and in the direction from the 5' terminal to the 3' terminal, the base composition of the nucleotide sequence III is CAG; or, the nucleotide sequences III and IV both have a length of four nucleotides, and in the direction from the 5' terminal to the 3' terminal, the base composition of the nucleotide sequence III is CCAG; or
iv) the nucleotide sequence I is the nucleotide sequence shown in SEQ ID NO: 183, and the nucleotide sequence II is the nucleotide sequence shown in SEQ ID NO: 184; and the nucleotide sequences III and IV both have a length of one nucleotide, and the base of the nucleotide sequence III is G; or, the nucleotide sequences III and IV both have a length of two nucleotides, and in the direction from the 5' terminal to the 3' terminal, the base composition of the nucleotide sequence III is GG; or, the nucleotide sequences III and IV both have a length of three nucleotides, and in the direction from the 5' terminal to the 3' terminal, the base composition of the nucleotide sequence III is AGG; or, the nucleotide sequences III and IV both have a length of four nucleotides, and in the direction from the 5' terminal to the 3' terminal, the base composition of the nucleotide sequence III is CAGG; or
v) the nucleotide sequence I is the nucleotide sequence shown in SEQ ID NO: 243, and the nucleotide sequence II is the nucleotide sequence shown in SEQ ID NO: 244; and the nucleotide sequences III and IV both have a length of one nucleotide, and the base of the nucleotide sequence III is G; or, the nucleotide sequences III and IV both have a length of two nucleotides, and in the direction from the 5' terminal to the 3' terminal, the base

composition of the nucleotide sequence III is GG; or, the nucleotide sequences III and IV both have a length of three nucleotides, and in the direction from the 5' terminal to the 3' terminal, the base composition of the nucleotide sequence III is AGG; or, the nucleotide sequences III and IV both have a length of four nucleotides, and in the direction from the 5' terminal to the 3' terminal, the base composition of the nucleotide sequence III is CAGG; or

vi) the nucleotide sequence I is the nucleotide sequence shown in SEQ ID NO: 303, and the nucleotide sequence II is the nucleotide sequence shown in SEQ ID NO: 304; and the nucleotide sequences III and IV both have a length of one nucleotide, and the base of the nucleotide sequence III is A; or, the nucleotide sequences III and IV both have a length of two nucleotides, and in the direction from the 5' terminal to the 3' terminal, the base composition of the nucleotide sequence III is UA; or, the nucleotide sequences III and IV both have a length of three nucleotides, and in the direction from the 5' terminal to the 3' terminal, the base composition of the nucleotide sequence III is UUA; or, the nucleotide sequences III and IV both have a length of four nucleotides, and in the direction from the 5' terminal to the 3' terminal, the base composition of the nucleotide sequence III is GUUA.

**17.** The siRNA conjugate according to claim 16, wherein the nucleotide sequence III is completely reverse complementary to the nucleotide sequence IV.

**18.** The siRNA conjugate according to any one of claims 1-17, wherein the antisense strand further comprises a nucleotide sequence V, the nucleotide sequence V has a length of 1-3 nucleotides, is linked to the 3' terminal of the antisense strand, thereby constituting a 3' overhang of the antisense strand; or, the nucleotide sequence V has a length of 2 nucleotides; or the nucleotide sequence V is two continuous thymidine deoxyribonucleotides or two continuous uridine ribonucleotides; or the nucleotide sequence V is complementary to the nucleotides at the corresponding site of the target mRNA.

**19.** The siRNA conjugate according to any one of claims 1-18, wherein,

the sense strand of the siRNA comprises the nucleotide sequence shown in SEQ ID NO: 5, and the antisense strand comprises the nucleotide sequence shown in SEQ ID NO: 6; or the sense strand of the siRNA comprises the nucleotide sequence shown in SEQ ID NO: 7, and the antisense strand comprises the nucleotide sequence shown in SEQ ID NO: 8;

or the sense strand of the siRNA comprises the nucleotide sequence shown in SEQ ID NO: 65, and the antisense strand comprises the nucleotide sequence shown in SEQ ID NO: 66; or the sense strand of the siRNA comprises the nucleotide sequence shown in SEQ ID NO: 67, and the antisense strand comprises the nucleotide sequence shown in SEQ ID NO: 68;

or the sense strand of the siRNA comprises the nucleotide sequence shown in SEQ ID NO: 125, and the antisense strand of the siRNA comprises the nucleotide sequence shown in SEQ ID NO: 126; or the sense strand of the siRNA comprises the nucleotide sequence shown in SEQ ID NO: 127, and the antisense strand of the siRNA comprises the nucleotide sequence shown in SEQ ID NO: 128;

or the sense strand of the siRNA comprises the nucleotide sequence shown in SEQ ID NO: 185, and the antisense strand comprises the nucleotide sequence shown in SEQ ID NO: 186; or the sense strand of the siRNA comprises the nucleotide sequence shown in SEQ ID NO: 187, and the antisense strand comprises the nucleotide sequence shown in SEQ ID NO: 188;

or the sense strand of the siRNA comprises the nucleotide sequence shown in SEQ ID NO: 245, and the antisense strand of the siRNA comprises the nucleotide sequence shown in SEQ ID NO: 246; or the sense strand of the siRNA comprises the nucleotide sequence shown in SEQ ID NO: 247, and the antisense strand of the siRNA comprises the nucleotide sequence shown in SEQ ID NO: 248;

or the sense strand of the siRNA comprises the nucleotide sequence shown in SEQ ID NO: 305, and the antisense strand of the siRNA comprises the nucleotide sequence shown in SEQ ID NO: 306; or the sense strand of the siRNA comprises the nucleotide sequence shown in SEQ ID NO: 307, and the antisense strand of the siRNA comprises the nucleotide sequence shown in SEQ ID NO: 308.

**20.** The siRNA conjugate according to any one of claims 11-19, wherein the siRNA has the nucleotide sequence shown in siC5a1, siC5a2, siC5b1, siC5b2, siC5c1, siC5c2, siC5d1, siC5d2, siC5e1, siC5e2, siC5f1 or siC5f2.

**21.** The siRNA conjugate according to any one of claims 1-20, wherein at least one nucleotide in the sense strand or antisense strand is a modified nucleotide, and/or at least one phosphate group is a phosphate group with modified group.

**22.** The siRNA conjugate according to claim 21, wherein each nucleotide in the sense strand and the antisense strand is independently a fluoro modified nucleotide or a non-fluoro modified nucleotide;

or, the fluoro modified nucleotides are located in the nucleotide sequence I and the nucleotide sequence II, and in the direction from 5' terminal to 3' terminal, the nucleotides at positions 7, 8 and 9 of the nucleotide sequence I are fluoro modified nucleotides; and in the direction from 5' terminal to 3' terminal, the nucleotides at positions 2, 6, 14 and 16 of the nucleotide sequence II are fluoro modified nucleotides;
or, in the direction from 5' terminal to 3' terminal, the nucleotides at positions 7, 8 and 9 or 5, 7, 8 and 9 of the nucleotide sequence I in the sense strand are fluoro modified nucleotides, and the nucleotides at the rest of positions in the sense strand are non-fluoro modified nucleotides; and in the direction from 5' terminal to 3' terminal, the nucleotides at positions 2, 6, 14 and 16 or 2, 6, 8, 9, 14 and 16 of the nucleotide sequence II in the antisense strand are fluoro modified nucleotides, and the nucleotides at the rest of positions in the antisense strand are non-fluoro modified nucleotides.

**23.** The siRNA conjugate according to claim 21 or 22, wherein each non-fluoro modified nucleotide is independently selected from a nucleotide formed by substituting the 2'-hydroxy of the ribose group of a nucleotide with a non-fluoro group, or a nucleotide analogue;

or, the nucleotide formed by substituting the 2'-hydroxy of the ribose group of the nucleotide with the non-fluoro group is selected from one of a 2'-alkoxy modified nucleotide, a 2'-substituted alkoxy modified nucleotide, a 2'-alkyl modified nucleotide, a 2'-substituted alkyl modified nucleotide, a 2'-amino modified nucleotide, a 2'-substituted amino modified nucleotide, and a 2'-deoxy nucleotide; and the nucleotide analogue is selected from one of an isonucleotide, an LNA, an ENA, a cET, a UNA and a GNA;
or, each non-fluoro modified nucleotide is a methoxy modified nucleotide, and the methoxy modified nucleotide refers to a nucleotide formed by substituting the 2'-hydroxy of the ribose group with a methoxy group.

**24.** The siRNA conjugate according to any one of claims 21-23, wherein in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and in the direction from 5' terminal to 3' terminal, the nucleotides at positions 2, 6, 8, 9, 14 and 16 of the nucleotide sequence II in the antisense strand of the siRNA are fluoro modified nucleotides, and the nucleotides at the rest of positions in the antisense strand of the siRNA are methoxy modified nucleotides;

or, in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides;
or, in the direction from 5' terminal to 3' terminal, the nucleotides at positions 7, 8 and 9 of the nucleotide sequence I in the sense strand of the siRNA are fluoro modified nucleotides, and the nucleotides at the rest of positions in the sense strand of the siRNA are methoxy modified nucleotides; and in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides.

**25.** The siRNA conjugate according to any one of claims 1-24, wherein the siRNA is any one of siC5a1-M1, siC5a2-M1, siC5a1-M2, siC5a2-M2, siC5a1-M3, siC5a2-M3, siC5b1-M1, siC5b2-M1, siC5b1-M2, siC5b2-M2, siC5b1-M3, siC5b2-M3, siC5c1-M1, siC5c2-M1, siC5c1-M2, siC5c2-M2, siC5c1-M3, siC5c2-M3, siC5d1-M1, siC5d2-M1, siC5d1-M2, siC5d2-M2, siC5d1-M3, siC5d2-M3, siC5e1-M1, siC5e2-M1, siC5e1-M2, siC5e2-M2, siC5e1-M3, siC5e2-M3, siC5f1-M1, siC5f2-M1, siC5f1-M2, siC5f2-M2, siC5f1-M3 or siC5f2-M3.

**26.** The siRNA conjugate according to claim 21, wherein the phosphate group with modified group is a phosphorothioate group formed by substituting at least one oxygen atom in a phosphodiester bond in the phosphate group with a sulfur atom; or, the phosphate group with modified group is a phosphorothioate group having a structure as shown by Formula (1):

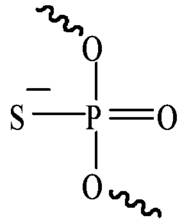

Formula (1).

**27.** The siRNA conjugate according to claim 26, wherein, in the siRNA, a phosphorothioate linkage exists in at least one of the following positions:

the position between the first nucleotide and the second nucleotide at 5' terminal of the sense strand;
the position between the second nucleotide and the third nucleotide at 5' terminal of the sense strand;
the position between the first nucleotide and the second nucleotide at 3' terminal of the sense strand;
the position between the second nucleotide and the third nucleotide at 3' terminal of the sense strand;
the position between the first nucleotide and the second nucleotide at 5' terminal of the antisense strand;
the position between the second nucleotide and the third nucleotide at 5' terminal of the antisense strand;
the position between the first nucleotide and the second nucleotide at 3' terminal of the antisense strand; and
the position between the second nucleotide and the third nucleotide at 3' terminal of the antisense strand.

**28.** The siRNA conjugate according to any one of claims 1-27, wherein the siRNA is any one of siC5a1-M1S, siC5a2-M1S, siC5a1-M2S, siC5a2-M2S, siC5a1-M3S, siC5a2-M3S, siC5b1-M1S, siC5b2-M1S, siC5b1-M2S, siC5b2-M2S, siC5b1-M3S, siC5b2-M3S, siC5c1-M1S, siC5c2-M1S, siC5c1-M2S, siC5c2-M2S, siC5c1-M3S, siC5c2-M3S, siC5d1-M1S, siC5d2-M1S, siC5d1-M2S, siC5d2-M2S, siC5d1-M3S, siC5d2-M3S, siC5e1-M1S, siC5e2-M1S, siC5e1-M2S, siC5e2-M2S, siC5e1-M3S, siC5e2-M3S, siC5f1-M1S, siC5f2-M1S, siC5f1-M2S, siC5f2-M2S, siC5f1-M3S or iC5f2-M3S.

**29.** The siRNA conjugate according to any one of claims 1-28, wherein the 5'-terminal nucleotide in the antisense strand of the siRNA is a 5'-phosphate nucleotide or a 5'-phosphate analogue modified nucleotide;

or, the 5'-phosphate nucleotide is a nucleotide as shown by Formula (2); and the 5'-phosphate analogue modified nucleotide is selected from a nucleotide represented by any one of Formulae (3) to (6):

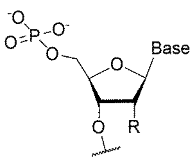
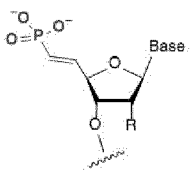
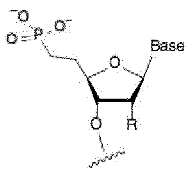
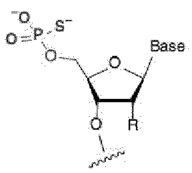
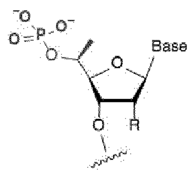

Formula (2) Formula (3) Formula (4) Formula (5) Formula (6)

wherein, R is selected from H, OH, methoxy or F; and Base represents a base selected from A, U, C, G, or T.

**30.** The siRNA conjugate according to any one of claims 1-29, wherein the siRNA is any one of siC5a1-M1P1, siC5a1-M2P1, siC5a1-M3P1, siC5a2-M1P1, siC5a2-M2P1, siC5a2-M3Pl, siC5a1-M1SP1, siC5a1-M2SP1, siC5a1-M3SP1, siC5a2-M1SP1, siC5a2-M2SP1, siC5a2-M3SP1, siC5b1-M1P1, siC5b1-M2P1, siC5b1-M3P1, siC5b2-M1P1, siC5b2-M2P1, siC5b2-M3P1, siC5b1-M1SP1, siC5b1-M2SP1, siC5b1-M3SP1, siC5b2-M1SP1, siC5b2-M2SP1, siC5b2-M3SP1, siC5c1-M1P1, siC5c1-M2P1, siC5c1-M3P1, siC5c2-M1P1, siC5c2-M2P1, siC5c2-M3P1, siC5c1-M1SP1, siC5c1-M2SP1, siC5c1-M3SP1, siC5c2-M1SP1, siC5c2-M2SP1, siC5c2-M3SP1, siC5d1-M1P1, siC5d1-M2P1, siC5d1-M3P1, siC5d2-M1P1, siC5d2-M2P1, siC5d2-M3P1, siC5d1-M1SP1, siC5d1-M2SP1, siC5d1-M3SP1, siC5d2-M1SP1, siC5d2-M2SP1, siC5d2-M3SP1, siC5e1-M1P1, siC5e1-M2P1, siC5e1-M3P1, siC5e2-M1P1, siC5e2-M2P1, siC5e2-M3P1, siC5e1-M1SP1, siC5e1-M2SP1, siC5e1-M3SP1, siC5e2-M1SP1, siC5e2-M2SP1, siCSe2-M3SP1, siCSf1-M1P1, siC5f1-M2P1, siC5f1-M3P1, siC5f2-M1P1, siC5f2-M2P1, siC5f2-M3P1, siC5f1-M1SP1, siC5f1-M2SP1, siC5f1-M3SP1, siC5f2-M1SP1, siC5f2-M2SP1 or siC5f2-M3SP1.

**31.** An siRNA, wherein the siRNA comprises a sense strand and an antisense strand, and each nucleotide in the sense strand and the antisense strand is independently a fluoro modified nucleotide or a non-fluoro modified nucleotide; the sense strand comprises a nucleotide sequence I, the antisense strand comprises a nucleotide sequence II, the nucleotide sequence I and the nucleotide sequence II are at least partly reverse complementary to form a double-stranded region, the fluoro modified nucleotides are located in the nucleotide sequence I and the nucleotide sequence II, and, in the direction from 5' terminal to 3' terminal, the nucleotides at positions 7, 8 and 9 of the nucleotide sequence I in the sense strand are fluoro modified nucleotides, and the nucleotides at the rest of positions in the sense strand are non-fluoro modified nucleotides; in the direction from 5' terminal to 3' terminal, the nucleotides at positions 2, 6, 14 and 16 of the nucleotide sequence II in the antisense strand are fluoro modified nucleotides, and the nucleotides at the rest of positions in the antisense strand are non-fluoro modified nucleotides; and,

i) the nucleotide sequence I has the same length and no more than three nucleotide differences from the nucleotide sequence shown in SEQ ID NO: 1; and the nucleotide sequence II has the same length and no more than three nucleotide differences from the nucleotide sequence shown in SEQ ID NO: 2; the nucleotide sequence I comprises a nucleotide $Z_3$ at a corresponding site to $Z_1$, the nucleotide sequence II comprises a nucleotide $Z_4$ at a corresponding site to $Z_2$, and $Z_4$ is the first nucleotide from the 5' terminal of the antisense strand; or,
ii) the nucleotide sequence I has the same length and no more than three nucleotide differences from the nucleotide sequence shown in SEQ ID NO: 61; and the nucleotide sequence II has the same length and no more than three nucleotide differences from the nucleotide sequence shown in SEQ ID NO: 62; the nucleotide sequence I comprises a nucleotide $Z_7$ at a corresponding site to $Z_5$, the nucleotide sequence II comprises a nucleotide $Z_8$ at a corresponding site to $Z_6$, and $Z_8$ is the first nucleotide from the 5' terminal of the antisense strand; or,
iii) the nucleotide sequence I has the same length and no more than three nucleotide differences from the nucleotide sequence shown in SEQ ID NO: 121; and the nucleotide sequence II has the same length and no more than three nucleotide differences from the nucleotide sequence shown in SEQ ID NO: 122; the nucleotide sequence I comprises a nucleotide $Z_{11}$ at a corresponding site to $Z_9$, the nucleotide sequence II comprises a nucleotide $Z_{12}$ at a corresponding site to $Z_{10}$, and $Z_{12}$ is the first nucleotide from the 5' terminal of the antisense strand; or,
iv) the nucleotide sequence I has the same length and no more than three nucleotide differences from the nucleotide sequence shown in SEQ ID NO: 181; and the nucleotide sequence II has the same length and no more than three nucleotide differences from the nucleotide sequence shown in SEQ ID NO: 182; the nucleotide sequence I comprises a nucleotide $Z_{15}$ at a corresponding site to $Z_{13}$, the nucleotide sequence II comprises a nucleotide $Z_{16}$ at a corresponding site to $Z_{14}$, and $Z_{16}$ is the first nucleotide from the 5' terminal of the antisense strand; or,
v) the nucleotide sequence I has the same length and no more than three nucleotide differences from the nucleotide sequence shown in SEQ ID NO: 241; and the nucleotide sequence II has the same length and no more than three nucleotide differences from the nucleotide sequence shown in SEQ ID NO: 242; the nucleotide sequence I comprises a nucleotide $Z_{19}$ at a corresponding site to $Z_{17}$, the nucleotide sequence II comprises a nucleotide $Z_{20}$ at a corresponding site to $Z_{18}$, and $Z_{20}$ is the first nucleotide from the 5' terminal of the antisense strand; or,
vi) the nucleotide sequence I has the same length and no more than three nucleotide differences from the nucleotide sequence shown in SEQ ID NO: 301; and the nucleotide sequence II has the same length and no more than three nucleotide differences from the nucleotide sequence shown in SEQ ID NO: 302; the nucleotide sequence I comprises a nucleotide $Z_{23}$ at a corresponding site to $Z_{21}$, the nucleotide sequence II comprises a nucleotide $Z_{24}$ at a corresponding site to $Z_{22}$, and $Z_{24}$ is the first nucleotide from the 5' terminal of the antisense strand.

**32.** The siRNA according to claim 31, wherein each non-fluoro modified nucleotide is independently selected from a nucleotide formed by substituting the 2'-hydroxy of the ribose group of a nucleotide with a non-fluoro group, or a nucleotide analogue.

**33.** The siRNA according to claim 32, wherein the nucleotide formed by substituting the 2'-hydroxy of the ribose group of the nucleotide with the non-fluoro group is selected from one of a 2'-alkoxy modified nucleotide, a 2'-substituted alkoxy modified nucleotide, a 2'-alkyl modified nucleotide, a 2'-substituted alkyl modified nucleotide, a 2'-amino modified nucleotide, a 2'-substituted amino modified nucleotide, and a 2'-deoxy nucleotide; and the nucleotide analogue is selected from one of an isonucleotide, an LNA, an ENA, a cET, a UNA and a GNA.

**34.** The siRNA according to any one of claims 31-33, wherein each non-fluoro modified nucleotide is a methoxy modified

nucleotide, and the methoxy modified nucleotide refers to a nucleotide formed by substituting the 2'-hydroxy of the ribose group of a nucleotide with a methoxy group.

**35.** The siRNA according to any one of claims 31-34, wherein, i) the nucleotide sequence I has no more than one nucleotide difference from the nucleotide sequence shown in SEQ ID NO: 1; and/or the nucleotide sequence II has no more than one nucleotide difference from the nucleotide sequence shown in SEQ ID NO: 2; or ii), the nucleotide sequence I has no more than one nucleotide difference from the nucleotide sequence shown in SEQ ID NO: 61, and/or the nucleotide sequence II has no more than one nucleotide difference from the nucleotide sequence shown in SEQ ID NO: 62; or, iii) the nucleotide sequence I has no more than one nucleotide difference from the nucleotide sequence shown in SEQ ID NO: 121, and/or the nucleotide sequence II has no more than one nucleotide difference from the nucleotide sequence shown in SEQ ID NO: 122; or, iv) the nucleotide sequence I has no more than one nucleotide difference from the nucleotide sequence shown in SEQ ID NO: 181, and/or the nucleotide sequence II has no more than one nucleotide difference from the nucleotide sequence shown in SEQ ID NO: 182; or, v) the nucleotide sequence I has no more than one nucleotide difference from the nucleotide sequence shown in SEQ ID NO: 241, and/or the nucleotide sequence II has no more than one nucleotide difference from the nucleotide sequence shown in SEQ ID NO: 242; or, vi), the nucleotide sequence I has no more than one nucleotide difference from the nucleotide sequence shown in SEQ ID NO: 301, and/or the nucleotide sequence II has no more than one nucleotide difference from the nucleotide sequence shown in SEQ ID NO: 302.

**36.** The siRNA according to any one of claims 31-35, wherein the nucleotide difference between the nucleotide sequence II and the nucleotide sequence shown in SEQ ID NO: 2 comprises a difference at the site of $Z_4$, and $Z_4$ is selected from A, C or G; or ii) the nucleotide difference between the nucleotide sequence II and the nucleotide sequence shown in SEQ ID NO: 62 comprises a difference at the site of $Z_8$, and $Z_8$ is selected from A, C or G; or iii) the nucleotide difference between the nucleotide sequence II and the nucleotide sequence shown in SEQ ID NO: 122 comprises a difference at the site of $Z_{12}$, and $Z_{12}$ is selected from A, C or G; or iv) the nucleotide difference between the nucleotide sequence II and the nucleotide sequence shown in SEQ ID NO: 182 comprises a difference at the site of $Z_{16}$, and $Z_{16}$ is selected from A, C or G; or v) the nucleotide difference between the nucleotide sequence II and the nucleotide sequence shown in SEQ ID NO: 242 comprises a difference at the site of $Z_{20}$, and $Z_{20}$ is selected from A, C or G; or vi) the nucleotide difference between the nucleotide sequence II and the nucleotide sequence shown in SEQ ID NO: 302 comprises a difference at the site of $Z_{24}$, and $Z_{24}$ is selected from A, C or G.

**37.** The siRNA according to claim 36, wherein $Z_3$ is a nucleotide complementary to $Z_4$; or $Z_7$ is a nucleotide complementary to $Z_8$; or $Z_{11}$ is a nucleotide complementary to $Z_{12}$; or $Z_{15}$ is a nucleotide complementary to $Z_{16}$; or $Z_{19}$ is a nucleotide complementary to $Z_{20}$; or $Z_{23}$ is a nucleotide complementary to $Z_{24}$.

**38.** The siRNA according to any one of claims 31-37, wherein the nucleotide sequence I is basically reverse complementary, substantially reverse complementary, or completely reverse complementary to the nucleotide sequence II; the basically reverse complementary refers to no more than three base mispairings between two nucleotide sequences; the substantially reverse complementary refers to no more than one base mispairing between two nucleotide sequences; and the completely reverse complementary refers to no mispairing between two nucleotide sequences.

**39.** The siRNA according to any one of claims 31-38, wherein the sense strand further comprises a nucleotide sequence III, the antisense strand further comprises a nucleotide sequence IV, the nucleotide sequence III and the nucleotide sequence IV each independently has a length of 1-4 nucleotides, the nucleotide sequence III is linked to the 5' terminal of the nucleotide sequence I, the nucleotide sequence IV is linked to the 3' terminal of the nucleotide sequence II, and the nucleotide sequence III has the same length and is substantially reverse complementary or completely reverse complementary to the nucleotide sequence IV; the substantially reverse complementary refers to no more than one base mispairing between two nucleotide sequences; and the completely reverse complementary refers to no mispairing between two nucleotide sequences.

**40.** The siRNA according to any one of claims 31-39, wherein,

i) the nucleotide sequence I is the nucleotide sequence shown in SEQ ID NO: 3, and the nucleotide sequence II is the nucleotide sequence shown in SEQ ID NO: 4; and the nucleotide sequences III and IV both have a length of one nucleotide, and a base of the nucleotide sequence III is U; or, the nucleotide sequences III and IV both have a length of two nucleotides, and in the direction from the 5' terminal to the 3' terminal, the base composition of the nucleotide sequence III is CU; or, the nucleotide sequences III and IV both have a length of

three nucleotides, and in the direction from the 5' terminal to the 3' terminal, the base composition of the nucleotide sequence III is UCU; or, the nucleotide sequences III and IV both have a length of four nucleotides, and in the direction from the 5' terminal to the 3' terminal, the base composition of the nucleotide sequence III is UUCU; or

ii) the nucleotide sequence I is the nucleotide sequence shown in SEQ ID NO: 63, and the nucleotide sequence II is the nucleotide sequence shown in SEQ ID NO: 64; and the nucleotide sequences III and IV both have a length of one nucleotide, and the base of the nucleotide sequence III is A; or, the nucleotide sequences III and IV both have a length of two nucleotides, and in the direction from the 5' terminal to the 3' terminal, the base composition of the nucleotide sequence III is UA; or, the nucleotide sequences III and IV both have a length of three nucleotides, and in the direction from the 5' terminal to the 3' terminal, the base composition of the nucleotide sequence III is AUA; or, the nucleotide sequences III and IV both have a length of four nucleotides, and in the direction from the 5' terminal to the 3' terminal, the base composition of the nucleotide sequence III is CAUA; or

iii) the nucleotide sequence I is the nucleotide sequence shown in SEQ ID NO: 123, and the nucleotide sequence II is the nucleotide sequence shown in SEQ ID NO: 124; and the nucleotide sequences III and IV both have a length of one nucleotide, and the base of the nucleotide sequence III is G; or, the nucleotide sequences III and IV both have a length of two nucleotides, and in the direction from the 5' terminal to the 3' terminal, the base composition of the nucleotide sequence III is AG; or, the nucleotide sequences III and IV both have a length of three nucleotides, and in the direction from the 5' terminal to the 3' terminal, the base composition of the nucleotide sequence III is CAG; or, the nucleotide sequences III and IV both have a length of four nucleotides, and in the direction from the 5' terminal to the 3' terminal, the base composition of the nucleotide sequence III is CCAG; or

iv) the nucleotide sequence I is the nucleotide sequence shown in SEQ ID NO: 183, and the nucleotide sequence II is the nucleotide sequence shown in SEQ ID NO: 184; and the nucleotide sequences III and IV both have a length of one nucleotide, and the base of the nucleotide sequence III is G; or, the nucleotide sequences III and IV both have a length of two nucleotides, and in the direction from the 5' terminal to the 3' terminal, the base composition of the nucleotide sequence III is GG; or, the nucleotide sequences III and IV both have a length of three nucleotides, and in the direction from the 5' terminal to the 3' terminal, the base composition of the nucleotide sequence III is AGG; or, the nucleotide sequences III and IV both have a length of four nucleotides, and in the direction from the 5' terminal to the 3' terminal, the base composition of the nucleotide sequence III is CAGG; or

v) the nucleotide sequence I is the nucleotide sequence shown in SEQ ID NO: 243, and the nucleotide sequence II is the nucleotide sequence shown in SEQ ID NO: 244; and the nucleotide sequences III and IV both have a length of one nucleotide, and the base of the nucleotide sequence III is G; or, the nucleotide sequences III and IV both have a length of two nucleotides, and in the direction from the 5' terminal to the 3' terminal, the base composition of the nucleotide sequence III is GG; or, the nucleotide sequences III and IV both have a length of three nucleotides, and in the direction from the 5' terminal to the 3' terminal, the base composition of the nucleotide sequence III is AGG; or, the nucleotide sequences III and IV both have a length of four nucleotides, and in the direction from the 5' terminal to the 3' terminal, the base composition of the nucleotide sequence III is CAGG; or

vi) the nucleotide sequence I is the nucleotide sequence shown in SEQ ID NO: 303, and the nucleotide sequence II is the nucleotide sequence shown in SEQ ID NO: 304; and the nucleotide sequences III and IV both have a length of one nucleotide, and the base of the nucleotide sequence III is A; or, the nucleotide sequences III and IV both have a length of two nucleotides, and in the direction from the 5' terminal to the 3' terminal, the base composition of the nucleotide sequence III is UA; or, the nucleotide sequences III and IV both have a length of three nucleotides, and in the direction from the 5' terminal to the 3' terminal, the base composition of the nucleotide sequence III is UUA; or, the nucleotide sequences III and IV both have a length of four nucleotides, and in the direction from the 5' terminal to the 3' terminal, the base composition of the nucleotide sequence III is GUUA.

**41.** The siRNA according to claim 40, wherein the nucleotide sequence III is completely reverse complementary to the nucleotide sequence IV.

**42.** The siRNA according to any one of claims 31-41, wherein the antisense strand further comprises a nucleotide sequence V, the nucleotide sequence V has a length of 1-3 nucleotides, is linked to a 3' terminal of the antisense strand, thereby constituting a 3' overhang of the antisense strand; or, the nucleotide sequence V has a length of 2 nucleotides; or the nucleotide sequence V is two continuous thymidine deoxyribonucleotides or two continuous uridine ribonucleotides; or the nucleotide sequence V is complementary to a nucleotide at a corresponding site of

the target mRNA.

**43.** The siRNA according to any one of claims 31-42, wherein,

the sense strand of the siRNA comprises the nucleotide sequence shown in SEQ ID NO: 5, and the antisense strand comprises the nucleotide sequence shown in SEQ ID NO: 6; or the sense strand of the siRNA comprises the nucleotide sequence shown in SEQ ID NO: 7, and the antisense strand comprises the nucleotide sequence shown in SEQ ID NO: 8;
or the sense strand of the siRNA comprises the nucleotide sequence shown in SEQ ID NO: 65, and the antisense strand comprises the nucleotide sequence shown in SEQ ID NO: 66; or the sense strand of the siRNA comprises the nucleotide sequence shown in SEQ ID NO: 67, and the antisense strand comprises the nucleotide sequence shown in SEQ ID NO: 68;
or the sense strand of the siRNA comprises the nucleotide sequence shown in SEQ ID NO: 125, and the antisense strand comprises the nucleotide sequence shown in SEQ ID NO: 126; or the sense strand of the siRNA comprises the nucleotide sequence shown in SEQ ID NO: 127, and the antisense strand comprises the nucleotide sequence shown in SEQ ID NO: 128;
or the sense strand of the siRNA comprises the nucleotide sequence shown in SEQ ID NO: 185, and the antisense strand comprises the nucleotide sequence shown in SEQ ID NO: 186; or the sense strand of the siRNA comprises the nucleotide sequence shown in SEQ ID NO: 187, and the antisense strand comprises the nucleotide sequence shown in SEQ ID NO: 188;
or the sense strand of the siRNA comprises the nucleotide sequence shown in SEQ ID NO: 245, and the antisense strand of the siRNA comprises the nucleotide sequence shown in SEQ ID NO: 246; or the sense strand of the siRNA comprises the nucleotide sequence shown in SEQ ID NO: 247, and the antisense strand of the siRNA comprises the nucleotide sequence shown in SEQ ID NO: 248;
or the sense strand of the siRNA comprises the nucleotide sequence shown in SEQ ID NO: 305, and the antisense strand of the siRNA comprises the nucleotide sequence shown in SEQ ID NO: 306; or the sense strand of the siRNA comprises the nucleotide sequence shown in SEQ ID NO: 307, and the antisense strand of the siRNA comprises the nucleotide sequence shown in SEQ ID NO: 308.

**44.** The siRNA according to any one of claims 31-43, wherein the siRNA has the nucleotide sequence shown in siC5a1, siC5a2, siC5b1, siC5b2, siC5c1, siC5c2, siC5d1, siC5d2, siC5e1, siC5e2, siC5f1 or siC5f2.

**45.** The siRNA according to any one of claims 31-44, wherein the siRNA is any one of siC5a1-M1, siC5a2-M1, siC5b1-M1, siC5b2-M1, siC5c1-M1, siC5c2-M1, siC5d1-M1, siC5d2-M1, siC5e1-M1, siC5e2-M1, siC5f1-M1 or siC5f2-M1.

**46.** The siRNA according to any one of claims 31-45, wherein at least one phosphate group in the sense strand or the antisense strand is a phosphate group with modified group.

**47.** The siRNA according to claim 46, wherein the phosphate group with modified group is a phosphorothioate group formed by substituting at least one oxygen atom in a phosphodiester bond in the phosphate group with a sulfur atom; or, the phosphate group with modified group is a phosphorothioate group having a structure as shown by Formula (1):

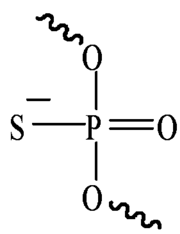

Formula (1).

**48.** The siRNA according to claim 47, wherein, in the siRNA, a phosphorothioate linkage exists in at least one of the following positions:

the position between the first nucleotide and the second nucleotide at 5' terminal of the sense strand;
the position between the second nucleotide and the third nucleotide at 5' terminal of the sense strand;
the position between the first nucleotide and the second nucleotide at 3' terminal of the sense strand;

the position between the second nucleotide and the third nucleotide at 3' terminal of the sense strand;
the position between the first nucleotide and the second nucleotide at 5' terminal of the antisense strand;
the position between the second nucleotide and the third nucleotide at 5' terminal of the antisense strand;
the position between the first nucleotide and the second nucleotide at 3' terminal of the antisense strand; and
the position between the second nucleotide and the third nucleotide at 3' terminal of the antisense strand.

**49.** The siRNA according to any one of claims 31-48, wherein the siRNA is any one of siC5a1-M1S, siC5a2-M1S, siC5b1-M1S, siC5b2-M1S, siC5c1-M1S, siC5c2-M1S, siC5d1-M1S, siC5d2-M1S, siC5e1-M1S, siC5e2-M1S, siC5f1-M1S or siC5f2-M1S.

**50.** The siRNA according to any one of claims 31-49, wherein the 5'-terminal nucleotide in the antisense strand of the siRNA is a 5'-phosphate nucleotide or a 5'-phosphate analogue modified nucleotide;

or, the 5'-phosphate nucleotide is a nucleotide as shown by Formula (2); and the 5'-phosphate analogue modified nucleotide is selected from a nucleotide represented by any one of Formula (3) to (6):

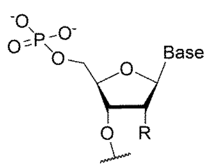
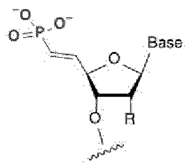
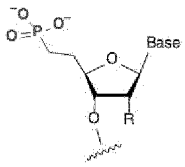
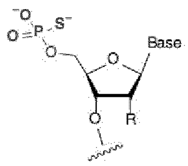
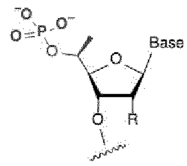

Formula (2) Formula (3) Formula (4) Formula (5) Formula (6)

wherein, R is selected from H, OH, methoxy or F; and Base represents a base selected from A, U, C, G, or T.

**51.** The siRNA according to any one of claims 31-50, wherein the siRNA is any one of siC5a1-M1P1, siC5a2-M1P1, siC5a1-M1SP1, siC5a2-M1SP1, siC5b1-M1P1, siC5b2-M1P1, siC5b1-M1SP1, siC5b2-M1SP1, siC5c1-M1P1, siC5c2-M1P1, siC5c1-M1SP1, siC5c2-M1SP1, siC5d1-M1P1, siC5d2-M1P1, siC5d1-M1SP1, siC5d2-M1SP1, siC5e1-M1P1, siC5e2-M1P1, siC5e1-M1SP1, siC5e2-M1SP1, siC5f1-M1P1, siC5f2-M1P1, siC5f1-M1SP1 or siC5f2-M1SP1.

**52.** The siRNA according to any one of claims 31-51, wherein the siRNA is any one of siC5a1-M1SP, siC5b1-M1SP, siC5c1-M1SP, siC5d1-M1SP, siC5e1-M1SP or siC5f1-M1SP.

**53.** A pharmaceutical composition, wherein the pharmaceutical composition comprises the siRNA according to any one of claims 31-52 and a pharmaceutically acceptable carrier.

**54.** An siRNA conjugate, wherein the siRNA conjugate comprises the siRNA according to any one of claims 31-52 and a conjugating group conjugatively linked to the siRNA.

**55.** Use of the siRNA conjugate according to any one of claims 1-30 and 54, the siRNA according to any one of claims 31-52, and/or the pharmaceutical composition according to claim 53 in the manufacture of a medicament for treating and/or preventing myasthenia gravis.

**56.** A method for treating and/or preventing myasthenia gravis, wherein the method comprises administering an effective amount of the siRNA conjugate according to any one of claims 1-30 and 54, the siRNA according to any one of claims 31-52, and/or the pharmaceutical composition according to claim 53 to a subject suffering from myasthenia gravis.

**57.** A method for inhibiting expression of a C5 gene in a hepatocyte, comprising contacting an effective amount of the siRNA conjugate according to any one of claims 1-30 and 54, the siRNA according to any one of claims 31-52, and/or the pharmaceutical composition according to claim 53 to the hepatocyte.

**58.** A kit, wherein the kit comprises the siRNA conjugate according to any one of claims 1-30 and 54, the siRNA according to any one of claims 31-52, and/or the pharmaceutical composition according to claim 53.

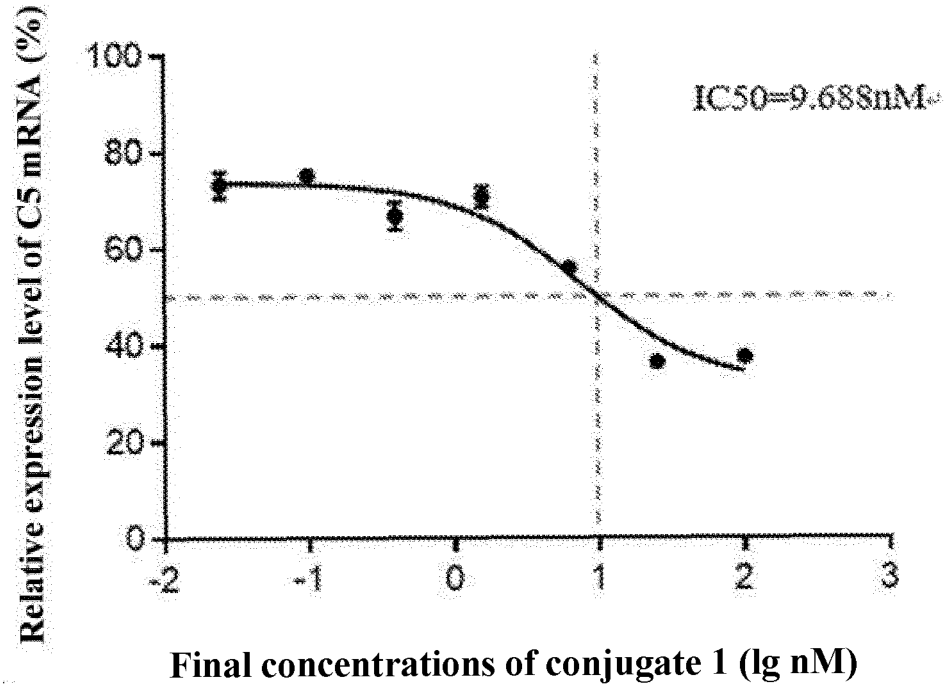


Figure 1A

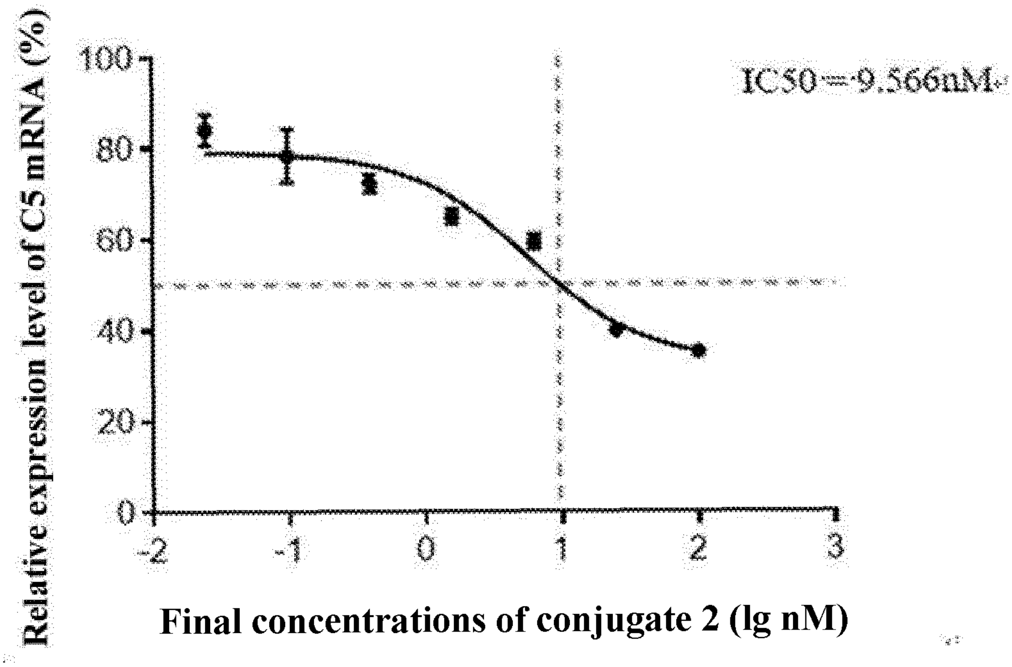


Figure 1B

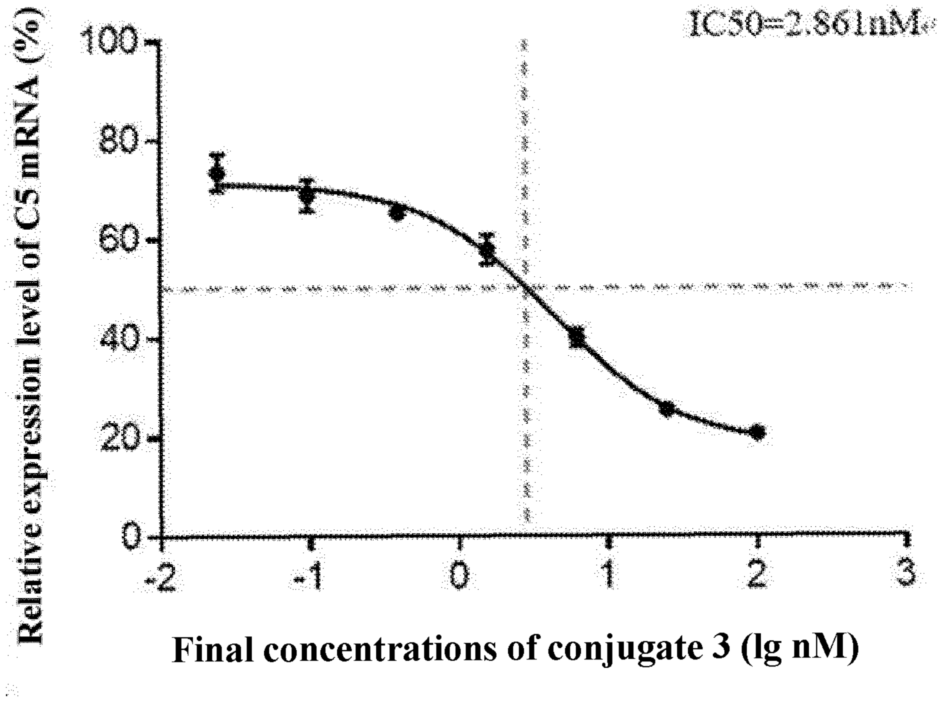


Figure 1C

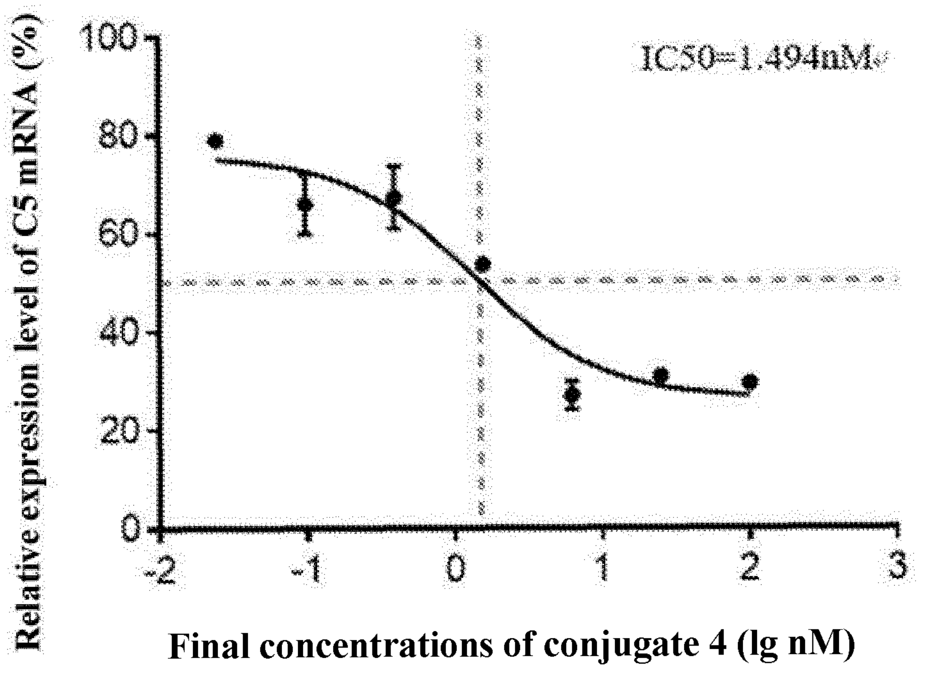


Figure 1D

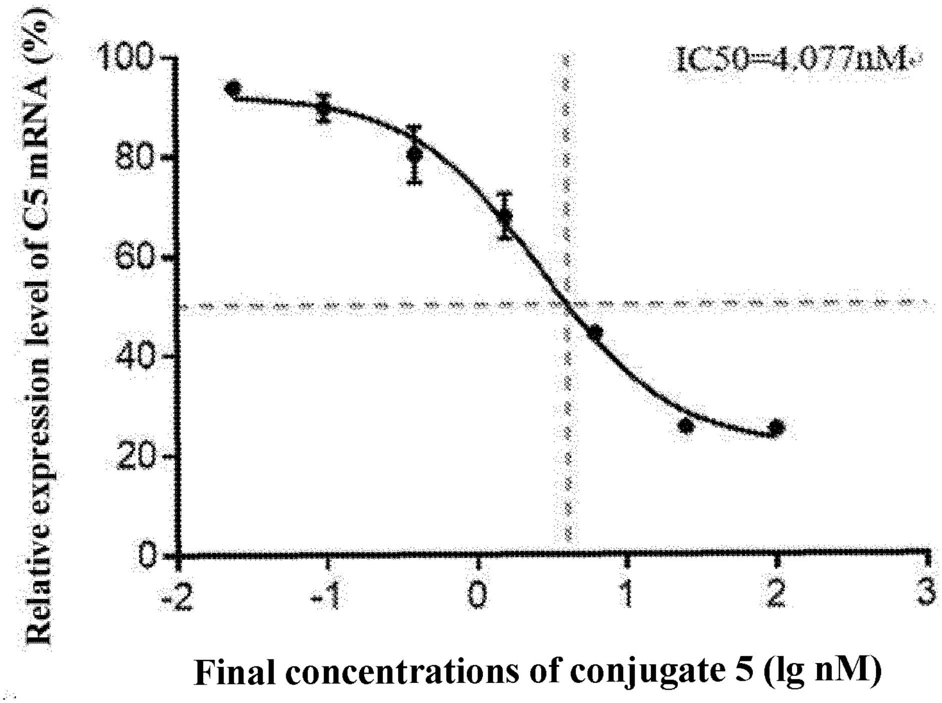


Figure 1E

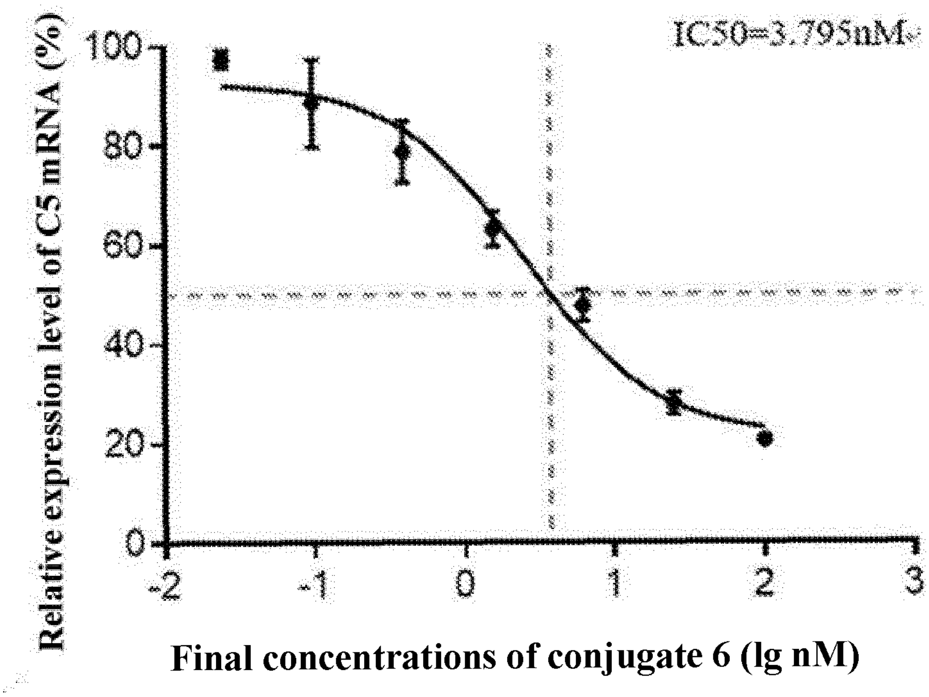


Figure 1F

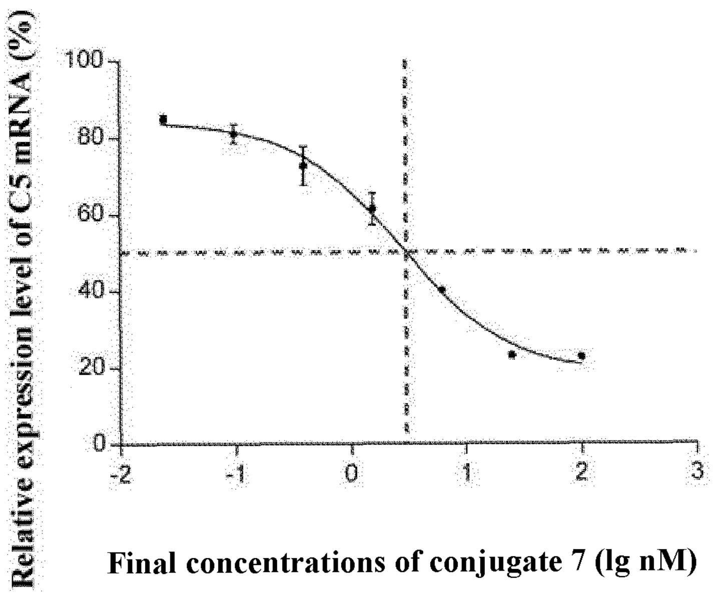


Figure 1G

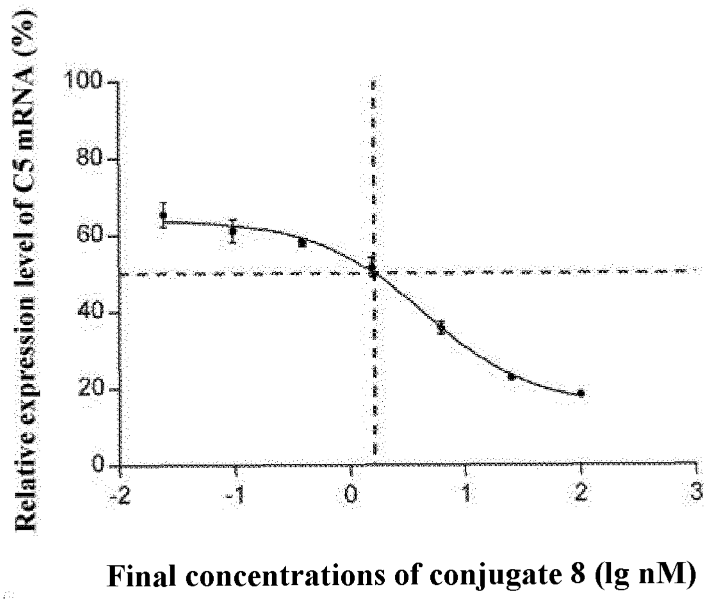


Figure 1H

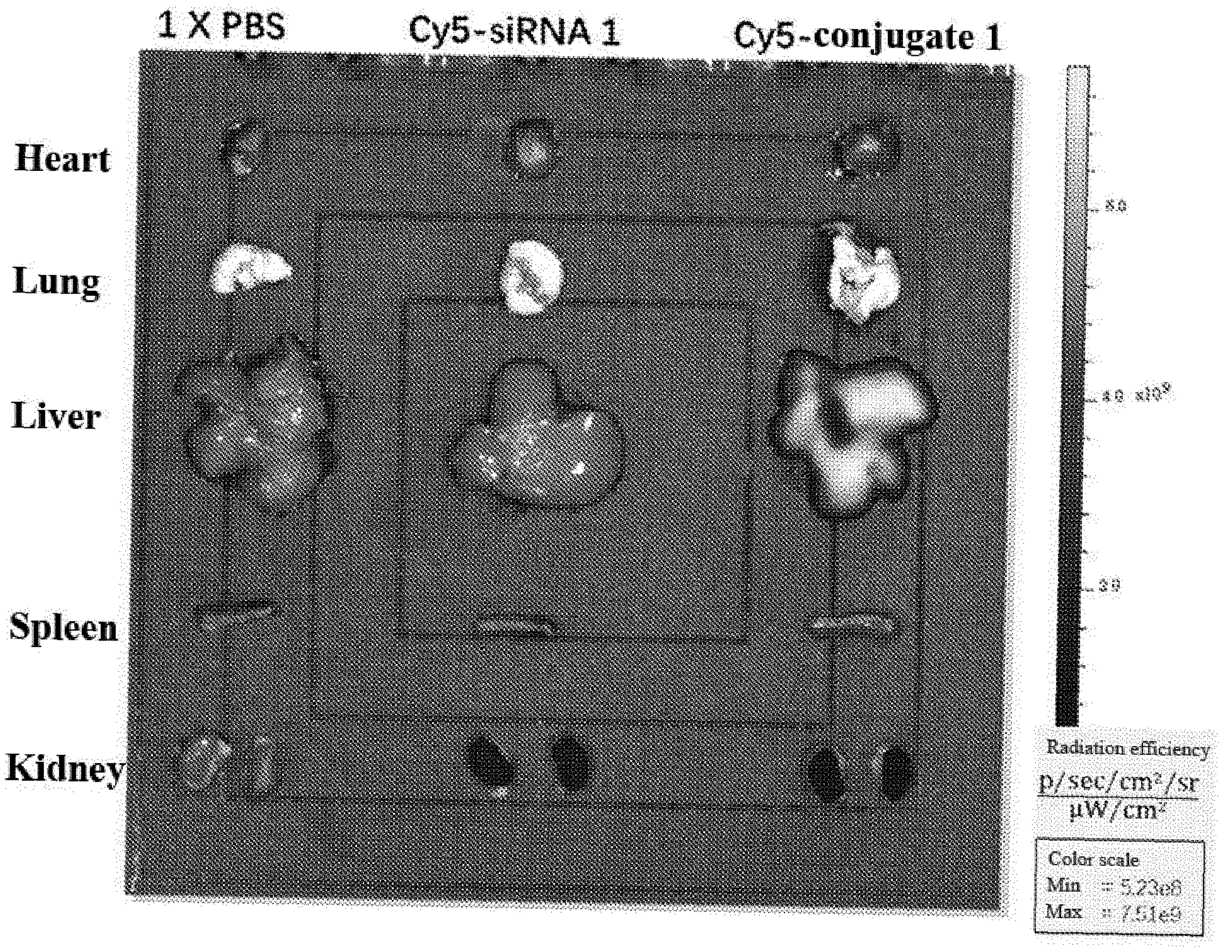
1 X PBS
Cy5-siRNA 1
Cy5-conjugate 1
Heart
Lung
Liver
Spleen
Kidney
Radiation efficiency
p/sec/cm²/sr
µW/cm²
Color scale
Min = 5.23e8
Max = 7.51e9

Figure 2A

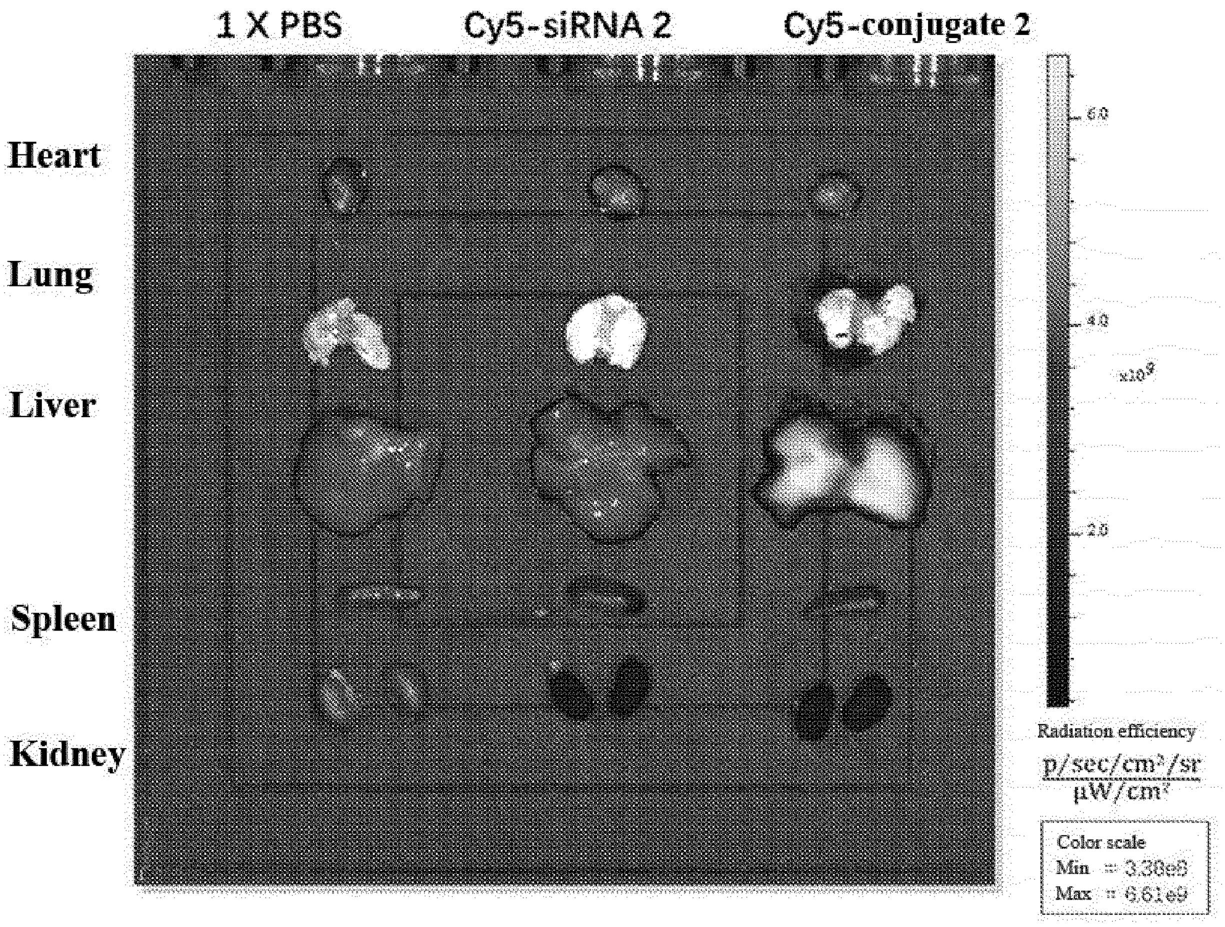
1 X PBS
Cy5-siRNA 2
Cy5-conjugate 2
Heart
Lung
Liver
Spleen
Kidney
6.0
4.0
x10⁹
2.0
Radiation efficiency
p/sec/cm²/sr
µW/cm²
Color scale
Min = 3.30e8
Max = 6.61e9

Figure 2B

**INTERNATIONAL SEARCH REPORT**

International application No.
**PCT/CN2020/091624**

**A. CLASSIFICATION OF SUBJECT MATTER**

A61K 48/00(2006.01)i；C12N 15/113(2010.01)i；A61P 21/04(2006.01)i；A61P 37/00(2006.01)i

According to International Patent Classification (IPC) or to both national classification and IPC

**B. FIELDS SEARCHED**

Minimum documentation searched (classification system followed by classification symbols)

A61K,C12N,A61P

Documentation searched other than minimum documentation to the extent that such documents are included in the fields searched

Electronic data base consulted during the international search (name of data base and, where practicable, search terms used)

CNABS, SIPOABS, DWPI, 超星读秀学术, 中国专利生物序列检索系统, CNKI, 万方, ISI web of knowledge, Elsevier Science Direct, Springerlink, pubmed, genbank, ENA, EMBL, Uniport, STN, 百度, 百度学术, patentics: 苏州瑞博, SUZHOU RIBO, 张鸿雁, ZHANG HONGYAN, 补体蛋白C5, 补体组分C5, complement C5, siRNA, RNAi, iRNA, 缀合物, conjugates, GalNAc, SEQ ID NOs: 1-2, 61-62, 121-122, 181-182, 241-242, 301-302

**C. DOCUMENTS CONSIDERED TO BE RELEVANT**

| Category* | Citation of document, with indication, where appropriate, of the relevant passages | Relevant to claim No. |
|---|---|---|
| X | CN 105324485 A (ALNYLAM PHARMACEUTICALS, INC.) 10 February 2016 (2016-02-10)<br>claims 24-69, 89-100, description, paragraph 723 | 31-58 |
| PY | WO 2019105437 A1 (SUZHOU RIBO LIFE SCIENCE CO., LTD.) 06 June 2019 (2019-06-06)<br>description, page 21, line 15 to page 28, line 5 | 1-30, 55-58 |
| PY | WO 2019105435 A1 (SUZHOU RIBO LIFE SCIENCE CO., LTD.) 06 June 2019 (2019-06-06)<br>description, page 19, line 5 to page 25, line 5 | 1-30, 55-58 |
| A | CN 107109405 A (SOLSTICE BIOLOG LTD.) 29 August 2017 (2017-08-29)<br>entire document | 1-58 |
| A | CN 105378082 A (ISIS PHARMACEUTICALS INC.) 02 March 2016 (2016-03-02)<br>entire document | 1-58 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 July 2020** | **24 August 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/091624**

**C. DOCUMENTS CONSIDERED TO BE RELEVANT**

| Category* | Citation of document, with indication, where appropriate, of the relevant passages | Relevant to claim No. |
|---|---|---|
| A | SPRINGER, A.D. et al. "GalNAc-siRNA Conjugates: Leading the Way for Delivery of RNAi Therapeutics" *Nucleic Acid Therapeutics,* Vol. 28, No. 3, 24 May 2018 (2018-05-24), pp. 109-118 | 1-58 |

Form PCT/ISA/210 (second sheet) (January 2015)

**INTERNATIONAL SEARCH REPORT**

International application No.
**PCT/CN2020/091624**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

**INTERNATIONAL SEARCH REPORT**

International application No.
**PCT/CN2020/091624**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **56-57**
because they relate to subject matter not required to be searched by this Authority, namely:

[1] Claims 56-57 relate to methods for treating or preventing myasthenia gravis and inhibiting C5 gene expression in liver cells (and thereby treating a corresponding disease), and therefore belong to subject matter which does not require an international searching authority to perform a search, as defined by PCT Rule 39.1(iv). Nevertheless, a search has still been performed on the basis of corresponding siRNA pharmaceutical uses.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/091624**

| Patent document cited in search report | Publication date (day/month/year) | Patent family member(s) | Publication date (day/month/year) |
|---|---|---|---|
| CN 105324485 A | 10 February 2016 | US 9249415 B2 | 02 February 2016 |
| | | CA 2904654 A1 | 02 October 2014 |
| | | GT 201500293 A | 18 December 2018 |
| | | WO 2014160129 A3 | 04 December 2014 |
| | | HU E034987 T2 | 02 May 2018 |
| | | JP 2016518331 A | 23 June 2016 |
| | | MX 2015012796 A | 03 February 2016 |
| | | TW I660731 B | 01 June 2019 |
| | | JP 6524060 B2 | 05 June 2019 |
| | | DK 2970974 T3 | 04 December 2017 |
| | | TW 201932597 A | 16 August 2019 |
| | | PT 2970974 T | 29 November 2017 |
| | | CR 20150493 A | 28 March 2016 |
| | | MX 366660 B | 18 July 2019 |
| | | US 2016244763 A1 | 25 August 2016 |
| | | US 2020032259 A1 | 30 January 2020 |
| | | SI 2970974 T1 | 29 December 2017 |
| | | NZ 712336 A | 27 March 2020 |
| | | HR P20171813 T1 | 29 December 2017 |
| | | US 9850488 B2 | 26 December 2017 |
| | | RS 56663 B1 | 30 March 2018 |
| | | EP 2970974 A2 | 20 January 2016 |
| | | EP 3312281 A2 | 25 April 2018 |
| | | EA 201591707 A1 | 31 March 2016 |
| | | CL 2015002710 A1 | 22 April 2016 |
| | | EP 3312281 A3 | 27 June 2018 |
| | | HK 1252501 A1 | 31 May 2019 |
| | | AU 2014244116 B2 | 26 March 2020 |
| | | CL 2019000107 A1 | 03 April 2020 |
| | | LT 2970974 T | 11 December 2017 |
| | | BR 112015022876 A2 | 07 November 2017 |
| | | ES 2649490 T3 | 12 January 2018 |
| | | KR 20150127243 A | 16 November 2015 |
| | | TW 201505637 A | 16 February 2015 |
| | | HK 1221484 A1 | 02 June 2017 |
| | | US 2017268005 A1 | 21 September 2017 |
| | | CY 1120289 T1 | 10 July 2019 |
| | | SG 10201912286T A | 27 February 2020 |
| | | US 9701963 B2 | 11 July 2017 |
| | | AU 2014244116 A1 | 01 October 2015 |
| | | MX 2019008380 A | 09 September 2019 |
| | | UY 35442 A | 31 October 2014 |
| | | US 2015247143 A1 | 03 September 2015 |
| | | PE 20160046 A1 | 14 February 2016 |
| | | CL 2017002014 A1 | 27 April 2018 |
| | | CL 2016000882 A1 | 09 December 2016 |
| | | EP 2970974 B1 | 23 August 2017 |
| | | ME 03043 B | 20 October 2018 |
| | | PE 00462016 A1 | 14 February 2016 |
| WO 2019105437 A1 | 06 June 2019 | AU 2018377716 A1 | 09 April 2020 |

Form PCT/ISA/210 (patent family annex) (January 2015)

**INTERNATIONAL SEARCH REPORT**
**Information on patent family members**

International application No.
**PCT/CN2020/091624**

| Patent document cited in search report | Publication date (day/month/year) | Patent family member(s) | Publication date (day/month/year) |
|---|---|---|---|
| | | TW 201928057 A | 16 July 2019 |
| | | CN 110945132 A | 31 March 2020 |
| WO 2019105435 A1 | 06 June 2019 | CN 110944675 A | 31 March 2020 |
| | | TW 201925468 A | 01 July 2019 |
| | | WO 2019105435 A9 | 22 August 2019 |
| CN 107109405 A | 29 August 2017 | US 2017114341 A1 | 27 April 2017 |
| | | CA 2950960 A1 | 10 December 2015 |
| | | WO 2015188197 A2 | 10 December 2015 |
| | | WO 2015188197 A3 | 25 February 2016 |
| | | EP 3152308 A2 | 12 April 2017 |
| | | AU 2015269053 A1 | 22 December 2016 |
| | | EP 3152308 A4 | 27 December 2017 |
| | | JP 2017522046 A | 10 August 2017 |
| CN 105378082 A | 02 March 2016 | NZ 631537 A | 26 May 2017 |
| | | RU 2018136140 A | 17 December 2018 |
| | | CA 2921518 A1 | 06 November 2014 |
| | | JP 6592486 B2 | 16 October 2019 |
| | | EP 2992097 A2 | 09 March 2016 |
| | | US 2018273953 A1 | 27 September 2018 |
| | | AU 2017200365 A1 | 23 February 2017 |
| | | EP 2991656 B1 | 18 December 2019 |
| | | RU 2015151200 A3 | 14 January 2019 |
| | | RU 2015151199 A | 05 June 2017 |
| | | US 2016090596 A1 | 31 March 2016 |
| | | US 2019055554 A1 | 21 February 2019 |
| | | MX 2015015264 A | 12 August 2016 |
| | | PE 14302016 A1 | 06 January 2017 |
| | | US 2016076030 A1 | 17 March 2016 |
| | | RU 2019110030 A | 06 May 2019 |
| | | US 2018273952 A1 | 27 September 2018 |
| | | AU 2014259750 A1 | 22 October 2015 |
| | | CN 105378082 B | 09 June 2020 |
| | | JP 2018027091 A | 22 February 2018 |
| | | CR 20150612 A | 03 March 2016 |
| | | HK 1221475 A1 | 02 June 2017 |
| | | US 9181550 B2 | 10 November 2015 |
| | | RU 2015151199 A3 | 27 March 2018 |
| | | AU 2018267625 A1 | 13 December 2018 |
| | | WO 2014179627 A9 | 26 February 2015 |
| | | AU 2019200820 B2 | 30 April 2020 |
| | | AU 2017200365 C1 | 18 April 2019 |
| | | SG 11201508870V A | 27 November 2015 |
| | | PL 2992098 T3 | 30 September 2019 |
| | | EP 2992009 A1 | 09 March 2016 |
| | | AU 2017200950 B2 | 17 January 2019 |
| | | EP 2991661 A4 | 15 February 2017 |
| | | AU 2014259755 B2 | 30 August 2018 |
| | | JP 6456362 B2 | 23 January 2019 |
| | | WO 2014179629 A8 | 02 June 2016 |
| | | PH 12015502493 A1 | 22 February 2016 |

Form PCT/ISA/210 (patent family annex) (January 2015)

**INTERNATIONAL SEARCH REPORT**
**Information on patent family members**

International application No.
**PCT/CN2020/091624**

| Patent document cited in search report | Publication date (day/month/year) | Patent family member(s) | | | Publication date (day/month/year) |
|---|---|---|---|---|---|
| | | KR | 20180051678 | A | 16 May 2018 |
| | | IL | 264241 | D0 | 28 February 2019 |
| | | JP | 2020039355 | A | 19 March 2020 |
| | | AU | 2019202598 | A1 | 02 May 2019 |
| | | AU | 2017203436 | A1 | 08 June 2017 |
| | | NZ | 631552 | A | 24 February 2017 |
| | | RU | 2019124314 | A | 21 August 2019 |
| | | IL | 263843 | D0 | 31 January 2019 |
| | | AU | 2014259756 | B2 | 23 February 2017 |
| | | BR | 112015027319 | A2 | 26 September 2017 |
| | | KR | 20160002976 | A | 08 January 2016 |
| | | CN | 110079524 | A | 02 August 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103380113 A
- WO 2009082607 A2
- CN 105378082 A
- WO 2015006740 A2
- WO 2014025805 A1
- US 8106022 B2


### Non-patent literature cited in the description


- **BEAUCAGE.** *Tetrahedron,* 1992, vol. 48, 2223-2311
- **GREENE ; WUTS.** Protective Groups in Organic Synthesis. John Wiley & Sons, 1991
- **ANASTASIA KHVOROVA ; JONATHAN K. WATTS.** The chemical evolution of oligonucleotide therapies of clinical utility. *Nature Biotechnology,* 2017, vol. 35 (3), 238-48
- **MUTHIAH MANOHARAN.** siRNA conjugates carrying sequentially assembled trivalent N-acetylgalactosamine linked through nucleosides elicit robust gene silencing in vivo in hepatocytes. *ACS Chemical biology,* 2015, vol. 10 (5), 1181-7
- **RAJEEV et al.** *ChemBioChem,* 2015, vol. 16, 903-908
- *J. Am. Chem. Soc.,* 2014, vol. 136, 16958-16961
- Molecular Cloning. Cold Spring Harbor Laboratory Press, 1989
- *CHEMICAL ABSTRACTS,* 1772-03-8
- *CHEMICAL ABSTRACTS,* 27607-77-8
- *CHEMICAL ABSTRACTS,* 821-41-0
- *CHEMICAL ABSTRACTS,* 7790-28-5
- *CHEMICAL ABSTRACTS,* 14898-67-0